# EUROPEAN PATENT SPECIFICATION

(11) **EP 1 867 724 B1**
(45) Date of publication and mention of the grant of the patent: **29.08.2012**
(21) Application number: 07011474.9
(22) Date of filing: 12.06.2007
(51) Int. Cl.: C12N 9/24, C12N 15/82

(54) **Production of ß-glucosidase, hemicellulase and ligninase in E1 and FLC-cellulase-transgenic plants**
Herstellung von ß-Glucosidase, Hemicellulase and Ligninase in transgenen Pflanzen mit Cellulase E1 und FLC
Production d'hémicellulase et de lignisase, et de glucosidase ß dans les plantes transgéniques à cellulase E1 et FLC

(30) Priority: 12.06.2006 US 451162; 19.07.2006 US 489234
(43) Date of publication of application: 19.12.2007
(73) Proprietor: Michigan State University, East Lansing, Michigan 48824 (US)
(72) Inventor: Sticklen, Masomeh B., East Lansing, MI 48823 (US)
(74) Representative: Grünecker, Kinkeldey, Stockmair & Schwanhäusser

(56) References cited:
- WO-A-2007/100897
- SALEHI H ET AL: "Delay in flowering and increase in biomass of transgenic tobacco expressing the Arabidopsis floral repressor gene FLOWERING LOCUS C" JOURNAL OF PLANT PHYSIOLOGY, FISCHER, STUTTGART, DE, vol. 162, no. 6, 14 June 2005 (2005-06-14), pages 711-717, XP004939720 ISSN: 0176-1617
- BLAYLOCK M J: "Cellulose production and increased biomass multifunction crop plants (STTR Phase II Project)" INTERNET CITATION, [Online] November 2005 (2005-11), XP002455836 Retrieved from the Internet: URL:http://www.hydrogen.energy.gov/annual_ progress05.html> [retrieved on 2007-10-19]
- ZIEGLER M T ET AL: "ACCUMULATION OF A THERMOSTABLE ENDO-1,4-BETA-D-GLUCANASE IN THE APOPLAST OF ARABIDOPSIS THALIANA LEAVES" MOLECULAR BREEDING, KLUWER ACADEMIC PUBLISHERS, DORDRECHT, NL, vol. 6, no. 1, February 2000 (2000-02), pages 37-46, XP008020043 ISSN: 1380-3743
- STICKLEN ET AL: "Plant genetic engineering to improve biomass characteristics for biofuels" CURRENT OPINION IN BIOTECHNOLOGY, LONDON, GB, vol. 17, no. 3, June 2006 (2006-06), pages 315-319, XP005495090 ISSN: 0958-1669

## Description

### REFERENCE TO A "NUCLEOTIDE/AMINO ACID SEQUENCE LISTING APPENDIX SUBMITTED ON A COMPACT DISC".

The application contains nucleotide and amino acid sequences which are identified with SEQ ID NOs. A compact disc is provided which contains the Sequence Listings for the sequences. The Sequence Listing on the compact disc and is identical to the paper copy of the Sequence Listing provided with the application.

### BACKGROUND OF THE INVENTION

### (1) Field of the Invention

The present invention relates to transgenic plants. The transgenic plants are capable of expressing one or more cell wall degrading enzymes and a flowering locus c gene coding region. The cell wall degrading enzyme are directed to a plastid, vacuole, vesicle, cytosol or apoplast of the transgenic plant. The flowering locus c gene delays flowering while increasing biomass and enabling isolation of increased amounts of the hydrolyzing enzyme from the transgenic plant as compared to a non-transgenic plant from which the transgenic plant is derived.

### (2) Description of Related Art

If human economies are to become more truly sustainable, we will need to learn how to use the solar energy and carbon fixed in plant biomass to meet a much larger fraction of our energy and raw material needs. The potential of plant biomass to help these needs is certainly real: approximately 180 billion tons of new plant matter is produced annually across the globe, or about 30 tons per person on the planet per year. (Khan, A., Jameel, A.M., Jameel, M. (1984). Energy from Biomass: Resources and expectations. In: Renewable Energy Sources: International Progress, Part B, ed. T.N. Veziroglu, pp. 87-98). The energy value of this plant matter is roughly equivalent to 10 times the total human use of all types of energy. However, because of the difficulty in extracting the energy from plant biomass, most of the energy potential of the biomass goes unused.

Much research and engineering remains to be done to actually realize the potential of plant matter to meet a greater portion of our fuel and raw material needs. Specifically, cellulose and hemicellulose are polymers of five and six carbon sugars that represent approximately 70-80% of most plant matter. These could be converted into fermentable sugars, and the rest be used for other purposes. These sugars could form the raw material and energy basis for a renewable chemical and fuel industry if the sugars could be made available at significantly lower cost.

It is encouraging that in recent years, much progress has been made toward realizing this goal of reduced cost of production of sugars from biomass. In particular, hydrolysis methods have been improved (Vlasenko E. and Cherry J. (2005). Improving cellulose hydrolysis with the new cellulase compositions. Proceedings of the 27th Symposium on Biotechnology for Fuels and Chemicals. Denver, May 1-4, 2005. Page 6: 1B-03) and recombinant microorganisms have been developed that can ferment the mixed five and six carbon sugars from plant biomass to ethanol in high yield (Moniruzzaman M., Dien B. S., Ferrer B., Hespel R. B. Dale B. E., Ingram L. O., and Bothast R. J. (1996). Ethanol production from AFEX pretreated corn fiber by recombinant bacteria. Biotechnology Letters. 18 (8): 985-990) and their efficiency has been increased (Cherry J. R. (2005). Progress on enzymes for biomass utilization and prospects for the future. Proceedings of the 27th Symposium on Biotechnology for Fuels and Chemicals. Denver, May 1-4, 2005. Page 35: CA-11). Also, different promising pretreatments have been developed and compared that make the cellulose and hemicellulose much more reactive and accessible to hydrolytic enzymes.

Scientists have made important strides in reducing the costs of production of hydrolysis enzymes through molecular enzymology and other molecular techniques. However the costs of these enzymes produced from microbes in conventional deep tank fermentation systems is still far too high to meet the economics of the commercial production of biofuels from plant biomass. An alternative technology to be tested is production of high levels of biologically active hydrolysis enzymes directly in biomass plants.

Much of the cellulose in plant biomass is in the form of lignocellulose. Lignin is a complex macromolecule consisting of aromatic units with several types of interunit linkages. In the plant, the lignin physically protects the cellulose polysaccharides in complexes called lignocellulose. To degrade the cellulose in the lignocellulose complexes, the lignin must first be degraded. While lignin can be removed in chemi-mechanical processes that free the cellulose for subsequent conversion to fermentable sugars, the chemi-mechanical processes are expensive and inefficient. Ligninase and cellulase enzymes, which are produced by various microorganisms, have been used to convert the lignins and cellulose, respectively, in plant biomass to fermentable sugars. However, the cost for these enzymes is expensive. As long as the cost to degrade plant biomass remains expensive, the energy locked up in the plant biomass will largely remain unused.

An attractive means for reducing the cost of degrading plant biomass is to make transgenic plants that contain cellulases. For example, WO 98/11235 to Lebel et al. discloses transgenic plants that express cellulases in the chloroplasts of the transgenic plants or transgenic plants wherein the cellulases are targeted to the chloroplasts. Preferably, the cellulases are operably linked to a chemically-inducible promoter to restrict expression of the cellulase to an appropriate time. However, because a substantial portion of the cellulose in plants is in the form of lignocellulose, extracts from the transgenic plants are inefficient at degrading the cellulose in the lignocellulose.

U.S. Patent Nos. 5,981,835 and 6,818,803 to Austin-Phillips et al. discloses transgenic tobacco and alfalfa which express the cellulases E2, or E3 from Thermomononospora fusca. The genes encoding the E2 or E3, which were modified to remove their leader sequence, were placed under the control of a constitutive promoter and stably integrated into the plant genome. Because the leader sequence had been removed, the E2 or E3 product preferentially accumulated in the cytoplasm of the transgenic plants. However, when produced at high level in cytoplasm, the heterologous enzyme will interact with normal cytoplasmic metabolic activities and the growth of the transgenic plants can be impaired.

U.S. Patent No. 5,536,655 to Thomas et al. discloses a gene encoding Acidothermus cellulolyticus E1 endoglucanase and correponding protein sequences. U.S. Patent No. 6,013,860. to Himmel et al. discloses transgenic plants which express the cellulase E1 from Acidothermus cellulolyticus. The gene encoding E1, which was modified to remove the leader region, was placed under the control of a plastid specific promoter and preferably integrated into the plastid genome. Because the leader sequence had been removed, the E1 product accumulated in the plastid. U.S. Patent Application Publication Nos. 2003/0109011 and 2006/0026715 to Hood et al. teach expression of recombinant polysaccharide degrading enzymes in plants.

The accumulation of hydrolytic enzymes in the cytoplasm of a plant is undesirable since there is the risk that the cellulase will interfere with cytoplasmic biochemical activities causing harm to the plant growth and development. For example, research has shown that plants such as the avocado, bean, pepper, peach, poplar, and orange also contain, cellulase genes, which are activated by ethylene during ripening and leaf and fruit abscission. Therefore, transgenic plants which contain large quantities of cellulase in the cytoplasm are particularly prone to damage. Furthermore, the cellulases accumulate in all tissues of the plant which can be undesirable. Restriction of cellulase expression to plastids or apoplast is desirable because it reduces the risk of plant damage due the cellulases interfering with the cytoplasmic chemical reactions. However, for most crop plants, it has been difficult to develop a satisfactory method for introducing heterologous genes into the genome of plastids.

For production of ligninases to use in degrading lignins, the ligninases of choice are from the white-rot fungus Phanerochaete chrysosporium. One of the major lignin-degrading, extracellular enzymes produced by P. chrysosporium is lignin peroxidase (LIP). Potential applications of LIP include not only lignin degradation but also biopulping of wood and biodegradation of toxic environmental pollutants. To produce large quantities of LIP, the fungus can be grown in large reactors and the enzyme isolated from the extracellular fluids. However, the yields have been low and the process has not been cost-effective. Production of recombinant LIP in E. coli, in the fungus Trichoderma reesei, and baculovirus have been largely unsuccessful. Heterologous expression of lignin-degrading manganese peroxidase in alfalfa plants has been reported; however, the transgenic plants had reduced growth and expression of the enzyme was poor (Austin et al., Euphytica 85: 381-393 (1995)).

Finally, U.S. Patent No. 6,693,228 to Amasino et al.*,* hereby incorporated herein by reference in its entirety, discloses Flowering Locus C (*flc*) genes, and teaches the use of *flc* to delay flowering in transgenic plants as compared to non-transgenic plants. Amasino et *al.* disclose Flowering Locus C (*flc*) genes from *Arabidopsis thaliana* and *B. rapa.* Amasino et *al.* also teach overexpression of the *A.* thaliana gene under control of the constitutive 35S promoter in *A. thaliana* is sufficient to delay flowering in transformed plants. U.S. Patent Application Publication No. 2004/0126843 A1 to Demmer et al. suggest that the ability to control flowering in C3 monocotyledonous plants, such as forage grasses and cereals has wide ranging applications. Demmer *et al*. propose that controlling flowering offers the ability to control the spread of genetically modified organisms. Demmer et *al.* mentions genes such as *flc* as one of a number of genes important in regulating flowering time. U.S. Patent Application Publication No. 2004/0045049 A1 to Zhang generally teaches *flc* and transgenic plants having modified traits.

Thus, a need remains for improved transgenic plants expressing enzymes that degrade cellulose, hemicellulose, and/or lignin in lignocellulose to fermentable sugars. The ability to control the spread of the transgenic plants is important.

WO 2007/100897 discloses transgenic plants (tobacco, rice and corn) comprising an E1 cellulase gene linked to a signal peptide which directs E1 to the apoplast and a flowering locus C gene (FLC). Salehi, et al.; J. Plant Physiol.; Vol. 162 (2005); pp. 711-717 discloses that FLC from *Arabidopsis thaliana* can increase biomass when transgenically expressed in tobacco. Blaylock, M.J.; Internet citation (2005) "2005 Annual Progress Report"; URL: http://www.hydrogen.energy.gov/pdfs/progress05/xi_11_blaylock.pdf; is a project outline on the generation of transgenic plants constitutively producing an endoglucanase, a hemicellulase and a ligninase and exhibiting delayed flowering for increasing biomass. Ziegler, M.T. et al.; "Molecular Breeding" Vol. 6, No. 1 (2000); pp. 37-46 discloses transgenic *A. thaliana,* expressing the catalytic domain of E1 glucanase from *Acidothermus cellulolyticus* in the apoplast. Sticklen, M. et al.; Curr. Op. Biotech. Vol. 17 (2006); pp. 315-319 summarizes the various attempts to increase the biomass to biofuels conversion including E1 endoglucanase expression in maize and rice and targeting of lignocellulolytic enzymes to the apoplast as well as use of the FLC gene for biomass increase. WO 2002/034926 discloses a transgenic lignocellulose degrading plant comprising (a) at least one DNA encoding a cellulase and (b) at least one DNA encoding a ligninase.

### Summary of the Invention

### The present invention pertains to the following embodiments:

(1) A transgenic plant capable of expressing cell wall degrading enzymes comprising: (a) at least one DNA encoding a beta-glucosidase as a cell wall degrading enzyme which is operably linked to a nucleotide sequence encoding a signal peptide directing the beta-glucosidase to a plastid, vacuole or apoplast, in the leaves of the transgenic plant; and (b) at least one DNA comprising a flowering locus C gene coding region operably linked to a constitutive promoter, wherein the transgenic plant expresses the one or more cell wall degrading enzymes and a transcription factor encoded by the flowering locus C gene that delays flowering while increasing biomass and enabling isolation of increased amounts of the cell wall degrading enzymes from the transgenic plant as compared to a non-transgenic plant from which the transgenic plant is derived.
(2) The transgenic plant of (1), wherein the transgenic plant is a monocot. (3) The transgenic plant of (2), wherein the monocot is switchgrass, rice or maize.
(4) The transgenic plant of (1), wherein a DNA encoding one or more additional cell wall degrading enzymes selected from the group consisting of a hemicellulase and a ligninase is expressed, in addition to the DNA encoding the cell wall degrading β-glucosidase enzyme. (5) The transgenic plant of (4), further comprising DNA encoding heterologous endoglucanase or exoglucanase expressed in said transgenic plant. (6) The transgenic plant of (5), wherein the beta-glucosidase is a beta-glucosidase from *Butyrivibrio fibrisolvens.* (7) The transgenic plant of (6), wherein the beta-glucosidase is encoded by a DNA comprising the nucleotide sequence set forth in SEQ ID NO:23. (8) The transgenic plant of (5), wherein the DNA encodes a ligninase is from *Phanerochaete chrysosporium.*
(9) The transgenic plant of (4) comprising in addition at least one DNA encoding a xylanase as the hemicellulase which is operably linked to a nucleotide sequence encoding a signal peptide directing the xylanase to a plastid or apoplast of the transgenic plant along with the DNA encoding the ligninase. (10) The transgenic plant of (9), wherein the transgenic plant is a monocot. (11) The transgenic plant of (10) wherein the monocot is switchgrass, rice or maize.
(12) The transgenic plant of (4) wherein the xylanase is encoded by a DNA comprising the nucleotide sequence set forth in SEQ ID NO:24, SEQ ID NO:33, SEQ ID NO:34 or SEQ ID NO:35.
(13) The transgenic plant of (1), wherein at least one DNA encoding the cell wall degrading enzyme is operably linked to a leaf-specific promoter. (14) The transgenic plant of (13), wherein the leaf-specific promoter is a promoter for rbcS.
(15) The transgenic plant of (1), wherein at least one DNA encoding a cell wall degrading enzyme is operably linked to a Cauliflower Mosaic Virus 35S promoter.
(16) The transgenic plant of (1), wherein at least one DNA encoding a cell wall degrading enzyme is operably linked to a Tobacco Mosaic Virus translational enhancer.
(17) The transgenic plant of (1), wherein the nucleotide sequence encoding the signal peptide encodes a signal peptide of rbcSand the β-glucosidase is as set forth in SEQ ID NO: 23. (18) The transgenic plant of (17), wherein the rbcS comprises the nucleotide sequence set forth in SEQ ID NO:1.
(19) The transgenic plant of (1) further comprising at least one DNA encoding a selectable marker operably linked to a constitutive promoter. (20) The transgenic plant of (19) wherein the DNA encoding the selectable marker provides the transgenic plant with resistance to an antibiotic, a herbicide, or to environmental stress. (21) The transgenic plant of (20) wherein the DNA encoding resistance to the herbicide is a DNA encoding phosphinothricin acetyl transferase which confers resistance to the herbicide phosphinothricin.
(22) A method for making an enzyme extract comprising one or more cell wall degrading enzymes comprising: (a) providing a transgenic plant according to (1); (b) growing the transgenic plant for a time to accumulate the one or more cell wall degrading enzymes due to the delay in flowering; (c) harvesting the transgenic plant which has accumulated the one or more cell wall degrading enzymes; and (d) grinding the transgenic plant to provide an enzyme extract comprising the one or more cell wall degrading enzymes that accumulated in the transgenic plant.
(23) The method of (22) further comprising: (e) incubating the lignocellulosic material in the enzyme extract to produce the fermentable sugars from the lignocellulose in the plant material; and (f) extracting the fermentable sugars produced from the lignocellulosic material.
(24) The transgenic plant of (1), wherein the beta-glucosidase is directed to a plastid or apoplast. (25). The transgenic plant of (1), wherein the beta-glucosidase is directed to a vacuole.

The present invention provides transgenic plants expressing cell wall degrading enzymes that degrade cellulose, hemicellulose and/or lignin in lignocellulose to fermentable sugars and lignin to aromatic compounds. The fermentable sugars can further be fermented to ethanol or other products. When the transgenic plants are harvested, the plants are ground to release the cellulase, hemicellulase, and/or ligninase enzymes which then can be used to degrade the lignin and cellulose of the transgenic plants or other plants to produce the fermentable sugars.

Also disclosed herein is a transgenic plant capable of expressing one or more cell wall degrading enzymes comprising: at least one DNA comprising a cell wall degrading enzyme coding region operably linked to a nucleotide sequence encoding a signal peptide directing the cell wall degrading enzyme encoded by the DNA to an apoplast, plastid or vacuole of the transgenic plant; and at least one DNA comprising a flowering locus c gene coding region operably linked to a constitutive promoter, wherein the transgenic plant expresses the one or more cell wall degrading enzymes and a transcription factor encoded by the flowering locus c gene that delays flowering while increasing biomass and enabling isolation of increased amounts of the hydrolyzing enzyme from the transgenic plant as compared to a non-transgenic plant from which the transgenic plant is derived.

In further embodiments, the transgenic plant is a monocot. In further embodiments, the monocot is switchgrass and other perennial grasses, rice or maize. In further embodiments, the one or more cell wall degrading enzymes are selected from the group consisting of a cellulase, a hemicellulase and a ligninase. In further embodiments, the cellulase is an endoglucanase, an exoglucanase or a β-glucosidase.
A DNA encoding the cellulase is selected from the group consisting of an *e1* gene from *Acidothermus cellulyticus,* a *cbh1* gene from *Trichoderma reesei,* a dextranase gene from Streptococcus *salivarius,* and a β-glucosidase gene from *Actinomyces naeslundi.* An *e1* gene comprises the nucleotide sequence set forth in SEQ ID NO:4, the *cbh1* gene comprises the nucleotide sequence set forth in SEQ ID NO:10, the dextranase gene comprises the nucleotide sequence set forth in SEQ ID NO:8, and the β-glucosidase gene comprises the nucleotide sequence set forth in SEQ ID NO:6. In one embodiment, the DNA encodes a β-glucosidase from *Butyrivibrio fibrisolvens.* In further embodiments, the DNA encoding the β-glucosidase comprises the nucleotide sequence set forth in SEQ ID NO:23. In further embodiments, the DNA encodes a ligninase from Phanerochaete *chrysosporium.*
A DNA encoding ligninase is ckg4 comprising the nucleotide sequence set forth in SEQ ID NO:11 or ckg5 comprising the nucleotide sequence set forth in SEQ ID NO:13. In further embodiments, the DNA encoding the xylanase comprises the nucleotide sequence set forth in SEQ ID NO:24, SEQ ID NO:33, SEQ ID NO:34 or SEQ ID NO:35. In further still embodiments, the transgenic plant further comprises at least one DNA encoding a selectable marker operably linked to a constitutive promoter. [0020] In further still embodiments, the at least one DNA comprising a cell wall degrading enzyme coding region is operably linked to a leaf-specific promoter. In further still embodiments, the leaf-specific promoter is a promoter for rbcS. In further still embodiments, the at least one DNA comprising a cell wall degrading enzyme coding region is operably linked to a Cauliflower Mosaic Virus 35S promoter. In further still embodiments, the at least one DNA comprising a cell wall degrading enzyme coding region is operably linked to a Tobacco Mosaic Virus Ω translational enhancer. In further still embodiments, the nucleotide sequence encoding the signal peptide encodes a signal peptide of rbcS. In further still embodiments, the rbcS comprises the nucleotide sequence set forth in SEQ ID NO:1. In further still embodiments,the nucleotide sequence encoding the signal peptide encodes a signal peptide of tobacco pathogenesis-related protein 1a (Pr1a). In further still embodiments, the transgenic plant further comprises at least one DNA encoding a selectable marker operably linked to a constitutive promoter. In further embodiments, the DNA encoding the selectable marker provides the transgenic plant with resistance to an antibiotic, an herbicide, or to environmental stress. In further still embodiments, wherein the DNA encoding resistance to the herbicide is a DNA encoding phosphinothricin acetyl transferase which confers resistance to the herbicide phosphinothricin.

Further disclosed is a transgenic plant capable of expressing cell wall degrading enzymes comprising: at least one DNA encoding a β-glucosidase as a first cell wall degrading enzyme which is operably linked to a nucleotide sequence encoding a signal peptide directing the β-glucosidase to an apoplast, plastid or vacuole of the transgenic plant; at least one DNA encoding a ligninase as a second cell wall degrading enzyme which is operably linked to a nucleotide sequence encoding a signal peptide directing the ligninase to an apoplast, plastid or vacuole of the transgenic plant, at least one DNA encoding a xylanase as a third cell wall degrading enzyme which is operably linked to a nucleotide sequence encoding a signal peptide directing the xylanase to an apoplast, plastid or vacuole of the transgenic plant; and at least one DNA comprising a flowering locus c gene coding region operably linked to a constitutive promoter, wherein the transgenic plant expresses the cell wall degrading enzymes and a transcription factor encoded by the flowering locus c gene that delays flowering while increasing biomass and enabling isolation of increased amounts of the cell wall degrading enzymes from the transgenic plant as compared to a non-transgenic plant from which the transgenic plant is derived.

In further embodiments, the transgenic plant further comprises at least one DNA encoding a selectable marker operably linked to a constitutive promoter. In further embodiments, the DNA encoding the selectable marker provides the transgenic plant with resistance to an antibiotic, an herbicide, or to environmental stress. In further embodiments, the DNA encoding resistance to the herbicide is a DNA encoding phosphinothricin acetyl transferase which confers resistance to the herbicide phosphinothricin. In still further embodiments, the DNA encoding the β-glucosidase is from *Butyrivibrio fibrisolvens.* In further still embodiments, the DNA encoding the β-glucosidase comprises the nucleotide sequence set forth in SEQ ID NO:23. In further embodiments, the DNA encoding the ligninase is from *Phanerochaete chrysosporium.* In further embodiments, the DNA encoding the xylanase comprises the nucleotide sequence set forth in SEQ ID NO:24, SEQ ID NO:33, SEQ ID NO:34 or SEQ ID NO:35.

The present invention provides a method for making an enzyme extract comprising one or more cell wall degrading enzymes comprising: providing a transgenic plant capable of expressing one or more cell wall degrading enzymes comprising at least one DNA comprising a cell wall degrading enzyme coding region operably linked to a nucleotide sequence encoding a signal peptide directing the cell wall degrading enzyme encoded by the DNA to an apoplast, plastid or vacuole of the transgenic plant; and at least one DNA comprising a flowering locus c gene coding region operably linked to a constitutive promoter, wherein the transgenic plant expresses the one or more cell wall degrading enzymes and a transcription factor encoded by the flowering locus c gene that delays flowering while increasing biomass and enabling isolation of increased amounts of the hydrolyzing enzyme from the transgenic plant as compared to a non-transgenic plant from which the transgenic plant is derived; growing the transgenic plant for a time to accumulate the one or more cell wall degrading enzymes;harvesting the transgenic plant which has accumulated the one or more cell wall degrading enzymes; grinding the transgenic plant to provide an enzyme extract comprising the one or more cell wall degrading enzymes that accumulated in the transgenic plant.

The present invention provides a method for converting lignocellulosic material to fermentable sugars comprising: providing a transgenic plant capable of expressing one or more cell wall degrading enzymes comprising at least one DNA encoding the one or more cell wall degrading enzymes which is operably linked to a nucleotide sequence encoding a signal peptide directing a cellulose, lignin or lignocellulose hydrolyzing enzyme encoded by the DNA to an apoplast, plastid or vacuole of the transgenic plant; and at least one DNA comprising a flowering locus c gene coding region operably linked to a constitutive promoter, wherein the transgenic plant expresses the one or more cell wall degrading enzymes and a transcription factor encoded by the flowering locus c gene that delays flowering while increasing biomass and enabling isolation of increased amounts of the hydrolyzing enzyme from the transgenic plant as compared to a non-transgenic plant from which the transgenic plant is derived; growing the transgenic plant for a time sufficient for the transgenic plant to accumulate the one or more cell wall degrading enzymes; harvesting the transgenic plant which has accumulated the one or more cell wall degrading enzymes; grinding the transgenic plant to provide an enzyme extract comprising the one or more cell wall degrading enzymes that accumulated in the transgenic plant; incubating the lignocellulosic material in the enzyme extract to produce the fermentable sugars from the lignocellulose in the plant material; and extracting the fermentable sugars produced from the lignocellulosic material.

### BRIEF DESCRIPTION OF THE DRAWINGS

Figure 1 is a diagram of a plasmid containing a heterologous gene expression cassette containing cbh1 operably linked to the rbcS promoter and DNA encoding the rbcS signal peptide and a heterologous gene expression cassette containing the bar gene operably linked to the Act1 promoter. rbcSP is the rbcS gene promoter, SP is DNA encoding the rbcS signal peptide, pin3' is the 3' untranslated region of the potato inhibitor II-chloramphenicol acetyltransferase gene, Act1 is the promoter for the act1 gene, and nos is the 3' untranslated region of the Agrobacterium nopaline synthase gene.

Figure 2 is a diagram of a plasmid containing a heterologous gene expression cassette containing e1 operably linked to the rbcS promoter and DNA encoding the rbcS signal peptide and a heterologous gene expression cassette containing the bar gene operably linked to the Act1 promoter. The terms in the diagram are as in Figure 1.

Figure 3 is a diagram of a heterologous gene expression cassette containing the bar gene in plasmid pDM302. Act1 is the promoter for the act1 gene and nos is the 3' untranslated region of the Agrobacterium nopaline synthase gene.

Figure 4 is a diagram of plasmid pSMF13 which is plasmid pSK containing a heterologous gene expression cassette containing cbh1 operably linked to the rbcS promoter. The terms in the diagram are as in Figure 1.

Figure 5 is a diagram of plasmid pMSF14 which is plasmid pSK containing a heterologous gene expression cassette containing cbh1 operably linked to the rbcS promoter and DNA encoding the rbcS signal peptide. The terms in the diagram are as in Figure 1.

Figure 6 is a diagram of plasmid pMSF15 which is plasmid pBI221 containing a heterologous gene expression cassette containing syn-cbh1 operably linked to the rbcS promoter and DNA encoding the rbcS signal peptide. The terms in the diagram are as in Figure 1.

Figure 7. is a diagram of plasmid pTZA8 which is plasmid pBI121 containing a heterologous gene expression cassette containing e1 operably linked to the CaMV35S promoter and DNA encoding the SSU signal peptide. SSU is the glycine max (soybean) rbcS signal peptide. CaMV35S is the cauliflower mosaic virus 35S promoter. The remainder of the terms are as in the diagram are as in Figure 1.

Figure 8 is a diagram of plasmid pZA9 which is plasmid pBI121 containing a heterologous gene expression cassette containing e1 operably linked to the CaMV35S promoter and DNA encoding the VSP signal peptide. VSP is the soybean vegetative storage protein beta-leader sequences. The remainder of the terms in the diagram are as in Figure 7.

Figure 9 is a diagram of plasmid pZA10 which is plasmid pBI121 containing a heterologous gene expression cassette containing e1 operably linked to the CaMV35S promoter. The remainder of the terms in the diagram are as in Figure 7.

Figure 10 is a diagram of a plasmid containing a heterologous gene expression cassette containing ckg4 operably linked to the rbcS promoter and DNA encoding the rbcS signal peptide and a gene expression cassette containing the bar gene operably linked to the Act1 promoter. The remainder of the terms in the diagram are as in Figure 1.

Figure 11 is a diagram of a plasmid containing a heterologous gene expression cassette containing ckg5 operably linked to the rbcS promoter and DNA encoding the rbcS signal peptide and a gene expression cassette containing the bar gene operably linked to the Act1 promoter. The remainder of the terms in the diagram are as in Figure 1.

Figure 12 is a diagram of plasmid pSMF18 containing a heterologous gene expression cassette containing ckg4 operably linked to the rbcS promoter. The remainder of the terms in the diagram are as in Figure 1.

Figure 13 is a diagram of plasmid pSMF19 containing a heterologous gene expression cassette containing ckg5 operably linked to the rbcS promoter. The remainder of the terms in the diagram are as in Figure 1.

Figure 14 is a diagram of plasmid pSMF16 containing a heterologous gene expression cassette containing ckg4 operably linked to the rbcS promoter and DNA encoding the rbcS signal peptide. The remainder of the terms in the diagram are as in Figure 1.

Figure 15 is a diagram of plasmid pSMF17 containing a heterologous gene expression cassette containing ckg5 operably linked to the rbcS promoter and DNA encoding the rbcS signal peptide. The remainder of the terms in the diagram are as in Figure 1.

Figure 16 is an RNA gel blot analysis of FLC in T0 and T1 tobacco plants. Lanes 1-5 are transgenic lines; Lane C is a negative control.

Figure 17 illustrates FLC transgenic tobacco plants delayed flowering two (2) or more weeks. Figure 17A: Right plant is FLC transgenic and left plant is untransformed control. Figure 17B: Plants from Line 4 (right) compared to control plants (left). Figure 17C: FLC transgenic versus control flowers from the same age. (A=anther, S=stigma). Note the pollen grains on control anthers and stigma.

Figure 18 is a restriction map of the plasmid pGreen. RB= T-DNA right border; LB= T-DNA left border; FLC= FLC coding region (0.59 kb); 35S= CaMV 35S promoter; bar= phosphinothricin acetyltransferase gene; Nos= nopaline synthase terminator. Plasmid size: about 6 kb.

Figure 19 is an RNA-blot analysis of FLC in E1cd-FLC transgenic plants. Lanes: 1 to 6 transgenic lines; C, E1cd control.

Figure 20 is an E1cd-FLC transgenic tobacco (line 1) plant (right) compared to control E1cd plant (left). Note the short stem and larger leaves of transgenic plant.

Figure 21 is a schematic representation of ApoE1 binary vector containing the *Acedothermus cellulolyticus* E1 catalytic domain driven by Cauliflower Mosaic Virus 35S Promoter (CaMV 35S), tobacco Mosaic Virus translational enhancer (Ω), and the sequence encoding the tobacco pathogenesis-related protein 1a (Pr1a) signal peptide for apoplast-targeting of the E1 enzyme, and the polyadenylation signal of nopaline synthase (nos).

Figure 22A illustrates Gus expression in plantlets of transgenic rice as compared to the untransformed control. Figure 22B illustrates greenhouse grown A. cellulolyticus E1 transgenic rice plants.

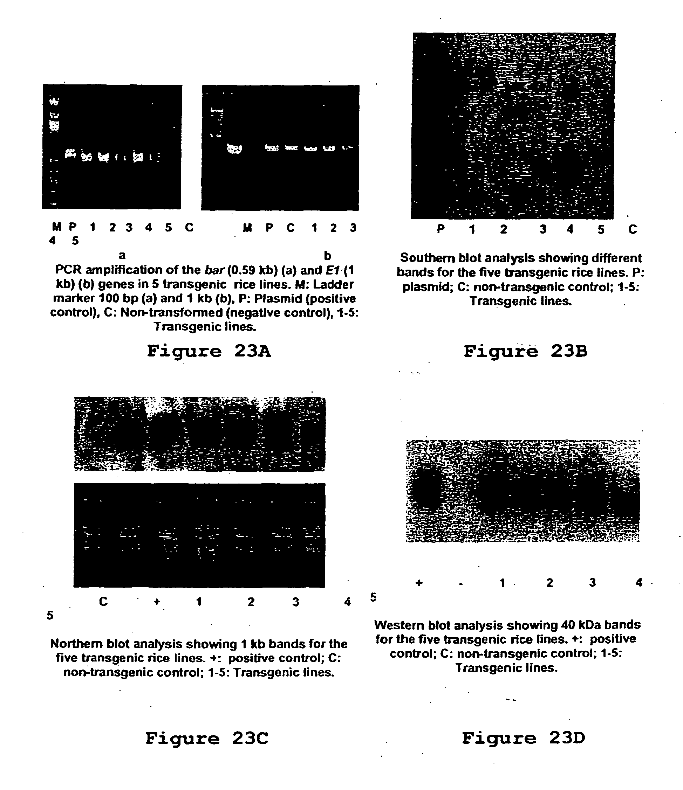

Figures 23A-D illustrate a PCR (A), Southern (B), Northern (C) and Western (D) Blot analysis that show the presence of the transgenes in five transgenic rice lines. Figures 23A illustrates Left; PCR amplification of the bar (0.59 kb), and right; E1 (1 kb) (b) genes in 5 transgenic rice lines. M: Ladder marker 100 bp (a) and 1 kb (b), P: Plasmid (positive control), C: Non-transformed (negative control), 1-5: Transgenic lines. Figures 23B illustrates a Southern blot analysis for E1 transgene showing different bands for the five transgenic rice lines. P: plasmid; C: non-transgenic control; 1-5: Transgenic lines. Figures 23C illustrates a Northern blot analysis showing 1 kb bands for the five transgenic rice lines. +: positive control; C: non-transgenic control; 1-5: Transgenic lines. Figures 23D illustrates a Western blot analysis showing 40 kDa bands for the five transgenic rice lines. +: positive control; C: non-transgenic control; 1-5: Transgenic lines.

Figures 24A and Figures 24B illustrate immunofluorescence confocal microscopy for the transgenic (Figures 24A) and untransformed (Figures 24B) rice showing apoplast localization of the E1 enzyme in transgenic rice leaves.

Figure 25 shows the detection of the E1 enzyme activity using CMCase activity assay. Zones of CMC hydrolysis were decolorized with washing leaving yellow regions in the transgenic as compared to red background in the control.

Figure 26 illustrates in Figure 26A the amount of glucose released from the enzymatic hydrolysis of CMC (1%, 5%, 10%) and Avicel (1%, 5%, 10%) using total protein extracted from E1 expressed rice straw. In Figure 26B is shown the comparison of percentage of glucan converted in the enzymatic hydrolysis of corn stover (CS) and rice straw (RS). CE, commercial enzyme, UT, untreated biomass, CS1, RS1, CS2, and RS2 represent, reaction done using 0.5 ml and 4 ml of total protein (with 4.9% of E1) and commercial β-glucosidase (6.5mg/15ml) respectively.

Figure 27 is a schematic drawing of the plasmid pMZ766 used to produce transgenic maize plants. A 1.076-kb Sac I restriction fragment was used as the probe for Southern blot analysis.

Figure 28 is a PCR analysis of the DNA from plants recovered after transformation with the pMZ766. Lanes 1, untransformed maize control; 2-6, five independent transgenic maize plants; 7, plasmid control, and 8, 1-kb plus DNA ladder.

Figure 29 is a Southern blot analysis of genomic DNA from maize plants, probed with the E1-cd. Lane 1, 10 pg of Sac I digest E1-cd fragment from p MZ766; Lanes 2-3, untransformed maize control (lane 2; DNA undigested and lane3; DNA digested); Lanes 4-13; Five independent pMZ766 transformants; (4, 6, 8, 10, 12) DNA not digested; (5, 7, 9, 11, 13) DNA digested with Sac I. Size of bands is 1kb.

Figure 30 is a Western blot of total soluble protein from transgenic maize plants expressing E1-cd. Lanes +C, positive tobacco control; -C, untransformed maize control; 1-6; transgenic maize plants.

Figure 31 illustrates a schematic representation of plasmid vectors containing two cassettes, one containing the *Acedothermus cellulolyticus* E1 catalytic domain or the ligninase (CGL4) driven by maize rubisco promoter (rbcS) or Cauliflower Mosaic Virus 35S Promoter (CaMV 35S), tobacco Mosaic Virus translational enhancer (Ω), and the sequence encoding the tobacco pathogenesis-related protein 1a (Pr1a) signal peptide for apoplast-targeting or the maize rbcs signal peptide for enzyme targeting, and the polyadenylation signal of nopaline synthase (Nos).

Figure 32 is an illustration of a plasmid used in a 1:1 ratio with the plasmid of Figure 27 in maize transformation experiments. In the two constructs, Ω represents for the tobacco Mosaic Virus translational enhancer, Pr1a SP for the sequence encoding the tobacco pathogenesis-related protein 1a signal peptide for apoplast-targeting of the E1 enzyme, Nos for the polyadenylation signal of nopaline synthase, and bar for the herbicide resistance sequences.

Figure 33A and B illustrates immunofluorescent confocal laser microscopy of apoplast-targeted E1 transgenic maize leaf tissue (Figure 33A) using the E1 primary antibody and the FITC anti-mouse secondary antibody. Figure 33B illustrates immunofluorescent confocal laser microscopy of leaf tissue from an untransformed control maize plant.

Figure 34 illustrates biological activity and conversion ability of maize-produced E against corn stover (CS), Avaeil and CMC. As was performed in our rice work, commercial β-glucosidase (6.5 mg/15 ml) was added to convert cellubiose into glucose.

Figure 35 is immunofluorescent localization of PHBC. Immunofluorescent confocal laser microscopy of choloroplast-targeted polyhydroxybutyrate C in transgenic maize leaf tissue (left) using the PHBC primary antibody and the FITC secondary antibody. Photo on the right is leaf tissue from an untransformed control maize plant.

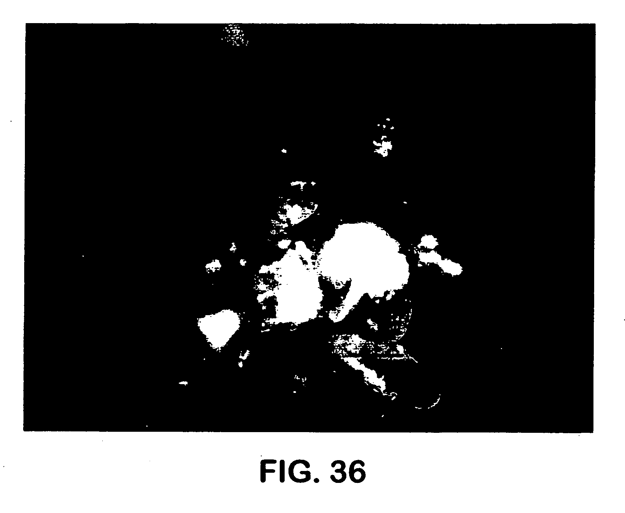

Figure 36 shows corn GFP-chloroplast embryos.

Figure 37 is a Western blot of E1 transgenic maize as compared to E1 transgenic rice and tobacco plants produced in the inventor's laboratory. Lane "+" = Transgenic tobacco as positive control. Lane "-C" = Maize control (untransformed). Lanes 1 to 10 represent at least 5 different transformation events. Lane 11 = E1 Transgenic rice as another positive control

Figure 38 is a schematic of a plasmid containing the *Butyrivibrio fibrisolvens* β-glucosidase (Yao, 2004) cDNA regulated by the 35S promoter and enhancer. This construct too contains the sequences encoding the tobacco pathogenesis-related protein 1a (Pr1a) signal peptide for targeting of β-glucosidase enzyme into plant apoplast.

Figure 39 is a schematic of a plasmid containing the xylanase cDNA regulated by the 35S promoter and enhancer. This construct contains the sequences encoding the tobacco pathogenesis-related protein 1a (Pr1a) signal peptide for targeting of *Cochliobolus carbonum* endoxylanase (Apel et al., Mol. Plant-Microbe Interact, 6:467-473 (1993)) into plant apoplast.

Figure 40 is a schematic of a plasmid containing the *Phanerochaete chrysosporium* ligninase (de Boer et *al.,* 1988) gene regulated by the 35S promoter and enhancer.

Figure 41 is a schematic of a more detailed map of plasmid pGreen, which has the *Arabidopsis* Flowering Locus C (FLC) coding sequences regulated by 35S promoter and Nos terminator. The bar herbicide resistance selectable marker is regulated by 35S promoter and Nos terminator. The sizes of each are shown in kilobases (kb).

### DETAILED DESCRIPTION OF THE INVENTION

The term "cell wall degrading enzyme" as used herein refers to any cellulase, hemicellulase or ligninase.

The term "cellulase" as used herein is a generic term that includes endoglucanases, exoglucanases and β-glucosidases. The term includes endoglucanases such as the EI beta-1,4-endoglucanase precursor gene (e1) of Acidothermus cellulolyticus and exoglucanases such as the cellobiohydrolase gene (cbh1) of Trichoderma reesei (also classified by some as Trichoderma longibrachiatum), the dextranase gene of Streptococcus salivarius encoding the 1,6-alpha-glucanhydrolase gene, and the β-glucosidase gene from Butyrivibrio fibrisolvens or Actinomyces naeslundi. Endoglucanases randomly cleave cellulose chains into smaller units. Exoglucanases include cellobiohydrolases, which liberate glucose dimers (cellobiose) from the ends of cellulose chains; glucanhydrolases, which liberate glucose monomers from the ends of cellulose chains; and, β-glucosidases, which liberate D-glucose from cellobiose dimers and soluble cellodextrins. When all four of the above enzymes are combined, they work synergistically to rapidly decrystallize and hydrolyze cellulose to fermentable sugars.

The term "hemicellulase" as used herein is a generic term which encompasses all varieties of enzymes that degrade any type of hemicellulose such as xylan, glucuronoxylan, arabinoxylan, glucomannan and xyloglucan. Some examples include, but are not limited to xylanase. Examples of beta-1,4-xylanase genes include XYL1 (SEQ ID NO:24), and XYL2 (SEQ ID NO:33), XYL3 (SEQ ID NO:34) and XYL4 (SEQ ID NO:35) of *Cochliobolus carbonum* as described by Apel-Birkhold et al., Applied and Environmental Microbiology, Nov. 1996, pp. 4129-4135. Further examples of xylanases include those described in U.S. Patent No. 6,682,923 to Bentzien et al.,

The term "lignin" is used herein as a generic term that includes both lignins and lignocelluloses.

The term "ligninase" is used herein as a generic term that includes all varieties of enzymes which degrade lignins such as the lignin peroxidase gene of Phanerochaete chrysosporium. Ligninase enzymes degrade lignin into phenolics, unlike cellulases that hydrolyse cellulase into sugars. Ligninase can be provided in the transgenic plants of the present invention so that after harvesting and grinding the plants, ligninase will remove lignin of the lignicellulosic matter for better access of cellulases to the cellulose in the plant matter. Thus, the present invention reduces or eliminates the need for expensive heat and/or chemical pretreatments to remove lignin.

The term "monocot" as used herein refers to all monocotyledonae plants including, but not limited to cereal plants such as maize, wheat, barley, oats, rye, rice, buckwheat, millet, and sorghum. Additionally monocot plants include switchgrass, and other perennial grasses. Other monocots include such plants as sugar cane.

The term "Pr1a" as used herein refers to tobacco pathogenesis-related protein 1a signal peptide encoding a sequence for apoplast-targeting of proteins. U.S. Patent No. 6,750,381 to Mitsuhara et al., teaches the Pr1a signal peptide.

A variety of fungi and bacteria produce ligninase and cellulase enzymes, and based on evolutionary pressures, these fungi are able to degrade lignin or cellulose and hemicellulose of plant residues in the soil. In the laboratory, cellulases have been used to hydrolyze or convert cellulose and hemicellulose into mixtures of simple sugars that can be used in fermentation to produce a wide variety of useful chemical and fuel products, including but not limited to, ethanol, lactic acid, and 1,3-propanediol, which is an important molecular building block in the production of environmentally-friendly plastics.

The biodegradation of lignin, which comprises 20-30% of the dry mass of woody plants, is of great economic importance because this process is believed to be an important rate-limiting step in the earth's carbon cycle. Furthermore, there is considerable potential for the transformation of lignin into aromatic chemical feedstock. Also, delignification of lignocellulosic feeds has been shown to increase their digestibility by cattle by about 30%, therefore, contributing to enhanced cost effectiveness for producing milk and meat. Moreover, research on lignin biodegradation has important implications in biopulping and biobleaching in the paper industry.

The present invention provides transgenic plants which produce ligninases, cellulases, or both in the leaves and straw/stalks of the plant. While the transgenic plant can be any plant which is practical for commercial production, it is preferable that the transgenic plants be constructed from plants which are produced in large quantities and which after processing produce a substantial amount of leaves and stalks as a byproduct. Therefore, it is desirable that the transgenic plant be constructed from plants including, but not limited to, maize, wheat, barley, rye, hops, hemp, rice, potato, soybean, sorghum, sugarcane, clover, tobacco, alfalfa, arabidopsis, coniferous trees, and deciduous trees. Most preferably, the transgenic plant is constructed from maize.

Maize is a preferred plant because it is a major crop in the United States; approximately 60 million acres of maize are produced per year. Further, there is already a large industry built around the processing of maize grain to industrial products, which includes the production of over 1.2 billion gallons of fuel ethanol per year. Thus, fermentable sugars produced by the hydrolysis of maize stalks and leaves according to the present invention can be utilized within the large existing maize refining infrastructure. Leaves and stalks from transgenic maize made according to the present invention can be made available to this refining infrastructure in large quantities, about tens of millions of tons annually) at a current cost of about 30 dollars per ton. This cost is about one quarter of the cost of maize grain which further enhances the value of the present invention for the economical production of a wide variety of industrial products from the residue of transgenic plants made according to the present invention. Furthermore, maize is preferred because it is a C-4 monocot that has very large chloroplasts. The large chloroplasts enables the chloroplasts of the transgenic maize of the present invention to accumulate higher levels of ligninases and cellulases than could be accumulated in the chloroplasts of other transgenic plants, e.g., C-3 dicots and monocots. Therefore, transgenic maize of the present invention is a particularly useful source of ligninases and cellulases.

Thus, the transgenic plants of the present invention provide a plentiful, inexpensive source of fungal or bacterial ligninases and cellulases which can be used to degrade lignins and cellulose in plants to fermentable sugars for production of ethanol or for other uses which require ligninases and cellulases such as pre-treating silage to increase the energy value of lignocellulosic feeds for cows and other ruminant animals, pre-treating lignocellulosic biomass for fermentative conversion to fuels and industrial chemicals, and biodegradation of chloroaromatic environmental pollutants. Because the transgenic plants of the present invention produce the ligninases, cellulases, or both therein, the external addition of ligninases and cellulases for degradation of the plant material is no longer necessary. Therefore, the present invention enables the plant biomass, which is destined to become ethanol or other products, to serve as the source of ligninase and cellulase. Furthermore, the plant material from the transgenic plants of the present invention can be mixed with non-transgenic plant material. The ligninases, cellulases, or both produced by the transgenic plants will degrade the lignin and cellulose of all the plant material, including the non-transgenic plant material. Thus, ligninase and cellulase degradation of plant material can be carried out more economically.

The transgenic plants of the present invention comprise one or more heterologous gene expression cassettes containing DNA encoding at least one fungal or bacterial ligninase, cellulase, or both inserted into the plant's nuclear genome. The preferred cellulase is encoded by a DNA from the microorganism Acidothermus cellulolyticus, Thermomonospora fusca, and Trichoderma reesei (Trichoderma longibrachiatum). Other microorganisms which produce cellulases suitable for the present invention include Zymomonas mobilis, Acidothermus cellulolyticus, Cloostridium thermocellum, Eiwinia chrysanthemi, Xanthomonas campestris, Alkalophilic Baccilus sp., Cellulomonas fimi, wheat straw mushroom (Agaricus bisporus), Ruminococcus flavefaciens, Ruminococcus albus, Fibrobacter succinogenes, and Butyrivibrio fibrisolvens.

The preferred ligninase is lignin peroxidase (LIP) encoded in DNA from Phanerochaete chrysosporium or Phlebia radiata. One of the major lignin-degrading, extracellular enzymes produced by P. chrysosporium is LIP. The LIPs are glycosylated heme proteins (MW 38 to 46 kDa) which are dependent on hydrogen peroxide for activity and catalyze the oxidative cleavage of lignin polymer. At least six heme proteins (H1, H2, H6, H7, H8, and H10) with LIP activity have been identified in P. chrysosporium strain BKMF-1767 of which isozymes H2, H6, H8, and H10 are the major LIPs in both static and agitated cultures of P. chrysosporium. However, other fungi which produce ligninases suitable for use in the present invention include Bjerkandera adusta, Trametes hirsuta, Plebia radiata, Pleurotus spp., Stropharia aurantiaca, Hypholoma fasciculare, Trametes versicolor, Gymnopilus penetrnas, Stereum hirsutum, Mycena haematopus, and Armillaria mellea.

In the present invention, the transgenic plant comprises a DNA encoding one or more cellulase fusion proteins wherein the DNA encoding the cellulases are operably linked to a DNA encoding a signal peptide which directs the cellulase fusion protein to a plant organelle such as the nucleus, a microbody (e.g., a peroxisome, or specialized version thereof, such as a glyoxysome), an endoplasmic reticulum, an endosome, a vacuole, a mitochondria, a chloroplast, or a plastid. By sequestering the cellulase fusion proteins in the plant organelle, the cellulase fusion protein is prevented from leaking outside the cytoplasm to harm the plant by degrading the cellulose in the plant's cell wall while the plant is being cultivated. In particular embodiments of the present invention, the gene encoding the cellulase is modified by replacing the amino acid codons that encode the leader region of the cellulase with amino acid codons that encode the signal peptide.

In a preferred embodiment of the invention, the amino acid codons that encode the signal peptide that directs the protein to which it is attached to the plant organelle, the chloroplasts, are the nucleotide codons that encode the rice rubisco synthase gene (rbcS) small subunit signal peptide (rbcSSP). The nucleotide sequence of the rbcS is set forth in SEQ ID NO:1 (GenBank Accession No. X07515). The 47 amino acid signal peptide of the rbcS protein has the amino acid sequence MAPPS VMASS ATIVA PFQGS SPPPA CRRPP SELQL RQRQH GGRIR CM (SEQ ID N0:2). The rbcS SP directs proteins to which it is operably linked to the chloroplasts of the transgenic plant. Therefore, in the preferred embodiment of the present invention, the transgenic plant comprises a DNA encoding the cellulase operably linked with a DNA encoding the rbcS SP to produce the cellulase fusion protein. The rbcS SP directs the cellulase fusion protein to the chloroplasts. Thus, the cellulase fusion protein produced by the transgenic plant accumulates in the chloroplasts of the transgenic plant which protects the transgenic plant from degradation by the cellulase fusion protein while it is being cultivated. Alternatively, the DNA encoding the cellulase is modified at its 3'end to encode a transit peptide such as the peptide RAVARL (SEQ ID NO:3), which targets the ligninase fusion protein to the peroxisomes (U.S. Patent 6,103,956 to Srienc et al.*).* Preferably, the leader region of the cellulase is also removed. In any one of the above embodiments, the cellulase can be further modified to include a GC content that approximates the GC content of the genomic DNA of the plant by methods well known in the art.

In a preferred embodiment, the cellulase comprising the cellulase fusion protein is encoded by the EI beta-1,4-endoglucanase precursor gene (e1) of Acidothermus cellulolyticus, the cellobiohydrolase gene (cbh1) of Trichoderma reesei (Trichoderma longibrachiatum), the beta-glucosidase gene from Actinomyces naeslundi, or the glucanhydrolase (dextranase) gene from Streptococcus salivarius. The nucleotide sequence of the e1 DNA is set forth in SEQ ID NO:4 (GenBank Accession No. U33212), which encodes the cellulase with the amino acid sequence set forth in SEQ ID NO:5. SEQ ID NO:6 provides the nucleotide sequence of the beta-glucosidase gene from Actinomyces naeslundi (GenBank Accession No. AY029505), which encodes the beta-glucosidase with the amino acid sequence set forth in SEQ ID NO:7. SEQ ID NO:8 provides the nucleotide sequence of the dextranase gene from Streptococcus salivarius (GenBank Accession No. D29644), which encodes a glucanhydrolase with the amino acid sequence set forth in SEQ ID NO:9. The nucleotide sequence of cbh1 is set forth in SEQ ID NO:10 (GenBank Accession No. E00389), which encodes the cellulase that includes the joined exons from positions 210 to 261, 738 to 1434, and 1498-1881.

In the present invention, the transgenic plant comprises a DNA encoding one or more ligninase fusion proteins wherein a DNA encoding the ligninase is operably linked to a DNA encoding a signal peptide which directs the ligninase fusion protein to a plant organelle. By sequestering the ligninase fusion proteins in the plant organelles, the modified ligninase is prevented from leaking outside the cytoplasm to harm the plant by degrading the ligninase in the plant's cell wall while the plant is being cultivated. In particular embodiments of the present invention, the leader sequence of the gene encoding the ligninase is modified by replacing the amino acid codons that encode the leader region of the ligninase with amino acid codons that encode the signal peptide.

In a preferred embodiment of the invention, the amino acid codons that encode the signal peptide are the amino acid codons which encode the rice rubisco synthase gene (rbcS) small subunit signal peptide (rbcSSP). The nucleotide sequence of the rbcS is set forth in SEQ ID NO:1 (GenBank Accession No. X07515). The 47 amino acid signal peptide of the rbcS protein has the amino acid sequence MAPPS VMASS ATIVA PFQGS SPPPA CRRPP SELQL RQRQH GGRIR CM (SEQ ID NO:2). Therefore, in the preferred embodiment of the present invention, the transgenic plant comprises a DNA encoding the ligninase operably linked to a DNA encoding the rbcS SP. The rbcS SP directs the ligninase fusion protein to the chloroplasts. Thus, the ligninase fusion protein produced by the transgenic plant accumulates in the chloroplasts of the transgenic plant which protects the transgenic plant from degradation by the ligninase fusion protein while it is being cultivated. Alternatively, the DNA encoding the ligninase is modified at its 3'end to encode a transit peptide such as the peptide RAVARL (SEQ ID NO: 3) . Optionally, the leader region of the ligninase is also removed. In any one of the above embodiments, the ligninase can be further modified to include a GC content that approximates the GC content of the genomic DNA of the plant by methods well known in the art.

In a preferred embodiment of the invention, the ligninase comprising the ligninase fusion protein is encoded by the lignin peroxidase gene (LIP) genes ckg4 (H2) and ckg5 (H10) of Phanerochaete crysosporium (de Boer et al., Gene 60: 93-102 (1987), Corrigendum in Gene 69: 369 (1988)). The nucleotide sequence of the ckg4 gene is set forth in SEQ ID NO:11 (GenBank Accession No. M18743), which encodes the amino acid with the sequence set forth in SEQ ID NO:12. The nucleotide sequence of the ckg5 gene is set forth in SEQ ID NO:13 (GenBank Accession No. M18794), which encodes the amino acid with the sequence set forth in SEQ ID NO:14.

In the present invention, transcription and, therefore, expression of the ligninase and cellulase fusion proteins are effected by a promoter that is active in a particular tissue of the plant, e.g., a promoter that is active primarily in the leaves of a plant. A leaf-specific promoter that is preferred for transcription (expression at the RNA level) is the rice rubisco synthase gene promoter (rbcSP), which has the nucleotide sequence prior to the rbcS gene coding region included in SEQ ID NO:1. In some embodiments of the present invention, it is desirable to relegate transcription of the heterologous gene expression cassette to the seeds using a seed-specific promoter. Seed-specific promoters that are suitable include, but are not limited to, the seed-specific promoters such as the maize 19 kDa zein (cZ19B1) promoter, the maize cytokinin-induced message (Cim1) promoter, and the maize myo-inositol-1-phosphate synthase (mi1ps) promoter, which are disclosed in U.S. Patent 6,225,529 to Lappegard et al. Therefore, in the heterologous gene expression cassettes, the nucleotide sequence comprising rbcS promoters are operably linked to the nucleotide sequences encoding the ligninase and cellulase fusion proteins. Thus, in a transgenic plant of the present invention, transcription of the ligninase and cellulase fusion proteins occurs primarily in the leaves of the plant, and because the ligninase and cellulase fusion proteins each has a signal peptide that directs its transport to plastids, the ligninase and cellulase fusion proteins accumulate in the plastids.

In the preferred embodiment of the present invention, the 3' ends of the nucleotide sequence encoding the above ligninase and cellulase fusion proteins are operably linked to a 3' noncoding sequence wherein the noncoding sequence contains a poly(A) cleavage/addition site and other regulatory sequences which enables the RNA transcribed therefrom to be properly processed and polyadenylated which in turn affects stability, transport and translation of the RNA transcribed therefrom in the plant cell. Examples of 3' noncoding sequences include the 3' noncoding sequence from the potato protease inhibitor II gene, which includes nucleotides 871 to 1241 of SEQ ID NO:15 (GenBank Accession No. M15186) and the 3' noncoding sequence from the Agrobacterium nopaline synthase gene, which includes nucleotides 2001 to 2521 of SEQ ID NO:16 (GenBank Accession No. V00087 J01541).

The above heterologous gene expression cassettes can be constructed using conventional molecular biology cloning methods. In a particularly convenient method, PCR is used to produce the nucleotide fragments for constructing the gene expression cassettes. By using the appropriate PCR primers, the precise nucleotide regions of the above DNAs can be amplified to produce nucleotide fragments for cloning. By further including in the PCR primers restriction enzyme cleavage sites which are most convenient for assembling the heterogenous gene expression cassettes (e.g., restriction enzyme sites that are not in the nucleotide fragments to be cloned), the amplified nucleotide fragments are flanked with the convenient restriction enzyme cleavage sites for assembling the nucleotide fragments into heterogenous gene expression cassettes. The amplified nucleotide fragments are assembled into the heterogeneous gene expression cassettes using conventional molecular biology methods. Based upon the nucleotide sequences provided herein, how to construct the heterogenous gene expression cassettes using conventional molecular biology methods with or without PCR would be readily apparent to one skilled in the art.

In a further embodiment of the present invention, the transgenic plant comprises more than one heterogeneous gene expression cassette. For example, the transgenic plant comprises a first cassette which contains a DNA encoding a ligninase fusion protein, and one or more cassettes each containing a DNA encoding a particular cellulase fusion protein. Preferably, both the ligninase and cellulase fusion proteins comprise amino acids of a signal peptide which directs the fusion proteins to plant organelles. In a preferred embodiment, the signal peptide for each is the rbcS SP or the SKL motif.

In a further still embodiment, the transgenic plant comprises DNA encoding the ligninase fusion protein such as the ckg4 or ckg5 LIP, an endoglucanase fusion protein such as the e1 fusion protein, and a cellobiohydrolase fusion protein such as the cbh1 fusion protein. In a further still embodiment, the transgenic plant comprises DNA encoding the ligninase fusion protein such as the ckg4 or ckg5 LIP, an endoglucanase fusion protein such as the e1 fusion protein, a cellobiohydrolase fusion protein such as the cbh1 fusion protein, a beta-glucosidase, and a glucanhydrolase. Preferably, both the ligninase and cellulase fusion proteins comprise amino acids of a signal peptide which directs the fusion proteins to plant organelles. In a preferred embodiment, the signal peptide for each is the rbcS SP or the SKL motif.

To make the transgenic plants of the present invention, plant material such as meristem primordia tissue is transformed with plasmids, each containing a particular heterogenous gene expression cassette using the Biolistic bombardment method as described in Example 5 and in U.S. Patent No. 5,767,368 to Zhong et al. Further examples of the Biolistic bombardment method are disclosed in U.S. application Ser. No. 08/036,056 and U. S. Patent No. 5,736,369 to Bowen et al. Each heterogenous gene expression cassette is separately introduced into a plant tissue and the transformed tissue propagated to produce a transgenic plant that contains the particular heterogenous gene expression cassette. Thus, the result is a transgenic plant containing the heterogenous gene expression cassette expressing a ligninase such as ckg4 or ckg5, a transgenic plant containing a heterogenous gene expression cassette expressing endoglucanase such as e1, a transgenic plant containing a heterogenous gene expression cassette expressing a cellobiohydrolase such as cbh1, a transgenic plant containing a heterogenous gene expression cassette expressing an exoglucanase such as beta-glucosidase, and a transgenic plant containing a heterogenous gene expression cassette expressing an exoglucanase such as glucanhydrolase.

Alternatively, transformation of corn plants can be achieved using electroporation or bacterial mediated transformation using a bacterium such as Agrobacterium tumefaciens to mediate the transformation of corn root tissues (see Valvekens et al. Proc. Nat'1. Acad. Sci. USA. 85: 5536-5540 (1988)) or meristem primordia.

In a preferred embodiment of the present invention, the transgenic plant comprises one or more ligninase fusion proteins and one or more cellulase fusion proteins. Construction of the preferred transgenic plant comprises making first generation transgenic plants as above, each comprising a ligninase fusion protein, and transgenic plants as above, each comprising a cellulase fusion protein. After each first generation transgenic plant has been constructed, progeny from each of the first generation transgenic plants are cross-bred by sexual fertilization to produce second generation transgenic plants comprising various combinations of both the ligninase fusion protein and the cellulase fusion protein.

For example, various combinations of progeny from the first generation transgenic plants are cross-bred to produce second generation transgenic plants that contain ckg4 and cbh1, e1, beta-glucosidase, or ckg5; second generation transgenic plants that contain ckg5 and cbh1, e1, or beta-glucosidase; second generation transgenic plants that contain e1 or beta glucosidase, and a second generation transgenic plant that contains e1 and beta-glucosidase.

Progeny of the second generation transgenic plants are cross-bred by sexual fertilization among themselves or with first generation transgenic plants to produce third generation transgenic plants that contain one or more ligninases, one or more cellulases, or combinations thereof.

For example, cross-breeding a second generation transgenic plant containing ckg4 and cbh1 with a second generation transgenic plant containing e1 and beta-glucosidase produces a third generation transgenic plant containing ckg4, cbh1, e1, and beta-glucosidase. The third generation transgenic plant can be cross-bred with a first generation transgenic plant containing ckg5 to produce a fourth generation transgenic plant containing ckg4, ckg5, cbh1, e1, and beta-glucosidase.

It will be readily apparent to one skilled in the art that other transgenic plants with various combinations of ligninases and cellulases can be made by cross-breeding progeny from particular transgenic plants. Zhang et al, Theor. Appl. Genet. 92: 752-761, (1996), Zhong et al, Plant Physiol. 110: 1097-1107, (1996);, and Zhong et al, Planta, 187: 483-489, (1992) provide methods for making transgenic plants by sexual fertilization.

Alternatively, plant material is transformed as above with a plasmid containing a heterologous gene expression cassette encoding the ligninase fusion protein. The transgenic plant is recovered from the progeny of the transformed plant material. Next, plant material from the transgenic plant is transformed with a second plasmid containing a heterologous gene expression cassette encoding the cellulase fusion protein and a second selectable marker. The transgenic plant is recovered from the progeny of the transformed plant material. It will be readily apparent to one skilled in the art that transgenic plants containing any combination of ligninases and cellulases can be made by the above method.

In a preferred embodiment, the above heterologous gene expression cassettes further include therein nucleotide sequences that encode one or more selectable markers which enable selection and identification of transgenic plants that express the modified cellulase of the present invention. Preferably, the selectable markers confers additional benefits to the transgenic plant such as herbicide resistence, insect resistence, and/or resistence to environmental stress.

Alternatively, the above transformations are performed by co-transforming the plant material with a first plasmid containing a heterologous gene expression cassette encoding a selectable marker and a second plasmid containing a heterologous gene expression cassette encoding a ligninase or cellulase fusion protein. The advantage of using a separate plasmid is that after transformation, the selectable marker can be removed from the transgenic plant by segregation, which enables the selection method for recovering the transgenic plant to be used for recovering transgenic plants in subsequent transformations with the first transgenic plant.

Examples of preferred markers that provide resistence to herbicides include, but are not limited to, the bar gene from Streptomyces hygroscopicus encoding phosphinothricin acetylase (PAT), which confers resistance to the herbicide glufonsinate; mutant genes which encode resistance to imidazalinone or sulfonylurea such as genes encoding mutant form of the ALS and AHAS enzyme as described by Lee at al. EMBO J. 7: 1241 (1988) and Miki et al., Theor. Appl. Genet. 80: 449 (1990), respectively, and in U.S. Patent No. 5,773,702 to Penner et al.; genes which confer resistance to glycophosphate such as mutant forms of EPSP synthase and aroA; resistance to L-phosphinothricin such as the glutamine synthetase genes; resistance to glufosinate such as the phosphinothricin acetyl transferase (PAT and bar) gene; and resistance to phenoxy proprionic acids and cycloshexones such as the ACCAse inhibitor-encoding genes (Marshall et al. Theor. Appl. Genet. 83: 435 (1992)). The above list of genes which can import resistance to an herbicide is not inclusive and other genes not enumerated herein but which have the same effect as those above are within the scope of the present invention.

Examples of preferred genes which confer resistance to pests or disease include, but are not limited to, genes encoding a Bacillus thuringiensis protein such as the delta-endotoxin, which is disclosed in U.S. Patent 6,100,456 to Sticklen et al.; genes encoding lectins, (Van Damme et al.,Plant Mol. Biol. 24: 825 (1994)); genes encoding vitamin-binding proteins such as avidin and avidin homologs which can be used as larvicides against insect pests; genes encoding protease or amylase inhibitors, such as the rice cysteine proteinase inhibitor (Abe et al., J. Biol. Chem. 262: 16793(1987)) and the tobacco proteinase inhibitor I (Hubb et al., Plant Mol. Biol. 21: 985(1993)); genes encoding insect-specific hormones or pheromones such as ecdysteroid and juvenile hormone, and variants thereof, mimetics based thereon, or an antagonists or agonists thereof; genes encoding insect-specific peptides or neuropeptides which, upon expression, disrupts the physiology of the pest; genes encoding insect-specific venom such as that produced by a wasp, snake, etc.; genes encoding enzymes responsible for the accumulation of monoterpenes, sesquiterpenes, asteroid, hydroxaminc acid, phenylpropanoid derivative or other non-protein molecule with insecticidal activity; genes encoding enzymes involved in the modification of a biologically active molecule (see U.S. Patent No. 5,539,095 to Sticklen et al., which discloses a chitinase that functions as an anti-fungal); genes encoding peptides which stimulate signal transduction; genes encoding hydrophobic moment peptides such as derivatives of Tachyplesin which inhibit fungal pathogens; genes encoding a membrane permease, a channel former or channel blocker (for example cecropin-beta lytic peptide analog renders transgenic tobacco resistant to Pseudomonas solanacerum) (Jaynes et al. Plant Sci. 89: 43 (1993)); genes encoding a viral invasive protein or complex toxin derived therefrom (viral accumulation of viral coat proteins in transformed cells of some transgenic plants impart resistance to infection by the virus the coat protein was derived as shown by Beachy et al. Ann. Rev. Phytopathol. 28: 451 (1990); genes encoding an insect-specific antibody or antitoxin or a virus-specific antibody (Tavladoraki et al. Nature 366: 469(1993)); and genes encoding a developmental-arrestive protein produced by a plant, pathogen or parasite which prevents disease. The above list of genes which can import resistance to disease or pests is not inclusive and other genes not enumerated herein but which have the same effect as those above are within the scope of the present invention.

Examples of genes which confer resistence to environmental stress include, but are not limited to, mtld and HVA1, which are genes that confer resistance to environmental stress factors; rd29A and rd19B, which are genes of Arabidopsis thaliana that encode hydrophilic proteins which are induced in response to dehydration, low temperature, salt stress, or exposure to abscisic acid and enable the plant to tolerate the stress (Yamaguchi-Shinozaki et al., Plant Cell 6: 251-264 (1994)). Other genes contemplated can be found in U.S. Patents Nos. 5,296,462 and 5,356,816 to Thomashow.

Thus, it is within the scope of the present invention to provide transgenic plants which express one or more ligninase fusion proteins, one or more cellulase fusion proteins, and one or more of any combination of genes which confer resistance to an herbicide, pest, or environmental stress.

In particular embodiments of the present invention, the heterologous gene expression cassettes can further be flanked with DNA containing the matrix attachment region (MAR) sequence. While use of MAR in the present invention is optional, it can used to increase the expression level of transgenes, to get more reproducible results, and to lower the average copy number of the transgene (Allen et al., The Plant Cell 5: 603-613 (1993); Allen et al., The Plant Cell 8: 899-913 (1996); Mlynarova et al., The Plant Cell 8: 1589-1599 (1996)).

To degrade the lignocellulose in the leaves and stalks of the transgenic plants of the present invention, the transgenic plant is ground up to produce a plant material using methods currently available in the art to disrupt a sufficient number of the plant organelles containing the ligninase and cellulase therein. The ligninase and cellulase degrade the lignocellulose of the transgenic plant into fermentable sugars, primarily glucose, and residual solids. The fermentable sugars are used to produce ethanol or other products.

The transgenic plants can be processed to ethanol in an improvement on the separate saccharification and fermentation (SHF) method (Wilke et al., Biotechnol. Bioengin. 6: 155-175 (1976)) or the simultaneous saccharification and fermentation (SSF) method disclosed in U.S. Patent 3,990,944 to Gauss et al. and U.S. Patent 3,990,945 to Huff et al. The SHF and SSF methods require pre-treatment of the plant material feedstock with dilute acid to make the cellulose more accessible followed by enzymatic hydrolysis using exogenous cellulases to produce glucose from the cellulose, which is then fermented by yeast to ethanol. In some variations of the SHF or SSF methods, the plant material is pre-treated with heat or with both heat and dilute acid to make the cellulose more accessible.

An SHF or SSF method that uses the transgenic plant material of the present invention as the feedstock is an improvement over the SHF or SSF method because the transgenic plant material contains its own cellulases and ligninases or cellulases. Therefore, exogenous ligninases and/or cellulases do not need to be added to the feedstock. Furthermore, because particular embodiments of the transgenic plant material produce ligninase, the need for pre-treatment of the plant material in those embodiments before enzymatic degradation is not necessary. In a further improvement over the SHF method, the transgenic plant material is mixed with non-transgenic plant material and the mixture processed to ethanol.

The following examples are intended to promote a further understanding of the present invention.

### EXAMPLE 1

This example shows the construction of plasmids comprising a heterologous gene expression cassette comprising a DNA encoding a cellulase fusion protein and a heterologous gene expression cassette comprising a DNA encoding the bar gene (Table 1).

| **Table 1** | | |
|---|---|---|
| | Construct | Plasmid features |
| 1 | rbcSP/e1/pin 3'//Act1 P/*bar*/*nos* 3' | rbcSP leaf-specific promoter driving cellulase cDNA of A. *cellulolyticus* |
| 2 | *rbc*SP/*cbh*1/*pin* 3'//*Act*1 P/*bar*/*nos* 3' | rbcSP leaf-specific promoter driving cellulase cDNA of T. *reesi* |
| 3 | *rbc*SP/*rbc*S SP/*e*1/*pin* 3'//*Act*1 P/bar/nos 3' | The *rbc*S SP targets cellulase of A. *cellulolyticus* into maize chloroplasts |
| 4 | *rbc*SP/*rbc*S SP/*cbh*1/*pin* 3'// *Act*1 *P*/*bar*/*nos* 3' | The rbcs SP targets cellulase of T. reesi into maize chloroplasts |

### Abbreviations:

The term "rbcSP" means the rice rubisco rbcS promoter region. The rbcSP is a leaf-specific promoter that limits transcription of rbcS to the leaves (Schaeffer and Sheen, Plant Cell 3: 997-1012 (1991)). The nucleotide sequence for the rbcS promoter region is set forth in SEQ ID NO:1.

The term "e1" means the cDNA isolated from Acidothermus cellulolyticus which encodes the cellulase EI beta-1,4-endoglucanase precursor. The nucleotide sequence for the gene encoding e1 is set forth in SEQ ID NO:4. In this example, the codons for the 41 amino acid leader sequence (nucleotides 824 to 946 of SEQ ID NO:4) are removed.

The term "cbh1" means the cDNA isolated from Trichoderma reesi that encodes the cellulase cellobiohydrolase. The nucleotide sequence for the gene encoding cbh1 is set forth in SEQ ID N0:10. In this example, the codons for the 54 amino acid leader sequence (nucleotides 210 to 671 of SEQ ID NO:10) are removed.

The term "pin3"' means the potato protease inhibitor II-chloramphenicol acetyltransferase gene's 3' untranslated sequence which contains transcription termination signals (Thornburg et al., Proc. Natl. Acad. Sci. USA 84: 744-748 (1987)). The pin3' untranslated sequence includes nucleotides 882 to 1241 of the nucleotide sequence set forth in SEQ ID NO:15.

The term "bar" means the phosphinothricin acetyl transferase gene (Thompson et al., EMBO J. 6: 2519-2523 (1987)). The bar gene is a selectable marker for herbicide resistance. The 5' end of bar is operably linked to the rice actin 1 gene promoter which has been shown to operable in maize (Zhong et al., Plant Physiology 110: 1097-1107 (1996); Zhang et al., Theor. Appl. Genet. 92: 752-761 (1996); Zhang et al., Plant Science 116: 73-84 (1996)). The 3' end of bar is operably linked to the nos 3' untranslated sequences. The nucleotide sequence of the bar gene is set forth in SEQ ID NO:18 (GenBank Accession No. X05822), which encodes the bar having the amino acid sequence from nucleotides 160 to 711.

The term "Act1 P" means the rice Act1 gene promoter which further includes the 5' intron region (McElroy et al., Mol. Gen. Genet. 231: 150-160 (1991). The sequence of the Act1 gene and its promoter is set forth in SEQ ID NO:19 (GenBank Accession No. X63830).

The term "nos3"' means the 3' untranslated sequence from the Agrobacterium nopaline synthase gene encoding nopaline synthase of the amino acid sequence as set forth in SEQ ID NO:17 which includes nucleotides 2002 to 2521 of SEQ ID NO:16 (GenBank Accession No. V00087 J01541). The Nos3' sequence contains transcription termination signals.

The term "rbcS SP" means the rice rubisco small subunit signal peptide which consists of 47 codons encoding the peptide with the amino acid sequence set forth in SEQ ID NO:2. The rbcS SP directs the translocation of the rbcS small subunit or any polypeptide to which it is attached to the chloroplasts (Loza-Tavera et al., Plant Physiol. 93: 541-548 (1990)).

Construct 1 contains the rice rubisco rbcS leaf-specific promoter which limits expression of the cellulase encoded by e1 to the cells of the leaves of the maize plant.

Construct 2 contains the rice rubisco rbcS leaf-specific promoter which limits expression of the cellulase encoded by cbh1 to the cells of the leaves of the maize plant.

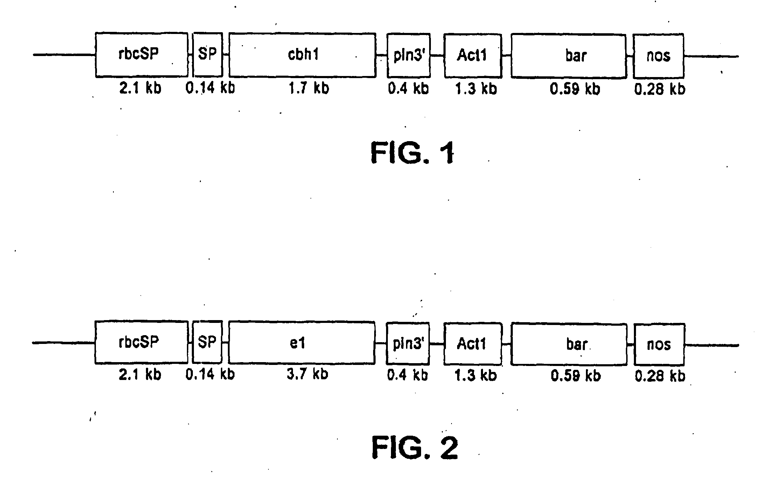

Construct 3, which is shown in Figure 1, is like construct 1 except that DNA encoding the rbcS SP signal peptide is operably linked to the 5' end of the e1, and construct 4, which is shown in Figure 2, is like construct 2 except that DNA encoding the rbcS SP signal peptide is operably linked to the 5' end of cbh1. Therefore, expression of cellulase from construct 3 or 4, which is limited to the cells of the leaves, directed to the chloroplasts in the cells. All of the above constructs are adjacent to a heterologous gene expression cassette containing the bar gene operably linked to the Act1 promoter.

Construction of plasmid rbcSP/rbcS SP/cbh1//pin3'//Act1 P/bar/nos3'. The starting plasmid was pBR10-11 which contained the crylA(b) gene upstream of the pin3'. Between the crylA(b) and the pin3' is a DNA polylinker containing in the following order a SmaI, BamHI, SpeI, XbaI, NotI, and EagI restriction enzyme recognition site. The plasmid pBR10-11 (available from Silan Dai and Ray Wu, Department of Molecular Biology and Genetics, Biotechnology Building, Cornell University, Ithaca, New York 14853-2703) was digested with restriction enzymes SpeI and XbaI to produce a 9.2 kb DNA fragment. The 9.2 kb DNA fragment (pBR10-11/SpeI/XbaI/9.2 kb fragment) was purified by agarose gel electrophoresis.

The plasmid pB210-5a (available from William S. Adney, Mike Himmel, and Steve Thomas, National Renewable Energy Laboratory, 1670 Cole Boulevard, Golden Colorado 80401) containing the cbh1 gene from Trichoderma reesei (Trichoderma longibrachiatum)was digested with SpeI and XbaI. The digested plasmid was electrophoresed on an agarose gel and a 1.8 kb fragment (pB210-5a/SpeI/XbaI/1.8 kb fragment containing cbh1) was purified from the gel.

The above 9.2 kb and the 1.8 kb DNA fragments were ligated together using T4 DNA ligase to make plasmid "pBR10-11-cbh1" which was used to transform E. coli XL1 Blue. Transformed bacteria containing plasmid pBR10-11-cbhl were identified by plating on LB agar gels containing ampicillin.

The plasmid pBR10-11-cbh1 was digested with SmaI and PstI. The PstI end was made blunt with mung bean exonuclease. The digested plasmid was electrophoresed on an agarose gel and the 2.8 kb DNA fragment containing cbh1 and pin3' was purified from the gel. The purified DNA fragment was designated "cbh1-pin3'/blunt-ended."

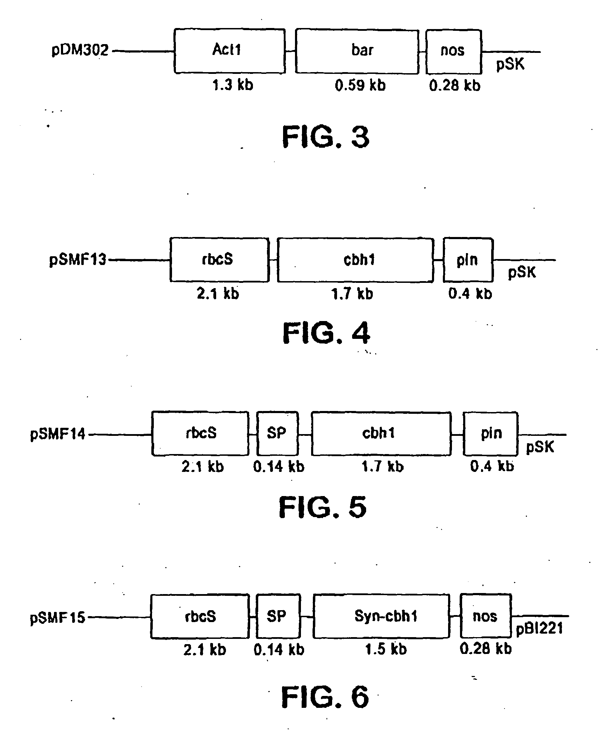

The plasmid pDM302 (Cao et al., Plant Cell Reports 11: 586-591 (1992)), shown in Figure 3, containing upstream of a ClaI site, a gene cassette consisting of the bar gene flanked by an upstream Act1 promoter and a downstream nos3', was digested with ClaI. The ClaI ends of the digested plasmid were made blunt with Taq DNA polymerase and the digested plasmid electrophoresed on an agarose gel. The digested plasmid was designated "pDM302/ClaI/blunt-ended."

The pDM302/ClaI/blunt-ended plasmid and the cbhl-pin3'/blunt-ended DNA fragment were ligated together using T4 DNA ligase to make plasmid "pDM302-cbh1-pin3"' which was used to transform E. coli XLlBlue. Transformed bacteria containing plasmid pDM302-cbh1-pin3' were identified by plating on LB agar gels containing ampicillin.

Plasmid pDM302-cbhl-pin3' was digested with SpeI, the ends made blunt with Taq DNA polymerase, and purified by agarose gel electrophoresis. The purified DNA fragment was designated "pDM302-cbh1-pin3'/SpeI/blunt-ended."

Plasmid pRRI (available from Silan Dai and Ray Wu, Department of Molecular Biology and Genetics, Biotechnology Building, Cornell University, Ithaca, New York 14853-2703), which contains the rice rbcS small subunit gene, was digested with PstI. The rbcS promoter is flanked by PstI sites. The PstI ends were made blunt with mung bean nuclease and the 2 kb DNA fragment (rice rbcS/PstI/blunt-ended) containing the promoter was purified by agarose gel electrophoresis.

Rice rbcSP/PstI/blunt-ended and plasmid pDM-cbh1-pin3'/SpeI/blunt-ended were ligated using T4 DNA ligase to make rbcSP/cbh1/pin3'//Act1P/bar/nos3' which was then used to transform E. coli XL Blue. Transformed bacteria containing plasmid rbcSP/cbhl/pin3'//ActlP/bar/nos3' were identified by plating on LB agar gels containing ampicillin.

PCR was used to insert NotI sites into rbcSP/cbh1/pin3'//Act1P/bar/nos3'. These sites were used to insert the rice rubisco signal peptide in place of the cbh1 signal peptide. The pRRI plasmid was the source of the rice rubisco signal peptide. It was also the used as a PCR template to produce the PCR product containing the rice rubisco signal peptide flanked by NotI cohesive termini. The rice rubisco signal peptide and the rbcSP/cbhl/pin3'//ActlP/bar/nos3' plasmid were ligated together using T4 DNA ligase to make rbcSP/rbcS SP/cbhl/pin3'//ActlP/bar/nos3' which was then used to transform E. coli XL Blue. Transformed bacteria containing plasmid rbcSP/rbcS SP/cbh1/pin3'//Act1P/bar/nos3' were identified by plating on LB agar gels containing ampicillin.

Construction of plasmid rbcSP/rbcS SP/e1/pin3'//ActlP/bar/nos3'. Plasmid pMPT4-5 (available from William S. Adney, Mike Himmel, and Steve Thomas, national Renewable Energy laboratory, 1670 Colorado Boulevard, Golden, Colorado 80401) contains the e1 gene encoding endoglucanase I from Acidothermus cellulolyticus as a 3.7 kb PvuI DNA fragment cloned into pGEM7 (Promega Corporation, Madison, Wisconsin). PCR was used to produce a DNA fragment containing the e1 gene flanked by AscI recognition sites. Plasmid rbcSP/cbhl/pin3'//Act1P/bar/nos3' was also mutagenized by PCR to introduce AscI sites flanking the cbh1 gene. Next, the plasmid rbcSP/cbh1/pin3'//Act1P/bar/nos3' was digested with AscI and the plasmid free of the cbh1 gene was purified by agarose gel electrophoresis. The AscI flanked e1 gene was ligated using T4 DNA ligase into the rbcSP/cbhl/pin3'//ActlP/bar/nos3' free of the cbh1 gene to produce plasmid rbcSP/el/pin3'//ActlP/bar/nos3', which then used to transform E. coli XL Blue. Transformed bacteria containing plasmid rbcSP/e1/pin3'//Act1P/bar/nos3' were identified by plating on LB agar gels containing ampicillin.

PCR was used to insert NotI sites into rbcSP/e1/pin3'//ActlP/bar/nos3'. These sites-were used to insert the rice rubisco signal peptide in place of the cbh1 signal peptide. The pRRI plasmid was the source of the rice rubisco signal peptide. It was also the used as a PCR template to produce the PCR product containing the rice rubisco signal peptide flanked by NotI cohesive termini. The rice rubisco signal peptide and the rbcSP/e1/pin3'//Act1P/bar/nos3' plasmid were ligated together using T4 DNA ligase to make rbcSP/rbcS SP/e1/pin3'//Act1P/bar/nos3' which was then used to transform E. coli XL Blue. Transformed bacteria containing plasmid rbcSP/rbcS SP/e1/pin3'//Act1P/bar/nos3' were identified by plating on LB agar gels containing ampicillin.

Both heterologous gene expression cassettes are contiguous and the contiguous cassettes can be flanked by MAR sequences.

### EXAMPLE 2

This example shows the construction of plasmids comprising a heterologous gene expression cassette comprising a DNA encoding a cellulase fusion protein. The plasmid constructs are shown in Table 2.

| **Table 2** | | |
|---|---|---|
| | Construct | Plasmid features |
| 1 | *rbc*SP/*cbh*1/*pin* 3' | *rbc*SP leaf-specific promoter driving cellulase cDNA of *T*. *reesei* |
| 2 | *rbcSP*/*rbcS* SP/*cbh1*/*pin* 3' | The *rbcS* SP targets cellulase of *T. reesi* into maize chloroplasts |
| 3 | *rbcSP*/*rbcS* SP/syn-*cbh1*/*pin* 3' | The *rbcS* SP targets modified cellulase of *T*, *reesei* into maize chloroplasts |
| 4 | CaMv35s/SSU/e1/*nos*3' | The SSU targets the cellulase of *A*. *cellulolyticus* into maize chloroplasts |
| 5 | CaMv35s/VSP/el/*nos*3' | The VSP targets the cellulase of *A*. *cellulolyticus* into maize apoplasts |
| 6 | CaMv35s/el/*nos*3' | No signal peptide |

### Abbreviations:

The term "syn-cbh1" refers to a cbh1 gene that has been codon-modified for use in transformation of tobacco plants. It is available from.

The term "CaMV35s" refers to the cauliflower mosaic virus promoter.

The term "SSU" refers to the glycine max rbcS signal peptide. Glycine max is a soybean and not a rice variety.

The term "VSP" refers to the soybean vegetative storage protein beta signal peptide.

The remainder of the terms in Table 2 are the same as those for table 1.

Construct 1, which is shown in Figure 4, is plasmid pSMF13 which is plasmid pSK (Stratagene, La Jolla, California) which contains cbh1, operably linked to the rice rubisco rbcS leaf-specific promoter which limits expression of the cellulase encoded by cbh1 to the cells of the leaves of the maize plant.

Construct 2, which is shown in Figure 5, is plasmid pSF15 which is plasmid pSK which contains cbh1 operably linked to the rice rubisco rbcS leaf-specific promoter which limits expression of the cellulase encoded by cbh1 to the cells of the leaves of the maize plant and a DNA encoding the rbcS SP which targets the cellulase to the chloroplasts.

Construct 3, which is shown in Figure 6, is like construct 2 except that the cbh1 has been modified to decrease the GC content of the cbh1 to an amount similar to the GC content of the tobacco plant genome. The nucleotide sequence of the modified cbh1 (syn-cbh1) in plasmid pBI221 is set forth in SEQ ID NO:20.

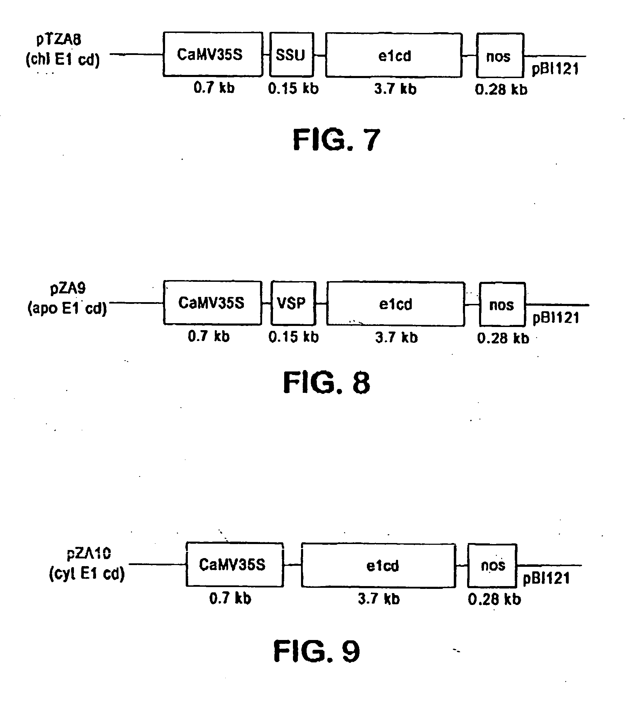

Construct 4, which is shown in Figure 7, is plasmid pTZA8 which is plasmid pBI121 which contains the caMV35s promoter, which is a constitutive promoter that is active in most plant tissues, to drive expression of e1 which is operably linked to a DNA encoding the SSU signal peptide which targets the cellulase to the chloroplasts.

Construct 5, which is shown in Figure 8, is plasmid pZA9 which is similar to construct 4 except the signal peptide is encoded by DNA encoding the VSP signal peptide which targets the cellulase to the apoplasts. Construct 6, which is shown in Figure 9, is plasmid pZA10 which is similar to construct 4 or 5 except that e1 is not operably linked to a DNA encoding a signal peptide that targets the cellulase to a plant organelle.

The constructs were prepared as follows.

First, the plasmid pRR1, which contains the rice rbcS gene was obtained from Ray Wu and Silan Dai, Cornell University. The rice rubisco (rbcS) small subunit was cleaved from pRR1 using EcoRI and EcoRV restriction sites to release a 2.1 kb DNA fragment containing the rbcS. The 2.1 kb DNA fragment was ligated into the plasmid pSK between the EcoR1 and EcoRV sites to produce plasmid pSMF8. The 2.1 kb DNA fragment provided the promoter for the cbh1 constructs below.

Next, the cbh1 gene was cloned downstream of rbcS promoter in plasmid pSMF8. First, a 1.7 kb DNA fragment containing the cbh1 gene from Trichoderma reesei was isolated from plasmid pB210-5A (available from William S. Adney, Mike Himmel, and Steve Thomas, National Renewable Energy Laboratory; described in Shoemaker et al., Bio/Technology 1: 691-696 (1983)) by digesting with SalI and XhoI. The ends of the 1.7 kb DNA fragment were made blunt end using DNA polymerase I (large fragment). The blunt-ended DNA fragment was cloned into plasmid pSMF8, which had been digested with BamHI and the ends made blunt with DNA polymerase I, to make plasmid pSMF9.

Next, to complete the heterologous gene expression cassette, the pin3' transcription termination nucleotide sequence was inserted at the 3' end of the cbh1 gene in plasmid pSMF9. The pin3' transcription termination nucleotide sequence was cleaved from pBR10-11 with PstI. However, to remove the pin3' transcription termination nucleotide sequence from pBR10-11, a PstI site had to be introduced upstream of the pin3' transcription termination nucleotide sequence.

To generate the PstI site upstream of the pin3' transcription termination nucleotide sequence in pBR10-11, the pBR10-11 was digested with NotI and XhoI and a 70 bp multi-cloning site nucleotide sequence, which had been isolated from the pSK vector by digesting with NotI and XhoI, was cloned between the NotI and XhoI sites of the pBR10-11 to produce plasmid pSMF11. The pin3' transcription termination nucleotide sequence was then removed from pSMF11 by digesting with PstI to produce a 1 kb DNA fragment which was then cloned into the PstI site of pSK, which had been digested with PstI, to produce plasmid pSMF12. PSMF12 was then digested with NotI to produce a 1 kb DNA fragment containing the pin3' transcription termination nucleotide sequence. The 1 kb DNA fragment cloned into the NotI site downstream of the cbh1 gene in pSMF9, which had been digested with NotI, to produce plasmid pSMF13 (construct 1 in Table 2).

Next, a DNA encoding a signal peptide which targets proteins to which it is attached to the chloroplasts was inserted upstream of the cbh1 and in the same reading frame as the cbh1. Thus, a fusion protein is produced from translation of RNA transcribed from the cbh1 DNA linked to the DNA encoding the signal peptide. DNA encoding the signal peptide (SP) was isolated from the rbcS in the pRR1 plasmid. Because there were no convenient restriction enzyme sites available which flanked the DNA encoding the SP for cloning, it was planned to PCR amplify that region containing the DNA encoding the SP using PCR primers with PCR primers that contained convenient restriction enzyme sites for cloning. At the end of the rbcS promoter pSMF13 is a unique AvrII site and upstream of the first ATG of the cbh1 gene is a unique BsrGI. A DNA encoding the SP that was flanked with an AvrII site on one end and a BsrGI site on the opposite end would be able to be cloned between the AvrII and BsrGI sites in PSMF13. That would place the DNA encoding the SP between the rbcS promoter and the cbh1 gene and would enable a fusion protein containing the SP fused to the cellulase.

Therefore, PCR primers were synthesized using DNA sequences for the AvrII and BsrGI sites and the SP DNA sequences. The upstream PCR primer (SP1F) had the nucleotide sequence 5'-CCGCCTAGGCGCATGGCCCCCTCCGT-3' (SEQ ID NO:21) and the downstream PCR primer (SP3R) had the nucleotide sequence 5'-CGCTGTACACGCACCTGATCCTGCC-3' (SEQ ID NO:22). Plasmid pRR1 encoding the SP was PCR amplified with the PCR primers and the 145 bp amplified product was purified using 2% agarose gel. The purified 145 bp product was sequenced to confirm that the 145 bp amplified product contained the SP nucleotide sequences. The amplified product was digested with AvrII and BsrGI and cloned between the AvrII and BsrGI sites of pSMF13 digested with AvrII and BsrGI to produce plasmid pSMF14.

To produce pSMF15 which contains a cbh1 gene codon-modified to decrease the GC content of the cbh1 gene to an amount similar to the GC content of the tobacco genome, a synthetic cbh1 (syn-cbh1) gene was obtained from plasmid pZD408 (available from Ziyu Dai, Pacific Northwest national Laboratory, 902 Battelle Boulvard, Richland, Washington 99352). The syn-cbh1 is a cbh1 which had been codon-modified for use in tobacco plant transformations. The nucleotide sequence of syn-cbh1 is set forth in SEQ ID NO:20. Plasmid pZD408 was linearized with NcoI and the ends made blunt. Then, the blunt-ended pZD408 was digested with HindIII to remove the CaMV35S promoter. A 4.5 kb DNA fragment containing the syn-cbh1 was isolated from the CaMV35S promoter by agarose gel electrophoresis. The 4.5 kb DNA fragment was dephosphorylated and the DNA fragment containing a blunt end and a HindIII end was named pZD408B.

Plasmid pSMF14 was digested with BsrGI, the BsrGI ends made blunt, and then pSMF14 was digested with HindIII to produce a DNA fragment containing the rbcS promoter with the DNA encoding the SP flanked by a blunt end and a HindIII end. The DNA fragment was purified by agarose gel electrophoresis and ligated to the pZD408B DNA fragment to produce plasmid pSMF15 (construct 3 of Table 2) .

The heterologous gene expression cassettes are contiguous can be flanked by MAR sequences.

### EXAMPLE 3

This example shows the construction of plasmids comprising a heterologous gene expression cassette comprising a DNA encoding a ligninase fusion protein and a heterologous gene expression cassette comprising a DNA encoding the bar gene. The constructs are shown in Table 3.

| Table 3 | | |
|---|---|---|
| | Construct | Plasmid features |
| 1 | *rbcSP*/*ckg4*/*pin* 3'//Act1 *P*/*bar*/*nos* 3' | *rbc*SP leaf-specific promoter driving *ckg*4 cDNA of *P*. *chrysosporium* |
| 2 | *rbc*SP/*ckg*5/*pin* 3'//Act1 *P*/*bar*/*nos* 3' | *rbc*SP leaf-specific promoter driving *ckg*5 cDNA of *P*. *chrysosporium* |
| 3 | *rbc*SP/*rbcS* SP/*ckg*4/pin 3'//Act1 *P*/*bar*/*nos* 3' | The *rbcS* SP targets *ckg*4 into maize chloroplasts |
| 4 | *rbcSP*/*rbcS* SP/*ckg*5/*pin* 3'// Act1 *P*/*bar*/*nos* 3' | The *rbcS* SP targets *ckg*5 into maize chloroplasts |

### Abbreviations:

The terms "ckg4" and "ckg5" mean the ligninase cDNAs isolated from the basidiomycete Phanerochaete. chrysosporium, SEQ ID NO:11 and SEQ ID NO:13, respectively. The codons for the 28 amino acid leader are deleted so that the expressed gene product remains inside the cells.

The remainder of the terms in Table 3 are the same as those for Table 1. All plasmid constructs contain the selectable marker gene (bar) driven by the rice actin 1 gene promoter. The rice actin gene and its promoter are disclosed in U.S. Patent 5,641,876 to McElroy et al.

Construct 1 contains the rice rubisco rbcS leaf-specific promoter which limits expression of the ligninase encoded by ckg4 to the cells of the leaves of the maize plant.

Construct 2 contains the rice rubisco rbcS leaf-specific promoter which limits expression of the ligninase encoded by ckg5 to the cells of the leaves of the maize plant.

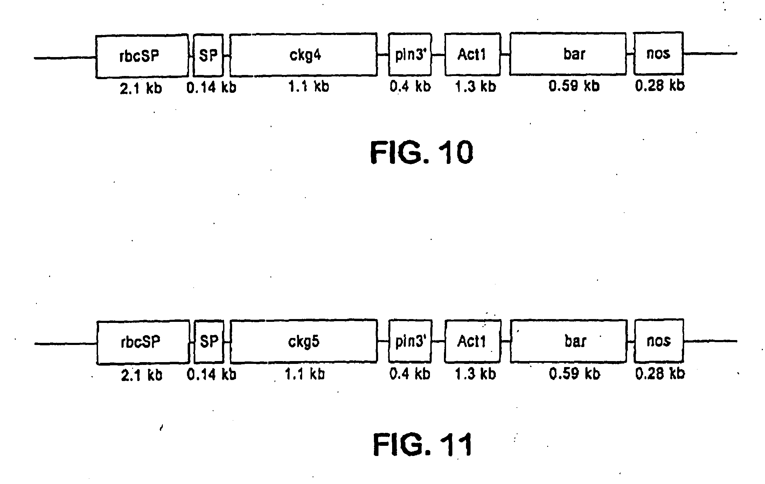

Construct 3, which is shown in Figure 10, contains the rice rubisco rbcS leaf-specific promoter which limits expression of the ligninase encoded by ckg4 to the cells of the leaves of the maize plant and further contains DNA encoding the rbcS SP which targets the ligninase to the chloroplasts.

Construct 4, which is shown in Figure 11, contains the rice rubisco rbcS leaf-specific promoter which limits expression of the ligninase encoded by ckg5 to the cells of the leaves of the maize plant and further contains DNA encoding the rbcs SP which targets the ligninase to the chloroplasts. All of the above constructs are adjacent to a heterologous gene expression cassette containing the bar gene operably linked to the Act1 promoter. Both heterologous gene expression cassettes are contiguous and the contiguous cassettes can be flanked by MAR sequences.

### EXAMPLE 4

This example shows the construction of plasmids comprising a heterologous gene expression cassette comprising a DNA encoding a ligninase fusion protein. The constructs are shown in Table 4.

| **Table 4** | | |
|---|---|---|
| | Construct | Plasmid features |
| 1 | *rbc*SP/*ckg*4/*pin* 3' | *rbc*SP leaf-specific promoter driving *ckg*4 cDNA of *P. chrysosporium* |
| 2 | *rbc*SP/*cka*5/*pin* 3' | *rbc*SP leaf-specific promoter driving *ckg*5 cDNA of *P*. *chrysosporium* |
| 3 | *rbc*SP/*rbc*S SP/*ckg*4/pin 3' | The *rbc*S SP targets *ckg*4 into maize chloroplasts |
| 4 | *rbc*SP/*rbc*S SP/*ckg*5/*pin* 3' | The *rbcS* SP targets *ckg*5 into maize chloroplasts |

The terms in table 4 are the same as those for Tables 1 and 3.

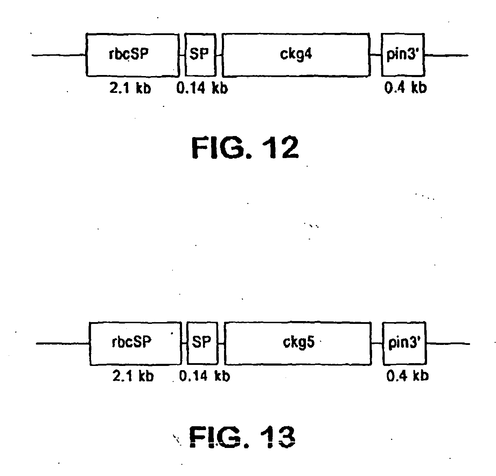

Construct 1, which is shown in Figure 12, is plasmid pSMF18 which is plasmid pSK which contains the rice rubisco rbcS leaf-specific promoter which limits expression of the ligninase encoded by ckg4 to the cells of the leaves of the maize plant.

Construct 2, which is shown in Figure 13, is plasmid pSMF19 which is plasmid pSK which contains the rice rubisco rbcS leaf-specific promoter which limits expression of the ligninase encoded by ckg5 to the cells of the leaves of the maize plant.

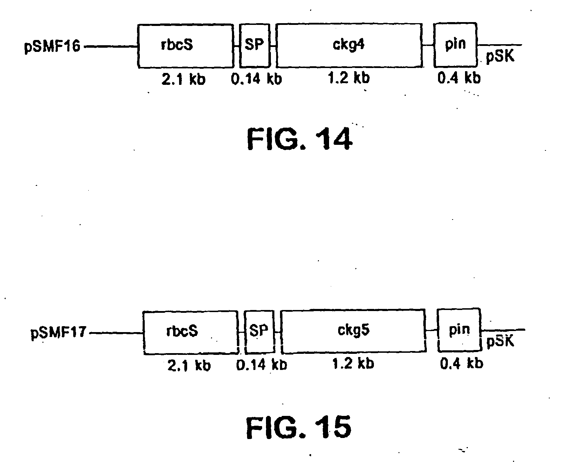

Construct 3, which is shown in Figure 14, is plasmid pMSF16 which is plasmid pSK which contains the rice rubisco rbcS leaf-specific promoter which limits expression of the ligninase encoded by ckg4 to the cells of the leaves of the maize plant and further contains DNA encoding the rbcS SP which targets the ligninase to the chloroplasts.

Construct 4, which is shown in Figure 15, is plasmid pSMF17 which is plasmid pSK which contains the rice rubisco rbcS leaf-specific promoter which limits expression of the ligninase encoded by ckg5 to the cells of the leaves of the maize plant and further contains DNA encoding the rbcS SP which targets the ligninase to the chloroplasts. The above heterologous gene expression cassettes can be flanked by MAR sequences.

The ligninase constructs shown in Table 4 are prepared as described below.

Two plasmids, pCLG4 and pCLG5, the former containing a cDNA clone encoding the ligninase gene ckg4 and the latter containing a cDNA clone encoding the ckg5 were obtained from Dr. C. Adinarayana Reddy, Department of Microbiology and Public Health, Michigan State University and described in de Boer et al., Gene 60: 93-102 (1987), Corrigendum in Gene 69: 369 (1988). These ligninase cDNA clones were prepared from a white-rot filamentous fungus (Phanerochaete chrysosporium). The cDNAs for ckg4 and ckg5 had each been cloned into the PstI site of the pUC9 plasmid to make pCLG4 and pCLG5, respectively. The codons for the 28-amino acid leader sequence is deleted from both cDNAs before cloning so that expressed gene product remains inside the cell.

Plasmid pSMF16 is made as follows. The ckg4 gene is removed from pCLG4 by digesting the plasmid with the restriction enzymes XbaI and BstEII to produce a 1.2 kb DNA fragment containing the ckg4 without the nucleotide sequence encoding the transit peptide. The BstEII removes the nucleotide sequences encoding the transit peptide of the ligninase.

The ends of the DNA fragment containing the ckg4 gene are made blunt and the blunt-ended DNA fragment is ligated into pSMF14 in which the cbh1 has been removed by digesting with BsrGI and XhoI and the ends made blunt to produce pSMF16.

Plasmid pSMF18 is made as follows. The nucleotide sequence encoding the rbcS signal peptide and cbh1 are removed from pSMF14 by digesting pSMF14 with AvrII and XhoI instead of BsrGI and XhoI. The ends of the digested pSMF14 are made blunt and the blunt-ended DNA fragment containing the ckg4 gene, prepared as above, is ligated into the digested pSMF14 to make plasmid pSMF18.

Plasmid pSMF17 is made as follows. The ckg5 gene is removed from pCLG5 by digesting the plasmid with the restriction enzymes XbaI and EagI to produce a 1.2 kb DNA fragment containing the ckg5 without the nucleotide sequence encoding the transit peptide. The EagI removes the nucleotide sequences encoding the transit peptide of the ligninase.

The ends of the DNA fragment containing the ckg5 are made blunt and the blunt-ended DNA fragment is ligated into pSMF14 in which the cbh1 has been removed by digesting with BsrGI and XhoI and the ends made blunt to produce pSMF17.

Plasmid pSMF19 is made as follows. The nucleotide sequence encoding the rbcS signal peptide and cbh1 are removed from pSMF14 by digesting pSMF14 with AvrII and XhoI instead of BsrGI and XhoI. The ends of the digested pSMF14 are made blunt and the blunt-ended DNA fragment containing the ckg5 gene, prepared as above ,is ligated into the digested pSMF14 to make plasmid pSMF19.

### EXAMPLE 5

This example shows the transformation of maize multi-meristem primordia via Biolistic bombardment with the plasmid constructs of Examples 1-4, regeneration of the transgenic plants, confirmation of the integration of the plasmid constructs into the plant genome, and confirmation of the expression of the cellulase or ligninase fusion proteins in the transgenic plant. For transformations with the constructs of Examples 2 and 4, which do not contain a selectable marker, a selectable marker comprising the bar gene in the plasmid pDM302 (Cao et al., Plant Cell Reports 11: 586-591 (1992)) is cotransfected into the cells with the plasmid containing the ligninase or cellulase heterologous gene expression cassette.

Maize seeds have been germinated in Murashige and Skoog (MS) medium (Murashige and Skoog, Physiol. Plant 15: 473-497 (1962)) supplemented with the appropriate growth regulators (Zhong et al., Planta 187: 490-497 (1992)). Shoot meristems have been dissected and cultured for 2-3 weeks until an initial multiplication of meristem have been produced for bombardment.

The multi-meristem primordia explants are bombarded with tungsten particles coated with particular plasmids of Example 1 or 3 or with particular plasmids of Example 2 or 4 along with the plasmid containing the heterogenous gene expression cassette containing the bar gene. The bombarded explants are gently transferred onto meristem multiplication medium for further multiplication, about 6 to 8 more weeks. This step is required to reduce the degree of chimerism in transformed shoots prior to their chemical selection. Shoots are transferred to the above medium containing 5 to 10 mg per liter glufosinate ammonium (PPT) selectable chemical for another 6 to 8 weeks. Chemically selected shoots are rooted in rooting medium containing the same concentration of PPT. Plantlets are transferred to pots, acclimated, and then transferred to a greenhouse.

When the plantlets or shoots are small, the quantity of transgenic plant material is insufficient for providing enough DNA for Southern blot hybridization; therefore, polymerase chain reaction (PCR) is used to confirm the presence of the plasmid constructs the plantlets. The amplified DNA produced by PCR is resolved by agarose or acrylamide gel electrophoresis, transferred to membranes according standard Southern transfer methods, and probed with the appropriate DNA construct or portion thereof according to standard Southern hybridization methods. Those shoots or plantlets which show they contain the construct in its proper form are considered to have been transformed. The transformed shoots or plantlets are grown in the greenhouse to produce sufficient plant material to confirm that the plasmid constructs has been properly integrated into the plant genome. To confirm proper integration of the plasmid constructs into the plant genome, genomic DNA is isolated from the greenhouse grown transgenic plants and untransformed controls and analyzed by standard Southern blotting methods as in Zhong et al., Plant Physiology 110: 1097-1107 (1996); Zhang et al., Theor. Appl. Genet. 92: 752-761 (1996); Zhang et al., Plant Science 116: 73-84 (1996); and, Jenes et al., In Transgenic Plants. Vol. 1. Kung, S-D and Wu, R (eds.). Academic Press, San Diego, CA. pp- 125-146 (1992).

To confirm expression of the ligninase or cellulase fusion protein, total cellular RNA is isolated from the greenhouse grown plant tissues as described in Zhong et al., Plant Physiology 110: 1097-1107 (1996). The mRNA encoding the cellulase or ligninase fusion protein is detected by RNA Northern blot analysis using the same probes used for the Southern blot analyses. Briefly, the RNA is electrophoresed on a denaturing formaldehyde agarose gel, transferred to nitrocellulose or nylon membranes, hybridized to the appropriate ligninase or cellulase probe, and then exposed to X-ray autoradiology film. The hybridization bands are scanned using a densitometer which enables determination of the expression level of the specific mRNA.

Translation of the mRNA is confirmed by Western blot analysis according to the standard methods of Towbin et al., Proc. Natl. Acad Sci. USA 76: 4350 (1979) and Burnette, Anal. Biochem. 112: 195 (1981) using antibodies specific for ligninase or cellulase.

### EXAMPLE 6

Transgenic maize containing both a ligninase and a cellulase fusion protein is made by crossing-breeding the abovementioned transgenic plants one of which contains cbh1 or e1 stably integrated into the genome and the other of which contains ckg4 or ckg5 stably integrated into the genome using the method provided in (Zhang et al, Theor. Appl. Genet. 92: 752-761, (1996); Zhong et al, Plant Physiol. 110: 1097-1107, (1996); Zhong et al, Planta, 187: 483-489, (1992)). Transgenic plants that carry a low copy number of the DNA encoding the ligninase or cellulase fusion proteins are used for cross-breeding.

Briefly, transgenic maize plants that produce the ligninase fusion protein are made as disclosed in Example 5 to make a first transgenic plant and transgenic maize plants that produce the cellulase fusion protein are made as disclosed in Example 5 to make a second transgenic plant. The first and second transgenic plants are cross-pollinated to create a transgenic plant which produces both a ligninase and a cellulase fusion protein. The progeny are analyzed for homozygosity and transgenic plants that are homozygous for both the ligninase gene cassette and the cellulase gene cassette are selected for further propagation for seeds.

The progeny in the above crosses are used in subsequent crosses to produce transgenic maize with both ligninase gene cassettes and one, two, or three cellulase gene cassettes or transgenic maize with two or three cellulase gene cassettes and one ligninase gene cassette.

### EXAMPLE 7

Production levels and activity of the cellulase fusion protein in transgenic maize made as in Example 5 or 6 is determined as follows.

Cellulase activity in transgenic maize is first assayed by standard methods (Ghose. In Analytical Method B-304, rev. A, IUPAC Commission on Biotechnology. A short Report (1984)) based on the time course assay for hydrolysis of a pre-weighed sample of filter paper at pH 4.8-5.2 and temperature of 50o C. While the filter paper assay is a standard substrate for cellulase activity, results using the filter paper assay are not particularly representative of the actual activity of the cellulase in plant materials containing cellulose, hemicellulose, and other sugars or sugar polymers. Therefore, a more accurate method for determining cellulase activity is used.

Plant material is ground and the ground material is suspended to a concentration of up to about 5% in 0.05 M citrate buffer at pH 4.8 and incubated with shaking at 50o C. Over a 48 hour time period, samples are removed at intervals of 0, 1, 3, 12, 24, and 48 hours. A minimal amount of sodium azide, about 0.05%, is added to the citrate buffer during incubation to control microbial activity. For analysis by high pressure liquid chromatography (HPLC), the supernatant fraction of each sample is removed, capped, and heated to inactivate the enzymes. The inactivated supernatant fraction is filtered through a syringe filter and analyzed by HPLC to measure the glucose, cellobiose, and xylose content of the samples according to established methods (Dale et al., Biosource Technol. 56: 11-116 (1996)).

Cellulase activity is manifested by an increasing level of glucose, xylose and/or cellobiose levels in the supernatant fractions during the 48 hour period. The control for the above assay is to treat samples from non-transgenic plants with varying amounts of commercially available cellulase enzymes such as CYTOLASE 300 which is a cellulase from Genencor, Inc. and NOVOZYME 188 which is a cellobiose from Novo Laboratories, Inc. to confirm that the ground plant material is susceptible to hydrolysis.

### EXAMPLE 8

Comparison of cellulase activity in transgenic maize prepared as in Example 5 or 6 treated to enhance cellulose accessibility.

Generally, cellulose and hemicellulose in plant material are not very accessible to hydrolytic enzymes such as cellulase. Therefore, it is possible that even if the cellulase fusion protein is produced in the transgenic plants of the present invention, its cellulase activity would not be measurable. Therefore, to demonstrate accessibility, samples of the transgenic maize plants of the present invention are treated by the ammonia fiber explosion process to increase cellulose and hemicellulose accessibility (Dale et al., Biosource technol. 56: 11-116 (1996)). Samples treated are analyzed as in Example 3.

In previous experiments with coastal Bermuda grass, the ammonia fiber explosion process disrupted the plant cell walls sufficiently to permit over 80% extraction of plant protein, compared with less than 30% extraction under the same conditions prior to ammonia treatment (de la Rosa et al., Appl. Biochem. Biotechnol. 45/46: 483-497 (1994). The process increased the hydrolytic effectiveness of the added cellulases by at least six-fold (Dale et al., Biosource Technol. 56: 11-116 (1996)). Thus, it is expected that the ammonia fiber explosion process helps release cellulase from the transgenic maize chloroplasts and will also increase the access of the cellulase released to the cellulose in the plant material.

### EXAMPLE 9

Production levels and activity of the ligninase fusion protein in transgenic maize made as in Example 5 or 6 can be determined as follows.

Maize leaves from the transgenic maize made as in Examples 5 or 6 are ground using a pestle and mortar. Chloroplasts are isolated from leaves of transgenic plants by Ficoll (Pharmacia) gradient centrifugation and ground as above.

The ground materials (leaves, grains, chloroplasts) are suspended in 50 mM L-tartrate buffer (pH 4.5), mixed well by vortexing, and centrifuged for 10 minutes at 14,000 rpm (16,000 x g) at 40 C and the supernatant fraction tested for lignin peroxidase (LIP) activity as described in Tien et al., Meth. Enzymol. 161: 238-249 (1988). The LIP assay measures the production of veratraldehyde (as an increase in absorbance at 310 nm) from veratryl alcohol (substrate) in the presence of hydrogen peroxide. Control assays are done on non-transgenic maize seeds to measure endogenous peroxidase activity. The assay is sensitive and is able to detect very low levels of lignin peroxidase activity, e.g., conversion of 0.1 mmole substrate per minute per liter of test sample.

Soluble protein content is determined by the Bradford procedure (Bradford, Anal. Biochem. 72: 248-254 (1976)) using bovine serum albumen (BSA) as the standard. LIP enzyme in the extracted fluid is purified by Fast Protein liquid Chromatography (FPLC) analysis using the Mono Q anion exchange system (Pharmacia) and a gradient of 0 to 1 M Na-acetate to elute the various isozymes (Yadav et al., Appl. Environ. Microbiol. 61: 2560-2565 (1995); Reddy et al., FEMS Microbiol. Rev. 13: 137-152 (1994)). The relative activity, yield, pH optimum, stability, and other characteristics of the LIP in the transgenic plant are compared to that determined for the LIP isolated from the fungus. Furthermore, ground maize seeds or leaf extracts containing the LIP is used to treat various lignocellulosic feeds in small laboratory reactor systems and the extent of delignification can be analyzed per established procedures (Van Soest et al., Assoc. Off. Anal. Chem. J. 51: 780-785 (1968)).

Detection of ligninase mRNA is by isolating the mRNA from the transgenic plants as above, resolving the mRNA by denaturing RNA gel electrophoresis, transferring the resolved mRNA to membranes, and probing the membranes with ckg4 or ckg5 cDNA probes.

Western blots are performed to determine whether the LIP protein is in an active or inactive form. The total protein from the transgenic plants is resolved by SDS-polyacrylamide gel electrophoresis and transferred to membranes. The membranes are probed with antibodies to LIP H2 (ckg4) or LIP H10 (ckg5).

### EXAMPLE 10

This Example illustrates the delay in flowering and increase in biomass of transgenic tobacco expressing the *Arabidopsis* floral repressor gene *Flowering Locus C.*

*Flowering Locus C* (*FLC*), a gene from *Arabidopsis thaliana* (L.) Heynh. that acts as a flowering represser, was expressed in tobacco (*Nicotiana tabacum* L. 'Samsun'). Five putative transgenic lines were selected and examined for the presence of *FLC.* Genomic DNA and total RNA were isolated from the leaves and used for polymerase chain reaction (PCR) and RNA blot analysis, respectively. Both DNA and RNA tests confirmed the integration and transcription of *FLC* in all five lines and their *T*₁ progenies. Transgenic plants in one line showed an average of 36 d delay in flowering time compared to control plants, and the overall mean for all lines was 14 d. Transgenic plants also displayed increased leaf size and biomass yield and reduced height at flowering time. It is important to note that the delay in flowering might have been caused by a slower rate of leaf initiation (i.e. nodes/day) rather than by a change in the flowering mechanism itself.

Flowering, the switch from vegetative to reproductive growth, is a key developmental change in the life cycle of the plant (Simpson and Dean, Science, 296: 285-289 (2002); Henderson and Dean, Development 131: 3829-3838 (2004)) and is controlled by both environmental and developmental signals (Jang et al., J. Plant Biotechnol, 5: 209-214 (2003)). The control of flowering and genes associated with the mechanism have recently been reviewed (Reeves and Coupland, Curr Opin Plant Biol 3: 37-42 (2000); Samach and Coupland, Bioassays, 22: 38-47 (2000); Araki, Curr Opin Plant Biol, 4: 63-68 (2001); Mouradov et al., Plant Cell, 14: 5111-5130 (2002); Simpson and Dean, ibid, 2002; Henderson and Dean, ibid, 2004). Because of its importance, flowering is the subject of intense studies, but it is still poorly understood at the molecular level partly due to the complexity of the flower initiation process (Koornneef et al., Genetics 148: 885-892 (1998)). Regardless, progress is regularly being made in this area. Most molecular and genetic studies on flowering have been carried out on the model plant, *Arabidopsis thaliana* (L.) Heynh.

The most current flowering model (Henderson and Dean, ibid, 2004) shows the involvement of at least eight distinct pathways regulating the change from vegetative growth to reproductive organ development. Although these pathways act largely independent of one another, some interaction does take place among them (Koornneef et al., ibid, 1998; Rouse et al., Plant J., 29: 183-191 (2002)). Flowering is promoted by the light quality, ambient temperature, gibberellin, circadian clock, and photoperiod pathways (Henderson and Dean, ibid, 2004). Acting antagonistically to these pathways is the floral repressor gene *FLOWERING LOCUS C* (*FLC*). Several genes act to promote *FLC* expression; however, *FLC* is down-regulated by vernalization (a long period of near-freezing temperatures) and the autonomous pathway genes (Michaels and Amasino, Plant Cell, 11: 949-956 (1999) and Michaels and Amasino, Plant Cell, 13: 935-941 (2001); Sheldon et al., Plant Cell, 11: 445-458 (1999) and Sheldon et al., Proc Natl Acad Sci USA 97: 3753-3758 (2000); Henderson and Dean, ibid, 2004). *FLC* encodes a MADS box transcription factor that is expressed mainly in vegetative shoot apices and roots (Michaels and Amasino, ibid, 1999). *FLC* works to inhibit flowering by suppressing a group of floral promotion genes termed 'floral pathway integrators' (Michaels and Amasino, .ibid, 1999 and Michaels and Amasino, ibid, 2001; Sheldon et al., ibid, 1999; Henderson and Dean, ibid, 2004). Plants over-expressing *FLC* experience an extended vegetative growth phase unless a vernalization requirement is met (Michaels-and Amasino, ibid, 1999).

Control of flowering time is essential for efficient seed production and for summer cultivation of biennial leafy crops (Jang et al., ibid, 2003). Since delay in flowering time results in prolonged vegetative growth, it theoretically may produce higher yields in crops grown for their leaves and/or biomass. Another key application for flowering delay is bioconfinement of transgenic pollen to avoid transfer of transgenes to cross-breedable non-target crops.

To test the delay in flowering and increase in biomass production, we used the model plant tobacco (*Nicotiana tabacum* L.). In this study, we describe *Agrobacterium*-mediated transformation of tobacco with constitutively expressed *FLC* and the molecular and physiological analyses of the transgenic plants.

Materials and methods

Plant materials: Tobacco (*Nicotiana tabacum* L. 'Samsun') seeds were prewashed in water with 0.2% Tween-20 for 10 min and rinsed three times with distilled water. The seeds were surface sterilized with 70% (*v*/*v*) ethanol for 1 min followed by immersion in 20% (*v*/*v*) Clorox (5.25% sodium hypochlorite) for 20 min and then rinsed three times with sterilized double-distilled water. Seeds were germinated on Murashige and Skoog, ibid, (1962) (MS) basal medium (Sigma-Aldrich, St. Louis, Mo) with 30 g/L sucrose, and solidified with 2.5 g/L gelrite (Sigma-Aldrich, St. Louis, Mo). Cultures were kept under 30 µmol/m²/s continuous white deluxe fluorescent light at 25 °C. Leaf segments (0.5×0.5 cm²) were aseptically excised from the second and third fully expanded in vitro produced leaves (Horsch et al., Science, 227: 1229-1231 (1985)) for infection with *Agrobacterium tumefaciens*.

***Agrobacterium* strain, plasmid, and in vitro culture:** The transformation experiments were conducted using *A*. *tumefaciens* strain GV 3101 (pMP90RK) (Koncz and Schell, Mol. Gen. Genet 204: 383-396 (1986)) containing the 3.232 kb binary vector pGreen (Hellens et al., Plant Mol. Biol., 42: 819-832 (2000a)). The plasmid contains *FLC* from *Arabidopsis thaliana* and the phosphoinothricin acetyltransferase gene (*bar*), both under the control of the constitutive cauliflower mosaic virus (CaMV) 35S promoter and the nopaline synthase (nos) terminator.

*Agrobacterium* containing the transgenes was grown in 10 mL YEP medium (containing 10 g/L Bactopeptone, 10 g/L Bacto yeast extract, 5 g/L NaCl, pH 7.2) supplemented with 25 mg/L of both kanamycin and gentamycin (Hellens et al., Trends Plant Sci, 5: 446-451 (2000b)), incubated at 28 °C and 250 rpm for 48 h, and the cultures (cell density 0.6-0.8 at A₆₀₀) were used for transformation.

**Sensitivity of tobacco leaves to glufosinate ammonium**. Since optimization of the herbicide concentration is a prerequisite for the selection efficiency of transformed lines, a kill curve was developed to test the sensitivity of tobacco leaf segments to glufosinate ammonium. Glufosinate ammonium was used as a selection agent since the binary vector used in this study contained *bar* gene. Twenty of the 3-week old tobacco leaf segments were placed on MS medium containing 0, 2.5, 5.0, 7.5, 10.0, 12.5 and 15.0 mg/L of glufosinate ammonium for 2 weeks. The explant survival was recorded. All explants turned brown and died after being cultured on glufosinate ammonium with concentrations of 5 mg/L or more. Therefore, 5 mg/L glufosinate ammonium was used for further transgenic plant selection.

**Inoculation and co-cultivation:** Leaf segments were infected using the *Agrobacterium* culture at room temperature for 20-25 min. After inoculation, the leaf explants were blotted on sterilized filter papers and placed abaxial side down on MS medium supplemented with 4.5 µmol/L *N*⁶-benzylamino purine (BAP) and 0.5 µmol/L α-naphthaleneacetic acid (NAA) (Ziegelhoffer T., Will, J., and Austin-Phillips, S. (1999). Expression of bacterial genes in transgenic alfalfa (Medicago sativa L.), potato (Solanum tuberosum L.) and tobacco (Nicotiana tabacum L.) Mol. Breed. 5: 309-318), 30 g/L sucrose and 2.5 g/L gelrite. They were co-cultivated for 2 d under continuous light as described above for seed culture. Then, they were rinsed three times with sterilized distilled water containing 400 mg/L carbencillin to prevent *Agrobacterium* overgrowth, blotted onto sterilized filter papers and placed adaxial side down on the same co-cultivation medium supplemented with 400 mg/L carbencillin and 5 mg/L glufosinate ammonium for selection of the putative transformants. The callus containing the adventitious shoots was subcultured in the same medium, and then shoots were excised and rooted on half strength MS medium containing 400 mg/L carbencillin and 5 mg/L glufosinate ammonium in Magenta boxes (Sigma-Aldrich, St. Louis, Mo). Plantlets were transferred to the greenhouse after acclimatization. Greenhouse conditions were 25-28 °C, 90-95% humidity and 190 µmol/m²/s light.

**Polymerase chain reaction (PCR) screening:** After selection on glufosinate ammonium-containing medium, PCR analysis was used to screen the *T₀* plants for *FLC* transgene incorporation. Five independent putative transgenic lines were selected for PCR analysis. Total genomic DNA of control and *T₀* plants were extracted from leaves as described by Edwards et al., Nucl. Acids Res, 19: 1349 (1991). The following set of primers was used: *FLC* F, 5'-CGA TAA CCT GGT CAA GAT CC-3' (forward primer, SEQ ID NO:25) and *FLC* R, 5'-CTG CTC CCA CAT GAT GAT TA-3' (reverse primer, SEQ ID NO:26). The predicted size of the amplified DNA fragments of the transgene was 338 bp. DNA amplifications were performed in a thermo cycler (Perkin Elmer/Applied Biosystem, Foster City, CA) using REDTaq™ ReadyMix™ PCR Reaction Mix with MgCl₂ (Sigma-Aldrich, St. Louis, MO). The PCR profile had an initial denaturation step at 94 °C for 1 min, followed by 30 cycles of 1 min at 94 °C (denaturation), 2 min at 60 °C (annealing) and 3 min at 72 °C (extension). The reaction mixture was loaded directly onto a 1.0% (*w*/*v*) agarose gel, stained with ethidium bromide and visualized with UV light.

**RNA gel blot analysis:** Total RNA of control, T₀ and *T*₁ plants from five putative lines was isolated from leaves of 6-weeks-old greenhouse plants using the TRI Reagent (Sigma-Aldrich, St. Louis, Mo) according to the manufacturer's instructions. Aliquots of RNA (20 µg) were fractionated in 1.2% agarose formaldehyde denaturing gel and blotted on a Hybond-N⁺ nylon membrane (Amersham Pharmatica Biotech) as specified by the manufacturer. The probe was generated by digesting plasmid DNA with *Xho*I and *Spe*I, releasing the 0.59-kb fragment containing the *FLC* coding region. The digestion reaction mixture was gel-purified using the QIAquick Gel Extraction Kit (QIAGEN Inc., Valencia, CA). Probe labeling and transcript detection were obtained using the DIG-High Prime DNA Labeling and Detection Starter Kit II (Kit for chemiluminescent detection with CSPD, Roche Co.) following the manufacturer's protocol.

**Flowering delay**, **biomass and yield studies:** Control and *T*₀ plants were compared regarding plant height, number of leaves produced before flowering, leaf area, days to flowering after transferring to the greenhouse, mean biomass fresh and dry weight, seed yield and thousand seed weight. The experimental design was a completely randomized design with four replications. Data were analyzed using MSTAT-C software (Freed and Eisensmith, MSTAT-C; A Softward Package for the Design, Management, and Analysis of Agronomic Experiments. East Lansing, MI: Michigan State University; (1989)). Means were separated using Tukey's test at the 1% level. *T*₁ plants were only compared for the delay in flowering.

**Segregation analysis:** *T*₁ generation seeds were obtained from self-pollination of the *T*₀ of the five putative transgenic lines. The segregation of the *T*₁ progeny was tested by culturing 40 seeds of each line on half-strength basal MS medium containing 5 mg/L glufosinate ammonium. Numbers of germinated and non-germinated seeds were recorded after 2 weeks. The chi square (*χ*²) test at *P*=0.01 was performed to determine if the observed segregation was consistent with a Mendelian ratio.

**Identification of transgenic *FLC* tobacco plants:** *Polymerase chain reaction*: In addition to the plasmid, a band of the expected size of 338 bp revealed five independent transformation events. No band was detected in the untransformed control (data not shown).

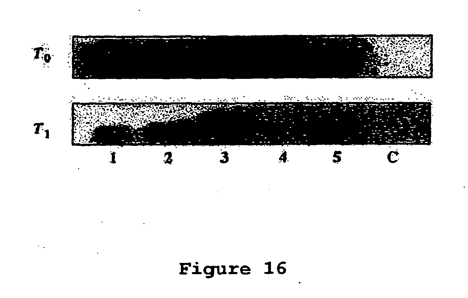

*RNA gel blot analysis*: The levels of *FLC* mRNA transcripts in all the transgenic lines for *T*₀ were high but varied in *T*₁ (Figure 16). The results also showed the lack of detectable transcript for *FLC* in the control plants. Figure 16 is an illustration of the RNA gel blot analysis of FLC in T0 and T1 tobacco plants. Lanes 1-5 are transgenic lines; Lane C is a negative control.

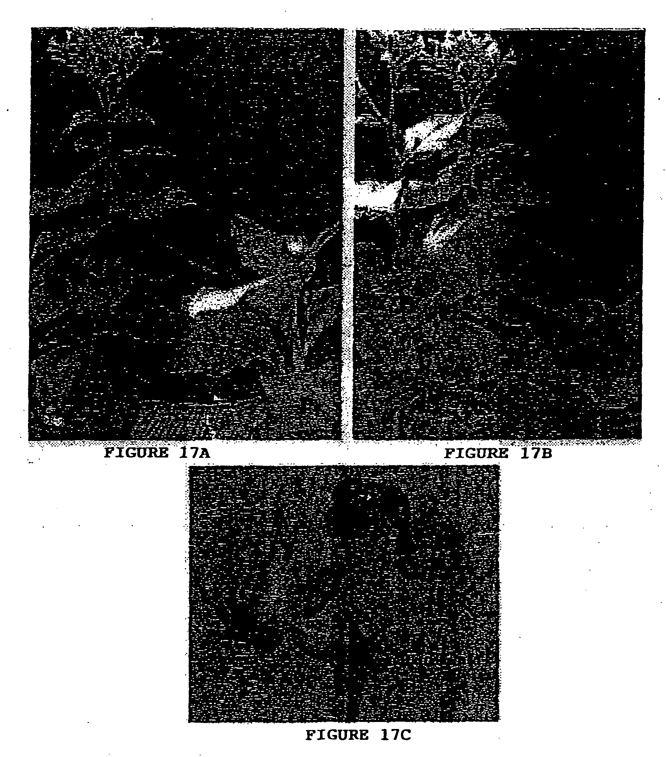

**Flowering delay, biomass and yield studies:** Analysis of flowering time of the *T*₀ transgenic lines and the control yielded informative results about the functionality of *FLC* in flowering delay. All transformants produced visible flowers later than the control plants with an average of 7 (Line 1) to 36 (Line 4) d with an overall mean of 14 d for all lines (Table 5 and Figure 17A). This observation was confirmed by anthesis time, as the mature anthers of the control shed pollen though the *T*₀ lines were still immature (Figure 17) . While there was no significant difference between the five lines and the control regarding number of leaves produced before flowering (Table 5, Figure 17B), there was significant difference between four lines (lines 1, 3, 4 and 5) and the control regarding plant height at flowering time (Table 5). All the transgenic lines produced leaves larger than the control plants (Table 5). Preliminary experiments with *T*₁ plants showed more than 4 weeks flowering delay in Line 4. However, plants in the other transgenic lines began flowering about 2 weeks before control.

**Table 5. Comparison between control and T₀ tobacco plants with regard to flowering delay and vegetative growth before flowering.**

| **Plants** | **Days to flowering after transfer to greenhouse** | **Flowering delay (d)** | **No. of leaves produced before flowering** | **Leaf area^{a} (cm²)** | **Plant height at flowering time (cm)** |
|---|---|---|---|---|---|
| Control | 15 c^{b} | 0 c | 19 ab | 187. 5 d | 60 a |
| *T*₀: | | | | | |
| Line 1 | 22 b | 7 b | 18 ab | 213. 8 c | 35 b |
| Line 2 | 23 b | 8 b | 20 a | 236. 5 bc | 65 a |
| Line 3 | 24 b | 9 b | 21 a | 239. 5 b | 35 b |
| Line 4 | 51 a | 36 a | 16 b | 369. 7 a | 22 c |
| Line 5 | 23 b | 8 b | 20 a | 219. 9 bc | 40 b |
| Overall mean of five lines | 29 | 14 | 19 | 256 | 39 |

| | | | | | |
|---|---|---|---|---|---|
| ^{a} Measured with second fully expanded leaf from the bottom. ^{b} In each column; means followed by the same letters are not significantly different using Tukey's test at 1% level. | | | | | |

Figure 17. *FLC* transgenic tobacco plants delayed flowering 2 or more weeks. Figure 17A: Right plant is *FLC* transgenic and left plant is untransformed control. Figure 17B: Plants from Line 4 (right) compared to control plants (left). Figure 17C: *FLC* transgenic versus control flowers from the same age. (A=anther, S=stigma). Note the pollen grains on control anthers and stigma.

The phenotype of Line 4 showed the most extreme flowering delay (approximately 5 weeks). Also, due to its short internodes, this line showed a statistically significant short stem length before flowering. Moreover, this line produced the largest leaves among all the other lines and the control (Table 5). There was a significant difference between biomass fresh weight of three lines (lines 2, 4 and 5) and the control but only Line 4 had more biomass dry weight compared to the control (Table 6). With exception of Line 2, all other lines produced lower seed yield compared to the control but the thousand seed weight of all transgenic lines was significantly more than the control plants (Table 6). Line 4 produced the lowest seed yield but the seeds were bigger and heavier (Table 6).

**Table 6. Differences between control and T₀ tobacco plants in biomass, thousand seed weight and seed yield per plant**

| **Plants** | **Biomass FW/plant (g)** | **Biomass DW/plant (g)** | **Thousand seed weight (mg)** | **Seed yield/ plant (g)** |
|---|---|---|---|---|
| [control | 192.31 c^{a} | 35.22 bc | 66 c | 6.19 b |
| *T*₀: | | | | |
| Line 1 | 233.33 c | 33.47 c | 76 b | 3.18 d |
| Line 2 | 338.52 ab | 50.48 ab | 83 b | 7.12 a |
| Line 3 | 260.81 bc | 35.58 c | 81 b | 5.31 c |
| Line 4 | 335.03 ab | 51. 65 a | 101 a | 0.17 e |
| Line 5 | 346.06 a | 45.79 abc | 81 b | 3.36 d |
| Overall mean of five lines | 377 | 43 | 84 | 4 |

| | | | | |
|---|---|---|---|---|
| ^{a} In each column, means followed by the same letters are not significantly different using Tukey's test at 1% level. | | | | |

Expression of bar gene in the *T*₁ progeny: A segregation ratio of 3:1 was obtained from all the transgenic lines (data not shown). None of the non-transformed seeds germinated on the selection medium.

### Discussion

*FLC* acts to prevent premature flowering in *Arabidopsis* (Koornneef et al., Plant J., 6: 911-919 (1999); Lee et al., Plant J., 6: 903-909 (1999); Michaels and Amasino, ibid, 1999; Sheldon et al., ibid, 1999), and was shown to have a similar function when expressed in rice (Tadege et al., Plant Biotechnol J., 1: 361-369 (2003)) and in *Brassica napus* (Tadege et al., Plant J., 28: 545-553 (2001)), and here, when expressed in tobacco. In *Arabidopsis, FLC* delays flowering by suppressing the 'floral pathway integrators,' including *SUPRESSOR OF OVEREXPRESSION OF CO 1* (*SOC1*) (Henderson and Dean, ibid, 2004; Michaels and Amasino, ibid, 1999 and Michaels and Amasino, ibid, 2001; Sheldon et al., ibid, 1999). Similarly, when expressed in rice, *FLC* was shown to delay flowering by down-regulating rice *SOC1* (Tadege et al., ibid, 2003), causing a similar flowering time effect. In addition, *FLC* caused reduced fertility and even sterility due to lack of pollen shed in transgenic rice, suggesting that expression of *FLC* could interfere with other elements of reproductive development (Tadege et al., ibid, 2003). Interestingly, the extended vegetative phase observed when *FLC* was expressed constitutively in tobacco seems to be caused by a slower rate of leaf initiation (i.e. nodes/day) rather than by a change in the flowering mechanism itself. Transgenic leaves and floral organs were significantly larger than control. It has been suggested that in this case, *FLC* causes the misdistribution of apical cells into leaf anlagen instead of internodes (S. van Nocker, personal communication). One line (Line 4) had significantly reduced internode length compared with other lines and the control, creating an almost rosette-like phenotype. This is interesting, as *FLC* comes from *Arabidopsis*, a rosette plant, while tobacco is not. Perhaps in tobacco, *FLC* also plays a role in determining internode length, in addition to its role in distribution of apical cells. This would indicate that the function of *FLC* differs depending on the species it is introduced to.

Delay in flowering time is relative to the level of *FLC* activity in *Arabidopsis* and rice: when *FLC* was introduced under the control of the CaMV 35S promoter in *Arabidopsis* and under the maize ubiquitin promoter in rice, a wide range of flowering times was observed (Michaels and Amasino, ibid, 1999; Sheldon et al., ibid, 1999; Tadege et al., ibid, 2003), indicating that transgene copy number and/or position effects could greatly affect flowering time (Michaels and Amasino, ibid, 1999). In rice it was shown that the higher the expression of *FLC,* the greater the delay in flowering and the more severe the floral defects and infertility (Tadege et al., ibid, 2003). In the present work, the delay in flowering time of To plants was 7-49 d, with an overall mean of 14 d. This is more or less consistent with the previous results in *Arabidopsis* and rice. One line (Line 4) showed up to 49 d (with mean of 36 d) flowering delay.

In the To, all the transgenic lines showed a similar level of *FLC* mRNA expression, with Line 4 showing a slightly higher level. However, our results in the *T*₁ seem to suggest an opposite relationship: the lower the level of *FLC* transcripts, the greater the delay in flowering. This is especially true for Line 4 which has the most severe phenotypic effect among all transgenic lines, yet the lowest amount of *FLC* transcripts in the *T*₁ generation. Further experiments must be conducted to determine the reasons behind these phenomena in second and further generations.

The delay in flowering resulted in higher biomass yield in all the transgenic lines that was significantly different from control. This higher biomass yield along with production of larger leaves would be useful for efficient production of tobacco, leafy vegetables, and biomass crops such as switchgrass and maize.

Comparing stages of flower development at anthesis of plants that were of the same age, control flowers were completely mature and shed pollen while *T*₀ plants were still immature (Figure 17C).

This study shows that expression of *FLC* in biomass crops may have the desired effect of extending the vegetative stage, with the added benefit of larger leaves and fresh weight biomass. A major problem in maize and other transgenic open-pollinated crops is concern about the movement of transgenes via pollen flow (Riegel et al., Science, 296: 2386-2388 (2002)). Therefore, delay in flowering may not only increase biomass production, but reduce the likelihood of unintended cross-pollination between genetically modified and native cross-breedable plants in the field as their flowering times would be less likely to coincide.

### EXAMPLE 11

This Example relates to Expression of *Flowering Locus C* in an *Acidothermus cellulolyticus* E1 endo-1,4-β-Glucanase-Producing Transgenic Tobacco *(Nicotiana tabacum* L.) and its Effect on Delay in Flowering and Increase in Biomass.

With a hypothesis that delay in flowering increases the biomass of an industrial enzyme-producing transgenic plant, the T₄ generation of an *Acidothermus cellulolyticus* E1 endo-1,4-β-glucanase-producing transgenic tobacco *(Nicotiana tabacum* L.) was used for *Agrobacterium-mediated* transformation and expression of *FLOWERING LOCUS C* (*FLC*), a gene from *Arabidopsis thaliana* that acts as a flowering repressor. *Agrobacterium* strain GV 3101 containing the *FLC* and *bar* selectable marker cassettes was employed for transformation. Six putative transgenic lines, resistant to 5 mg l⁻¹ glufosinate ammonium, were selected for molecular analyses. Genomic DNA and total RNA were isolated from leaves of 6 week old greenhouse plants and used for polymerase chain reaction (PCR) and RNA-blot analysis. The DNA and RNA tests respectively confirmed the integration and transcription of *FLC* in all six *E1cd-FLC* lines. Transgenic *E1cd-FLC* plants showed an average of 9 to 21 d delay in flowering time compared to Elcd-expressing control plants, and the overall mean for all lines was 14 d. *E1cd-FLC* transgenic plants also displayed significant increases in leaf size and biomass yield. All transgenic *E1cd-FLC* plants were significantly shorter than control at flowering time. The enzymatic activity of E1cd in the *Elcd-FLC* expressing plants was similar to the E1cd enzymatic activity in *E1cd* transgenic control plants. Delay in flowering of transgenic plants could be a useful bioconfinement system to avoid or reduce the chance of cross contamination of transgenic pollen with cross-breedable plants in the field, and the increase in biomass could be a useful trait to increase production of biomass per plant.

Air pollution and global warming as a result of burning fossil fuels, and the high costs of fossil fuel have compelled researchers to develop alternative energy sources such as lignocellulosic biomass (Ziegelhoffer et al., ibid, 2001). Cellulases are a class of enzymes with great potential for bioconversion of lignocellulosic biomass to ethanol and other important industrial chemicals (Wright, Energy Prog. 8:71-78 (1988); Lynd et al., Science, 251: 1318-1323 (1991); Halliwell and Halliwell, Outlook Agric. 24: 219-225 (1995)). However, the high cost of cellulase enzyme production in bacterial fermentation tanks is a barrier to the utilization of these enzymes at commercial level (Ziegelhoffer et al., ibid, 1999). Technology to produce hydrolysis enzymes in transgenic crops may become very valuable in reducing these costs (Teymouri et al., Appl. Biochem. Biotechnol. 116: 1183-1192 (2004)). To test whether plants could produce biologically active microbial cellulases, *Arabidopsis thaliana* (Ziegler et al., Mol. Breed. 6: 37-46 (2000)), tobacco, alfalfa and potato (Ziegelhoffer et al., ibid, 1999) have been genetically engineered with microbial cellulase gene. Also, cellulase-producing transgenic tobacco has been used to test the stability of activity of the herterologues cellulase in plant material after Ammonia Fiber Explosion (AFEX) pretreatment (Teymouri et al., ibid, 2004).

It is well understood that the biomass production decreases after transition from vegetative plant growth (i.e. production of leaves) to a reproductive stage (i.e. production of flowers). Therefore, if the onset of flowering could be delayed, this is assumed to give the plant a longer vegetative growth period resulting in a higher biomass.

Flowering, the transition from the vegetative to the reproductive stage, is a key developmental change in the life cycle of the plant (Simpson and Dean, ibid, 2002; Henderson and Dean, ibid, 2004) and is controlled by both environmental and developmental signals (Jang et al., ibid, 2003). Flowering has been the subject of many studies. However, it is still not well understood at the molecular level due to the complexity of the flower initiation phenomena (Koornneef et al., ibid, 1998).

Henderson and Dean, ibid, (2004) recently presented a model that shows the current understanding of flowering in *Arabidopsis*. This model represented the participation of at least eight distinct pathways regulating the transition from vegetative growth to reproductive organ development. While these pathways perform largely independent of one another, certain interaction takes place among them (Koornneef et al., ibid, 1998; Rouse et al., ibid, 2002). Several factors, including the light quality, ambient temperature, gibberellin, circadian clock, and photoperiod pathways may promote flowering (Henderson and Dean, ibid, 2004). Acting against these pathways is the floral repressor gene *FLOWERING LOCUS C* (*FLC*). A number of genes act to promote *FLC* expression. It has been shown that *FLC* is down-regulated by vernalization (i.e. long exposure to near-freezing temperatures) and the autonomous pathway genes (Michaels and Amasino, ibid, 1999; Sheldon et al., ibid, 1999; Sheldon et al., ibid, 2000; Michaels and Amasino, ibid, 2001; Henderson and Dean, ibid, 2004). Here we report an *Agrobacterium*-mediated transformation of *E1cd* transgenic tobacco (Ziegelhoffer et al., Mol. Breed. 8: 147-158 (2001)), a cellulase-producing bioreactor plant, with a constitutively regulated *FLC*, and the molecular and physiological analyses of the transgenic plants.

### MATERIALS AND METHODS

*Plant materials.* Seeds of T₃ transgenic tobacco *(Nicotiana tabacum* L.) plants expressing *E1cd* (catalytic domain fragment of E1 endo-1, 4-β-glucanase from *Acidothermus cellulolyticus*) we used from our previous research (Teymouri et at., ibid, 2004). Initially, the T1 seeds were obtained from Dr. Sandra Austin-Phillips of the University of Wisconsin. In their *E1cd* transformation research, the team used the pZA9 containing *E1cd* regulated by the CaMV 35S (Cauliflower Mosaic Virus 35S) promoter, the apoplast-targeting leader VSPβ of soybean, and nopaline synthase terminator (Nos); and used *nptII* as the selectable marker gene (Ziegelhoffer et al., ibid, 2001).

Seeds of T₃ plants were prewashed in water with 0.2% Tween-20 for 10 min and rinsed three times with distilled water. The seeds were surface sterilized with 70% (v/v) ethanol for 1 min, immersed in 20% (v/v) Clorox (5.25% sodium hypochlorite) for 20 min and then rinsed three times with sterilized double distilled water. Seeds were germinated on Murashige and Skoog (1962) (MS) basal medium (Sigma-Aldrich, St. Louis, MO) containing 30 g 1⁻¹. sucrose and 2.5 g 1⁻¹ gelrite (Sigma-Aldrich, St. Louis, MO). Cultures were kept under 30 µmol m⁻² s⁻¹ continuous white deluxe fluorescent light at 25 °C. Leaf segments (0.5 cm × 0.5 cm squares) were aseptically excised from the second and third fully expanded in vitro produced leaves for infection with *Agrobacterium tumefaciens* (Horsch et al., ibid, 1985).

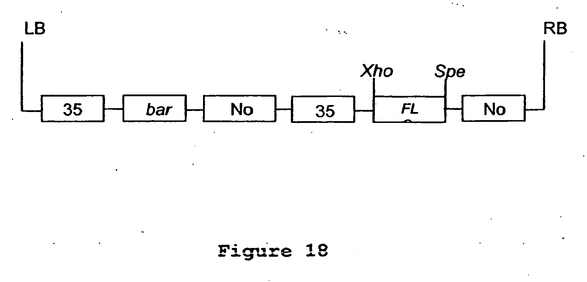

*Agrobacterium strain and plasmid. Agrobacterium tumefaciens* strain GV 3101 (pMP90RK) (Koncz and Schell, ibid, 1986) containing the 3.232 kb binary vector pGreen (Hellens et al., ibid, 2000a) was employed for transformation experiments. The plasmid contains *FLC* from *Arabidopsis thaliana* and phosphoinothricin acetyltransferase gene (*bar*), both under the control of the cauliflower mosaic virus (CaMV) 35S promoter and the nopaline synthase (hos) terminator (Figure 18).

The *Agrobacterium* containing the transgenes was grown in 10 ml YEP medium (containing 10 g 1⁻¹ Bactopeptone, 10 g 1⁻¹ Bacto yeast extract, 5 g 1⁻¹ NaCl, pH 7.2) supplemented with 25 g 1⁻¹ of both kanamycin and gentamycin (Hellens et al., ibid, 2000b), incubated at 28 °C and 250 rpm for 48 h, and the cultures (cell density 0.6-0.8 at A₆₀₀) were used for transformation.

*Agrobacterium-mediated transformation.* Leaf segments were infected using the *Agrobacterium* culture at room temperature for 25 min. Then, the leaf explants were blotted on sterilized filter papers and placed upside down on MS medium supplemented with 4.5 µM N⁶-benzylamino purine (BAP) and 0.5 µM α-naphthaleneacetic acid (NAA). (Ziegelhoffer et al., ibid, 1999), 30 g 1⁻¹ sucrose and 2.5 g 1⁻¹ gelrite (co-cultivation medium). The leaf segments were kept in co-cultivation medium for two days under continuous light as described above for seed culture. Then, they were rinsed three times with sterilized distilled water containing 400 mg 1⁻¹ carbencillin, blotted onto sterilized filter papers and placed on the same co-cultivation medium supplemented with 400 mg l⁻¹ carbencillin and 5 mg l⁻¹ glufosinate ammonium for selection of the putative transformants. The produced calli were subcultured in the same medium, and then shoots were excised and rooted on half- strength MS medium containing 400 mg l⁻¹ carbencillin and 5 mg l⁻¹ glufosinate ammonium in Magenta boxes (Sigma-Aldrich, St. Louis, MO). Well-rooted plantlets were transferred to the greenhouse after acclimatization. Greenhouse conditions were 25 to 28 °C, 90-95% relative humidity and 190 µmol m⁻² s⁻¹ light.

*Polymerase chain reaction (PCR) analysis.* After selection on glufosinate ammonium-containing medium, PCR analysis was used to screen the transgenic plants for *FLC* and *E1cd* transgene incorporation. Six independent transgenic lines were selected for PCR screening. Total genomic DNA of control and transgenic plants were extracted from leaves as described by Edwards et al. (1991). The following set of primers were used: *FLC* F, 5'-CGA TAA CCT GGT CAA GAT CC-3' (forward primer, SEQ ID NO:25) and *FLC* R, 5'-CTG CTC CCA CAT GAT GAT TA-3' (reverse primer, SEQ ID NO:26), and *E1cd* F, 5'-GCG GGC GGC GGC TAT TG-3' (forward primer, SEQ ID NO:27) and *E1cd* R, 5'-GCC GAC AGG ATC GAA AAT CG-3' (reverse primer, SEQ ID NO:28). The predicted size of the amplified DNA fragments of the transgene was 338 bp for *FLC,* and 1.07 kb for *E1cd.* DNA amplifications were performed in a thermo cycler. (Perkin Elmer/Applied Biosystem, Foster City, CA) using REDTaq^{™} ReadyMix^{™} PCR Reaction Mix with MgCl₂ (Sigma-Aldrich, St. Louis, MO). The PCR profile had an initial denaturation step at 94 °C for 1 min, followed by 30 cycles of 1 min at 94 °C (denaturation), 2 min at 60 °C (annealing) and 3 min at 72 °C (extension). The reaction mixture was loaded directly onto a 1.0 % (w/v) agarose gel, stained with ethidium bromide and visualized with UV light.

*RNA-blot analysis.* Total RNA of control plants and PCR-positive transgenic plants for both *FLC* and *E1cd* from six putative transgenic lines was isolated from leaves of six-week-old greenhouse plants using the TRI Reagent (Sigma-Aldrich, St. Louis, MO) according to the manufacturer's instructions. Twenty micrograms of RNA were fractionated in 1.2 % agarose formaldehyde denaturing gel and blotted onto a Hybond-N⁺ nylon membrane (Amersham Pharmatica Biotech.) as specified by the manufacturer. The probe was generated by digesting plasmid DNA with *Xho*I and SpeI, releasing the 0.59-kb fragment containing the *FLC* coding region. The digestion reaction mixture was gelpurified using the QIAquick Gel Extraction Kit (QIAGEN Inc., Valencia, CA). Probe labeling and transcript detection were obtained using the DIG-High Prime DNA Labeling and Detection Starter Kit II (Kit for chemiluminescent detection with CSPD, Roche Co.) following the manufacturer's protocol.

*Flowering delay*, *biomass and yield studies.* Control and *E1cd-FLC* transgenic plants were compared concerning plant height, number of leaves produced before flowering, leaf area, days to flowering after transferring to the greenhouse, mean biomass fresh and dry weight, seed yield and thousand seed weight. The experimental design was a completely randomized design (CRD) with four replications. Data were analyzed using MSTAT-C software (Freed and Eisensmith, ibid, 1989) and means were separated using Tukey's test at the 1 or 5% level.

*Segregation analysis.* Segregation analysis was conducted using the T₁ generation seeds of the *E1cd-FLC* self-pollinated plants of the six putative transgenic lines. Forty seeds of each line were cultured on half-strength basal MS medium containing 5 mg 1⁻¹ glufosinate ammonium. Numbers of germinated and non-germinated seeds were recorded after 2 weeks. The chi square (χ²) test at *P*= 0.01 was performed to determine if the observed segregation was consistent with a Mendelian ratio.

*E1cd enzymatic activity of E1cd-FLC transgenic plants*. Samples of *E1cd-FLC* and control untransformed plants were assayed for E1 activity as described (Ziegelhoffer et al., ibid, 2001; Teymouri et al., ibid, 2004). Briefly, a standard curve was generated using 4 to 160 pmol 4-methylumbelliferone (MU), the product of E1 hydrolization of the substrate 4-methylumbelliferone β-D-cellobioside (MUC). Total soluble protein was isolated from 100 mg fresh leaf tissue using the sodium acetate grinding buffer and ammonium sulfate precipitation described in Ziegelhoffer et al., ibid, (1999) and quantified by using the BioRad (Hercules, CA) Protein Dye Reagent, measuring the absorbance at 595 nm and comparing the value to the standard curve as specified by the manufacturer. A series of soluble protein dilutions ranging from 10⁻¹ to 10⁻³ were made. In a 96-well plate, 1 to 4 µl sample were mixed with 100 µl reaction buffer containing MUC. Plates were covered with adhesive lids and incubated at 65 °C for 30 minutes. The reaction was stopped and the fluorescence was read at 465 nm using SPECTRAmax M2 device (Molecular Devices Inc., Sunnyvale, CA) at an excitation wavelength of 360 nm. After subtracting background fluorescence contributed by the control, activity of samples was calculated using the standard curve and compared to the activity of pure E1 reported in Ziegelhoffer et al., ibid, (1999). Briefly, the nmol MU (from the standard curve) was divided by 30 minutes to calculate nmol MU/min; this number was divided by the µg total protein in the well to calculate the activity.

*Vernalization studies.* To test the effect of vernalization on delay in flowering, seeds of control untransformed tobacco plants and seeds of E1cd-FLC tobacco were allowed to germinate on wetted filter papers in petri dishes. Petri dishes were kept in the dark at 4 °C (14) for 30 d. Then, the seedlings were planted in the soil and transferred to the greenhouse, where they were grown until flowering.

### Results and discussion

*Polymerase chain reaction*. PCR analysis showed the integration of both *FLC* and *E1cd* in all the lines. The *E1cd* transgenic control plants showed only- presence of *E1cd* (data not shown).

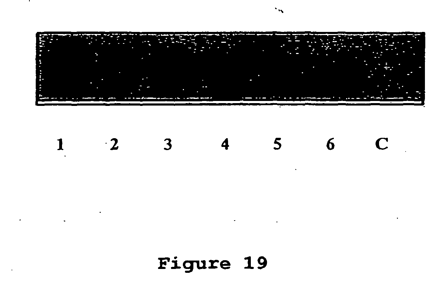

*RNA-blot analysis*. All the transgenic lines showed high levels of *FLC* mRNA transcripts (Figure 19). No band was detected for control plants. Based on PCR and RNA-blot analyses, transformation and selection efficiency can be estimated as 100%.

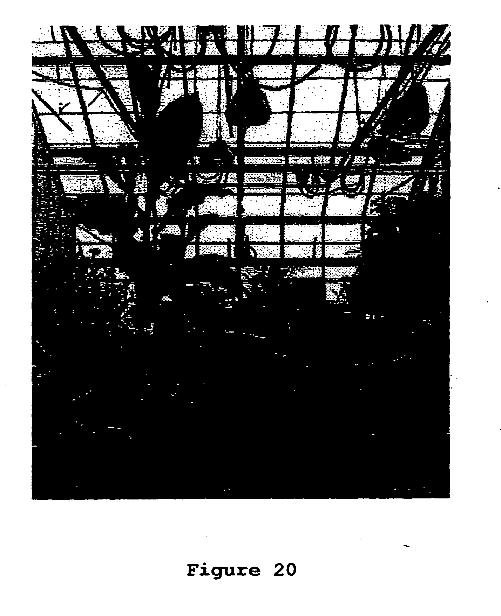

*Flowering delay, biomass and yield studies.* Control plants flowered 23 days after transfer to the greenhouse (Table 7). Transgenic plants showed 32 to 44 days of vegetative growth before to switching to the reproductive stage, after transfer to the greenhouse (Table 7). Therefore, transgenic plants showed a delay in flowering of 9 to 21 days, with a mean of 15 days greater control plants (Table 7). This is more or less consistent with the previous results in *Arabidopsis* (Koornneef et al., ibid, 1994; Lee et al., ibid, 1994; Michaels and Amasino, ibid, 1999; Sheldon et al., ibid, 1999), *Brassica napus* (Tadege et al., The Plant Journal, 28: 548-553 (2001)) rice (Tadege et al., ibid, 2003) and our previous study on tobacco (Salehi et al., J. Plant Physiol., 162: 711-717 (2005)). Lines 1 and 4 showed the greatest delays in flowering, 18 to 25 days, with a mean of 21 days, and 17 to 26 days, with a mean of 20 days, respectively. *FLC* is known to prevent premature flowering in *Arabidopsis* (Koornneef et al., ibid, 1994; Lee et al., ibid, 1994; Michaels and Amasino, ibid, 1999; Sheldon et al., ibid, 1999), *Brassica napus* (Tadege et al., ibid, 2001) rice (Tadege et al., ibid, 2003) and tobacco (Salehi et al., ibid, 2005). In our experiment, all the transgenic lines were shorter than control at flowering time, with no significant difference in nodes or leaf number (Table 7, Figure 20). Practically speaking, the shorter stem should be one the advantages of biomass plants considering normal lodging or stem breakage in the field. Lines 1 and 4 produced leaves significantly larger than control and other lines (Table 7, Figure 20).

**Table 7: Comparison between control and E1cd-FLC transgenic tobacco plants WITH REGARD TO flowering delay and vegetative growth before flowering.**

| Plants | Days to flowering after transferring to the greenhouse | Flowering delay (d) | No. of leaves produced before flowering | Leaf area* (cm²) | Plant height at flowering time (cm) |
|---|---|---|---|---|---|
| Control | 23 | b 0b | 20 a | 333.5 c | 113.3 a |
| Transgenic : | | | | | |
| Line 1 | 44 a | 21 a | 19 a | 518.0 a | 50.0 b |
| Line 2 | 39 a | 16 a | 20 a | 218.5 e | 56.8 b |
| Line 3 | 32 ab | 9 ab | 19 a | 345.8 c | 45.5 b |
| Line 4 | 43 a | 20 a | 19 a | 477.5 b | 47.0 b |
| Line 5 | 38 b | 15 a | 21 a | 239.5 de | 48.0 b |
| Line 6 | 35 ab | 12 ab | 20 a | 253.0 d | 54.5 b |
| Overall mean of six lines | 38 | 15 | 20 | 342 | 50 |

| | | | | | |
|---|---|---|---|---|---|
| * Measured with second fully expanded leaf from the bottom. In each column, means followed by the same letters are not significantly different using Tukey's test at *P*≤0.01. | | | | | |

Lines 1 and 4 had significantly more biomass fresh weight than all four other lines (*P*≤0.01) and the control (*P*≤0.05) (Table 8). Biomass dry weight was more or less the same in control and all the transgenic lines (Table 8). The higher fresh biomass yield along with production of larger leaves might be useful for more cellulase production from *E1cd-FLC* tobacco plants.

**Table 8: Differences between control and E1cd-FLC transgenic tobacco plants in biomass, thousand seed weight and seed yield per plant**

| Plants | Biomass FW/plant (g) | Biomass DW/plant | Thousand seed weight (mg) | Seed yield/plant |
|---|---|---|---|---|
| Control | 187.0 abc | 32.75 a | 658 c | 5.51 abc |
| Transgenic: | | | | |
| Line 1 | 275.3 ab* | 29.25 ab | 701 bc | 4.90 bcd |
| Line 2 | 181.8 bc | 30.50 ab | 740 ab | 3.87 cd |
| Line 3 | 164.3 c | 21.00 b | 792 a | 3.75 d |
| Line 4 | 291.3 a* | 35.50 a | 745 ab | 6.89 a |
| Line 5 | 159.8 c | 19.25 b | 726 b | 4.01 cd |
| Line 6 | 179.5 bc | 27.00 ab | 588 d | 6.30 ab |
| Overall mean of six lines | 209 | 27 | 715 | 5 |

| | | | | |
|---|---|---|---|---|
| In each column, means followed by the same letters are not significantly different using Tukey's test at *P*≤0.01. *Significantly different from control using Tukey's test at *P*≤0.05. | | | | |

Except line 3, seed yield per plant was not significantly different between control and transgenic plants. Also, except line 6, the thousand seed weight of all transgenic lines was significantly more than the control plants (Table 8). Line 3 produced the lowest seed yield but the seeds were larger and heavier (Table 8). In transgenic rice, *FLC* caused reduced fertility and even sterility, suggesting that expression of *FLC* could get in the way with other elements of reproductive development (Tadege et al., ibid, 2003). In transgenic rice, FLC caused reduced fertility and even sterility, suggesting that expression of FLC could get in the way with other elements of reproductive developments (Tadege et al., ibid, 2003), that we believe has been due to the transgene position effect rather than the transgene physiological effect. In our case, except line 3, FLC did not reduce the fertility, and transgenic plants produced seed weight the same amount as the control plants (Table 8).

*Expression of bar gene in the T₁ progeny*. Seeds of all the *E1cd-FLC* transgenic lines were germinated on selection medium with a segregation ratio of 3:1 (Table 9). None of the control non-transformed seeds germinated on the selection medium.

**Table 9: Segregation of glufosinate ammonium resistance (germinated vs. non-germinated seeds) in E1cd-FLC T₁ progeny**

| Lines | Number of germinated seeds | Number of non-germinated seeds | Expected ratio | χ² |
|---|---|---|---|---|
| 1 | 30 | 10 | 3:1 | 0.000^{ns} |
| 2 | 27 | 13 | 3:1 | 1.999^{ns} |
| 3 | 30 | 10 | 3: 1 | 0.000^{ns} |
| 4 | 32 | 8 | 3:1 | 0.533^{ns} |
| 5 | 31 | 9 | 3:1 | 0.133^{ns} |
| 6 | 29 | 11 | 3:1 | 0.133^{ns} |

| | | | | |
|---|---|---|---|---|
| * Forty seeds were used for each line. ^{ns} Non-significant. | | | | |

*Enzymatic activity of E1cd in E1cd-FLC transgenic plants*. According to Ziegelhoffer et al., ibid, (2001), E1 in E1cd plants hydrolyzed 4-methylumbelliferone β-D-cellobioside (MUC) to 4-methylumbelliferone (MU) at a rate of 40 nmol of substrate per microgram per minute. The enzymatic activity of E1 enzyme extracted from apoplast-targeted transgenic *E1cd* which were further transformed with *FLC* (so called *E1cd-FLC*) was 1.4726 nM/µg/min. This activity is similar to the E1 enzymatic activity that was originally reported by Ziegelhoffer et al. ibid, (2001) and confirmed by Teymouri et al. ibid, (2004) for earlier *E1cd* transgenic generations of these plants. This confirms that addition of *FLC* does not affect E1cd enzymatic activity.

*Vernalization studies*. As tobacco is an annual and a warm-season crop, it normally does not require vernalization to induce flowering. However, because vernalization has the ability to down-regulate *FLC* expression in *Arabidopsis*, the *FLC*-expressing transgenic plants were tested for vernalization effects in case of accidental cold exposure. As expected, vernalization had no effect on flowering time, which is consistent with a similar experiment in *Brassica napus* (Tadege et al., ibid, 2001). Vernalization is downstream of *FLC*, and because the CaMV35S promoter is a strong promoter, it would overturn any effects vernalization may have had on *FLC*, and as pointed out previously (Tadege et al., ibid, 2001), it is not cold-responsive.

### EXAMPLE 12

This Example illustrates the High-Level Production of Endo-1, 4-β-Glucanase in Transgenic Rice with Subsequent Enhanced Conversion of Biomass Polysaccharides into Fermentable Sugars.

The catalytic domain of *Acidothermus cellulolyticus*, thermostable endoglucanase gene (encoding for endo-1,4-β-glucanase enzyme or E1) was constitutively expressed in rice using the *Agrobacterium*-mediated transformation system in an apoplast-targeting manner. Molecular analyses of T1 plants confirmed presence and expression of the transgene. The amount of E1 enzyme accounted for up to 4.9% of the plant total soluble proteins, and its accumulation had no apparent deleterious effects on plant growth and development. Approximately 22 and 30% of the cellulose in the Ammonia Fiber Explosion (AFEX)-pretreated rice and maize biomass was converted into glucose using rice E1 heterologous enzyme respectively. As rice is the major food crop of the world with minimal use for its straw, the results may suggest a successful strategy for producing biologically active hydrolysis enzymes in rice to help generate alcohol fuel while substituting the wasteful and polluting practice of rice straw burning with an environmentally superior technology.

The fuel ethanol industry has been growing extensively in many countries worldwide (Renewable Fuels Association. Homegrown for the homeland: Industry Outlook Report 2005. p. 14 (2005)), and considerable efforts have been exerted towards improving ethanol yield and reducing its production costs during the last two decades (Ingledew, W.M. In: T.P. Lyons, D. Kelsall & Murtagh J. Editors. The Alcohol Textbook Nottingham University Press, Nottingham, UK, 55-79 (1995) and Lynd, L.R., et al, Consolidated bioprocessing of cellulosic biomass: an update. Curr. Opin. Biotechnol. 16, 577-583 (2005)). A vision to enhance U.S. economic security has set a target of using plant-derived materials to meet 10% of chemical feedstock demand by 2020-a fivefold increase (Singh, S.P. et al, International Food and Agribusiness Management Review 5, 1-15 (2003). In 2004, U.S. ethanol production capacity reached 3.535 billion gallons, about 303 million gallons more than 2003. Another production increase of more than 500 million gallons is projected for 2006 (MacDonald, T. et al, California Energy Commission Report. P. 6 (2003)). Until now, the vast majority of U.S. ethanol has been produced from maize seeds (Renewable Fuels Association, ibid, 2005), while Brazil produces similar quantities of ethanol from sugar derived from cane. The economic and environmental performance of maize and sugar ethanol would likely be improved by producing ethanol from lignocellulosic materials instead. Approximately 1.3 billion tons of crop and forest residues and energy crops are thought to be available in the United States with proper management. The energy value of this much lignocellulosic biomass is roughly equivalent to 3.5 billion barrels of petroleum per year; the total amount of petroleum produced in the United States in its peak production year-1972. Worldwide, over 1.7 billion tons of crop residues are available annually (Kim, S. and Dale, B.E. Biomass and Bioenergy. 26, 361-375 (2004)) and nearly half of this total is rice straw. Energy crops could add many more billions of tons of lignocellulosic biomass for processing to fuels and chemicals. A recent comprehensive study prepared under the leadership of the Natural Resources Defense Council highlights the potential economic and environmental benefits of very large scale conversion of lignocellulosic biomass to ethanol, electricity and other fuels (Greene, N., Growing Energy: How Biofuels Can Help End America's Oil Dependence. Natural Resources Defense Council. December (2004)).

For ethanol to be produced from plant biomass sources, enzymatic hydrolysis of cellulose to fermentable sugars is employed (Kabel, M.A. et al, Biotechnol. Bioeng. 93, 56-63 (2005)) using hydrolysis enzymes (Ziegler, ibid, 2000 and Ziegelhoffer, ibid, 2001). These enzymes are produced in large-scale microbial fermentation tanks (Howard, R.L. et al, Afr. J. Biotechnol. 2, 602-619 (2003) and Knauf, M. et al., Internat Sugar Jour. 106, 147-150 (2004)). Although the cost of enzyme production was reduced by about a factor of four from 1980 to 1999 (Wyman, C.E., Annu. Rev. Energ. Env. 24, 189-226 (1999)) and by another 10 fold since 2000 (Knauf, M., ibid, 2004), it still represents about $0.20 per gallon of lignocellulosic ethanol-a major cost factor. However, cost reduction might be achieved by producing crops that can sustainably and actively self-produce the desired hydrolysis enzymes (Sheehan, J. et al, Biotechnol. Progr. 15, 817-827 (1999) and Teymouri, F. et al, ibid, 2004).

In addition, the specific enzyme mixture was developed for corn stover treated with dilute acid and it is yet to be demonstrated whether or not the specific enzyme mixture will be suitable for other biomass materials or other pretreatments. In biological conversion of biomass to ethanol, the biomass raw material, the pretreatment and hydrolysis enzymes used after pretreatment to produce sugars must function _together as a system (Wyman, C.E. et al, Bioresource Technology. 96, 1959-1966 (2005)). Enzymes developed for acidic pretreatments are likely not suitable for pretreatments using neutral or alkaline conditions. One set of high value hydrolysis enzymes that might be alternatively produced within the biomass crops and utilized in fuel ethanol production in a biorefinery are the "cellulases"-enzymes that convert cellulose into fermentable sugars.

The E1 endo-1,4-β-glucanase enzyme of A. *cellulolyticus* is one of the most thermostable cellulases known (Baker, J.O. et al, Appl Biochem. Biotechnol. 45/46, 245-256 (1994)). The expression of E1cd endo-1,4-β-glucanase in tobacco (Ziegelhoffer et al, ibid, 2001) potato (Dai, ibid, 2000), and *Arabidopsis* (Ziegler, ibid, 2000) plants demonstrated the possibility of producing this enzyme in plants. Several prominent crops have been recommended for this purpose, especially those with a high-lignocellulosic biomass, which some presently cause disposal problems (Knauf, ibid, 2004 and Sticklen, M. et al, 2nd International Ukrainian Conference on biomass for energy, p. 133, 20-22 September 2004, Kyiv, Ukraine (2004)).

Rice, as the primary source of caloric intake for over half of the world's human population, is grown on over 148 million hectares worldwide (Chandra Babu, R., et al. Crop Sci. 43, 1457-1469 (2003)) with a total production of about 800 million tons of straw (Jiang, J. et al, Crop Sci. 40, 1729-1741 (2000)). Because of its central role in food supply, significant advances have already been made in the development of rice genetic transformation methods and incorporation of genes conferring important agronomic traits (Jiang, J. et al, ibid, 2000).

While rice seed is the traditionally useful portion of this important crop, its remaining biomass has to date shown limited use. Traditionally, farmers throughout the world burn rice straw in the field after harvest. Burning is inexpensive and mitigates rice diseases. However, emitted smoke give rise to health concerns such as increased incidence of asthma (McCurdy, S.A., et al., Am. J. Respir. Crit. Care Med. 153, 1553-1559 (1996); Jacobs, J. et al, Environ. Health Perspect. 105, 980-985 (1997); Torigoe, K. et al, Pediatr. Int. 42, 143-150 (2000); Golshan, M. et al, Int. J. Environ. Health Res. 12, 125-131 (2002) and Kayaba, H. et al, Tohoku J. Exp. Med. 204, 27-36 (2004)), among others. These concerns, for example, resulted in California legislation that limits rice straw burning to the lesser of 125,000 acres or 25% of rice area in 2001, and even then burning is allowed only if evidence of disease is present (California Rice Commission, http://www.calrice.org/ala_burning.htm). California harvested 508,000 acres of rice in 2005, down from a peak of 590,000 acres in 2004 but nearly ten percent higher than the 465,000 acres harvested a decade earlier (USDA-National Agricultural Statistical Service, http://www.nass.usda.gov/QuickStats/). As a result, California produces an excess of one million tons of rice straw each year. Of the rice straw produced in California, only about 3-4% is used in commercial applications (http://www.ethanolmarketplace.com/061305_news2.asp) and the rest must be incorporated into the soil by plowing and adding water to aid decomposition. The cost of this soil incorporation is about $43/acre, for a total of $15-18 million per year (California Rice Commission, Rice Straw, http://www.calrice.org/a5a_recycling.htm).

Therefore, rice may become a viable bio-based energy candidate with potential to lower pollution levels at the same time. Moreover, the production of enzymes in rice straw may prove fruitful for the manufacture of other valuable bio-based industrial enzymes and protein pharmaceuticals.

The following study examines the production of biologically active *A*. *cellulolyticus* endo-1,4-β-glucanase E1 enzyme in transgenic rice plants and conversion of crop biomass cellulose-to-glucose using rice-produced E1 heterologous enzyme.

Results

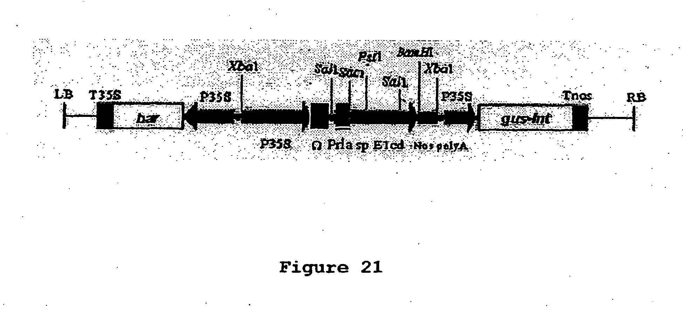

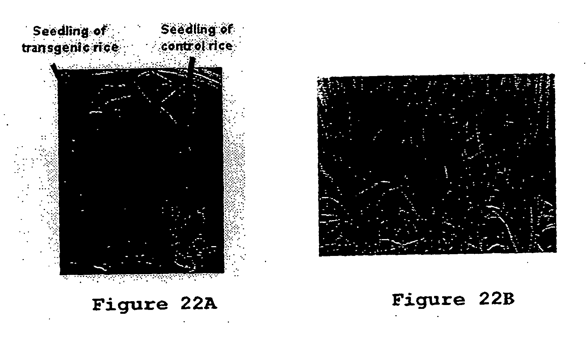

**Construct and genetic transformation.** The ApoE1 binary vector (Figure 21) containing the catalytic domain of the *A*. *cellulolyticus* thermostable endoglucanase (*E1*) gene (encoding for endo-1, 4-β-glucanase enzyme) was introduced into the nuclear genome of mature embryo-derived calli of the rice variety Taipei 309 (*Oryza sativa* L. subsp. Japonica) using the *Agrobacterium*-mediated transformation system (Ahmad, A. et al, In Vitro Cell. Devel. Biol. 38, 213-220 (2002) and Cheng, M. et al, In Virto Cell. Dev. Biol. 40, 31-45 (2004)). Transformation frequency, as defined in terms of percentage of glufosinate herbicide resistant calli was 32%. About 78% of these glufosinate-resistant embryogenic calli differentiated into plantlets in the presence of 15 mg/L glufosinate ammonium. Many transgenic plants were produced among which five independent transgenic events were selected for further molecular and biochemical analysis. T0 and T1 plants grew well under controlled environments with no apparent growth or developmental abnormalities (Figure 22B).

**Molecular analysis of the transgenic plants.** Combining the results of stable GUS expression patterns (for the *gus* gene) and PCR (for the *bar* and *E1* genes) assays confirmed the presence of intact, linked *gus*, *bar* and *E1* genes. The blue color of GUS expression patterns were observed in the transgenic plantlets (Figure 22A). The expected PCR bands (0.59 kb for *bar* and 1 kb for *E1*) were confirmed in the plasmid and the transgenic rice lines, but not in the untransformed control plants (Figures 23A).

Southern blot analysis confirmed the stable incorporation, copy number and independence of the transgenic lines (Figures 23B). The genomic DNA of the five transgenic lines showed bands of different sizes, as an indication of five independent transgenic events with 1-2 copies.

When Northern blot analysis was used to confirm the transcription of the *E1* gene, a transcript of approximately 1 kb for this gene was detected in the transgenic tobacco positive control (tobacco transformed with the same construct) as well as the rice transgenic lines, indicating that the transgenic lines possess the transcriptionally-active *E1* gene (Figures 23C).

Western blot analysis of leaf total soluble proteins (LTSP) using the mouse antibody raised against the E1 protein confirmed the expression of *E1* both in transgenic rice and transgenic tobacco positive control, with the expected molecular mass of 40-kDa (Figures 23D). Furthermore, the relative amount of transcript and 40 kDa E1 polypeptide in all five transgenic lines, judged from band intensity respectively in Northern and Western blots, correlated well with the amount of E1 produced in the transgenic lines using the 4-methylumbelliferyl β-D-cellobioside assay (MUCase) (Figures 23C,D and Table 10).

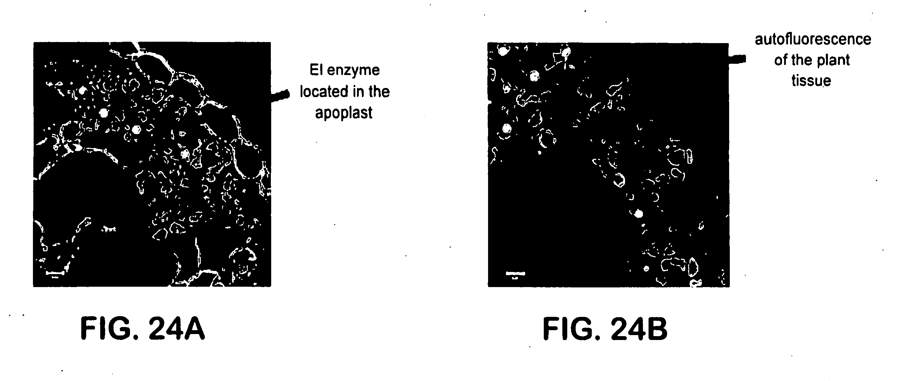

**Localization of the E1 enzyme in the apoplast.** Strong green fluorescent signals were detected in the apoplast of the transgenic tissues upon the application of immunoflouresence scanning laser confocal microscopy, confirming accumulation of the E1 enzyme. No signals were detected in the untransformed control plant tissues (Figures 24).

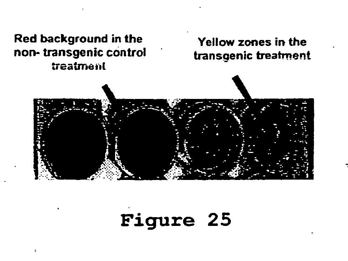

**High-level production of biologically active E1 enzyme.** E1 enzyme was expressed at relatively high levels of 2.4-4.9% of LTSP, as detected among the transgenic lines. The carboxymethyl cellulase activity assay (CMCase) confirmed that the rice-produced heterologous E1 is biologically active. In this confirmation, zones of carboxymethylcellulose (CMC) hydrolyzed by the enzyme were decolorized with a washing buffer, leaving yellow regions in the transgenic as compared with red background in the control untransformed plant samples (Figure 25). The results suggest that the microbial E1 enzyme remained biologically active in the transgenic ,rice plants while E1 activity was not present in the untransformed plants (Table 10).

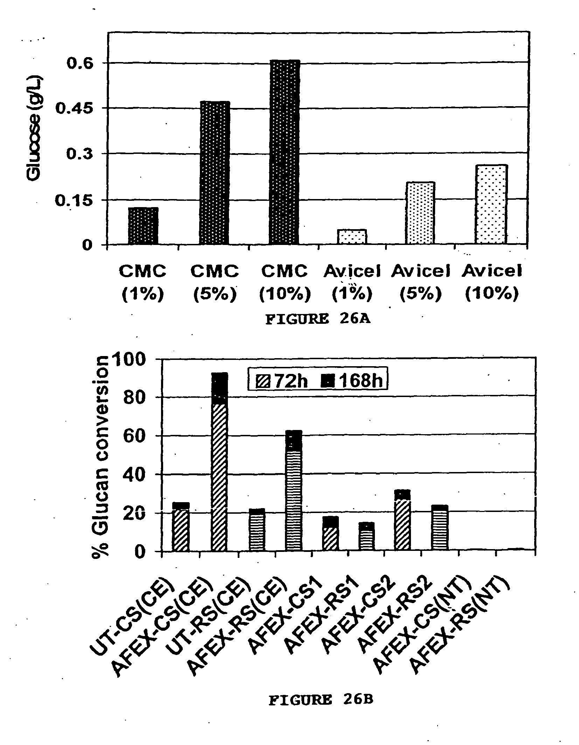

**Glucan to glucose conversion**. The Ammonia Fiber Explosion (AFEX)-pretreated (Teymouri, F. et al, ibid, 2004) maize and rice biomass (lignocellulosic substrates containing both amorphous and crystalline cellulose), as well as increasing concentrations of both CMC (amorphous cellulose) and Avicel (crystalline cellulose) were converted into glucose using the transgenic rice plant total soluble proteins containing the E1 enzyme. Using 10% CMC and Avicel concentrations, approximately 0.6 and 0.2 g/L glucose was released after 168 hour of hydrolysis, respectively (Figure 26A). Additionally, considerable amounts of polyoligosaccharides were released from the CMC substrate blank, and the apparent solution viscosity increased substantially. Conversely, when an aliquot of rice E1-containing total soluble proteins was added to the CMC substrate, viscosity declined with reduced polyoligosaccharide formation and a detectable increase in the glucose peak (results not shown).

Approximately 25% and 95% glucan conversion was achieved for untreated and AFEX-treated corn stover respectively, when the cellulase commercial enzyme (Spezyme CO, Genencore) along with β-glucosidase (Novo 188, Sigma) was used in each case. Under similar conditions, untreated and AFEX-treated rice straw showed 21% and 62% glucan conversion respectively. Since both untreated corn stover and rice straw showed much lower conversion compared to AFEX-treated biomass (less than 2% using E1-containg rice extract and 25% and 21% using cellulase commercial respectively), AFEX-treated biomass was used for further experiments. When 0.5 ml of rice extract containing 4.9% LTSP E1 along with commercial β-glucosidase were added to the substrates, 17% and 14% of glucan was respectively converted for AFEX-treated corn stover and AFEX-treated rice straw. When the amount of E1-bearing rice extract was increased to 4 ml, 30% and 22% were respectively converted under the same conditions. No activity was observed when substrates were treated with non-transgenic (NT) rice total soluble protein.

**Discussion**

Plants have been used as "green bioreactors" for the production of essential enzymes (Hong, C.Y., Chen, K.J., Liu, L.F., Tseng, T.H., Wang, C.S. & Yu, S.M. Production of two highly active bacterial phytases with broad pH optima in germinating transgenic rice seeds. Transgenic Res. 13, 29-39 (2004); Chiang, C.M et al. Expression of a bifunctional and thermostable amylopullulanase in transgenic rice seeds leads to starch autohydrolysis and altered composition of starch. Mol. Breed. 15, 125-143 (2005)) and other proteins (Liu., H.L., Li, W.S., Lei, T., Zheng, J., Zhang, Z., Yan, X.F., Wang, Z.Z., Wang, Y.L. & Si, L.S. Expression of Human Papillomavirus type 16 L1 protein in transgenic tobacco plants. Acta Biochim Biophys Sin. 37, 153-158 (2005).), carbohydrates (Schulman, A.H. Transgenic plants as producers of modified starch and other carbohydrates. In: Plant biotechnology and transgenic plants. Edited by Kirsi-Marja Oksman-Caldenetey and Wolfgang H.Barz., New York, Basel., pp.255-282 (2002); Sahrawy, M., Avila, C., Chueca, A., Canovas, F.M. & Lopez-Gorge, J. Increased sucrose level and altered nitrogen metabolism in Arabidopsis thaliana transgenic plants expressing antisense chloroplastic fructose-1,6-bisphosphatase. J. Exp. Bot. 55, 2495-2503 (2004)) and lipids (Qi, B. et al. Production of very long chain polyunsaturated omega-3 and omega-6 fatty acids in plants. Bio/technology 22, 739-745 (2004)) while requiring minimal inputs of raw materials and energy (Teymouri, F., Alizadeh, H., Laureano-Perez; L., Dale, B. E. & Sticklen, M. Effects of Ammonia Fiber Explosion Treatment on Activity of Endoglucanase from Acidothermus cellulolyticus in Transgenic Plant. Appl. Biochem. Biotechnol. 116, 1183-1192 (2004)). Production of biomolecules in plants, considered as molecular farming, is one approach to improve the economics and increase the low-cost production efficiency of these biomolecules (Bailey, M.R. et al. Improved recovery of active recombinant laccase from maize seed. Appl Microbiol Biotechnol. 63, 390-397 (2004); Breithaupt, H. GM plants for your health. EMBO. 5, 1031-1034 (2004); Fischer, R., Stoger, E., Schillberg, S., Christou, P. & Twyman, R. Plant-based production of biopharmaceuticals. Curr. Opin. Plant Biol. 7, 152-158 (2004)).

Several crops have been recommended for biomass-to-ethanol conversion, among them maize, rice, sugarcane and switchgrass (Knauf, M. & Moniruzzaman, M. Lignocellulosic biomass processing: A perspective. Internat Sugar Jour. 106, 147-150 (2004); Sticklen, M. et al. Production of microbial hydrolysis enzymes in biomass crops via genetic engineering. 2nd International Ukrainian Conference on biomass for energy, p. 133, 20-22 September 2004, Kyiv, Ukraine (2004)) -all with a high amount of lignocellulosic biomass, and some of which have caused disposal problems. Production of enzymes in plants used for biomass conversion is a potentially powerful tool to facilitate the conversion of cellulose to glucose in the commercial production of ethanol while solving the problems associated with accumulated agricultural waste biomass. In addition, as ethanol bioconversion enzyme costs are decreased, ethanol biorefineries may achieve financial advantages over petroleum refineries.

In the present study, rice was genetically transformed with the catalytic domain of the E1 gene encoding the endo-1, 4-β-glucanase (E1) enzyme. By immunoflouresence microscopic analysis, it was shown that the apoplast efficiently accumulated a high level of functional E1 enzyme, accounting for up to 4.9% of the plant total soluble protein (Table 10) with no apparent deleterious effects on plant growth, fertility and yield. Also, the *E1* gene was stably inherited in a Mendelian manner (data not shown) and its E1 product remained active at high levels in the T1 generation.

There are three possible explanations why the heterologous E1 accumulated in apoplast did not harm transgenic plant cell walls. First, lignocellulose is difficult to hydrolyze because it is associated with hemicellulose, and surrounded by a lignin seal, which has a limited covalent association with hemicellulose. Moreover, it has a crystalline structure with a potential formation of hydrogen bonds resulting in a tightly packed structure. Also as per Figure 26B, we conclude that pretreatment might be necessary to increase the surface area and consequently accessibility of cellulases by removing the lignin seal, solubilizing hemicellulose and disrupting crystallinity (Demain, A.L., Newcomb, M. & Wu, J.H.D. Cellulase, Clostridia, and Ethanol. Microbiol Mol Biol Rev. 69, 124-154 (2005)). Second, cellulases function in a synergistic enzyme complex. If only one enzyme of the complex is expressed such as E1, this single enzyme might not be sufficient to significantly affect the integrity of the cell wall without the pretreatment (Ziegelhoffer, T.J., Raasch, A. & Austin-Phillips, S. Dramatic effects of truncation and subcellular targeting on the accumulation of recombinant microbial cellulase in tobacco. Mol. Breed. 8,147-158 (2001)). Third, due to the thermophilic nature of the E1, the enzyme has limited activity at plant growth temperature (Dai, Z., Hooker, B.S., Anderson, D.B. & Thomas, S.R. Expression of Acidothermus cellulolyticus endoglucanase E1 in transgenic tobacco: biochemical characteristics and physiological effects. Trans. Res. 9, 43-54 (2000)).

When accumulated in cytosol, the normal level of heterologous protein production in plants is about 0.1-0.3% of plant total soluble proteins. In contrast when enzyme is targeted for accumulation in apoplast, this level has been increased to 4.9% in rice (Table 10) and up to 26% in *Arabidopsis* (Ziegler, M. T., Thomas, S. R. & Danna, K. J. Accumulation of a thermostable endo-1,4-b-D-glucanase in the apoplast of Arabidopsis thaliana leaves. Mol. Breed. 6, 37-46 (2000)). Among many factors (Cheng, M., Lowe, B.A., Spencer, T.M., Ye, X. & Armstrong, C.L. Factors influencing Agrobacterium-mediated transformation of monocotyledonous species. In Vitro Cell. Dev. Biol. 40, 31-45 (2004)), the use of the catalytic domain of the *E1* gene (Ziegler, M. T., Thomas, S. R. & Danna, K. J. Accumulation of a thermostable endo-1,4-b-D-glucanase in the apoplast of Arabidopsis thaliana leaves. Mol. Breed. 6, 37-46 (2000).), the use of the Tobacco Mosaic Virus translational enhancer (Ibid.), the strength of the CaMV 35S promoter (Cheng et al. In Vitro Cell. Dev. Biol. 40, 31-45 (2004)) and the targeting of E1 enzyme to the apoplast (Ziegler et al.) might have contributed to the overall level of production of E1 in rice.

It has been well documented that cellulases work together synergistically to decrystallize and hydrolyze the cellulose. Exo-glucanases act on crystalline cellulose (on cellulose chain ends) and endo-glucanase (E1) acts on amorphous cellulose (interior portions of the cellulose chain) (Bayer, E.A., Chanzy, H., Lamed, R. & Shoham, Y. Cellulose, cellulases and cellulosomes. Curr. Opin. In Str. Biol. 8, 548-557 (1998)). In contrast, the results in this study demonstrate production of glucose at from atypical endoglucanase activity on a solid substrate. This could be due to two system features. First, the presence of hemicellulase (from β-glucosidase) in the reaction stream helped to remove the hemicellulose from the biomass, which in turn gave higher accessibility for the E1 enzyme to act more on the cellulose chains. Second, E1 enzyme can cause multiple random attacks on the same cellulose chain resulting in small fragments of cellobiose, cellotriose and cellotetraose. These fragments can be further hydrolyzed by enzyme molecules in solution such as E1 itself or β-glycosidase enzyme used to avoid reaction inhibition by cellobiose (Medve, J., Karlsson, J., Lee, D. & Tjerneld, F. Hydrolysis of microcrystalline cellulose by cellobiohydrolase I and endoglucanase II from Trichoderma reesei: Adsorption, sugar production pattern and synergism. Biotechnol. Bioeng. 59, 621-634 (1998)).

Based on a previous study, AFEX pretreatment, similar to other methods of pretreatments which operate under harsh conditions, might destroy as much as 2/3^{rd} of the activity of plant produced heterologous E1 (Teymouri, F., Alizadeh, H., Laureano-Perez; L., Dale, B. E. & Sticklen, M. Effects of Ammonia Fiber Explosion Treatment on Activity of Endoglucanase from Acidothermus cellulolyticus in Transgenic Plant. Appl. Biochem. Biotechnol. 116, 1183-1192 (2004)). Therefore, we recommend either producing higher level of E1 needed for hydrolysis, or extracting the E1 protein or the plant total soluble proteins concentrate containing the biologically active E1 enzyme and then adding the enzyme or the crude extract after pretreatment for the enzymatic hydrolysis of pretreated lignocellulosic matter, as we did in this study.

Production of E1 enzyme in plant biomass could potentially be commercially viable, with the caveat that for this potential to be fulfilled additional work is needed to produce other cellulase and possibly hemicellulase enzymes along with E1 in plants in order to maximize production of glucose and other sugars. When all are produced in plants, this could compete with the full range of commercial hydrolysis enzymes currently used in ethanol production. The costs of plant-produced enzyme conversion technology might further be reduced, and research ensuring that cellulases produced within the plants will survive harvest, storage, transportation and the thermo-chemical cellulosic material pretreatment step itself. The latter is a particularly formidable obstacle but may be achievable for pretreatments that use alkaline pH and more moderate temperatures than the dilute acid pretreatment, which operates at around 200°C and pH 1.0.

This is the first report on production of a hydrolysis enzyme in rice. More experiments are needed to produce other hydrolysis enzymes in biomass crops, along with the use of bioconfinement methods to reduce seed contamination and controversies around genetically modified plants (NRC Report. Bioconfinement of genetically engineered organisms. The U.S. National Academy of Sciences. Natl. Acad. Sci. Press. 265 pages. (2004)).

**Experimental protocols**

**Transformation vector.** The pZM766-E1cat containing the *Acidothermus cellulolyticus E1* catalytic domain driven by the Cauliflower Mosaic Virus 35S Promoter (CaMV 35S), tobacco Mosaic Virus translational enhancer (Ω), and the tobacco pathogenesis-related protein 1a (Prla) signal peptide encoding sequence for apoplast-targeting of the E1 enzyme was removed from the pUC19 vector by digestion with *Xba*I. The removed cassette was transferred to the binary vector pCAMBIA 3301 containing the bar herbicide resistance selectable marker and the *gus* marker genes to generate the binary vector ApoE1.

**Selection of transformants using glufosinate herbicide**. A kill curve was developed to test the sensitivity of rice calli to glufosinate ammonium. Glufosinate ammonium was used as a selection agent since the binary vector used in this study contained the bar gene. Untransformed calli were placed on Murashige and Skoog (MS) and vitamins medium (Murashige, T. & Skoog, F.. A revised medium for rapid growth and bioassays tobacco tissue cultures. Physiol. Plant 15, 473-497 (1962)) containing 0, 5.0, 10.0, 15.0, 20.0 and 25.0 mg/L of glufosinate ammonium for 6-8 weeks. The calli survival was recorded. All calli turned brown and died after being cultured on glufosinate ammonium with concentrations of 15 mg/L or more. Therefore, 15 mg/L glufosinate ammonium was used for transgenic plant selection.

**Genetic transformation**. Mature seeds of rice (Oryza sativa L. subsp. Japonica) variety Taipei 309 were dehusked and sterilized in 70% (vol/vol) ethanol for 2-3 min and then transferred into 50% (vol/vol) Clorox solution for 20 min with gentle shaking. The sterilized seeds were plated for callus induction on MS salts and vitamins medium supplemented with 3% sucrose, 300 mg/L casein enzymatic hydrolyzate, 500 mg/L proline, 2mg/L 2,4-dichlorophenoxyacetic acid, 2.5 g/L Phytagel, pH 5.8 and grown for 21 days at 25°C in the dark. The *Agrobacterium* strain LBA 4404 containing the transgenes was grown in 10 ml YM medium (containing Yeast extract 0.4 g/L, Mannitol 10.0 g/L, NaCl 0.1 g/L, MgS04. 7H20 0.2 g/L, K2HP04. 3H20 0.5 g/L, pH 7.0) supplemented with 50 mg/L of kanamycin, streptomycin, rifampcin and 100 µ*M* acetosyringone, incubated at 28 °C and 250 rpm for 48 h. Then, the cultures were transferred to 40 ml MS medium supplemented with 100 µ*M* acetosyringone and incubated under the same conditions for another 24 h. The bacterial cells were harvested by centrifugation and resuspended in 15 ml of the same media. The cultures (cell density 0.9 at A₆₀₀) were used for transformation.

Three weeks after callus induction from the scutellar region of the rice embryo, embryogenic calli were immersed in *A*. *tumefaciens* suspension for 20 min under vacuum. Infected calli were co-cultivated in MS medium supplemented with MS salts and -vitamins, 3% sucrose, 1% glucose, 500 mg/L casein enzymatic hydrolyzate, 2 g/L Gelrite, 100 µM acetosyringone, pH 5.2. After 3-4 days of co-cultivation, calli were washed with sterile water containing 500 mg/L cefotaxime and blotted on filter paper. The calli were immediately plated on a selection medium, calli induction medium supplemented with 15 mg/liter glufosinate ammonium and 500 mg/L cefotaxime, pH 5.8, and incubated at 25°C in the dark for 3-4 weeks. The calli that had proliferated after the initial selection were further sub-cultured for two selection cycles on the same medium every 2 weeks. The actively dividing glufosinate ammonium-resistant calli were plated on MS plant regeneration medium containing MS salts and vitamins, 3% sucrose, 3% sorbitol, 3 mg/L N6-benzyladenine, 1mg/L naphthaleneacetic acid, 500 mg/L casein enzymatic hydrolyzate, 3 g/L Gelrite, 15 mg/L glufosinate ammonium, pH 5.8 and grown at 25°C for a 10-h light/14-h dark photoperiod for 4 weeks. The regenerated plantlets were rooted on half-strength MS salts and vitamins, 4 g/L Gelrite, 15 mg/L glufosinate ammonium, pH 5.7. Plantlets were transferred to the greenhouse after acclimatization in growth chamber under 27°C (day), 19°C (night), 13h photoperiod (13h-light: 11h-dark) and 210-300 mE light intensity.

**Histochemical Analysis of GUS**. Stable expression was assayed in plantlets from the transgenic lines and untransformed control via GUS histochemical staining using 5-bromo-4-chloro-3-indoyl-β-D-glucuronicacid salt (X-gluc). Plantlets were immersed in the GUS substrate mixture and incubated at 37°C (Jefferson, R.A., Kananagh, T.A. & Bevan, M.W. GUS fusions: β-glucuronidase as a sensitive and versatile gene fusion marker in higher plants. EMBO. 6, 3301-3306 (1987)), then incubated in 70% ethanol to remove the chlorophyll, and examined under a Zeiss SV8 stereomicroscope.

**PCR analysis**. PCR was used to detect *bar* and *E1* genes in transgenic rice using leaf disk DNA as a template and REDExtract-N-Amp^{™} Plant PCR Kit (Sigma-Aldrich, St. Louis, MO, Cat # XNA-P) based on the manufacturer's instruction. Primers for *bar* included the forward 5'-ATG AGC CCA GAA CGA CG-3' (SEQ ID NO:29) and the reverse 5'-TCA GAT CTC GGT GAC GG-3' (SEQ ID NO:30), and for the the *E1* included the forward 5'- GCG GGC GGC GGC TAT TG -3' (SEQ ID NO:27) and the reverse 5'- GCC GAC AGG ATC GAA AAT CG - 3' (SEQ ID NO:28). DNA amplifications were performed in a thermo cycler (Perkin Elmer/Applied Biosystem, Foster City, CA) using initial denaturation at 94°C for 4 min, followed by 35 cycles of 1 min at 94°C, 1 min at 55°C, 2 min at 72°C, and a final 10 minute extension at 72°C. The reaction mixture was loaded directly onto a 0.9 % (w/v) agarose gel, stained with ethidium bromide and visualized with UV light. The transgene product size was about 0.59 kb for the *bar* gene and 1 kb for the *E1* gene.

**DNA isolation and Southern blot hybridization analysis.** Confirmation of transgene integration into the plant genome, number of independent transgenic lines, and transgene copy numbers were performed by Southern blot hybridization using the *E1*-coding sequence as a probe. Genomic DNA from 5 randomly selected putative transgenic lines and untransformed rice plants was isolated using the protocol described in (Saghai-Maroof, M.A., Soliman, K.M., Jorgensen, R.A. & Allard, R.W. Ribosomal DNA spacer-length polymorphism in barley: Mendelian inheritance, chromosomal location, and population dynamics. Proc. Natl. Acad. Sci. USA. 81, 8014-8019 (1984); Ziegler, M. T., Thomas, S. R. & Danna, K. J. Accumulation of a thermostable endo-1,4-b-D-glucanase in the apoplast of Arabidopsis thaliana leaves. Mol. Breed. 6, 37-46 (2000)). For Southern blots, 8 µg of genomic DNA was digested with BstX1 restriction enzyme, electrophoresed in 1.0 % (w/v) agarose gel, transferred onto Hybond-N+ (Amersham-Pharmacia Biotech) membranes, and fixed with a UV crosslinker (Stratalinker UV Crosslinker 1800, Stratagene, CA) as recommended in the manufacturers' instructions. The E1 gene-specific probe was generated using PCR amplification of the *E1* gene to produce a 1.0-kb fragment. The amplified fragment was purified using the QIAquick kit (QIAGEN). Probe labeling and detection were obtained using the DIG High Prime DNA Labeling and Detection Starter Kit II (Kit for chemiluminescent detection with CSPD, Roche Co.), following the manufacturer's protocol.

**RNA isolation and Northern blot hybridization analysis**. Total RNA samples of the untransformed and transgenic plants were isolated from five different transgenic lines using the TRI Reagent (Sigma-Aldrich, St. Louis, Mo) according to the manufacturer's instructions. Aliquots of RNA (20 µg) were fractionated in 1.2% agarose formaldehyde denaturing gel and blotted on a Hybond-N+ nylon membrane (Amersham Pharmatica Biotech) as specified by the manufacturer. The E1 gene-specific probe was generated using PCR amplification of the E1 gene to produce a 1.0-kb fragment. The fragment was gel purified using the QIAquick Gel Extraction Kit (QIAGEN Inc., Valencia, CA). Probe labeling and transcript detection were obtained using the DIGHigh Prime DNA Labeling and Detection Starter Kit II (Kit for chemiluminescent detection with CSPD, Roche Co.), following the manufacturer's protocol.

**Protein extraction and Western blot analysis.**

**Electrophoresis and transfer**. Plant total soluble protein was extracted from a reported protocol (Ziegler, M. T., Thomas, S. R. & Danna, K. J. Accumulation of a thermostable endo-1,4-b-D-glucanase in the apoplast of Arabidopsis thaliana leaves. Mol. Breed. 6, 37-46 (2000)) using the Invitrogen NuPAGE^{®} Bis-Tris Discontinuous Buffer System with the 10% NuPAGE^{®} Novex Bis-Tris Pre-Cast Gel. Total soluble protein (1 µg), NuPAGE^{®} LDS Sample Buffer (5 µl), NuPAGE^{®} Reducing Agent (2 µl), and deionized water were mixed to a total volume of 20 µl. The samples were heated at 70°C for 10 minutes prior to electrophoresis using the XCell SureLock^{™} Mini-Cell with NuPage^{®} MES SDS Running Buffer. The gel was run for about 45 minutes at 200 V, and blotted onto a membrane using the XCell II® Blot Module and NuPAGE^{®} Transfer Buffer at 30 V for 1 hour, following the manufacturer's protocol.

**Blocking, incubation and detection**. The membrane was placed into blocking buffer (1x PBS, 5% non-fat dry milk, 0.1 % Tween 20) immediately after transfer and incubated at room temperature for 1 hour with gentle agitation. The primary antibody (mouse anti-E1, provided by Steven Thomas, National Renewable Energy Laboratories) was diluted in blocking buffer to a concentration of 1 µg/ml. The blocking buffer was decanted from the membrane, 10 ml of antibody solution was added, and the membrane was incubated at room temperature for 1 hour with gentle agitation. The primary antibody solution was decanted and the membrane was washed in washing buffer (1x PBS, 0.1% Tween 20) for 30 minutes with gentle agitation at room temperature, changing the wash solution every 5 minutes. The enzyme conjugate anti-mouse IgG:HRPO (Transduction Laboratories) was diluted 1:2000 in blocking solution and added to the membrane after decanting the wash buffer. The membrane was incubated with the secondary antibody solution for 1 hour at room temperature with gentle agitation; the antibody solution was decanted from the membrane and the membrane was washed in washing solution as before. For detection, 1 ml each of Stable Peroxide Solution and Luminol/Enhancer Solution (Pierce SuperSignal^{®} West Pico Chemiluminescent Substrate) were mixed and incubated with the membrane for 5 minutes. The membrane was blotted slightly to remove excess substrate and placed in a plastic envelope. Excess liquid and air bubbles were removed. The blot was exposed to X-ray film (Kodak BioMax XAR Scientific Imaging Film) and developed in a Kodak RP X-OMAT Processor.

**Immunofluorescence microscopic analysis.**

**Tissue preparation and immunofluorescence labeling**: Free-hand sections of fresh leaf tissue from transgenic and untransformed rice plants were isolated and hydrated in NaCl/Pᵢ buffer (0.8% NaCl, 0.02% KCl, 0.14% Na₂HPO₄·2H₂O, and 0.02% KH₂PO₄ in water) containing 0.5% BSA (BSA/NaCl/Pi) for 2 min. Sections were incubated in primary antibody (rabbit anti-(mouse IgG)) raised against the E1 enzyme diluted 1:250 in the same buffer, in a moist chamber for 3 hours. The primary antibody was rinsed off with the BSA/NaCl/Pi buffer and sections were incubated for 2 hours at room temperature with fluorescein isothiocyanate (FITC)-conjugated secondary antibody (goat anti-(rabbit whole molecule IgG)) diluted 1:250 in the same buffer using same moist chamber. The secondary antibody was then rinsed off with the same buffer.

**Fluorescence microscopy**: Intracellular localization of the FITC-labeled protein was observed and images were taken using a confocal laser scanning microscopy Zeiss LSM 5 Pascal (Carl Zeiss, Jena, Germany). FITC fluorescence and chloroplast autofluorescence was excited with an argon ion laser, λₑₓ = 488 nm. Fluorescence emission was detected through a Band Pass (BP) filter, λₑₘ = 530/30 nm for the FITC (images represented in green) and Long Pass (LP) filter, λₑₘ = 650 nm for the chloroplast (images represented in red). Either a 63X Plan-apochromat or a 20X Plan-neofluar objective lens was used.

**The biological activity assays of** heterologous E1 **enzyme**.

**MUCase**. After seedlings developed reasonable leaf size, the MUCase enzyme assay was conducted as reported (Ziegler, M. T., Thomas, S. R. & Danna, K. J. Accumulation of a thermostable endo-1,4-b-D-glucanase in the apoplast of Arabidopsis thaliana leaves. Mol. Breed. 6, 37-46 (2000); Ziegelhoffer, T.J., Raasch, A. & Austin-Phillips, S. Dramatic effects of truncation and subcellular targeting on the accumulation of recombinant microbial cellulase in tobacco. Mol. Breed. 8,147-158 (2001)). E1 enzyme activity was determined by subtracting the background contributed by Taipei 309 rice control extracts, from the spectrophotometer fluorescence readings. The resulting fluorescence signals without noise were used to calculate the activity and amount of biologically active E1 enzyme present in transgenic samples.

**CMCase**. 24-well agar plates containing 1% carboxymethylcellulose (CMC) were exposed to leaf total soluble protein extract. The plates were heated to 65° C for 30 min in an oven to activate the enzyme. The plates were cooled at 4°C for 5 min and then stained with 1 mg/ml Congo Red for 30 min as described (Wood, P.J., Erfle, J.D. & Teather, R.M. Use of complex formation between Congo Red and polysaccharides in detection and assay of polysaccharide hydrolases. Meth. Enzymol. 160, 59-75 (1988)). Samples were destained with 1 M NaCl for 5 min and fixed with 10 mM NaOH.

**Cellulose hydrolysis assay.**

**Pretreatment of Biomass**. Milled corn stover and rice straw (about 1 cm in length) were pretreated using Ammonia Fiber Explosion technique (AFEX). The biomass was transferred to a high pressure Parr reactor with 60% moisture (kg water/kg dry biomass) and liquid ammonia ratio 1.0 (kg of ammonia/kg of dry biomass) was added. As the temperature was slowly raised, the pressure in the vessel increased. The temperature was maintained at 90° C for five minutes before explosively releasing the pressure. The instantaneous drop of pressure in the vessel caused the ammonia to vaporize, causing an explosive decompression and considerable fiber disruption. The pretreated material was kept under a hood to remove residual ammonia and stored in a freezer until further use.

**Substrate hydrolysis:** E1 activity was measured by reacting total protein extracted from *E1*-expressed rice leaves with different substrates, namely: AFEX-treated corn stover (CS), AFEX-treated rice straw (RS), CMC and Avicel. Commercial cellulase enzyme (Spezyme CP, Genencor International) was used in this experiment as a control. The enzyme hydrolysis was done in a sealed scintillation vial. A reaction medium composed of 7.5 ml of 0.1 M, pH 4.8 sodium citrate buffer was added to each vial. In addition, 60 µl (600 µg) tetracycline and 45 µl (450 µg) cycloheximide were added to prevent the growth of microorganisms during the hydrolysis reaction. The reaction was supplemented with 30 CBU of β-glycosidase enzyme (Novo 188 from Sigma) to avoid inhibition by cellobiose. Distilled water was then added to bring the total volume in each vial to 15 ml. All the reactions were done in duplicate to test reproducibility. All hydrolysis reactions were carried out at 50° C with a shaker speed 90 rpm. About 1 ml of sample was collected at 168 hours of hydrolysis, filtered using a 0.2 µm syringe filter and kept frozen. The amount of glucose produced in the enzyme blank and substrate blank were subtracted from the respective hydrolyzed glucose levels.

**Sugar analysis**: Hydrolyzate was quantified using Waters HPLC by running the sample in Aminex HPX-87P (Biorad) column, against sugar standards. The amount of glucose produced in the enzyme blank and substrate blank were subtracted from the respective hydrolyzate glucose levels.

**Table 10. The amount of heterologous E1 enzyme in different independent transgenic rice events determined by the MUCase activity assay (average of 3 reps).**

| | Positive control | Negative control | Line 1 | Line 2 | Line 3 | Line 4 | Line 5 |
|---|---|---|---|---|---|---|---|
| E1 in the total soluble protein | 3.60 % | 0 | 3.87 % | 2.67 % | 2.41 % | 4.90 % | 2.85 % |

### EXAMPLE 13

This Example relates to the expression of biologically active *Acidothermus cellulolyticus* endoglucanase in transgenic maize plants.

Commercial production of ethanol from plant biomass sources employs enzymatic hydrolysis of cellulose to glucose. Transgenic plants that can produce their own hydrolysis enzymes offer an inexpensive and convenient system for the large-scale production of these enzymes. The catalytic domain of a endo-1,4-β-D-glucanase gene from the eubacterium, *Acidothermus cellulolyticus,* was transferred to the maize using particle bombardment, and 31 independent transgenic plants were regenerated from five independent experiments containing E1 catalytic domain. Several of these plants grown in the greenhouse reached maturity and a few of them set seeds. Stable integration of the transgene in the genome of these plants was confirmed by Southern blot analysis and expression of the transgene in plants by Western blot analysis. Expression of the recombinant E1-cd varied in independent transgenic plants and the protein was enzymatically active at elevated temperatures. The activity-based assays indicate that the enzyme accumulated to concentrations upto 2.1% and 2.08% of the total soluble protein in leaf and root tissues, respectively. The present data demonostrate the feasibility to produce bacterial cellulase in a widely grown biomass crop plant for biomass conversion.

Abbreviations: E1-cd, catalytic domain of *Acidothermus cellulolyticus* endo-1, 4-β-glucanase E1; MUC, 4-methylumbelliferyl-β-D-cellobiose; PCR, polymerase chain reaction; Pr1a, tobacco pathogenesis-related protein 1a; MES, 2-(N-morpholino)ethanesulfonic acid.

Maize (*Zea mays* L.) is one of the largest grown annual crops cultivated worldwide. In the USA alone, maize production reached nearly 300 million metric tons in 2003 (World wheat, corn, and rice production. http://nue.okstate.edu/Crop_Information/World_wheat_produc tion. htm). Large over production of this subsidized crop decreases its market value and forces producers to find new uses of their commodity. Genetic engineering has the potential to improve the economic value of maize by introducing genes to improve its adaptability and agronomic characteristics, and to increase its utilization in non-traditional areas such as production of industrial raw materials, enzymes and other useful compounds (J.K-C. Ma, P.M.W. Drake and P. Christou, The production of recombinant pharmaceutical proteins in plants. Nature genetics. 4 (2003), pp. 794-805). One attractive approach is to engineer maize to produce polysacaride-degrading enzymes such as cellulases to serve as a "green bioreactor". Because of its high biomass production, it has been identified as ideal candidiate for biomass fuel production. Approximately, 45 % of dry mass of plant biomass comprised of cellulosic materials, of which cellulose is the largest single fraction biopolymer, constitute of 30-50 % (C.E. Wyman. Production of low cost sugars from biomass : progress, opportunities, and challenges : In : R.P. Overend, E. Cornet, Editors, Biomass - a growth opportunity in green energy and value added products, Proceedings of the 4th Biomass conference of the Americas, pergamon, oxford, 1 (1999), pp. 867-872). Enzymatic conversion of cellulose to metabolizable sugars is a crucial step for further conversion to other useful products, including ethanol production. The conversion of cellulosic biomass into useful products is a complex process and involves synergistic action of three different enzymes, such as endo-1,4-β-glucanase (EC.3.2.1.4), exocellobiohydrolase (EC.3.2.1.91), and β-glucosidase (EC.3.2.21).

Genes for a variety of cellulase enzymes from fungi and bacteria have been cloned and characterized (S. Shoemaker, V. Schweickart, M. Lander, D. Gelfand , S. Kwok , K. Myambo and M. Innis, Molecular cloning of exocellobiohydrolase I derived from trichoderma reesei strain L27, Biotechnology 1 (1983), pp. 691-696.) and (J.O. Kim, S.R. Park, W.J. Lim, S.K. Ryu, M.K. Kim, C.L. An, S.J. Choo, Y.W. Park, J.H. Kim and H.D. Yun, Cloning and characterization of thermostable endoglucanase (Cel8Y) from the hyperthermophilic Aquifex aeolicus VF5, Biochemical and Biophyisical Research Communication 279 (2000), pp. 420-426). Production of cellulase in microbial systems has been studied extensively (A.L, Demain, M. Newcomb and J.H.D. Wu, Cellulase, clostridia and ethanol, Microbiology and Molecular biology Reviews 69 (1) (2005), pp. 124-154), but studies involved its production in crop plants is very limited (A.M. Nuutila, A. Ritala, R.W. Skadsen, L. Mannonen and V. Kauppinen, Expression of fungal thermotolerant endo-1, 4-β-glucanase in transgenic barley seeds during germination. Plant Molecular Biology 41 (1999), pp. 777-783). To date, cellulase enzymes are produced from microorganisms, however, the production costs of commercial cellulase enzyme preparation from these sources are very high and prohibit large-scale bioconversion from cellulosic biomass to ethanol.

Transgenic plants that overexpress different cellulases offer an alternative as "green bioreactors" to produce inexpensive and sufficient amounts of cellulases with almost limitless potential for scale-up, which has been a major goal for other important industrial enzymes and pharmaceutical proteins.

Earlier work has demonostrated the feasibility of the production of cellulase in dicotyledonous plants namely, Arabidopsis (M.T. Ziegler, S.R. Thomas and K.J. Danna, Accumulation of a thermostable endo-1,4-β-D-glucanase in the apoplast of Arabidopsis thaliana leaves. Molecular Breeding 6 (2000), pp. 37-46), tobacco (T. Ziegelhoffer, J.A. Raasch and S. Austin-phillips, Dramatic effects of truncation and sub-cellular targeting on the accumulation of recombinant microbial cellulase in tobacco. Molecular Breeding 8 (2001), pp. 147-158.) and potato (Z. Dai, B.S. Hooker, D.B. Anderson and S.R. Thomas, Improved plant-based production of E1 endoglucanase using potato : expression optimization and tissue targeting. Molecular breeding 6 (2000), pp. 277-285). In this study, we have examined the expression of the catalytic domain of a thermostable endo-1,4-β-D-glucanase E1 cloned from *Acidothermus cellulolyticus,* in transgenic maize plants.

Materials and methods

Preparation of embryogenic callus: Immature zygotic embryos (approximately 1.5-2.0 mm long) of Hi II maize germplasm (C.L. Armstrong, C.E. Green and R.L. Phillips, Development and availability of germplasm with high Type II culture formation response. Maize Genetics Cooperative Newsletter 65 (1991), pp. 92-93), were cultured embryo-axis-side down on N6-based media (C.C. Chu, Wang, C.S. Sun, C. Hsu, K.C. Yin, C.Y. Chu and F.Y. Bi, Establishment of an efficient medium for anther culture of rice through comparative experiments on the nitrogen source, Sci. Sin. 18 (1975), pp. 659-668) supplemented with 50 µM silver nitrate, 100 mg L⁻¹ casein hydrolysate , 25 mM L- proline (C.L. Armstrong and C.E. Green, Establishment and maintenance of friable, embryogenic maize callus and the involvement of L-proline, Planta 164 (1985), pp. 207-214), 2 mg L⁻¹ 2,4-dichlorophenoxy acetic acid, 30 g L⁻¹ sucrose and 2.5 g L⁻¹ Gelrite (Sigma chemical Co., St. Louis, MO) at pH 5.8 (referred to as N6E). Culture plates were wrapped with parafilm and incubated for 2 weeks at 28°C in the dark. After 2 weeks, calluses produced from the scutellum were selected and subcultured onto fresh N6E medium. As the number of embryogenic calluses increased, they were distributed to new plates of N6E medium and subcultured every two weeks until required.

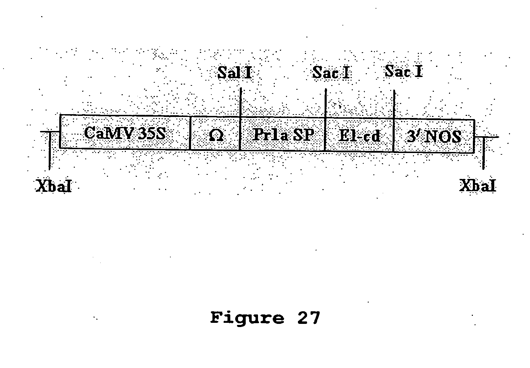

Transforming plasmids: The pMZ766 (M.R.L. Owen, J. Pen, Editors, Transgenic plants : A production system for industrial and pharmaceutical proteins. John wiley, London (1996)) containing the catalytic domain of a endo-1,4-β-D- glucanase gene isolated from the eubacterium, *Acidothermus cellulolyticus* (E1) was fused to the sequence encoding the tobacco Pr1a signal peptide. The fragment containing the signal peptide and E1 catalytic domain was fused in frame down stream of the Cauliflower mosaic 35S promoter, carrying tobacco mosaic virus Ω translational enhancer, and upstream of the polyadenylation signal of nopaline synthase (Figure 27) This plasmid was mixed in a 1 : 1 ratio with the plasmids pDM302 (J. Cao, X. Duan, D. McElroy and R. Wu, Regeneration of herbicide resistant transgenic rice plants following microprojectile - mediated transformation of suspension culture cells, Plant Cell Rep. 11 (1992) pp. 586-591) or pBY520 (D. Xu, X. Duan, B. Wang, B. Hong, T.D. Ho and R. Wu, Expression of a late embryogenesis abundant protein gene, hva1, from barley confers tolerance to water deficit and salt stress in transgenic rice, Plant Physiol. 110 (1996), pp. 249-257) containing the bar selectable marker gene.

Microprojectile bombardment: Tungsten particles of an average size of 0.7 µm (M10, Bio-Rad) were washed and coated with the plasmid DNA. The coated particles were bombarded according to the procedures described in the BioRad PDS 1000/He ® biolistic gun instruction manual. Briefly, prewashed 50 µl aliquots of tungsten particles (50 mg/ml of glycerol) were mixed with 10 µl of plasmid DNA (1.0 µg/ul), 50 µl Cacl₂ (2.5 M) and 20 µl spermidine (0.1 M) in a microfuge tube by vortexing after each addition. The mixture was centrifuged for 20 sec and the supernatant removed. The DNA-coated particles were washed in 250 µl ethanol and resuspended in 50 µl of ethanol. Five microliters of the DNA-coated particles was pipetted onto each macrocarriers while the suspension was continuously vortex. Prepared macrocarriers were placed in the laminar hood to maintain maximum dryness prior to bombardment. For bombardment, rapidly growing callus pieces 3-5 days after subculture were evenly distributed within a 1.5 cm diameter target area in the center of a plate on osmotic medium 2 h prior to bombardment. Osmotic medium was identical to the callus initiation medium but contained 0.2 M sorbitol and 0.2 M mannitol (P. Vain, M.D. McMullen and J.J. Finer, Osmotic treatment enhances particle bombardment-mediated transient and stable transformation of maize, Plant Cell Rep. 12 (1993), pp. 84-88). Each plate was bombarded once using the Biolistic particle acceleration device (PDS 1000, Bio-Rad) with a rupture disk pressure of 1100 psi ; 6.5 cm target distance (from middle of launch assembly to target plate) under a chamber pressure of 27 mm Hg. After bombardment, callus pieces were kept in respective plate for another 15 h and then transferred off the osmotic medium to callus inition medium. Culture plates were wrapped with parafilm and maintained at 28° C in the dark for 5 days before being placed under selection pressure.

Selection of stable transformants: The selection medium was similar to that used for callus initiation medium but without proline and casein hydrolysate, and with 2 mg L⁻¹ bialaphos. Callus pieces were transferred every 2 weeks to fresh selection media. After 3-4 cycles of selection, white, rapidly growing callus clusters were picked out from non proliferating and partially necrotic mother calli, and transferred onto fresh selection medium. They were subcultured every two weeks and maintained in the dark at 28°C during which embryogenic pieces were selected for plant regeneration.

Plant regeneration, acclimatization and transgenic seed recovery: Small pieces of resistant callus with clearly defined somatic embryos, were transferred to MS media (T. Murashige and F. Skoog, A revised medium for rapid growth and bioassays with tobacco tissue culture, Physiol. Plant. 15 (1962), pp. 473-497) containing 6 g L ⁻¹ maltose, 1 mg L ⁻¹ bialaphos, and 3 g L ⁻¹ Gelrite. Five to six callus pieces were transferred to 100 x 25 mm plastic petri plates containing 25 ml of medium. The plates were maintained under fluorescent light (60 µmol quanta m⁻². s⁻¹ from cool-white 40 W Econ-o-watt fluorescent lamp; Philips Westinghouse, USA) with a 16/8-h photoperiod at 25°C. Emerging plantlets with fully formed small shoots and roots were transferred onto 30 ml of rooting medium containing half-strength MS salts and vitamins, 15 g L⁻¹ sucrose and 2.0 g L⁻¹ gelrite without growth regulators in Magenta GA7 vessels (65 mm . 65 mm. 100 mm ; Magenta corp., Chicago, USA). Rooted plantlets were thoroughly washed with tap water from the medium and transferred to small pots (4 inch²) containing pre-wetted soil (BACTO high porosity professional planting mix ; Houston, TX). Plants were acclimated from the highly humid culture condition to the greenhouse environment under plastic bags, where small holes were made in the bag every other day for two weeks. The acclimated plants were transplanted to 2-gallon pots containing soil and grown under greenhouse condition. Plants were watered daily and fertilized with peter (20-20-20) granular fertilizer (Scotts company, Marysville, Ohio) weekly until they reached maturity. Transgenic plants were self-pollinated or cross-pollinated with plants originating from the same transformation events. In some cases, transgenic ears were pollinated with wild type pollen due to lack of transgenic pollen. Seed was dried down on the plant and harvested 35-45 days after pollination.

PCR and Southern blot analysis: Total genomic DNA was isolated from the leaves of greenhouse-grown independent plants. The leaves were exposed to liquid N₂ and ground to a fine powder. DNA was extracted with a modified CTAB-protocol (H.G. Murray and W.F. Thompson, Rapid isolation of high molecular weight plant DNA, Nucleic Acids Res. 8 (1980), pp. 4321-4326) and quantified after RNAse treatment. Plants were screened using PCR amplification for the introduced endo-1,4-β-D-glucanase gene. The oligonucleotide primers, 5'-GCGGGCGGCGGCTATTG-3' (SEQ ID NO:31) and 5'-GCCGACAGGATCGAAAATCG-3' (SEQ ID NO:32), were used to amplify a 1.0 kb fragment spanning the catalytic domain of the endo-1,4-β-D-glucanase gene which was analysed by electrophoresis in 0.8% agarose/ethidium bromide gels. For Southern analysis, genomic DNA (15 µg per lane) was loaded onto gels with or without digestion with *Sac* I endonuclase. Following electrophoresis in an 0.8% agarose gel, DNA was transferred to a Hybond-N membrane (Amersham-Pharmacia Biotech; Buckinghamshire, UK), fixed to the membrane by UV-crosslinking. The catalytic domain of the endo-1,4-β-D-glucanase gene was labeled with digoxigenin-11-dUTP and used for probing the domain. Hybridization and chemiluminescence signal detection were , performed according to the manufacturer's instructions. Transgene copy numbers were estimated by including on the Southern blot, plasmid DNA equivalent to 2, 4, 10, and 20 transgene copies added to non-transformed maize DNA.

Western blot analysis: Protein sample were obtained from approximately 100 mg of leaf material from the non-transgenic and transgenic lines, by grinding the tissue to a fine powder in liquid N₂. Subsequent homogenization in 200 ul plant protein extraction buffer (50 mM sodium acetate PH 5.5, 100 mM Nacl, 10 % v/v glycerol, 0.5 mM EDTA, 1 mM phenylmethylsulfonyl fluoride and 1 mg/l each of aprotinin, leupeptin and pepstatin) (T. Ziegelhoffer, J.A. Raasch and S. Austin-phillips, Dramatic effects of truncation and sub-cellular targeting on the accumulation of recombinant microbial cellulase in tobacco. Molecular Breeding 8 (2001), pp. 147-158) was performed, followed by a centrifugation at 15000 x g for 5 minutes to remove the precipitate. Total protein concentration in each sample was measured by the Bradford method (M. Bradford, A rapid and sensitive method for quantitation of microgram quantities of protein utilizing the principle of protein-dye binding. Anal Biochem.72 (1976), pp. 248-254) using Bio-rad protein assay reagent (Bio-Rad ; CA). Approximately, 1 µg of total soluble protein from each samples were heated at 70°C for 10 min and loaded onto 10% NuPAGE® Novex Bis-Tris Pre-Cast Gels (Invitrogen; Carlsbad, CA) and separated by electrophoresis, using the Xcell surelock Mini-cell with Nupage MES SDS running buffer (Invitrogen ; Carlsbad, CA). The separated proteins were then transferred to nitrocellulose membrane (Hybond™ ECL™; Amersham-Pharmacia Biotech, Buckinghamshire, UK). The membrane was blocked for 1 hour with blocking buffer (1 x PBS, 5 % non-fat dry milk, 0.1 % Tween 20). The membrane was probed with primary antibody (mouse anti-E1 monoclonal antibody), then rinsed with washing buffer (1 x PBS, 0.1 % Tween 20), and probed with secondary antibody (anti-mouse IgG: HRPO, Transduction Laboratories) for another hour. Finally, the membrane was washed in washing buffer for 30 minutes, follwed by incubation in SuperSignal®west pico (Pierce Biotechnology Inc; Rockford, IL) chemiluminescent substrate for the horseradish peroxidase reaction and developed in a Kodak RP X-OMAT processor.

MUCase assay: The 4-methylumbelliferyl-β-D-cellobiose (MUC) assay was bassed on the ability of endoglucanase E1 to hydrolyze the fluorogenic substrate 4-methylumbelliferyl-β-D-cellobioside to produce the fluorophore, 4-methylumbelliferone (MU). Approximately 100 mg leaf or root tissues from each sample was pulverized in liquid N₂ and total soluble proteins was extracted from the samples as described by (Ziegelhoffer et al., ibid, (2001)), but without cellulysin and macerase pretreatment. The total amount of protein in each of the extracts was measured by the Bradford method (Bradford, M., Anal. Biochem. 72: 248-254 (1976)) using the Bio-Rad protein assay reagent (Bio-Rad; CA). Subsequently the appropriate amount of extract containing the same amount of total protein (10 ng or 100 ng) was subjected to the activity assay as described (Ziegelhoffer, ibid, (2001)). The reaction was stopped by adding 100 µl of 150 mM glycine pH 10 and the relative amount of released MU was measured with a Spectramax M2 (Molecular Devices Corporation, Sunnyvale, CA) at excitation and emission wavelengths of 360 nm and 465 nm, respectively. The activity of the transgenic plants was calculated after substracting the background activity contributed by the wild type plants.

Results.

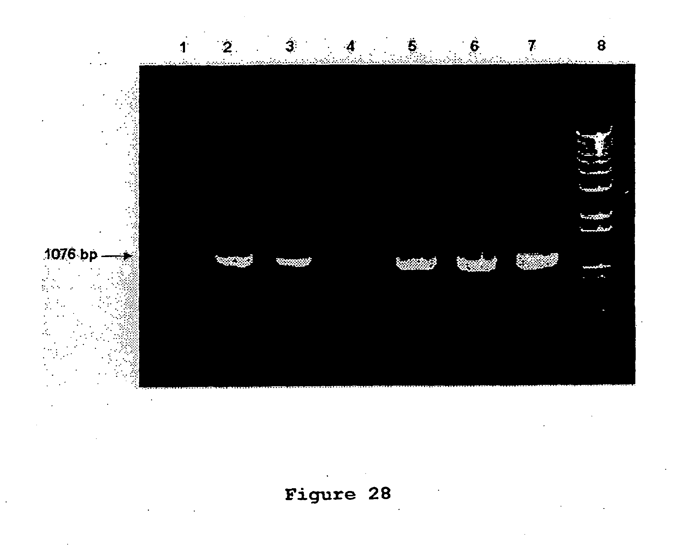

Recovery of transgenic maize plants: After 8 weeks of selection, the transformed calli became morphologically distinguishable from nontransformed calli. While control non-transformed calli died in selection medium, the bialaphos resistant calli continued to proliferate in presence of selection pressure. Bialaphos-resistant calli transferred to the maltose-containing regeneration medium readily regenerated to plantlets after 3-4 weeks. Over 2000 plantlets were regenerated from a total of 42 independent bialaphos-resistant calli (Table 11). Fourty two of these plantlets were grown to maturity in the greenhouse, where some set seeds after self or cross pollination. Of the 42 plants, 31 confirmed the presence of 1.0 kb E1-cd using PCR amplification. A representative sample of five lines transformed with the E1-cd cassette are shown in Figure 28.

**Table 11. Transformation frequencies and regeneration of transgenic plants based on fresh weight of maize type II callus.**

| Experiments | Total FW (g) of callus pieces bombarded | Number of independent bialaphos reistant callus clones | Plants containing E1-cd cassatte |
|---|---|---|---|
| 1 | 4 | 7 | 5 |
| 2 | 8 | 10 | 7 |
| 3 | 4 | 8 | 6 |
| 4 | 6.4 | 9 | 6 |
| 5 | 4 | 8 | 7 |

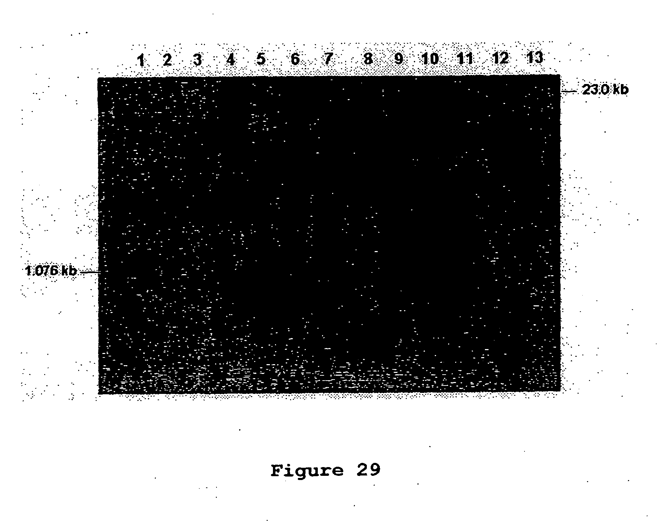

Southern blot analysis using undigested and digested total genomic DNA isolated from the leaves of PCR positive E1-cd plants confirmed the stable integration of E1-cd in the genome of these plants. After digestion of genomic DNA with *Sac* I, all of the five PCR positive plants showed the expected 1.0 kb diagnostic fragment (Figure 29), corresponding to the E1-cd construct used. A separate Southern blot (data not shown) showed that 2 to 20 copies of the E1-cd transgene were integrated into the maize geneome. With undigested genomic DNA, only fragments larger than 23.0 kb hybridized, indicating that the transferred gene had integrated into the genome of all plants analysed. No hybridization signal was present in any of the lanes containing DNA, digested or undigested from the control, untransformed plants.

Inheritance of the E1-cd transgene in 21 progenies of self-crossed plants was determined by PCR analysis and 3:1 segregation was found (data not shown), indicating that transgene was stably inherited and inserted in a single locus on one chromosome.

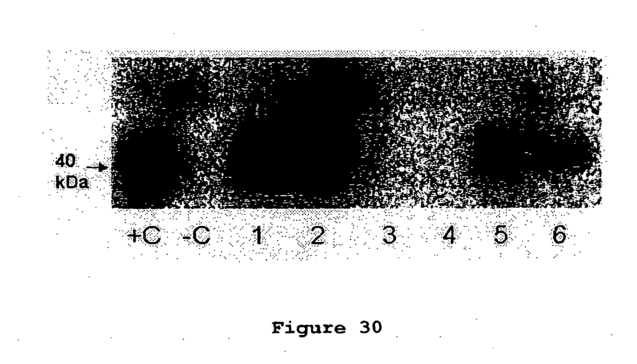

Expression of E1-cd in transgenic maize plants. Production of E1-cd protein in the regenerated plants was examined immunologically by using a monoclonal antibody against E1-cd. Protein extracts were prepared from the individual primary plants, representing six independent events and separated by electrophoresis on a SDS-PAGE gel. A band of approximately 40 KDa was detected in 4 events transformed with the chimeric E1-cd cassatte (Figure 30). The band was similar in size to authentic immunoreactive band of E1-cd of transgenic tobacco plants, which was included as a positive control. In contrast, a control sample from non-transformed maize plants exhibited no immunoreactive band.

Production of biologically active E1-cd in transgenic maize plants: Different transgenic plants showed different levels of E1 production. The accumulation of E1 in most of the plants was less than 2% of total plant soluble proteins. A broad range of endoglucanase activity was observed among 50 tested transformants (25 independent transgenic plants), of which 63% of the transgenic plants showed an extremely low level (0.000002 nMol/µg soluble protein /min) of activity and 27 % showed moderate level (i.e 0.0087 nMol/µg soluble protein/min) of activity (not shown in the table). The remaining 10% plants showed compairably high level (i.e 0.429 nMol/ug soluble protein /min) of activity (Table 12). The highest levels of activity achieved were observed in extracts from plant M4, with 0.845 nMol/min/µg protein in leaf and 0.835 nMol/min/µg protein in root tissue. In this plant, the recombinant E1-cd accumulated for ca. 2.1% of total soluble protein in leaf extracts and ca. 2.08% of total soluble protein in root extracts.

**Table 12. E1-cd specific activity in protein extracts of leaf and root tissues of selected individual transgenic maize plants expressed as nMol MU per microgram total soluble protein per min.**

| Transgenic Event | nMol/min/microgram protein | |
|---|---|---|
| | Leaf | Root |
| M1 | 0.432 | 0.537 |
| M2 | 0.330 | 0.029 |
| M3. | 0.360 | 0.131 |
| M4 | 0.845 | 0.835 |
| M5 | 0.178 | 0.017 |

The data presented in the table are the average values of three independent assays.

Microprojectile method of DNA transfer used in the present study has been used routinely to transform maize plants (Gordon-Kamm et al., Plant Cell 2, 603-618 (1990), Zhong et al., ibid, (1996) and Frame et al., In Vitro Cell Dev. Biol. Plant, 36: 21-29 (2000)). Stably transformed callus pieces were routinely recovered after 6-8 weeks of selection on bialaphos containing medium. Bialaphos has been used widely as a selective substrate for maize transformation (Dennehey et al., Plant Cell Tiss. Org. Cult., 36: 1-7 (1994) and Ishida et al, Nature Biotechnol., 14: 745-750 (1996)). It is well- documanted that maltose enhances plant regeneration frequency in rice (Ghosh, et al., J. Plant Physiol., 139: 523-527 (1993)) and, consequently, it is used in maize plant regeneration media. Interestingly, all the transformed callus pieces readily regenerated to plants upon transferred to the maltose-containing medium. The beneficial effect of maltose as a sole carbohydrate source on maize plant regeneration is likely attributable to better bioavailability of the maltose during embryo germination.

PCR and Southern analysis confirmed that 31 out of 42 transgenic plants carried the catalytic domain of E1 gene and in most cases, multiple copies of the transgene were integrated into the genome. Multiple copies of transgene integration pattern is common in transgenic maize plants produced by particle bombardment (Shou et al., Mol. Breeding, 13: 201-208 (2004) and Kennedy et al, Plant Cell Rep. 20, pp. 721-730 (2001). Functional transformation is associated with the normalized expression of the transgene, especially in translational level. Therefore, an E1-cd-specific monoclonal antibody was used to characterize the E1-cd expression in protein extracted from six independent transgenic plants. It was found that 4 out of 6 transgenic plants displayed E1-cd expression, although the accumulation of polypeptide varied substantially in independent transgenic plants. For example, transgenic plants 1 and 2, exhibited high expression and transgenic plants 5 and 6, exhibited relatively low expression. Conversely, transgenic plants 3 and 4, failed to express the transgene, presumably, due to transgene silencing. In nuclear transformation, transgene expression heterogenity could reflect the influence of many factors, including position effects, transgene structure, integrative fragmentation, transgene copy numbers, rearrangement and epigenetic phenomena such as homology-dependent transcriptional silencing and cosuppression (Allen, ibid, (1993), Rathore et al., Plant Mol. Biol. 21, pp. 871-884 (1993) and Hart, et al., Mol. Gen Genet. 235, pp. 179-188 (1992)), and the presence of boundary elements or MARs. Transgene Integrated at subtelomeric regions may be strongly expressed (Topping et al, Development, 112: pp. 1009-1019 (1991). Conversely, areas rich in heterochromatin, such as those surrounding centromeres, may exert strongly negative position effects, and transgenes integrated at such sites may be prone to silencing (Prols et al., Plant J. 2: pp. 465-475 (1992) and Rathore et al, ibid, (1993)).

Since the catalytic domain of E1 is from a thermophilic bacterium, the enzyme is most active at elevated temperatures (U.S. Patent No. 5,275,944 to Himmel et al). Therefore, the activity of the translational product was monitored with MUCase assay at 65°C. When we analyzed the E1 activity in the leaf and root protein extracts, the overall average activity was found to be slightly higher in the leaf protein extracts than in root protein extracts. Different tissues within plant are expected to have differing metabolic activity with corresponding differences in rates of translation and activity of the translational product, and our results may reflect such differences.

Production of cellulase (E1) in transgenic plants has been previously reported in tobacco (Ziegelhoffer et al, ibid, (2001)), Arabidopsis (Ziegler et al, ibid, (2000)) and Potato (Dai et al., Mol. Breeding: 6 pp. 277-285 (2000)). Ziegler et al. (ibid, 2000) examined the expression of E1 catalytic domain that targeted to the apoplast in *Arabidopsis thaliana* and observed ca. 26% of E1 accumulation in the apoplasts. In our case, the estimated E1 protein accumulation using the same construct in transgenic maize plants was upto 2.1% of total soluble protein. This is several fold lower than that reported in Arabidopsis (Ziegler et al, ibid, 2000), but is in the range reported in transgenic tobacco (Dai et al., Transgenic Research: 14(5): pp. 627-643 (2005)) and in transgenic potato plants (Dai et al, ibid, 2000). However, the exact comparison is difficult as the method of gene transfer and recipient plants are different.

Production of cellulases in a widely grown cultivated maize is important, as maize is considered the main U.S. biomass crop. This study for the first time shows that the biologically active cellulases such as E1 could be produced within the maize biomass at a relatively high level.

### EXAMPLE 14

This example shows the conversion of glucan (cellulose), rice and corn biomass into glucose using rice and corn-produced cellulase.

At present ethanol is mainly produced from corn kernel or corn starch, and today ethanol contributes to about 2% of the total of our transportation fuels mix. The goal of the DOE is to replace 30% of the total transportation petroleum fuel with biofuels by 2025. Since 2000, the repeated recommendations of the National Research Council have been to use crop biomass to supply most of these shortages in transportation liquid fuels because the current supply of sustainable global biomass energy potential is at about 10³⁰ joules per year, in which over 60% is not presently used.

Crop biomass is composed of crystalline cellulose embedded in a hemicellulose and lignin matrix. Cellulose and hemicellulose (called cellulose) are made of chains of fermentable sugars. In order to break the chemical bonds between the cellulose sugars, first one of the pretreatment methods are used to disrupt the biomass and to remove the lignin from the lignocellulosic matter, and then microbial cellulase enzymes are used to convert the cellulose into fermentable sugars for ethanol production.

Despite all efforts, the present technology still cannot economically convert the crop biomass into ethanol because of (1) the still very high cost of production of hydrolytic enzymes in deep microbial tanks, and (2) the costly operation of pretreatments of biomass to disrupt the lignocellulosic matter and remove lignin to help the exposure of cellulose to cellulase enzymes.

To reduce both of the above costs, one could design and sustainably produce cellulase and ligninase enzymes within the crop biomass in manners that these enzymes would remain in their biological active forms for further use, and would not harm the crop growth and development. Then recover and use these enzymes in pure or in crude forms before or during the biorefining process. The plant-produced ligninase can degrade lignin of biomass into phenolic compounds, and the plant-produced cellulase can convert cellulose into fermentable sugars.

Production of these enzymes in plants are possible since plants have been used as "green bioreactors" for the production of essential enzymes and many other proteins, carbohydrates and lipids while requiring minimal inputs of raw materials and energy. Production of biomolecules in plants, considered as molecular farming, is an ideal approach to improve the economics and increase the low-cost production efficiency of the cellulase and ligninase biomolecules.

For example, laccase (ligninase) has been produced under a seed specific promoter in seeds of maize and *Acidothermus cellulolyticus* endo-1, 4-β-D-glucanase (E1; cellulase) has been constitively produced in the whole (in biomass as well as in the seeds) alfalfa, potato, tobacco, and *Arabidopsis* plants.

**Project goals and specific objectives**

Cellulase enzymes which convert lignocellulosic matter into fermentable sugars for ethanol production are presently produced in microbial tanks at about $0.34/gallon of ethanol. Pretreatments are also performed to remove lignin from the lignocellulosic matter at a similar casts. The inventor's goal is to produce the cellulase and ligninase enzymes within the crop biomass cells in their biologically active forms at a level needed for such conversion of cellulose into fermentable sugars and lignin into phenolics at costs of $.03/gallon of ethanol. To achieve this goal, the inventor will reach the following specific objectives.

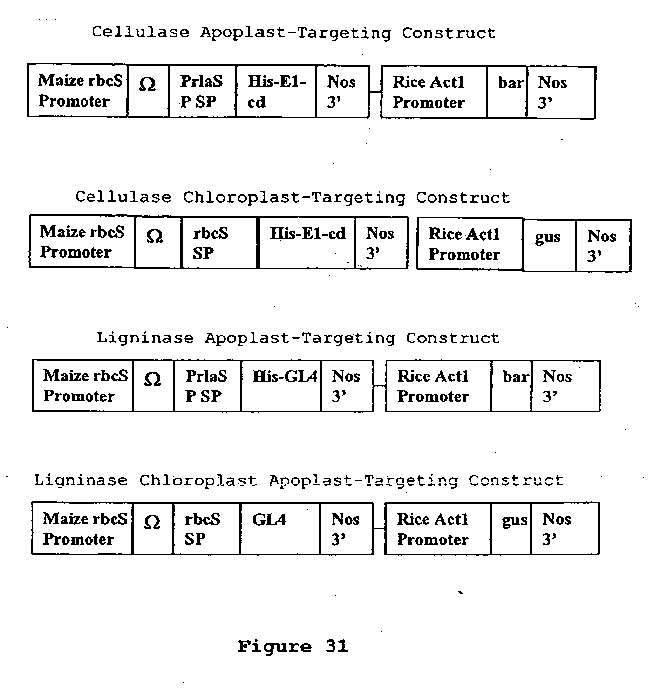

Develop a series of constructs containing the same thermostable celluluse (E1) used in her previous research, and the ligninase genes (CGL4) designed to be targeted into plant apoplast and chloroplast of the same or different plants (Figure 31).

As rice is the ideal cereal model crop and easy to genetically transgorm, we will also develop Ti plasmid vectors similar to the one in Figure 21, containing the E1 and CGL4 genes designed for apoplast and chloroplast targeting for rice genetic transformation.

Genetically engineer the model plant, rice with *Agrobacterium* containing each of the constructs.

Produce corn immature embryos in greenhouses and shoot apical multi-meristem primordial in laboratories, and genetically engineer these corn explants via the co-transformation technology using each of the above constructs and a construct containing a strong cornspecific selectable marker gene regulated by a strong developmentally controlled constitutive promoter, using the Biolistic gun device.

Grow a minimum of 10 independent transgenic plants (each with at least 10-20 plants) from each construct used, and confirm transgene copy number, integration and transcription via molecular techniques.

Develop more antibodies to E1 and to CGL4 enzymes, and confirm the translation of E1 and CGL4 in transgenic plants via Western blotting, and the localization of the enzymes in plant cell compartments via immunoflourescent confocal laser microscopy.

Extract transgenic plant total soluble proteins, and measure the enzymatic activity and determine the percentage of biologically active E1 and CGL4 in plant total soluable proteins via UV spectrophotometry.

Cross breed plants producing the highest level of E1 for maximum enzyme yield, and self breed for production of homozygous transformants.

Approaches:

Plasmid Construction: A set of four different constructs are made. The first; use the catalytic domain E1-tagged to coding sequences for a polyhistidine-regulated by rubisco promter, the tobacco mosaic virus translational enhancer, Nos terminator and the sequences encoding tobacco pathogenesis-related protein 1a (Prla) signal peptides for the targeting of E1 into the plant apoplast. The Second will be similar to the first except, we will use the maize rubisco transit peptide to direct the E1 into the chloroplast instead of its targeting into apoplast. The third construct will be similar to the first, except will use the CGL4 ligninase coding sequences instead of the E1. The fourth will be similar to the third construct, except we will use the maize rubisco transit peptide (rbcS SP) for targeting of the CGL4 into the chloroplast instead of apoplast.

Figure 31 illustrates a schematic representation of plasmid vectors containing two cassettes, one containing the *Acedothermus cellulolyticus E1* catalytic domain or the ligninase (CGL4) driven by maize rubisco promoter (rbcS) or Cauliflower Mosaic Virus 35S Promoter (CaMV 35S), tobacco Mosaic Virus translational enhancer (Ω), and the sequence encoding the tobacco pathogenesis-related protein 1a (Prla) signal peptide for apoplast-targeting or the maize rbcs signal peptide for enzyme targeting, and the polyadenylation signal of nopaline synthase (Nos). The second cassette contains either the bar selectable maker gene or the gus color marker gene regulated with the rice actin 1 promoter and intron (Act1) promoter and the Nos terminator. The constructs containing the gus gene will be usedc in a 1:1 ratio with a construct containg the *bar* for chemical selection. The above plasmids will be used alone or in combination to transform maize (**Table 13** below).

**Table 13. Transformation options to target E1 and/or CGL4 to apoplast and/or chloroplast**

| Transformation Options | I | II | III | IV | V | VI | VII | VIII |
|---|---|---|---|---|---|---|---|---|
| E1 to Apoplast | * | | * | | | | * | * |
| E1 to Chloroplast | | * | * | | | | * | * |
| CGL4 to Apoplast | | | | * | | * | | * |
| CGL4 to chloroplast | | | | | * | * | * | * |

Corn is not easily amenable to different chemical selections (antibiotics, etc). Because the bar gene is the ideal selectable marker gene known and it is routinely used by us since late 1980's in corn transformation, we will only use bar with an understanding that the use of gus color marker and the routine use of the PCR will select transgenic plants that contain 2-3 of the above gene constructs. To use more than one plasmid, the desired plasmids will be equally mixed (ratios of 1:1, 1:1:1 and 1:1:1:1) and biolistically bombarded into the maize explants in co-transformation fashion as will be explained in the next section.

The 35S can be used instead of the rbcS promoter, because our research has already shown high level production of E1 with the use of the 35S promoter. The construction of the above will follow the work performed in our research as shown in production of costructs with E1 for use in rice (Figure 26) and in maize transformation. The first cassette of each of the above four plasmids will be inserted in pTi binary vector pCAMBIA 3301 (Ti border vector) as we did for transforming rice with the E1, gus and bar transgenes (Figure 26).

Production of corn immature embryo-derived cell lines and the shoot apical multi-meristem primordial, transformation of rice via the Agrobacterium system and Co-transformation of corn via the Biolistic® gun:

Corn seeds can be germinated and plants can be grown in greenhouses to maturity. Immature embryos can be collected and cultured in appropriate medium supplemented with appropriate growth regulators. Corn shoot apical multi-meristem primordial can also be developed as per our previous patented technology.

Rice can be transformed using the *Agrobacterium* vector containing each of the above plasmids following our routine rice transformation research (Figure 26).

For corn transformation, the corn embryogenic cell lines and multi-meristems can be bombarded with tungsten particles coated with each plasmid containing both the gene of interests and the bar or the gus genes, or can be co-transformed (in equal ratios) with the plasmids containing the gene of interest and the *bar* selectable marker or the gus color marker gene. The bombarded explants can be selected in medium containing 6-10 mg/L glufosinate ammonium (PPT) and or can be tested via the Gus colorometric assay, and plantlets can be developed and transfered to greenhouses for testing, self and cross breeding, and seed production. Plants with different transgene expression can be cross bred to see whether both E1 and CGL4 could be produced at high levels in one plant.

Confirmation of integration, copy number and expression of transgenes in corn plants: Polymerase Chain Reaction (PCR) can be used to confirm the presence of the foreign genes in plants. Those shoots/plantlets, which show positive PCR signals, can be further tested for copy number via Southern blotting, for transcription via Northern blotting, and for translation via Western blotting.

Identification of the level of heterologous E1 enzyme production and the biological activity of this enzyme produced in corn plants: The E1 biological activity test can be performed as we have done previously. Soluble proteins can be extracted from leaf tissues by grinding in the sodium acetate buffer and precipitating with ammonium sulfate and quantifying using the BioRad (Hercules, CA) Protein Dye Reagent as specified by the manufacturer. The fluorescence can be read at 465 nm using SPECTRAmax M2 device (Molecular Devices Inc., Sunnyvale, CA) at an excitation wavelength of 360 nm. After subtracting background fluorescence contributed by the control, activity of samples can be calculated using a standard curve representing 4 to 160 pmol MU and compared to the activity of pure E1. The Ligninase activity test can be confirmed as described (Tien et al, ibid, 1988; Boominathan *et al.,* 1990).

Study of localization of heterologous E1 in transgenic corn cell compartments: We can confirm the localization of the heterologous E1 and CGL4 in apoplast of transgenic corn leaves using a monoclonal antibody specifically raised for this enzyme, as we performed in our apoplast-targeted E1 experiment. We can also confirm the targeting of E1 and CGL4 in chloroplasts by first isolating chloroplasts and using the same E1 antibody against these chloroplasts following our previous work on localization of three polyhydroxybutyrate enzymes (PHB) in transgenic corn chloroplasts (Teymouri F., Alizadeh H., Laureano-Preze L., Dale B., and Sticklen MB (2004). Effects of Ammonia fiber explosion (AFEX) on the activity of heterologous cellulose enzyme. Applied Biochemistry and Biotechnology. 16: 1183-1192.).

Testing the conversion of glucan into glucose and the lignin to phenolics using the corn-produced heterologous E1 and CGL4 enzymes:

Plant total soluble proteins can be extracted, and the amount of E1 in the total soluble proteins can be measured with the fluorescence dye assay using a UV spectrophotometer.

β-glucosidase can be added in each experiment in order to avoid cellabiose inhibition. The reactions are performed at 65 °C with 90 rpm, in a shaking incubator. Sampling can be done after 24, 48 and 72 hrs and checked for the amount of glucose using HPLC, Aminex 87P column against sugar standards as performed by our team before.

The plant produced CGL4 can be tested against commercially available ligninase.

Self breeding, and cross breeding of transgenic plants: Corn breeding is a routine in the inventor's facilities, and can take place in her greenhouses. Rice has been used as a model plant for corn genetic transformation because genetic engineering of rice is far less time consuming and easier than genetic engineering of corn. Therefore, while producing the thermostable *A*. *cellulololyticus* E1 in corn, she developed a rice-specific vector and conducted another experiment producing the thermostable *E1* (apoplast tragetted), the *gus* and the *bar* herbicide resistance genes (Figure 21) in rice via the Agrobacterium system (see Figure 22A, B and Figures 23A-D). In this research, rice produced the biologically active E1 up to 4.9% of plant total soluble proteins. She also found that the rice produced E enzyme was biologically active and could convert about 30% of rice biomass into glucose (Figure 26) using Ammonia Fiber Expossion (AFEX) pretreated rice straw (Dr. Bruce Dale's laboratory).

Figure 21 shows the schematic representation of ApoE1 binary vector containing the *Acedothermus cellulolyticus E1* catalytic domain driven by Cauliflower Mosaic Virus 35S Promoter (CaMV 35S), tobacco Mosaic Virus translational enhancer (Ω), and the sequence encoding the tobacco pathogenesis-related protein 1a (Prla) signal peptide for apoplast-targeting of the E1 enzyme, and the polyadenylation signal of nopaline synthase (nos).

Figure 22 illustrates genetic engineering of rice using the construct shown on Figure 21 above. Figure 22A shows Gus expression in plantlets of transgenic rice as compared to the untransformed control. Figure 22B illustrates greenhouse grown E1 transgenic rice plants. Figures 23 shows A) PCR, B) Southern, C) Northern and D) Western Blot analyses showing the presence and expression of the transgenes in five transgenic rice lines.

Figure 26 illustrates in panel Figure 26A the amount of glucose released from the enzymatic hydrolysis of CMC (1%, 5%, 10%) and Avicel (1%, 5%, 10%) using total protein extracted from E1 expressed rice straw. In panel (b) Comparison of percentage of glucan converted in the enzymatic hydrolysis of corn stover (CS) and rice straw (RS). CE, commercial enzyme, UT, untreated biomass, CS1, RS1, CS2, and RS2 represent, reaction done using 0.5 ml and 4 ml of total protein (with 4.9% of E1) and commercial β-glucosidase (6.5mg/15ml) respectively.

Please note that although we used AFEX as the pretreatment choice, we could in future use any other methods of pretreatment (for example, acid and/or heat used at NREL) because we have added the rice produced E1 after the pretreatment to convert glucan into glucose.

Our team also used the E1 cassette from the Fig. 1 and a construct containing the bar herbicide selectable marker genbe regulated by rice actin promoter and intron (Fig 4a and 4b below), co-transformed corn and constitutively produced this E1 in maize in an apoplast targeting manner (Fig. 5a and 5b).

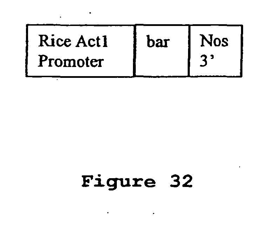

Figure 27 and Figure 32 are schematic drawings of two plasmids used in 1:1 ratio combination in maize transformation experiments. In these constructs, Ω represents for the tobacco Mosaic Virus translational enhancer, Pr1a SP for the sequence encoding the tobacco pathogenesis-related protein 1a signal peptide for apoplast-targeting of the E1 enzyme, Nos for the polyadenylation signal of nopaline synthase, and bar for the herbicide resistance seuences.

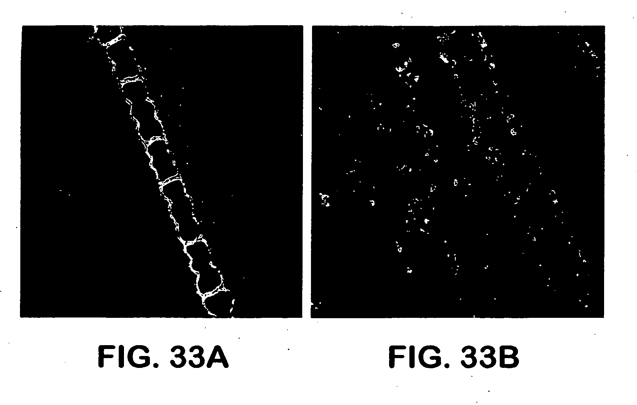

Figure 33 shows immunoflourescent confocal laser microscopy of apoplast-targeted E1 transgenic maize leaf tissue (left) using the E1 primary antibody and the FITC anti-mouse secondary antibody. Photo on right is leaf tissue from an untransformed control maize plant.

Figure 30 is a Western blot of transgenic maize plants (1 µg total soluble protein) expressing E1-cd. Lanes +C, positive transgenic tobacco control; -C, untransformed maize control; 1, 2, 5 and 6 transgenic maize plants.

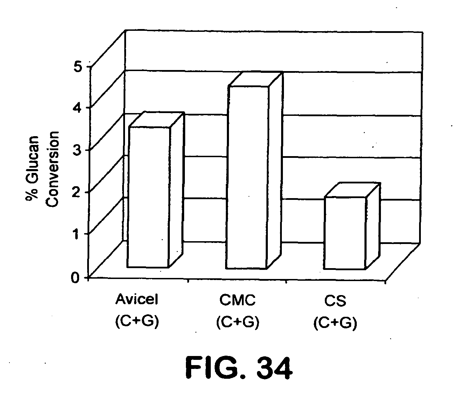

Furthermore, the E1 enzymes produced within the biomass was also biologically active and could convert AFEX pretreated corn stover, and commercially available Avicel and CMC into glucose (Figure 34). Glucan converted into glucose via the enzymatic hydrolysis of pretreated corn stover (CS), Avicel and CMC using maize-produced E1. Comparison of percentage of conversion to glucan is shown.

Transformation and heterologous enzymes targetting in maize cell compartments is a routine in our laboratory. We have also genetically engineered maize with three different polyhydroxybutyrate pathway key enzyme coding sequences while targetting these enzymes to maize chloroplasts (Fig. 7; Zhong et al, 2003).

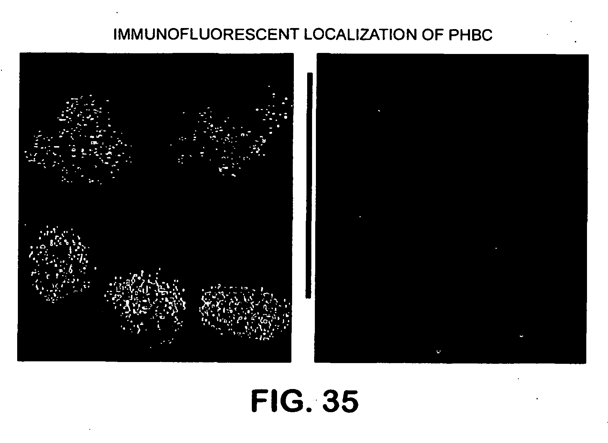

Figure 35 shows immunoflourescent confocal laser microscopy of chloroplast-targeted polyhydroxybutyrate C in transgenic maize leaf tissue (left) using the PHBC primary antibody and the FITC secondary antibody. Photo on right is leaf tissue from an untransformed control maize plant.

With our work on E1 transgenic rice and corn, there are three possible explanations why the heterologous E1 accumulated in apoplast did not harm transgenic plant cell walls. First, lignocellulose is difficult to hydrolyze because it is associated with hemicellulose, and surrounded by a lignin seal, which has a limited covalent association with hemicellulose. Moreover, cellulose has a crystalline structure with a potential formation of hydrogen bonds resulting in a tightly packed structure with less access to hydrolytic enzymes. Second, cellulases function in a synergistic enzyme complex. If only one enzyme of the complex is expressed such as E1, this single enzyme should not be sufficient to significantly affect the integrity of the cell wall by itself. Third, due to the thermophilic nature of the E1, the enzyme has limited activity under plant in vivo temperature.

We have accumulated the thermostable E1 in apoplast rather than keeping the enzyme in cytosol, where it is produced. There are two advantages associated with production of the E1 and other heterologous enzymes outside of cytosol. First; the foreign enzyme will not harm the cell by interfering with its cytosolic metabolic reactions. Second, the enzyme can accumulate at a much higher level in these compartments as compared to its production in cytosol (i.e. maximum of 0.1-0.3% of plant total soluble proteins). For example, E1 was accumulated in apoplast of transgenic *Arabidopsis* as high as 26% of plant total soluble proteins (Ziegler, M. T., Thomas, S. R. & Danna, K. J. (2000). Accumulation of a thermostable endo-1,4-β-D-glucanase in the apoplast of Arabidopsis thaliana leaves. Mol. Breed. 6, 37-46).

### EXAMPLE 15

Despite the very successful efforts on reducing the costs of production of fermentation tank microbial hydrolysis enzymes, the relatively high costs of these enzymes are still a barrier to commercial conversion of biomass into biofuel ethanol. Also, to compete economically with petroleum refineries, fuel ethanol "biorefineries", especially those based on lignocellulosic materials must make higher value, lower volume products. These high value products will significantly improve profit margins and thereby make attractive the very large capital investments required for ethanol biorefineries. Valuable proteins such as industrial enzymes including the hydrolysis enzymes produced within the plants themselves using genetic engineering would fit quite naturally into an ethanol biorefinery and might be recovered before or during the biorefining process. Industrial enzymes are worth at least ten times as much as ethanol product (per unit mass). The present invention provides these enzymes within the maize biomass cells in their biologically active form at a level sufficient for such conversion.

Maize biomass cell walls are composed of approximately 36.1% cellulose, 29.2% hemicellulose (including 21.4% xylan, 3.5% Arabinan, 2.5% galactan and 1.8% mannan) and 17.2% lignin. This means that to convert most of the maize lignocellulosic matter, one might need to hydrolyze the plant biomass with cellulase, hemicellulase, and ligninase enzymes. Three Cellulases including endoglucanase, exoglucanase and the β-glucosidase are involved with hydrolysis of cellulose. Similar combination of hemicellulases is involved with conversion of hemicellulose into fermentable sugars. Our team has already produced biologically active thermostable Acidothermus cellulolyticus endo-1, 4-β-D-glucanase (E1) in maize biomass at a level sufficient as compared to the amount of commercial endoglucanase presently added to the plant biomass for glucan conversion. Work is also in progress our laboratory for production of Trichoderma reesei exoglucanase (CBH1) in maize biomass, as CBH1 plays a synergistic effect on E1 for cellulose hydrolysis. Because CBH1 might not sustain its biological activity in transgenic plants, we are examining the effect of a histidine-tag and a molecular chaperon on activity of CBH1 in tobacco, and then in maize to test the easy purification and correct protein folding of this enzyme. With collaboration of Dr. Bruce Dale of Michigan State University, we also found that about 2/3 of the E1 enzymatic activity in transgenic plants is lost due to Ammonia Fiber Explosion (AFEX) pretreatment (Teymouri F., H. Alizadeh, L. Laureano-Preze, B. Dale, and M. B. Sticklen (2004). Effects of Ammonia fiber explosion (AFEX) on the activity of heterologous cellulose enzyme. Applied Biochemistry and Biotechnology. 16: 1183-1191; Dale BE., Leong CK., Pham TK., Esquivel V. M., Rios I., and Latimer V. (1996). Hydrolysis of lignocellulosics at low enzyme levels: Application of the AFEX Process. Biosource Technology. 56: 111-116). However, separation of E1 in transgenic plant total soluble proteins and addition of such protein to AFEX pre-treated dry biomass could replace the need for commercial E1 (Oraby H., Ransom C., Venkatesh B., Dale B., and Sticklen M. B. (2005). High level production of biologically active thermostable Acidothermus cellulolyticus endo-1, 4-β-D-glucanase (E1) in rice biomass converting maize and switchgrass biomass into fermentable sugars. Plant Biotech. Ready for submission).

All hydrolysis enzymes in their active forms are produced in the same or in different maize plant biomass, and these plant made enzymes are used for biomass conversion. The *Butyrivibrio fibrisolvens* β-glucosidase (Yao, ibid, 2004), *Cochliobolus carbonum* endoxylanase (Apel P.C., Panaccione D.G., Holden FR., J.D. Walton (1993) Cloning and targeted gene disruption of XYL1, a β-1, 4-xylanase gene from the maize pathogen Cochliobolus carbonum. Mol Plant-Microbe Interact 6: 467-473), and the white rot filamentous *Phanerochaete chrysosporium* ligninase (de Boer H.A., Zhang Y.Z., Collins and C.A. Reddy (1988). Analysis of nucleotide sequences of two ligninase cDNAs from a white-rot filamentous fungus, Phanerochaete chrysosporium. Gene: 69(2):369) are produced in maize biomass. Each of these enzymes are produced separately in maize, and then plants expressing the highest level of each biologically active enzyme are cross bred to produce some or all of the above in one plant without any apparent effect on transgenic plants growth and development. In the case where enzyme combinations cause damage to the maize plant cell walls, each heterologous enzyme can be isolated (in plant total soluble proteins) to be added to the biomass after pretreatment. The *Arabidopsis* Flowering Locus C (*flc)* is used in maize for delay in flowering and increase in biomass as was observed in FLC-transgenic tobacco (Salehi H., Ahmad R., Ransom C., Kravchenko A., and Sticklen M.B. B. (2005b). Convergence of goals: Expression of flowering locus C in an Acidothermus cellulolyticus endo-1, 4-β-D-glucanase producing tobacco (Nicotiana tobacum L.) for delay in flowering, increase in biomass and phytoremediation of lead. Appl. Biochemistry & Biotechnology. Submitted). The delay in flowering can reduce cross contamination of transgenes with other maize plants in the field, should maize be planted in different fields at more or less the same time

Petroleum refineries make very large-scale, low cost products (gasoline, diesel fuel, kerosene, etc.) that provide the economic "muscle" to compete in the marketplace while their profitability is greatly enhanced by higher value-lower volume products such as chemicals, plastics and solvents. If we are to develop "biorefining" industries based on renewable plant materials, we must simultaneously develop large-scale products such as fuel ethanol along with higher value-lower volume products such as industrial enzymes including hydrolysis enzymes. Protein co-production with biofuels will help make biorefineries more economically competitive and will lay the foundation for replacing much of our imported oil (at a cost of 6 million barrels per day imported at an average cost of $20 per barrel) with home grown fuels such as ethanol.

Our team has produced the microbial endoglucanase in maize biomass at the level comparable to the need for conversion. The inventor, along with Dr. Bruce Dale at Michigan State University has discovered that the E1 enzyme produced within the plant loses 2/3rd biological activity during the AFEX pretreatment conditions (Teymouri *et al. ibid*, 2004)*.* Therefore, in one embodiment of the present invention the hydrolysis enzymes are produced within the plants, extracted as total soluble proteins, stored under ideal conditions, and added to the lignocellulosic matter after pretreatment for the enzymatic hydrolysis of the biomass matter.

*Flc* in transgenic maize: The inventor have recently transferred the *Arabidopsis thaliana* Flowering Locus C (*flc*) gene to a non-early flowering tobacco and found up to 36 days of delay in flowering, fertile plants, and an increase in plant biomass as compared to non-transgenic plants (Salehi H., Seddighi, Z., C. Ransom, H. Oraby, R. Ahmad and M. Sticklen (2005a). Convergence of goals: Expression of flowering locus C in an Acidothermus cellulolyticus endo-1,4-β-D-glucanase (E1) transgenic tobacco (Nicotiana tobacum L.) and its effect on delay in flowering, increase in biomass and phytoremediation. Applied Biochemistry and Biotechnology Submitted.). Maize is an open pollinated crop that when genetically transformed, requires at least one method of bioconfinement to assure that its pollen grains will not transfer transgenes to other maize plants in the field (CRC Report, 2004). This *flc* gene encodes a novel protein that acts as a repressor of flowering. It has been cloned, characterized and confirmed to delay in flowering by about four weeks after it was transferred to an early-flowering Arabidopsis. Also, when this gene was null mutated, plants gave early flowering (Michaels S. and Amasino R., (1999). Flowering Locus C encodes a novel MADS domain protein that acts as a repressor of flowering. Plant Cell. 11(5): 949-956). Herein, the *flc* gene is transferred to maize (along with hydrolysis and bar genes) to meet *al*l or some of the bioconfinement of transgene flow requirements. The *flc* gene can work in maize as it did in early-flowering Arabidopsis and non-early flowering tobacco (Salehi *et al., ibid*, 2005). This delay in flowering (Van-Esbroeck G., Hussey M., and Sanderson (1998), Selection response and developmental basis for early and late panicle emergence in Alamo switchgrass. Crop Sci. 38 (2): 342-346) increases the vegetative production and therefore an increase in plant biomass, as it did in transgenic tobacco (Salehi *et al, ibid*, 2005).

Maize can be genetic engineered using the Biolistic® gun bombardment of multi-meristem primordia as well as the immature embryo-derived maize cell lines. The genetically engineered plants can be biologically confined to block or reduce the transfer of transgenes to their cross breedable field crops via pollen transfer (Kirk TK, Tien M., Kersten PJ., Mozouch MD., and Kalyanaraman B. (1986). Ligninase of Phanerochaete chrysosporium. Mechanism of its degradation of the non-phenolic arylglycerol beta-aryl ether substrate of lignin. Biochem. J. 236: 279-287). One method to accomplish this is to delay the flowering of transgenic plants by 3-4 weeks to bypass the flowering of other maize plants in the field as this is the window of time that maize plants flower in one geographic area when maize seeds are planted in different fields at more or less the same time. For example, we have used this method in tobacco via the transfer of *Arabidopsis flc* transgene. The delay in flowering also caused a significant increase in plant biomass (Salehi et *al.* ibid, 2005a, Salehi *et al,* ibid, 2005b).

### Background

Genetic Transformation of plants with hydrolysis enzymes. Northwest National Laboratory and the University of Wisconsin, and University of Colorado and NREL have produced E1 cellulase in alfalfa, potato, tobacco, and Arabidopsis (Dai Z.; Hooker B.S.; Queensberry R.D. and Gao J. (1999). Expression of Trichoderma reesei Exocellobiohydrolase I in transgenic tobacco leaves and calli. Applied Biochemistry and Biotechnology 77/79: 689-699; Ziegler, M., Thomas, S., and Danna, K. (2000). Accumulation of a thermostable endo-1,4 β-D-glucanase in the apoplast of Arabidopsis thaliana leaves. Mol. Breed. 6: 37-46 ; Ziegelhoffer, T., Raasch, J., and Austin-Phillips, S. (2001). Dramatic effects of truncation and sub-cellular targeting on the accumulation of recombinant microbial cellulase in tobacco. Mol. Breed. 8: 147-158, and Prodigene® (College Station, Texas) produced E1 in maize seeds. With the collaboration of Dr. Bruce Dale of MSU, the biological activity of E1 in transgenic tobacco after Ammonia Fiber Explosion (AFEX) pretreatment was tested and it was found that the enzyme loses two-thirds of its activity during AFEX (Teymouri *et al*., ibid, 2004). Therefore, the cellulases can be produced in biomass plants, and separated as total soluble proteins before adding to the lignocellulosic matter after the pretreatment. The biologically active *Acidothermus cellulolyticus,* endoglucanase has also been successfully produced by us in rice and maize biomass at a relatively high level (Oraby *et al*., ibid, 2005).

The following steps are involved in making the transgenic plants of the present invention. First, construct plasmids and bombard the plasmids containing the E1 and CBH1 into corn to produce typically at least 30 independent transgenic lines for each gene. Second, regenerate the R₀ and R₁ transgenic plants, and confirm the integration and expression of transgenes. Third, the localization of E1 and CBHI in transgenic maize cells is examined using immunofluorescent and laser microscopy. Fourth, the production level and activity of E1 and CBH1 in transgenic maize plants is determined. Fifth, AFEX (Ammonia Fiber Explosion) pretreatment is performed on transgenic tobacco and maize and then test for retention of cellulase (E1 and CBHI) activity.

These five steps have been successfully accomplished for genetic transformation of the *Acidothermus cellulolyticus* E1 as described below. The same is performed for the *Trichoderma reesei* CBH1 transgenesis in corn. We have used the *Acidothermus cellulolyticus* thermostable E1-transgenic tobacco to test whether this enzyme can sustain its biological activity during the mildest pretreatment conditions (i.e. Ammonia Fiber Explosion (AFEX) system). We discovered (Teymouri *et al.,* ibid, 2004) that this *Acidothermus cellulolyticus* E1 in E1-transgenic plants lost two-thirds (2/3^{rd}) of its biological activity during AFEX pretreatment. Therefore, we decided to separate E1 as total soluble proteins and then add it to the maize straw after AFEX pretreatment.

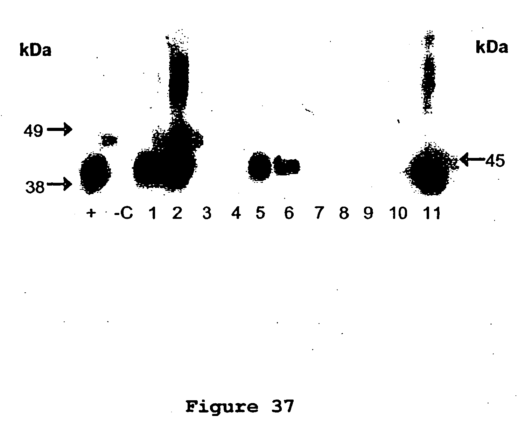

As rice is much easier and quicker than maize to transform, we first transformed rice with of E1 and the bar herbicide resistance genes, then transformed maize with the same transgenes. Figure 29 and Figure 37 confirm the integration and translation of microbial E1 in maize lines. Furthermore, Figure 33A and Figure 33B, in addition to Table 14 below show the localization of the E1 enzyme in maize leaf cells (Figure 33A and Figure 33B) and the biological activity of E1 in maize, rice and tobacco leaf biomass (Table 14).

Cross breeding of E1 transgenic maize: We have made self and crosses between E1 transgenic plants and the control untransformed to test the stability of transgene expression and enzymatic activity in future generations. T1 seeds have been collected for testing.

Preliminary work on apoplast localization of E1 in transgenic maize: Based on our previous experience on localization studies of other gene products (polyhydroxybutyrate) in maize via confocal microscopy, we used E1 primary and a corresponding secondary antibody and performed localization of E1 in transgenic plant apoplast. One sample showed possible localization of E1 in apoplast (Figure 33A and Figure 33B). Most samples showed strong non-specific binding of the fluorescence conjugate to plant tissues, however non-specific binding can be blocked using blocking agents.

Level of E1 production and its biological activity in transgenic plants: We measured the E1 enzyme produced and its biological activity in transgenic maize and compared with T₄ tobacco and To rice. The production levels and their activities varied among different transgenic lines due to the transgene position effect. In maize, we obtained E1 up to 9.07%; in rice up to 24.13% and in tobacco up to 3.8% of the plant total plant soluble proteins (see Table 14 below).

**Table 14: E1 enzymatic activity and percentage of E1 in total soluble proteins in To maize, T₄ tobacco and To rice. Maize lines are the same shown in the Western blot of Figure 37.**

| Plant lines | Activity (nmol/µg/min) | % E1 in total soluble proteins |
|---|---|---|
| + transgenic tobacco | 1.521 | 3.8 % |
| Control maize | 0.00 | 0.00 |
| Maize 1-1 | 0.1094 | 0.261 % |
| Maize 1-2 | 3.629 | 9.07 % |
| Maize 1-4 | 0.0798 | 0.199 % |
| Maize 1-6 | 0.0735 | 0.184 % |
| Maize 1-10 | 0.0124 | 0.0309 % |
| Maize 1-11 | 0.186 | 0.465 % |
| Maize 1-13 | 0.0331 | 0.0827 % |
| Maize 2-3 | 0.0727 | 0.182 % |
| Rice 8 | 9.654 | 24.134 % |

The hydrolysis enzymes in their active forms can be produced in the same or in different maize plant biomass to use for biomass conversion. Producing hydrolysis enzymes within the plant biomass can lead to cheaper enzyme production. *Acidothermus cellulolyticus* thermostable E1-transgenic tobacco was used to test whether this enzyme can sustain its biological activity during the mildest pretreatment conditions (*i.e*. Ammonia Fiber Explosion (AFEX) system). We discovered (Teymouri *et al*., ibid, 2004) that this *Acidothermus cellulolyticus* E1 in E1-transgenic plants lost two-thirds (2/3^{rd}) of its biological activity during AFEX pretreatment. Therefore, we decided to separate E1 as total soluble proteins from transgenic plants, and add to the maize straw after AFEX pretreatment.

New maize seeds are germinated and grown in greenhouses to maturity, then the immature embryos are collected and immature embryo-derived cell lines are produced *in vitro* for Biolistic® bombardment. New shoot apical meristem primordia are produced, multiplied by thousands through biweekly subculture for bombardment via the Biolistic® method as described in Zhong H., F. Teymouri, B. Chapman, S. Maqbool, R. Sabzikar, Y. E1-Maghraby, B. Dale, and M. B. Sticklen. (2003). the dicot pea (Pisum sativum L.) rbcS transit peptide directs the Alcaligenes eutrophus polyhydroxybutyrate enzymes into the monocot maize (Zea mays L.) chloroplasts. Plant Sci. 165: 455-462, Zhang H., D. Warkentin, B. Sun, H. Zhong, and M. B. Sticklen (1996). Variation in the inheritance of expression among subclones for unselected (uidA) and selected (bar) transgenes in maize (Zea mays L.). Theoretical and Applied Genetics. 92: 752-761. The system of shoot apical multimeristem bombardment, reproduction, and propagation has previously been developed for maize nuclear transgenesis as described also in U.S. Patent Nos. 5,281,529; 5,320,961; 5,767,368 to Zhong et al.*,* hereby incorporated herein by reference.

A set of plasmids is constructed containing: (1) *Cochliobolus carbonum* endoxylanase (Apel *et al.,* 1993), (2) *Butyrivibrio fibrisolvens β-*glucosidase (Yao, 2004), and (3) the white rot filamentous *Phanerochaete chrysosporium* ligninase (de Boer et *al.,* ibid, 1988). Each enzyme gene is regulated by the 35S promoter, tobacco mosaic virus translational enhancer, and the sequences encoding the tobacco pathogenesis-related protein 1a for the targeting of each of the enzyme gene products into the plant apoplast. In the plant apoplast, the pH is acidic and falls between approximately pH 5 to pH 5.5. The ideal pH for enzymatic activity of the enzymes fall between approximately pH 4.5 to pH 5.2. Therefore, the apoplast is an ideal storage location for sustaining the enzymatic activity of each of the enzymes.

The maize immature embryo-derived cell lines and multi-meristem primordia are co-transformed with each of the above three constructs along with another construct (for example pGreen) containing the *bar* herbicide resistance selectable marker and the *Arabidopsis* Flowering Locus C (FLC) genes in a manner that each plant will express one of the three hydrolysis genes, *bar* and *flc.* Next, greenhouse grown transgenic plants are produced from the co-transformed maize. The integration, copy number, transcription and translation of the above transgenes is confirmed in the transgenic plants by methods well known in the art. The delay in flowering and increase in the biomass of FLC-transgenic plants is then measured. The level of each of the heterologous enzymes is identified as percent of plant total soluble protein. The enzymatic activity of each of the three proteins in each of the transgenic plant lines is measured using specific substrates by enzymatic assay methods well known in the art. The localization of each of the three heterologous protein enzymes within the plant cells is confirmed via the use of specific primary and secondary antibodies followed by confocal microscopy as described for PHB-transgenic maize (Zhong *et al*., ibid, 2003) and E1 transgenic maize.

Thus, the procedure to provide the transgenic plants of the present invention include: (1) production of highly regenerable multi-meristems and immature embryo callus lines for bombardment; (2) development of three constructs, containing the xylanase, β-glucosidase and ligninase transgenes; (3) co-transformation of 5-10 independent transgenic maize lines, each containing a combination of bar selectable marker and flc, and either the β-glucosidase, xylanase or ligninase transgenes; (4) studying gene integration and transgene copy number via Southern blots, and gene expressions via northern and western blots; (5) studying translation level, enzymatic activity and localization of enzymes in cell maize cells; (6) Studying delays in flowering and increase in biomass of FLC-transgenic maize; and (7) self breeding, and cross breeding of To plants that show the highest level and activity of heterologous enzymes with E1 transgene plants.

### Methods:

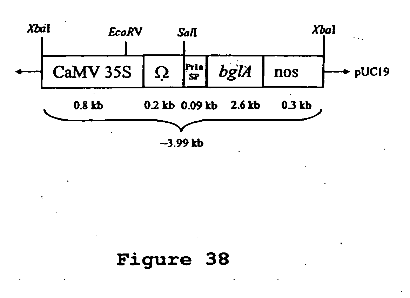

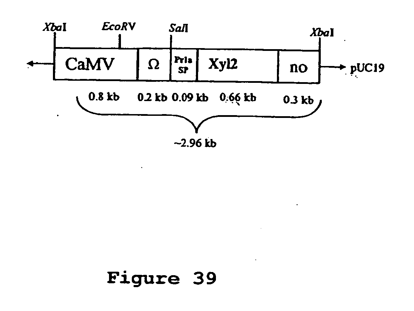

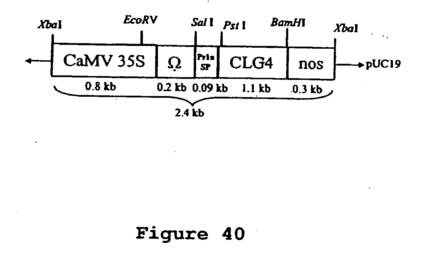

**Plasmid Constructions.** Construction of plasmids containing each of the β- glucosidase, xylanase and ligninase genes. Develop three new plasmid constructs (Figure 38, Figure 39 and Figure 40); containing the β-glucosidase (Figure 38), xylanase (Figure 39), and the ligninase (Figure 40) coding sequences regulated by the same regulatory sequences as used to produce E1-transgenic maize.

Figure 38 illustrates a schematic of the plasmid containing the *Butyrivibrio fibrisolvens* β-glucosidase (Yao JQ (2004). Genetic Transformation of Tobacco with a Beta-Glucosidase Gene to Induce Constitutive Systemic Acquired Resistance Against Tobacco Mosaic Virus. Ph. D. dissertation. Department of Biological Sciences, Western Michigan University) cDNA regulated by the 35S promoter and enhancer. This construct also contains the sequences encoding the tobacco pathogenesis-related protein 1a (Prla) signal peptide for targeting of β-glucosidase enzyme into plant apoplast. **CaMV 35S**: Cauliflower Mosaic Virus 35S Promoter. Ω: Tobacco Mosaic Virus Q translational enhancer. **Prla SP**: the sequence encoding the tobacco pathogenesis-related protein 1a (Prla) signal peptide. **bglA**: *Butyrivibrio fibrisolvens* β-glucosidase (Lin L., E. Rumbak, H. Zappe., JA Thompson and D. R. Woods (1990). Cloning, sequencing and analysis of expression of a Butyrivibrio fibrisolvens gene encoding a β-glucosidase. The J. Gen. Microbiol. 136: 1567-1576).

Figure 39 illustrates a schematic of the plasmid containing the xylanase cDNA regulated by the 35S promoter and enhancer. This construct contains the sequences encoding the tobacco pathogenesis-related protein 1a (Prla) signal peptide for targeting of *Cochliobolus carbonum* endoxylanase (Apel *et al.,* ibid, 1993) into plant apoplast. Abbreviations: **CaMV 35S**: Cauliflower Mosaic Virus 35S Promoter; **Ω**: Tobacco Mosaic Virus translational enhancer; **Pr1a SP:** the sequence encoding the tobacco pathogenesis-related protein 1a (Prla) signal peptide; **Xy12:** *Cochliobolus carbonum* endoxylanase cDNA.

Figure 40 illustrates a schematic of the plasmid containing the *Phanerochaete chrysosporium* ligninase (de Boer *et al*., ibid, 1988) gene regulated by the 35S promoter and enhancer. Abbreviations in pMZ766E1-cat: **CaMV 35S:** Cauliflower Mosaic Virus 35S Promoter; **Ω**: Tobacco Mosaic Virus Q translational enhancer; **Pr1a SP**: the sequence encoding the tobacco pathogenesis-related protein 1a (Prla) signal peptide; **CLG4:** *Phanerochaete chrysosporium* ligninase; **nos:** polydenylation signal of nopaline synthase.

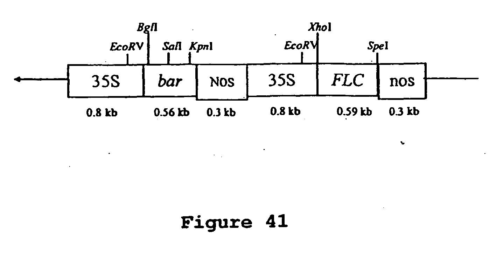

Construct for co-transformation with the bar herbicide resistance gene and *flc* for delay in flowering and an increase in plant biomass: The pGreen, as illustrated in Figure 41, was obtained from Dr. Richard Amasino of University of Wisconsin and was used in our research on tobacco (Salehi *et al.,* ibid, 2005). pGreen is one example of a plasmid that can be used to cotransform maize with the constructs described above. Figure 41 illustrates a schematic of the plasmid pGreen which contains the *Arabidopsis* Flowering Locus C (FLC) coding sequences regulated by 35S promoter and Nos terminator. It also has the *bar* herbicide resistance selectable marker regulated by 35S promoter and Nos terminator. Abbreviations in pGreen: *FLC: Arabidopsis Flowering Locus C* coding region; 35S: CaMV 35S promoter; *bar*: phosphinothricin acetyltransferase coding region.

Production of maize immature embryo-derived cell lines and the apical shoot multi-meristem primordia for genetic transformation: Maize seeds can be germinated and plants grown in greenhouses to maturity. Immature embryos are collected and cultured in Murashige and Skoog (Murashige T. and Skoog F. (1962). A revised medium for rapid growth and bioassay with tobacco tissue culture. Physiol. Plant. 15: 473-497) medium supplemented with appropriate growth regulators for proliferation of embryogenic cell lines as performed for production of E1 in maize. Maize apical shoot multi-meristem primordia can also be developed as previously published (Zhang *et al,* ibid, 1996; Zhong *et al*., ibid, 2003).

Maize cell lines are co-transformed via the Biolistic® gun. Embryogenic cell lines and multi-meristems are bombarded with tungsten particles coated with each plasmid and the plasmid containing bar and FLC genes. The bombarded explants are gently transferred onto selection medium containing 6-10 mg/L glufosinate ammonium (PPT) selectable chemical for another six to eight weeks. Chemically selected multi-meristems are further multiplied in selection medium for another three to four months, and the selected immature embryo cells are regenerated into somatic embryos, and germinated into plantlets in appropriate media containing the same concentration of PPT. The selected multi-meristems are rooted in vitro, and seven to ten centimeter plantlets produced from shoots or cell lines are transferred to pots, acclimated, and transferred to maize greenhouses where they are grown to maturity, and T₁ seeds are collected.

**Confirming integration, copy number and expression of transgenes in maize plants :**

**PCR test:** When the selected herbicide resistant plantlets are too many in number for Southern blot analyses, the Polymerase Chain Reaction (PCR) can be used to confirm the presence of the foreign genes in the plants. The shoots and/or plantlets which test positive will be considered as putatively transformed for further molecular analysis.

**Southern blot**: To find the copy numbers of transgenes in plants, genomic DNA is isolated from greenhouse-grown putatively transgenic and control (untransformed) plants, then Southern blot analysis is performed following our routine modification of Southern's method as we performed for E1 transgene.

**Northern blot**: To confirm the transcription of transgenes, total cellular RNA is isolated from plant tissues. The mRNA coding the foreign genes is detected by RNA blot analysis using the same probes used in Southern blot hybridization, as above. The mRNA is electrophoresed on a denaturing formaldehyde agarose gel, transferred to nitrocellulose or nylon filters, hybridized with the appropriate probe, and then exposed to X-ray film. After exposure of the probed RNA-containing filter to X-ray film, the hybridization bands can be scanned using a densitometer to determine the levels of specific mRNA present as performed for the transcription of E1 in transgenic maize.

**Western blot:** Primary polyclonal antibodies can be commercially custom-raised using synthetic peptides. Western blots are performed to find the translation of each transgenes in transgenic plants as performed for E1 heterologous protein.

**Identify the level of heterologous enzyme production and the biological activity of each enzyme produced in maize plants.** The E1 enzymatic activity and percentage E1 in total soluble proteins in transgenic maize, tobacco and rice has been measured as shown in Table 14. The β-glucosidase biological activity can be performed as described in Fisher, K. and Woods, J. (1999) Determination of β-glucosidase enzymatic function of Histoplasma capsulatum H antigen using a native expression system. Gene: 247 (1-2):191-197. Briefly, supernatants and column elute are electrophoresed in polyacrylamide gels, followed by a thirty minute room-temperature gel wash in enzyme buffer (20 mM Tris-HCl plus 0.6 mM CaCl₂, pH 8.0) and a 37°C incubation with the β-glucosidase substrate *p*-nitrophenyl- β-D-glucopyranoside (PNPG) (Sigma-Aldrich®, St. Louis, MO). Areas of substrate hydrolysis indicating β-glucosidase enzyme activity are visualized. Individual substrate gels are scanned with an scanner, such as an Agfa® brand (Agfa-Gevaert Aktiengesellschaft, Germany) scanner.

Xylanase activity is measured as described by Apel *et al.,* ibid, 1993. Briefly, the PAHBAH protocol is used. That is, four volumes of 0.5N NaOH is mixed with one volume of 5% PAHBAH stock including the p-hydroxybenzoic acid hydrazide (Catalog no. H-9882, Sigma-Aldrich®, St. Louis, MO) in 0.5 M HCl (conc. HCl = 12M; 300 ml 0.5 M HCl = 12.5 ml conc. HCl) and store for up to one month at 4°C. A standard curve is used (galacturonic acid H₂O MW 212.16. 100 ml of 10 mM = 0.21 g, or glucose for more intense reaction). The reaction mix is incubated at 37°C and at each time point (0 and 30 min), 25 µl is withdrawn and added to 1.5 ml of PAHBAH mixture in a 13×100 glass culture tube. The tube is then mixed by flicking or vortexing. Heat the sample to 100°C for ten minutes and allow it to cool. Then will read absorbance at 410 nm. For large number of samples (*e.g*. HPLC fractions), a reaction mixture can be made of acetate buffer, substrate and water and then aliquoted in microcentrifuge tubes. The volume of enzyme solution sampled initially and the volume sampled after incubation can be increased if necessary.

Ligninase biological activity is performed as described by Kirk TK, Tien M., Kersten PJ., Mozouch MD., and Kalyanaraman B. (1986). Ligninase of Phanerochaete chrysosporium. Mechanism of its degradation of the non-phenolic arylglycerol beta-aryl ether substrate of lignin. Biochem. J. 236: 279-287. The reaction buffer will contain a mixture of 2.2 ml sodium tartrate buffer (50 mM, pH; 405, 40 mM veratryl alcohol (2 mM) and 240 µl of ligninase transgenic plant total soluble proteins. The reaction will be initiated by adding 20 µl H₂O₂ (0. 2 mM). The absorbance will be measured immediately with an extinction coefficient ∈₃₁₀ = 9333 M⁻¹cm⁻¹. The activity will be defined as the quantity of ligninase enzyme that produces 1 µmol of oxidized product.

**Study of apoplast localization of heterologous enzymes in transgenic maize.** As was previously confirmed for the localization of three polyhydroxybutyrate enzymes (PHB) in maize chloroplasts (Zhong *et al*., ibid, 2003) and the localization of E1 in transgenic maize apoplast (Figure 33A and Figure 33B), commercially raised antibodies can be used against each of the enzymes, and immunofluorescent antibody staining can be performed to determine the localization of the xylanase, ligninase and β-glucosidase in transgenic maize cells. The transgenic leaf section samples are incubated in a solution containing the primary antibody against the xylanase, ligninase or the β-glucosidase. After washing to remove unbound antibodies, the tissues are incubated in the secondary antibody-fluorophore conjugate, which is a goat anti rabbit IgG-Alexa conjugate. After washing to remove unbound antibody-conjugate, the tissues will be mounted on microscope slides and viewed with a laser scanning confocal fluorescence microscope is used to determine the localization of these heterologous enzymes in the transgenic plants.

Confirmation of the FLC heterologous protein production and its effect on delay in flowering and increasing fresh and dry plant biomass production: The FLC has previously been expressed in transgenic tobacco and it was confirmed that this gene could delay flowering up to 36 days while increasing the fresh and dry biomass in the absence of and in the presence of the E1 transgene (Salehi *et al.,* ibid, 2005b; Salehi *et al.,* ibid, 2005b). The expression of FLC in maize is determined via Western blotting. The days for delay in flowering can be then counted as compared to the control plants. Measurements of the plant fresh and dry biomass, thousand seed weight and seed yield (gram per plant) can be taken.

Self breeding and cross breeding is started with transgenic plants showing the highest level and activity of E1 with hemicellulase, ligninase and β-glucosidase transformants. Self or cross breeding is performed with plants for the production of second generation plants, and testing is performed to determine whether combination of enzymes in plants have an effect on plant growth and development. Maize breeding has been described in Zhang *et al*, ibid, 1996 and Zhong *et al,* ibid, 2003.

Lignocellulosic biomass is composed of crystalline cellulose embedded in a hemicellulose and lignin matrix. The pretreatment methods are presently used to disrupt the lignicellulosic matter, and to mostly remove the lignin to allow the access of cellulose to cellulases. Plant genetic engineering can decrease lignin and/or change the composition of lignin for less need of expensive and harsh pretreatments. Plant genetic engineering can also produce microbial ligninases within the biomass crops, so the lignin content of biomass could be deconstructed during or before bioprocessing. There are three different groups of cellulases working in concert to convert cellulose into glucose. These enzymes include endoglucanase, exoglucanase and the β-glucosidase. Plant genetic engineering has been successfully used to produce these enzymes in plants. There might be ways to increase biomass via plant genetic engineering. These include genetic manipulation of plant growth regulators or photosynthetic pathways. Delay in flowering also can increase plant biomass.

**Production of cellulase enzymes within the crop biomass:** As an alternative to its production in microbial tanks, it has been recommended to produce these enzymes within the crop biomass (Sticklen, M, Teymouri, F, Maqbool, S, Salehi, H, Ransom C, Biswas, G, Ahmad, R, and Dale, B: Production of microbial hydrolysis enzymes in biomass crops via genetic engineering. 2nd International Ukrainian Conference on Biomass for Energy 2004, p. 133, 20-22). The apoplast targeting of the translation product of catalytic domain of the gene coding for thermostable *Acidothermus cellulolyticus* 1,4-endoglucanase E1 enzyme in *Arabidopsis* tobacco and potato plants demonstrated the possibility of producing this enzyme, in case of *Arabidopsis* at up to 25% plant total soluble proteins. Recently, the inventor's team constitutively expressed the catalytic domain of the A. *cellulolyticus* 1,4-endoglucanase E1 in rice (Oraby et *al.* unpublished) and maize in an apoplast targeting manner. The amount of E1 enzyme produced in rice and maize leaves respectively accounted for up to 4.9% and 2% of the plant total soluble proteins, and the E1 accumulation had no apparent deleterious effects on plant growth and development. Furthermore, when the crude extract of rice total soluble proteins was added to Ammonia Fiber Explosion (AFEX) pretreated rice straw and maize stover, Approximately 30 and 22% of the cellulose of these plants were respectively converted into glucose (Oraby *et al*. unpublished, Ransom *et al*: unpublished).

Initially, there were three concerns associated with production and use of the cellulase enzymes within the crop biomass. The first concern was whether the harsh conditions (acid, alkaline and/or heat.) of pretreatment would destroy the biological activity of these enzymes. The second concern was whether sufficient enzymes could be expressed within the biomass to convert polysaccharides into fermentable sugars without the need for the use of commercial enzymes. The third concern was whether increasing the level of production of these heterologous enzymes within the plant cells would cause harm to plant growth and development.

To address the first concern, the inventor's team used the mildest method of pretreatment *i.e*. Ammonia Fiber Explosion (AFEX) on the thermostable A. *cellulolyticus* E1-producing tobacco biomass. In this experiment, about 2/3^{rd} of the activity of this heterologous enzyme was lost. Therefore, the conclusion was to extract the heterologous enzyme in crude or pure forms, and then add to the pretreated matter for production of fermentable sugars. In a follow up study, up to 30% of rice and 22% of maize cellulose were respectively converted into glucose, when the rice-produced E1 was extracted in crude form, stored under freeze condition for 3 months and then added to the AFEX pretreated matter.

To address the second issue, it is possible to increase the level of gene expression by regulating the transcriptional, posttranscriptional and posttranslational factors. However, the increase in production of heterologous proteins is best possible by targeting these proteins from cytosol for accumulation into non-cytosolic cell compartments (Hood EE: Bioindustrial and biopharmaceutical products from plants. In: New directions for a diverse planet: Proc 4th Intl Crop Sci Congress, Brisbane, Austria, Sept. 26-Oct 1, 2004. ISBN 1 920842 20 9). This by itself would address the third concern because the enzyme accumulation inside these compartments will not interfere with the plant cytosolic metabolic activities.

Another advantage is that there are distinct molecular chaperon systems in targeted compartments to translocate or fold proteins. However, carefully addressing the targeting of heterologous proteins to cell compartments is very important because factors influencing transcription and translation efficiency, recombinant protein accumulation as well as the protein stability strongly depend on the compartment of the plant cell chosen for accumulation.

The question has been asked about the reasons that the E1 heterologous cellulase targeted and accumulated in the apoplast of rice, maize and other plants did not harm the plant cell wall cellulose. The answer is that it might be due to a combination of three reasons. First, heterologous E1 enzyme does not have a direct access to the plant cellulose because cellulose is in a compact mixture along with lignin and hemicellulose. Second, the plant cellulose is in crystalline form, less amenable to hydrolysis by cellulase. Third, the heterologous E1 inartistically from thermophilic *A*. *cellulolyticus* might have limited activity at plant in vivo temperature.

The question has also been which compartment should be chosen for targeting of heterologous enzymes? The apoplast targeting enzymes must have a significant influence on the production level rather than on transcription, meaning that the barrier to higher expression in cytosol and chloroplast is post transcriptional. In addition, since the apoplast is the extracellular pathway provided by the continuous matrix of cell walls, it may provide more space than other compartments for a higher level of accumulation.

The chloroplast targeting might require the first 24 amino acids of the mature rbcS protein in addition to the transit peptide. In a maize research (Zhong H, Teymouri F, Chapman B, Maqbool S, Sabzikar R, E1-Maghraby Y, Dale B, Sticklen MB: The dicot pea (Pisum sativum L.) rbcS transit peptide directs the Alcaligenes eutrophus polyhydroxybutyrate enzymes into the monocot maize (Zea mays L.) chloroplasts. Plant Sci 2003, 165: 455-462), the first 24 amino acid coding sequence of the mature rubisco small subunit protein was used in addition to the pea rubisco transit peptide to direct three polyhydroxybutyrate pathway enzymes into maize chloroplast.

Targeting of heterologous peptides to vacuole has been performed in several cases. For example, ProdiGene targeted the heterologous laccase into maize seed apoplast (Hood EE: Bioindustrial and biopharmaceutical products from plants. In: New directions for a diverse planet: Proc 4th Intl Crop Sci Congress, Brisbane, Austria, Sept. 26-Oct 1, 2004. ISBN 1 920842 20 9).

Because targeting of heterologous proteins for high accumulation without harm to plant growth and development has successfully been achieved, a battery of all different groups of polysaccharide- and lignin degrading enzymes can be tested within the same crop biomass by targeting each enzyme into the same or different compartments. Also, targeting of the same enzyme into all different cell compartments of the same plant can be tested to maximize the single enzyme production. Therefore, the future of large-scale production of these enzymes within the crop biomass is very bright, and the idea to replace the microbial tank reactors by plants as biofactories for commercial production of these and other industrial enzymes is indeed not impossible.

*Flc* Gene: Tobacco biomass was significantly increased with nuclear insertion of a single *Arabidopsis thaliana* Flowering Locus C *(flc)* gene (Salehi H, Ransom C, Oraby H, and Sticklen M: Delay in flowering and increase in biomass of plants expressing the Arabidopsis floral repressor gene FLC (FLOWERING LOCUS C). Plant Physiol 2005, 162: 711-717). Transfer of a single *flc* gene that delays flowering can significantly increase the plant biomass, because the energy needed for on time reproduction has shifted into biomass growth. An increase in photosynthesis does not increase the plant biomass, because several other factors such as plant nutrients, oxygen, water, and plant respiration also need to be regulated in order to increase the plant biomass. Increase in photosynthesis also relates to the correct matching of the plant circadian clock period (Dodd AN, Salathia N, Hall A, Kevei E, Toth R, Nagy F, Hibert JM, Miller AJ, Webb AAR: Plant circadian clocks increase photosynthesis, growth, survival, and competitive advantage. Science 2006, 309: 630-633) with that of the external light-dark cycle (Sinclair TR, Purcell LC, Sneller CH: Crop transformation and the challenge to increase yield potential. Trends Plant Sci. 2004, 9(2): 70-75). The fact that maize produced 20% more biomass under high CO₂ concentration might be because the C4 maize may have more capacity to synthesize sucrose, starch, and overall biomass under elevated CO₂. This observation needs to be tested in C3 plants.

Plant genetic engineering to improve biomass characterization for a better biofuel economy is a new technology. By definition, a new technology is economically feasible if the social benefits from adopting the technology are greater than its social costs (Communication with Dr. James Oehmke, Plant Biotechnology Economist, Michigan State University). Here, the social costs include costs of resources used without the government subsidies, and the social benefits are the low production costs.

Therefore, the present invention is limited only by the Claims attached herein.

## Claims

1. A transgenic plant capable of expressing cell wall degrading enzymes comprising:
(a) at least one DNA encoding a β-glucosidase as a cell wall degrading enzyme which is operably linked to a nucleotide sequence encoding a signal peptide directing the beta-glucosidase to a plastid, vacuole or apoplast in the leaves of the transgenic plant; and
(b) at least one DNA comprising a flowering locus C gene coding region operably linked to a constitutive promoter, wherein the transgenic plant expresses the one or more cell wall degrading enzymes and a transcription factor encoded by the flowering locus C gene that delays flowering while increasing biomass and enabling isolation of increased amounts of the cell wall degrading enzymes from the transgenic plant as compared to a non-transgenic plant from which the transgenic plant is derived.

2. The transgenic plant of Claim1, wherein the transgenic plant is a monocot.

3. The transgenic plant of Claim 2, wherein the monocot is switchgrass, rice or maize.

4. The transgenic plant of Claim 1, wherein a DNA encoding one or more additional cell wall degrading enzymes selected from the group consisting of a hemicellulase and a ligninase is expressed, in addition to the DNA encoding the cell wall degrading β-glucosidase enzyme.

5. The transgenic plant of Claim 4, further comprising DNA encoding heterologous endoglucanase or exoglucanase expressed in said transgenic plant.

6. The transgenic plant of Claim 5, wherein the beta-glucosidase is a beta-glucosidase from *Butyrivibrio fibrisolvens.*

7. The transgenic plant of Claim 6, wherein the beta-glucosidase is encoded by a DNA comprising the nucleotide sequence set forth in SEQ ID NO:23.

8. The transgenic plant of Claim 5, wherein the DNA encodes a ligninase is from *Phanerochaete chrysosporium.*

9. The transgenic plant of Claim 4 comprising in addition at least one DNA encoding a xylanase as the hemicellulase which is operably linked to a nucleotide sequence encoding a signal peptide directing the xylanase to a plastid or apoplast of the transgenic plant along with the DNA encoding the ligninase.

10. The transgenic plant of Claim 9, wherein the transgenic plant is a monocot.

11. The transgenic plant of Claim 10, wherein the monocot is switchgrass, rice or maize.

12. The transgenic plant of Claim 4, wherein the xylanase is encoded by a DNA comprising the nucleotide sequence set forth in SEQ ID NO:24, SEQ ID NO:33, SEQ ID NO:34 or SEQ ID NO:35.

13. The transgenic plant of Claim 1, wherein at least one DNA encoding the cell wall degrading enzyme is operably linked to a leaf-specific promoter.

14. The transgenic plant of Claim 13, wherein the leaf-specific promoter is a promoter for rbcS.

15. The transgenic plant of Claim 1, wherein at least one DNA encoding a cell wall degrading enzyme is operably linked to a Cauliflower Mosaic Virus 35S promoter.

16. The transgenic plant of Claim 1, wherein at least one DNA encoding a cell wall degrading enzyme is operably linked to a Tobacco Mosaic Virus translational enhancer.

17. The transgenic plant of Claim 1, wherein the nucleotide sequence encoding the signal peptide encodes a signal peptide of rbcS and the β-glucosidase is as set forth in SEQ ID NO: 23.

18. The transgenic plant of Claim 17, wherein the rbcS comprises the nucleotide sequence set forth in SEQ ID NO:1.

19. The transgenic plant of Claim 1, further comprising at least one DNA encoding a selectable marker operably linked to a constitutive promoter.

20. The transgenic plant of Claim 19, wherein the DNA encoding the selectable marker provides the transgenic plant with resistance to an antibiotic, a herbicide, or to environmental stress.

21. The transgenic plant of Claim 20, wherein the DNA encoding resistance to the herbicide is a DNA encoding phosphinothricin acetyl transferase which confers resistance to the herbicide phosphinothricin.

22. A method for making an enzyme extract comprising one or more cell wall degrading enzymes comprising:
(a) providing a transgenic plant according to claim 1;
(b) growing the transgenic plant for a time to accumulate the one or more cell wall degrading enzymes due to the delay in flowering;
(c) harvesting the transgenic plant which has accumulated the one or more cell wall degrading enzymes; and
(d) grinding the transgenic plant to provide an enzyme extract comprising the one or more cell wall degrading enzymes that accumulated in the transgenic plant.

23. The method of claim 22, further comprising:
(e) incubating the lignocellulosic material in the enzyme extract to produce the fermentable sugars from the lignocellulose in the plant material; and
(f) extracting the fermentable sugars produced from the lignocellulosic material.

24. The transgenic plant of Claim 1, wherein the beta-glucosidase is directed to a plastid or apoplast.

25. The transgenic plant of Claim 1, wherein the beta-glucosidase is directed to a vacuole.

## Patentansprüche

1. Eine transgene Pflanze, die in der Lage ist, Zellwand abbauende Enzyme zu exprimieren, umfassend:
(a) wenigstens eine DNA, die eine beta-Glucosidase als ein Zellwand abbauendes Enzym kodiert, funktionell verknüpft mit einer Nukleinsäuresequenz, die ein Signalpeptid kodiert, welches die beta-Glucosidase zu einem Plastiden, einer Vakuole oder einem Apoplasten in den Blättern der transgenen Pflanze dirigiert; und
(b) wenigstens eine DNA, die eine kodierende Region eines *flowering locus* C Gens, funktionell verknüpft mit einem konstitutiven Promotor, umfasst, wobei die transgene Pflanze das eine oder die mehreren Zellwand abbauende(n) Enzym(e) exprimiert und einen Transkriptionsfaktor, der vom *flowering locus* C Gen kodiert wird, welcher die Blüte verzögert und zur gleichen Zeit die Biomasse steigert und die Isolierung von erhöhten Mengen der Zellwand abbauenden Enzyme aus der transgenen Pflanze im Vergleich zu einer nicht-transgenen Pflanze, von welcher die transgene Pflanze abgeleitet ist, ermöglicht.

2. Die transgene Pflanze gemäß Anspruch 1, wobei die transgene Pflanze eine Monokotyledone ist.

3. Die transgene Pflanze gemäß Anspruch 2, wobei die Monokotyledone Rutenhirse, Reis oder Mais ist.

4. Die transgene Pflanze gemäß Anspruch 1, wobei eine DNA, die eine oder mehrere zusätzliche Zellwand abbauende Enzyme ausgewählt aus der Gruppe bestehend aus einer Hemicellulase und einer Ligninase exprimiert wird, zusätzlich zu der das Zellwand abbauende beta-Glucosidase-Enzym kodierenden DNA.

5. Die transgene Pflanze gemäß Anspruch 4, ferner umfassend heterologe Endoglucanase oder Exoglucanase kodierende DNA, die in besagter transgener Pflanze exprimiert wird.

6. Die transgene Pflanze gemäß Anspruch 5, wobei die beta-Glucosidase eine beta-Glucosidase aus *Butyrivibrio fibrisolvens* ist.

7. Die transgene Pflanze gemäß Anspruch 6, wobei die beta-Glucosidase durch eine DNA kodiert wird, welche die in SEQ ID NO:23 dargestellte Nukleotidsequenz umfasst.

8. Die transgene Pflanze gemäß Anspruch 5, wobei die DNA eine Ligninase kodiert, die aus *Phanerochaete chrysosporium* stammt.

9. Die transgene Pflanze gemäß Anspruch 4, welche zusätzlich mindestens eine DNA umfasst, die eine Xylanase als die Hemicellulase kodiert, welche funktionell mit einer Nukleinsäuresequenz verknüpft ist, die ein Signalpeptid kodiert, welches die Xylanase zu einem Plastiden, oder Apoplasten der transgenen Pflanze dirigiert, zusammen mit der DNA, welche die Ligninase kodiert.

10. Die transgene Pflanze gemäß Anspruch 9, wobei die transgene Pflanze eine Monokotyledone ist.

11. Die transgene Pflanze gemäß Anspruch 10, wobei die Monokotyledone Rutenhirse, Reis oder Mais ist.

12. Die transgene Pflanze gemäß Anspruch 4, wobei die Xylanase durch eine DNA kodiert wird, welche die in SEQ ID NO:24, SEQ ID NO:33, SEQ ID NO:34, oder SEQ ID NO:35 dargestellte Nukleotidsequenz umfasst.

13. Die transgene Pflanze gemäß Anspruch 1, wobei wenigstens eine DNA, die das Zellwand abbauende Enzym kodiert, funktionell mit einem Blatt-spezifischen Promotor verknüpft ist.

14. Die transgene Pflanze gemäß Anspruch 13, wobei der Blatt-spezifische Promotor ein Promotor für rbcS ist.

15. Die transgene Pflanze gemäß Anspruch 1, wobei wenigstens eine DNA, die ein Zellwand abbauendes Enzym kodiert, funktionell mit einem Cauliflower Mosaic Virus 35S Promotor verknüpft ist.

16. Die transgene Pflanze gemäß Anspruch 1, wobei wenigstens eine DNA, die ein Zellwand abbauendes Enzym kodiert, funktionell mit einem Tabakmosaikvirus-Translationsverstärker verknüpft ist.

17. Die transgene Pflanze gemäß Anspruch 1, wobei die Nukleinsäuresequenz, die das Signalpeptid kodiert, ein Signalpeptid von rbcS kodiert und die beta-Glucosidase wie in SEQ ID NO:23 dargestellt ist.

18. Die transgene Pflanze gemäß Anspruch 17, wobei das rbcS die Nukleinsäure wie in SEQ ID NO:1 dargestellt umfasst.

19. Die transgene Pflanze gemäß Anspruch 1, ferner umfassend wenigstens eine DNA, die einen selektierbaren Marker, funktionell verknüpft mit einem konstitutiven Promotor, kodiert.

20. Die transgene Pflanze gemäß Anspruch 19, wobei die DNA, die den selektierbaren Marker kodiert, der transgenen Pflanze eine Resistenz gegenüber einem Antibiotikum, einem Herbizid, oder gegenüber Umweltstress verleiht.

21. Die transgene Pflanze gemäß Anspruch 20, wobei die DNA, die für Herbizidresistenz kodiert, eine DNA ist, die Phosphinothricin-Acetyl-Transferase kodiert, welche Resistenz gegenüber dem Herbizid Phosphinotricin verleiht.

22. Ein Verfahren zur Herstellung eines Enzymextrakts, der ein oder mehrere Zellwand abbauende Enzyme umfasst, umfassend:
(a) Bereitstellen einer transgenen Pflanze gemäß Anspruch 1;
(b) Anziehen der Pflanze für eine Zeitspanne, um das eine oder die mehreren Zellwand abbauenden Enzyme aufgrund der Verzögerung der Blüte anzureichern;
(c) Ernten der transgenen Pflanze, welche das eine oder die mehreren Zellwand abbauenden Enzyme angereichert hat;
(d) Zerkleinern der transgenen Pflanze, um einen Enzymextrakt bereitzustellen, welcher das eine oder die mehreren Zellwand abbauenden Enzyme umfasst, welches bzw. welche in der transgenen Pflanze angereichert wurde bzw. wurden.

23. Das Verfahren gemäß Anspruch 22, ferner umfassend:
(e) Inkubieren des Lignocellulose-Materials in dem Enzymextrakt, um die fermentierbaren Zucker aus der Lignocellulose im Pflanzenmaterial zu produzieren; und
(f) Extrahieren der fermentierbaren Zucker, die aus dem Lignocellulose-Material produziert wurden.

24. Die transgene Pflanze gemäß Anspruch 1, wobei die beta-Glucosidase zu einem Plastiden oder Apoplasten dirigiert wird.

25. Die transgene Pflanze gemäß Anspruch 1, wobei die beta-Glucosidase zu einer Vakuole dirigiert wird.

## Revendications

1. Plante transgénique apte à exprimer des enzymes de dégradation des parois cellulaires comprenant :
(a) au moins un ADN codant pour une bêta-glucosidase en tant qu'enzyme de dégradation des parois cellulaires, qui est fonctionnellement lié à une séquence nucléotidique codant pour un peptide signal dirigeant la bêta-glucosidase vers un plastide, une vacuole ou un apoplaste dans les feuilles d'une plante transgénique ; et
(b) au moins un ADN comprenant une région codante du gène du locus de floraison C, fonctionnellement lié à un promoteur constitutif, où la plante transgénique exprime la ou les enzymes de dégradation des parois cellulaires et un facteur de transcription codé par le gène du locus de floraison C qui retarde la floraison tout en augmentant la biomasse et en permettant d'isoler des quantités accrues des enzymes de dégradation des parois cellulaires à partir de la plante transgénique comparativement à une plante non transgénique dont dérive la plante transgénique.

2. Plante transgénique selon la revendication 1, laquelle plante transgénique est une monocotylédone.

3. Plante transgénique selon la revendication 2, dans laquelle la monocotylédone est le panic raide, le riz ou le maïs.

4. Plante transgénique selon la revendication 1, dans laquelle un ADN codant pour une ou plusieurs enzymes de dégradation des parois cellulaires supplémentaires choisies dans le groupe constitué d'une hémicellulase et d'une ligninase est exprimé, en plus de l'ADN codant pour l'enzyme de dégradation des parois cellulaires β-glucosidase.

5. Plante transgénique selon la revendication 4, comprenant en outre de l'ADN codant pour une exoglucanase ou endoglucanase hétérologue exprimée dans ladite plante transgénique.

6. Plante transgénique selon la revendication 5, dans laquelle la bêta-glucosidase est une bêta-glucosidase de *Butyrivibrio fibrisolvens.*

7. Plante transgénique selon la revendication 6, dans laquelle la bêta-glucosidase est codée par un ADN comprenant la séquence nucléotidique présentée dans SEQ ID NO:23.

8. Plante transgénique selon la revendication 5, dans laquelle l'ADN code pour une ligninase de *Phanerochaete chrysosporium.*

9. Plante transgénique selon la revendication 4 comprenant en plus au moins un ADN codant pour une xylanase en tant qu'hémicellulase, qui est fonctionnellement lié à une séquence nucléotidique codant pour un peptide signal dirigeant la xylanase vers un plastide ou un apoplaste de la plante transgénique en même temps que l'ADN codant pour la ligninase.

10. Plante transgénique selon la revendication 9, laquelle plante transgénique est une monocotylédone.

11. Plante transgénique selon la revendication 10, dans laquelle la monocotylédone est le panic raide, le riz ou le maïs.

12. Plante transgénique selon la revendication 4, dans laquelle la xylanase est codée par un ADN comprenant la séquence nucléotidique présentée dans SEQ ID NO:24, SEQ ID NO:33, SEQ ID NO:34 ou SEQ ID NO:35.

13. Plante transgénique selon la revendication 1, dans laquelle au moins un ADN codant pour l'enzyme de dégradation des parois cellulaires est fonctionnellement lié à un promoteur spécifique des feuilles.

14. Plante transgénique selon la revendication 13, dans laquelle le promoteur spécifique des feuilles est un promoteur pour rbcS.

15. Plante transgénique selon la revendication 1, dans laquelle au moins un ADN codant pour une enzyme de dégradation des parois cellulaires est fonctionnellement lié à un promoteur 35S du virus de la mosaïque du chou-fleur.

16. Plante transgénique selon la revendication 1, dans laquelle au moins un ADN codant pour une enzyme de dégradation des parois cellulaires est fonctionnellement lié à un activateur de traduction du virus de la mosaïque du tabac.

17. Plante transgénique selon la revendication 1, dans laquelle la séquence nucléotidique codant pour le peptide signal code pour un peptide signal de rbcS et la β-glucosidase est telle que présentée dans SEQ ID NO: 23.

18. Plante transgénique selon la revendication 17, dans laquelle le rbcS comprend la séquence nucléotidique présentée dans SEQ ID NO:1.

19. Plante transgénique selon la revendication 1, comprenant en outre au moins un ADN codant pour un marqueur de sélection, fonctionnellement lié à un promoteur constitutif.

20. Plante transgénique selon la revendication 19, dans laquelle l'ADN codant pour le marqueur de sélection dote la plante transgénique d'une résistance à un antibiotique, à un herbicide, ou à un stress environnemental.

21. Plante transgénique selon la revendication 20, dans laquelle l'ADN codant pour une résistance à l'herbicide est un ADN codant pour la phosphinothricine acétyltransférase qui confère une résistance à l'herbicide phosphinothricine.

22. Procédé de fabrication d'un extrait enzymatique comprenant une ou plusieurs enzymes de dégradation des parois cellulaires comprenant les étapes consistant à :
(a) se procurer une plante transgénique selon la revendication 1 ;
(b) faire pousser la plante transgénique pendant un certain temps pour accumuler la ou les enzymes de dégradation des parois cellulaires du fait du retard de la floraison ;
(c) récolter la plante transgénique qui a accumulé la ou les enzymes de dégradation des parois cellulaires ; et
(d) broyer la plante transgénique pour obtenir un extrait enzymatique comprenant la ou les enzymes de dégradation des parois cellulaires qui se sont accumulées dans la plante transgénique.

23. Procédé selon la revendication 22, comprenant en outre les étapes consistant à:
(e) incuber la matière lignocellulosique dans l'extrait enzymatique pour produire les sucres fermentescibles à partir de la lignocellulose dans la matière végétale ; et
(f) extraire les sucres fermentescibles produits à partir de la matière lignocellulosique.

24. Plante transgénique selon la revendication 1, dans laquelle la bêta-glucosidase est dirigée vers un plastide ou un apoplaste.

25. Plante transgénique selon la revendication 1, dans laquelle la bêta-glucosidase est dirigée vers une vacuole.


### SEQUENCE LISTING

<110> Sticklen, Masomeh B
   Maqbool, shahina B
   Dale, Bruce E
<120> PRODUCTION OF BETA-GLUCOSIDASE, HEMICELLULASE AND LIGNINASE IN E1 AND FLC-CELLULASE-TRANSGENIC PLANTS
<130> MSU 4.1-804A
<150> US 60/242,408
   <151> 2000-10-20
<150> US 11/451,162
   <151> 2006-06-12
<150> US 11/354,310
   <151> 2006-02-14
<150> US 09/981,900
   <151> 2001-10-18
<160> 35
<170> PatentIn version 3.3
<210> 1
   <211> 1110
   <212> DNA
   <213> Oryza sativa
<400> 1
   gggtcggaga tgccaccacg gccacaaccc acgagcccgg cgcgacacca ccgcgcgcgt    60
   tgagccagcc acaaacgccc gcggataggc gcgccgcacg cggccaatcc taccacatcc   120
   ccggcctccg cggctcgagc gccgtgccat ccgatccgct gagttttggc tatttatacg   180
   taccgcggga gcctgtgtgc agagagtgca tctcaagaag tactcgagca aagaaggaga   240
   gagcttggtg agctgcagag atggccccct ccgtgatggc gtcgtcggcc accaccgtcg   300
   ctcccttcca gggctcaagt ccaccgccgg catgccgtcg cccgccgtcc gaactccagc   360
   ttcggcaacg tcagcatggc ggcaggatca ggtgcatgca ggtaattacc tactgatcca   420
   acacacattc ttcttcttct tcttcttctt aaccaacatt aaccaacaac tcaattatcg   480
   tttattcatt gaggtgtggc cgattgaggg catcaagaag ttcgagaccc tctcctacct   540
   gccaccgctc accgtggagg acctcctgaa gcagatcgag tacctagctc cgttccaagt   600
   ggtgccctgc ctcgagttca gcaaggtcgg atttgtctac cgtgagaacc acaagtcccc   660
   tggatactac gacggcaggt actggaccat gtggaagctg cccatgttcg ggtgcaccga   720
   cgccacccag gtcgtcaagg agctcgagga ggccaagaag gcgtaccctg atgcattcgt   780
   ccgtatcatc ggcttcgaca acgttaggca ggtgcagctc atcagcttca tcgcctacaa   840
   cccgggctgc gaggagtctg gtggcaacta agccgtcatc gtcatatata gcctcgttta   900
   attgttcatc tctgattcga tgatgtctcc caccttgttt cgtgtgttcc cagtttgttt   960
   catcgtcttt tgattttacc ggccgtgctc tgcttttgtt ttttcttttc acctgattct  1020
   ctctctgact tgatgtaaga gtggtatctg ctacgactat atgttgtttg ggtgaggcat  1080
   atgtgaatga aatctatgaa agctccggct                                   1110
<210> 2
   <211> 38
   <212> PRT
   <213> Oryza sativa
<400> 2
   Met Ala Pro Ser Val Met Ala Ser Ser Ala Thr Thr Val Ala Pro Phe
   1               5                   10                  15
   Gln Gly Ser Ser Pro Pro Pro Ala Cys Arg Arg Pro Pro Ser Glu Leu
               20                  25                  30
   Gln Leu Arg Gln Arg Gln
           35
<210> 3
   <211> 6
   <212> PRT
   <213> Artificial Sequence
<220>
   <223> signal peptide targets the peroxisomes of plants
<400> 3
   Arg Ala Val Ala Arg Leu
   1 5
<210> 4
   <211> 3004
   <212> DNA
   <213> Acidothermus cellulolyticus
<220>
   <221> CDS
   <222> (824)..(2512)
   <223> E I beta-1,4-endoglucanase precursor
<400> 4
   ggatccacgt tgtacaaggt cacctgtccg tcgttctggt agagcggcgg gatggtcacc 60
   cgcacgatct ctcctttgtt gatgtcgacg gtcacgtggt tacggtttgc ctcggccgcg 120
   attttcgcgc tcgggcttgc tccggctgtc gggttcggtt tggcgtggtg tgcggagcac 180
   gccgaggcga tcccaatgag ggcaagggca agagcggagc cgatggcacg tcgggtggcc 240
   gatggggtac gccgatgggg cgtggcgtcc ccgccgcgga cagaaccgga tgcggaatag 300
   gtcacggtgc gacatgttgc cgtaccgcgg acccggatga caagggtggg tgcgcgggtc 360
   gcctgtgagc tgccggctgg cgtctggatc atgggaacga tcccaccatt ccccgcaatc 420
   gacgcgatcg ggagcagggc ggcgcgagcc ggaccgtgtg gtcgagccgg acgattcgcc 480
   catacggtgc tgcaatgccc agcgccatgt tgtcaatccg ccaaatgcag caatgcacac 540
   atggacaggg attgtgactc tgagtaatga ttggattgcc ttcttgccgc ctacgcgtta    600
   cgcagagtag gcgactgtat gcggtaggtt ggcgctccag ccgtgggctg gacatgcctg    660
   ctgcgaactc ttgacacgtc tggttgaacg cgcaatactc ccaacaccga tgggatcgtt    720
   cccataagtt tccgtctcac aacagaatcg gtgcgccctc atgatcaacg tgaaaggagt    780
   acgggggaga acagacgggg gagaaaccaa cgggggattg gcg gtg ccg cgc gca      835
                                                   Val Pro Arg Ala
                                                   1
   ttg cgg cga gtg cct ggc tcg cgg gtg atg ctg cgg gtc ggc gtc gtc      883
   Leu Arg Arg Val Pro Gly Ser Arg Val Met Leu Arg Val Gly Val Val
   5                   10                  15                  20
   gtc gcg gtg ctg gca ttg gtt gcc gca ctc gcc aac cta gcc gtg ccg      931
   Val Ala Val Leu Ala Leu Val Ala Ala Leu Ala Asn Leu Ala Val Pro
                   25                  30                  35
   cgg ccg gct cgc gcc gcg ggc ggc ggc tat tgg cac acg agc ggc cgg      979
   Arg Pro Ala Arg Ala Ala Gly Gly Gly Tyr Trp His Thr Ser Gly Arg
               40                  45                  50
   gag atc ctg gac gcg aac aac gtg ccg gta cgg atc gcc ggc atc aac     1027
   Glu Ile Leu Asp Ala Asn Asn Val Pro Val Arg Ile Ala Gly Ile Asn
           55                  60                  65
   tgg ttt ggg ttc gaa acc tgc aat tac gtc gtg cac ggt ctc tgg tca     1075
   Trp Phe Gly Phe Glu Thr Cys Asn Tyr Val Val His Gly Leu Trp Ser
       70                  75                  80
   cgc gac tac cgc agc atg ctc gac cag ata aag tcg ctc ggc tac aac     1123
   Arg Asp Tyr Arg Ser Met Leu Asp Gln Ile Lys Ser Leu Gly Tyr Asn
   85                  90                  95                  100
   aca atc cgg ctg ccg tac tct gac gac att ctc aag ccg ggc acc atg     1171
   Thr Ile Arg Leu Pro Tyr Ser Asp Asp Ile Leu Lys Pro Gly Thr Met
                   105                 110                 115
   ccg aac agc atc aat ttt tac cag atg aat cag gac ctg cag ggt ctg     1219
   Pro Asn Ser Ile Asn Phe Tyr Gln Met Asn Gln Asp Leu Gln Gly Leu
               120                 125                 130
   acg tcc ttg cag gtc atg gac aaa atc gtc gcg tac gcc ggt cag atc     1267
   Thr Ser Leu Gln Val Met Asp Lys Ile Val Ala Tyr Ala Gly Gln Ile
           135                 140                 145
   ggc ctg cgc atc att ctt gac cgc cac cga ccg gat tgc agc ggg cag     1315
   Gly Leu Arg Ile Ile Leu Asp Arg His Arg Pro Asp Cys Ser Gly Gln
       150                 155                 160
   tcg gcg ctg tgg tac acg agc agc gtc tcg gag gct acg tgg att tcc     1363
   Ser Ala Leu Trp Tyr Thr Ser Ser Val Ser Glu Ala Thr Trp Ile Ser
   165                 170                 175                 180
   gac ctg caa gcg ctg gcg cag cgc tac aag gga aac ccg acg gtc gtc     1411
   Asp Leu Gln Ala Leu Ala Gln Arg Tyr Lys Gly Asn Pro Thr Val Val
                   185                 190                 195
   ggc ttt gac ttg cac aac gag ccg cat gac ccg gcc tgc tgg ggc tgc     1459
   Gly Phe Asp Leu His Asn Glu Pro His Asp Pro Ala Cys Trp Gly Cys
               200                 205                 210
   ggc gat ccg agc atc gac tgg cga ttg gcc gcc gag cgg gcc gga aac     1507
   Gly Asp Pro Ser Ile Asp Trp Arg Leu Ala Ala Glu Arg Ala Gly Asn
   215 220 225
   gcc gtg ctc tcg gtg aat ccg aac ctg ctc att ttc gtc gaa ggt gtg 1555
   Ala Val Leu Ser Val Asn Pro Asn Leu Leu Ile Phe Val Glu Gly Val
   230 235 240
   cag agc tac aac gga gac tcc tac tgg tgg ggc ggc aac ctg caa gga 1603
   Gln Ser Tyr Asn Gly Asp Ser Tyr Trp Trp Gly Gly Asn Leu Gln Gly
   245 250 255 260
   gcc ggc cag tac ccg gtc gtg ctg aac gtg ccg aac cgc ctg gtg tac 1651
   Ala Gly Gln Tyr Pro Val Val Leu Asn Val Pro Asn Arg Leu Val Tyr
   265 270 275
   tcg gcg cac gac tac gcg acg agc gtc tac ccg cag acg tgg ttc agc 1699
   Ser Ala His Asp Tyr Ala Thr Ser Val Tyr Pro Gln Thr Trp Phe Ser
   280 285 290
   gat ccg acc ttc ccc aac aac atg ccc ggc atc tgg aac aag aac tgg 1747
   Asp Pro Thr Phe Pro Asn Asn Met Pro Gly Ile Trp Asn Lys Asn Trp
   295 300 305
   gga tac ctc ttc aat cag aac att gca ccg gta tgg ctg ggc gaa ttc 1795
   Gly Tyr Leu Phe Asn Gln Asn Ile Ala Pro Val Trp Leu Gly Glu Phe
   310 315 320
   ggt acg aca ctg caa tcc acg acc gac cag acg tgg ctg aag acg ctc 1843
   Gly Thr Thr Leu Gln Ser Thr Thr Asp Gln Thr Trp Leu Lys Thr Leu
   325 330 335 340
   gtc cag tac cta cgg ccg acc gcg caa tac ggt gcg gac agc ttc cag 1891
   Val Gln Tyr Leu Arg Pro Thr Ala Gln Tyr Gly Ala Asp Ser Phe Gln
   345 350 355
   tgg acc ttc tgg tcc tgg aac ccc gat tcc ggc gac aca gga gga att 1939
   Trp Thr Phe Trp Ser Trp Asn Pro Asp Ser Gly Asp Thr Gly Gly Ile
   360 365 370
   ctc aag gat gac tgg cag acg gtc gac aca gta aaa gac ggc tat ctc 1987
   Leu Lys Asp Asp Trp Gln Thr Val Asp Thr Val Lys Asp Gly Tyr Leu
   375 380 385
   gcg ccg atc aag tcg tcg att ttc gat cct gtc ggc gcg tct gca tcg 2035
   Ala Pro Ile Lys Ser Ser Ile Phe Asp Pro Val Gly Ala Ser Ala Ser
   390 395 400
   cct agc agt caa ccg tcc ccg tcg gtg tcg ccg tct ccg tcg ccg agc 2083
   Pro Ser Ser Gln Pro Ser Pro Ser Val Ser Pro Ser Pro Ser Pro Ser
   405 410 415 420
   ccg tcg gcg agt cgg acg ccg acg cct act ccg acg ccg aca gcc agc 2131
   Pro Ser Ala Ser Arg Thr Pro Thr Pro Thr Pro Thr Pro Thr Ala Ser
   425 430 435
   ccg acg cca acg ctg acc cct act gct acg ccc acg ccc acg gca agc 2179
   Pro Thr Pro Thr Leu Thr Pro Thr Ala Thr Pro Thr Pro Thr Ala Ser
   440 445 450
   ccg acg ccg tca ccg acg gca gcc tcc gga gcc cgc tgc acc gcg agt 2227
   Pro Thr Pro Ser Pro Thr Ala Ala Ser Gly Ala Arg Cys Thr Ala Ser
   455 460 465
   tac cag gtc aac agc gat tgg ggc aat ggc ttc acg gta acg gtg gcc 2275
   Tyr Gln Val Asn Ser Asp Trp Gly Asn Gly Phe Thr Val Thr Val Ala
   470 475 480
   gtg aca aat tcc gga tcc gtc gcg acc aag aca tgg acg gtc agt tgg 2323
   Val Thr Asn Ser Gly Ser Val Ala Thr Lys Thr Trp Thr Val Ser Trp
   485                 490                 495                 500
   aca ttc ggc gga aat cag acg att acc aat tcg tgg aat gca gcg gtc      2371
   Thr Phe Gly Gly Asn Gln Thr Ile Thr Asn Ser Trp Asn Ala Ala Val
                   505                 510                 515
   acg cag aac ggt cag tcg gta acg gct cgg aat atg agt tat aac aac      2419
   Thr Gln Asn Gly Gln Ser Val Thr Ala Arg Asn Met Ser Tyr Asn Asn
               520                 525                 530
   gtg att cag cct ggt cag aac acc acg ttc gga ttc cag gcg agc tat      2467
   Val Ile Gln Pro Gly Gln Asn Thr Thr Phe Gly Phe Gln Ala Ser Tyr
           535                 540                 545
   acc gga agc aac gcg gca ccg aca gtc gcc tgc gca gca agt taa          2512
   Thr Gly Ser Asn Ala Ala Pro Thr Val Ala Cys Ala Ala Ser
       550                 555                 560
   tacgtcgggg agccgacggg agggtccgga ccgtcggttc cccggcttcc acctatggag    2572
   cgaacccaac aatccggacg gaactgcagg taccagagag gaacgacacg aatgcccgcc    2632
   atctcaaaac ggctgcgagc cggcgtcctc gccggggcgg tgagcatcgc agcctccatc    2692
   gtgccgctgg cgatgcagca tcctgccatc gccgcgacgc acgtcgacaa tccctatgcg    2752
   ggagcgacct tcttcgtcaa cccgtactgg gcgcaagaag tacagagcga acggcgaacc    2812
   agaccaatgc cactctcgca gcgaaaatgc gcgtcgtttc cacatattcg acggccgtct    2872
   ggatggaccg catcgctgcg atcaacggcg tcaacggcgg acccggcttg acgacatatc    2932
   tggacgccgc cctctcccag cagcagggaa ccacccctga agtcattgag attgtcatct    2992
   acgatctgcc gg                                                        3004
<210> 5
   <211> 562
   <212> PRT
   <213> Acidothermus cellulolyticus
<400> 5
   Val Pro Arg Ala Leu Arg Arg Val Pro Gly Ser Arg Val Met Leu Arg
   1               5                   10                  15
   Val Gly Val Val Val Ala Val Leu Ala Leu Val Ala Ala Leu Ala Asn
               20                  25                  30
   Leu Ala Val Pro Arg Pro Ala Arg Ala Ala Gly Gly Gly Tyr Trp His
           35                  40                  45
   Thr Ser Gly Arg Glu Ile Leu Asp Ala Asn Asn Val Pro Val Arg Ile
       50                  55                  60
   Ala Gly Ile Asn Trp Phe Gly Phe Glu Thr Cys Asn Tyr Val Val His
   65                  70                  75                  80
   Gly Leu Trp Ser Arg Asp Tyr Arg Ser Met Leu Asp Gln Ile Lys Ser
                   85                  90                  95
   Leu Gly Tyr Asn Thr Ile Arg Leu Pro Tyr Ser Asp Asp Ile Leu Lys
               100                 105                 110
   Pro Gly Thr Met Pro Asn Ser Ile Asn Phe Tyr Gln Met Asn Gln Asp
           115                 120                 125
   Leu Gln Gly Leu Thr Ser Leu Gln Val Met Asp Lys Ile Val Ala Tyr
       130                 135                 140
   Ala Gly Gln Ile Gly Leu Arg Ile Ile Leu Asp Arg His Arg Pro Asp
   145                 150                 155                 160
   Cys Ser Gly Gln Ser Ala Leu Trp Tyr Thr Ser Ser Val Ser Glu Ala
                   165                 170                 175
   Thr Trp Ile Ser Asp Leu Gln Ala Leu Ala Gln Arg Tyr Lys Gly Asn
               180                 185                 190
   Pro Thr Val Val Gly Phe Asp Leu His Asn Glu Pro His Asp Pro Ala
           195                 200                 205
   Cys Trp Gly Cys Gly Asp Pro Ser Ile Asp Trp Arg Leu Ala Ala Glu
       210                 215                 220
   Arg Ala Gly Asn Ala Val Leu Ser Val Asn Pro Asn Leu Leu Ile Phe
   225                 230                 235                 240
   Val Glu Gly Val Gln Ser Tyr Asn Gly Asp Ser Tyr Trp Trp Gly Gly
                   245                 250                 255
   Asn Leu Gln Gly Ala Gly Gln Tyr Pro Val Val Leu Asn Val Pro Asn
               260                 265                 270
   Arg Leu Val Tyr Ser Ala His Asp Tyr Ala Thr Ser Val Tyr Pro Gln
           275                 280                 285
   Thr Trp Phe Ser Asp Pro Thr Phe Pro Asn Asn Met Pro Gly Ile Trp
       290                 295                 300
   Asn Lys Asn Trp Gly Tyr Leu Phe Asn Gln Asn Ile Ala Pro Val Trp
   305                 310                 315                 320
   Leu Gly Glu Phe Gly Thr Thr Leu Gln Ser Thr Thr Asp Gln Thr Trp
                   325                 330                 335
   Leu Lys Thr Leu Val Gln Tyr Leu Arg Pro Thr Ala Gln Tyr Gly Ala
               340                 345                 350
   Asp Ser Phe Gln Trp Thr Phe Trp Ser Trp Asn Pro Asp Ser Gly Asp
   355 360 365
   Thr Gly Gly Ile Leu Lys Asp Asp Trp Gln Thr Val Asp Thr Val Lys
   370 375 380
   Asp Gly Tyr Leu Ala Pro Ile Lys Ser Ser Ile Phe Asp Pro Val Gly
   385 390 395 400
   Ala Ser Ala Ser Pro Ser Ser Gln Pro Ser Pro Ser Val Ser Pro Ser
   405 410 415
   Pro Ser Pro Ser Pro Ser Ala Ser Arg Thr Pro Thr Pro Thr Pro Thr
   420 425 430
   Pro Thr Ala Ser Pro Thr Pro Thr Leu Thr Pro Thr Ala Thr Pro Thr
   435 440 445
   Pro Thr Ala Ser Pro Thr Pro Ser Pro Thr Ala Ala Ser Gly Ala Arg
   450 455 460
   Cys Thr Ala Ser Tyr Gln Val Asn Ser Asp Trp Gly Asn Gly Phe Thr
   465 470 475 480
   Val Thr Val Ala Val Thr Asn Ser Gly Ser Val Ala Thr Lys Thr Trp
   485 490 495
   Thr Val Ser Trp Thr Phe Gly Gly Asn Gln Thr Ile Thr Asn Ser Trp
   500 505 510
   Asn Ala Ala Val Thr Gln Asn Gly Gln Ser Val Thr Ala Arg Asn Met
   515 520 525
   Ser Tyr Asn Asn Val Ile Gln Pro Gly Gln Asn Thr Thr Phe Gly Phe
   530 535 540
   Gln Ala Ser Tyr Thr Gly Ser Asn Ala Ala Pro Thr Val Ala Cys Ala
   545 550 555 560
   Ala Ser
<210> 6
   <211> 1467
   <212> DNA
   <213> Actinomyces naeslundii
<220>
   <221> CDS
   <222> (1)..(1467)
   <223> beta-glucosidase
<220>
   <221> misc_feature
   <222> (339)..(339)
   <223> nucleotide is uncertain
<220>
   <221> misc_feature
   <222> (443)..(443)
   <223> nucleotide is uncertain
<220>
   <221> misc_feature
   <222> (947)..(947)
   <223> nucleotide is uncertain
<400> 6
   atg acc gcc acg tcc act act tct aag agc aat ccg aac ttc ccc gac      48
   Met Thr Ala Thr Ser Thr Thr Ser Lys Ser Asn Pro Asn Phe Pro Asp
   1               5                   10                  15
   ggc ttc ctg tgg ggc ggg gcc acc gcc gcc aac cag atc gag ggc gct      96
   Gly Phe Leu Trp Gly Gly Ala Thr Ala Ala Asn Gln Ile Glu Gly Ala
               20                  25                  30
   tac aac gag gac ggc aag ggc ctg tcc gtc cag gac gtc atg cct cgg     144
   Tyr Asn Glu Asp Gly Lys Gly Leu Ser Val Gln Asp Val Met Pro Arg
           35                  40                  45
   ggc atc atg gcc cac ccc acc cag gct ccc aca ccg gat aac ctt caa     192
   Gly Ile Met Ala His Pro Thr Gln Ala Pro Thr Pro Asp Asn Leu Gln
       50                  55                  60
   gct cga ggc gat cga cct tct acc acc gct tac gcc gag gac atc tcc     240
   Ala Arg Gly Asp Arg Pro Ser Thr Thr Ala Tyr Ala Glu Asp Ile Ser
   65                  70                  75                  80
   ctg ttc gcg gag atg ggt ttc aag gtc ttc cgc ttc tcc atc gcc tgg     288
   Leu Phe Ala Glu Met Gly Phe Lys Val Phe Arg Phe Ser Ile Ala Trp
                   85                  90                  95
   agc cgc atc ttc ccg ctc ggc gac gag acc gag ccc aat gag gaa gga     336
   Ser Arg Ile Phe Pro Leu Gly Asp Glu Thr Glu Pro Asn Glu Glu Gly
               100                 105                 110
   ctn gcc ttc tac gac cgg gtc ctc gac gag ctc gag aag cac ggg atc     384
   Xaa Ala Phe Tyr Asp Arg Val Leu Asp Glu Leu Glu Lys His Gly Ile
           115                 120                 125
   gag cca ctg gtc acc atc agc cac tac gag acc ccg ctg cac ctg gcg     432
   Glu Pro Leu Val Thr Ile Ser His Tyr Glu Thr Pro Leu His Leu Ala
       130                 135                 140
   cgc acc tac gnc ggc tgg acc gac cgc cgc ctc atc ggc ttc ttc gag     480
   Arg Thr Tyr Xaa Gly Trp Thr Asp Arg Arg Leu Ile Gly Phe Phe Glu
   145                 150                 155                 160
   cgc tac gcc cgc acc ctg ttc gag cgc tat ggc aag cgg gtc aag tac     528
   Arg Tyr Ala Arg Thr Leu Phe Glu Arg Tyr Gly Lys Arg Val Lys Tyr
                   165                 170                 175
   tgg ctc acc ttc aac gag atc aac tcc gtg ctc cat gag ccc ttc cta     576
   Trp Leu Thr Phe Asn Glu Ile Asn Ser Val Leu His Glu Pro Phe Leu
               180                 185                 190
   tct ggg ggc gtc gcc acg ccc aag gac agg ccc ccc gag cag gac ctc     624
   Ser Gly Gly Val Ala Thr Pro Lys Asp Arg Pro Pro Glu Gln Asp Leu
           195                 200                 205
   tac cag gcc atc caa aac gag ctc gtc gcc tcc gcg gcc gcg acc agg      672
   Tyr Gln Ala Ile Gln Asn Glu Leu Val Ala Ser Ala Ala Ala Thr Arg
       210                 215                 220
   atc gcc cat gag acc aac ccc gac atc cag gtc ggc tgc atg atc ctg      720
   Ile Ala His Glu Thr Asn Pro Asp Ile Gln Val Gly Cys Met Ile Leu
   225                 230                 235                 240
   gcc gat ccc acc tac ccg ctc acc cct gat ccc cgg gac gtg tgg gcg      768
   Ala Asp Pro Thr Tyr Pro Leu Thr Pro Asp Pro Arg Asp Val Trp Ala
                   245                 250                 255
   gcc aag cag gca gag cgc gcc aac tac gcc ttc gga gac ctc cac gta      816
   Ala Lys Gln Ala Glu Arg Ala Asn Tyr Ala Phe Gly Asp Leu His Val
               260                 265                 270
   cgt ggt gag tac ccc gga tac ctg cgg cgg acc ctg cgg gac aag ggc      864
   Arg Gly Glu Tyr Pro Gly Tyr Leu Arg Arg Thr Leu Arg Asp Lys Gly
           275                 280                 285
   atc gag ctg gag atc acc gag gag gac cgc gtg ctg ctg cgg gag cac      912
   Ile Glu Leu Glu Ile Thr Glu Glu Asp Arg Val Leu Leu Arg Glu His
       290                 295                 300
   acc gtc gac ttc gtc tcc ttc tcc tac tac atg tnc gtg tgc gag acc      960
   Thr Val Asp Phe Val Ser Phe Ser Tyr Tyr Met Xaa Val Cys Glu Thr
   305                 310                 315                 320
   gtc acc cag tcg gcc gag gcc ggc cgg ggc aac ctc atg ggc ggc gtc      1008
   Val Thr Gln Ser Ala Glu Ala Gly Arg Gly Asn Leu Met Gly Gly Val
                   325                 330                 335
   ccc aat ccc acc ctc gag gcc tcc gag tgg gga tgg cag atc gac ccg      1056
   Pro Asn Pro Thr Leu Glu Ala Ser Glu Trp Gly Trp Gln Ile Asp Pro
               340                 345                 350
   gcg ggc ctg cgc acc atc ctg aac gac tac tgg gac cgc tgg ggc aag      1104
   Ala Gly Leu Arg Thr Ile Leu Asn Asp Tyr Trp Asp Arg Trp Gly Lys
           355                 360                 365
   cct ctg ttc atc gtc gag aac ggc ctg gga gcc aag gac gtc ctc gtt      1152
   Pro Leu Phe Ile Val Glu Asn Gly Leu Gly Ala Lys Asp Val Leu Val
       370                 375                 380
   gac gga ccc aac ggt ccc acg gtc gag gac gac tac cgc atc gcc tac      1200
   Asp Gly Pro Asn Gly Pro Thr Val Glu Asp Asp Tyr Arg Ile Ala Tyr
   385                 390                 395                 400
   atg aac gac cac ctg gtc cag gtc gcc gag gcc att gcc gac ggc gtc      1248
   Met Asn Asp His Leu Val Gln Val Ala Glu Ala Ile Ala Asp Gly Val
                   405                 410                 415
   gag gtc ctg ggc tac acc tcc tgg ggc tgc atc gac ctg gtc tcg gcc      1296
   Glu Val Leu Gly Tyr Thr Ser Trp Gly Cys Ile Asp Leu Val Ser Ala
               420                 425                 430
   tcc acc gcc cag atg tcc aag cgc tac ggg ttc atc tac gtg gac cgt      1344
   Ser Thr Ala Gln Met Ser Lys Arg Tyr Gly Phe Ile Tyr Val Asp Arg
           435                 440                 445
   gac gac ggc ggc aac ggc acc ctg gcc cgc tac cgc aag aag tcc ttc      1392
   Asp Asp Gly Gly Asn Gly Thr Leu Ala Arg Tyr Arg Lys Lys Ser Phe
       450                 455                 460
   ggc tgg tac cgc gac gtc atc gcc tcc aac ggt gcc tcc ctc gtg cct      1440
   Gly Trp Tyr Arg Asp Val Ile Ala Ser Asn Gly Ala Ser Leu Val Pro
   465                 470                 475                 480
   ccg gtg cag gaa ccg ccg cgg ggg tag 1467
   Pro Val Gln Glu Pro Pro Arg Gly
   485
<210> 7
   <211> 488
   <212> PRT
   <213> Actinomyces naeslundii
<220>
   <221> misc_feature
   <222> (113)..(113)
   <223> The 'xaa' at location 113 stands for Leu.
<220>
   <221> misc_feature
   <222> (148)..(148)
   <223> The 'xaa' at location 148 stands for Asp, Gly, Ala, or val.
<220>
   <221> misc_feature
   <222> (316)..(316)
   <223> The 'xaa' at location 316 stands for Tyr, cys, Ser, or Phe.
<400> 7
   Met Thr Ala Thr Ser Thr Thr Ser Lys Ser Asn Pro Asn Phe Pro Asp
   1 5 10 15
   Gly Phe Leu Trp Gly Gly Ala Thr Ala Ala Asn Gln Ile Glu Gly Ala
   20 25 30
   Tyr Asn Glu Asp Gly Lys Gly Leu Ser Val Gln Asp Val Met Pro Arg
   35 40 45
   Gly Ile Met Ala His Pro Thr Gln Ala Pro Thr Pro Asp Asn Leu Gln
   50 55 60
   Ala Arg Gly Asp Arg Pro Ser Thr Thr Ala Tyr Ala Glu Asp Ile Ser
   65 70 75 80
   Leu Phe Ala Glu Met Gly Phe Lys Val Phe Arg Phe Ser Ile Ala Trp
   85 90 95
   Ser Arg Ile Phe Pro Leu Gly Asp Glu Thr Glu Pro Asn Glu Glu Gly
   100 105 110
   Xaa Ala Phe Tyr Asp Arg Val Leu Asp Glu Leu Glu Lys His Gly Ile
   115 120 125
   Glu Pro Leu Val Thr Ile Ser His Tyr Glu Thr Pro Leu His Leu Ala
   130 135 140
   Arg Thr Tyr Xaa Gly Trp Thr Asp Arg Arg Leu Ile Gly Phe Phe Glu
   145 150 155 160
   Arg Tyr Ala Arg Thr Leu Phe Glu Arg Tyr Gly Lys Arg Val Lys Tyr
   165 170 175
   Trp Leu Thr Phe Asn Glu Ile Asn Ser Val Leu His Glu Pro Phe Leu
   180 185 190
   Ser Gly Gly Val Ala Thr Pro Lys Asp Arg Pro Pro Glu Gln Asp Leu
   195 200 205
   Tyr Gln Ala Ile Gln Asn Glu Leu Val Ala Ser Ala Ala Ala Thr Arg
   210 215 220
   Ile Ala His Glu Thr Asn Pro Asp Ile Gln Val Gly Cys Met Ile Leu
   225 230 235 240
   Ala Asp Pro Thr Tyr Pro Leu Thr Pro Asp Pro Arg Asp Val Trp Ala
   245 250 255
   Ala Lys Gln Ala Glu Arg Ala Asn Tyr Ala Phe Gly Asp Leu His Val
   260 265 270
   Arg Gly Glu Tyr Pro Gly Tyr Leu Arg Arg Thr Leu Arg Asp Lys Gly
   275 280 285
   Ile Glu Leu Glu Ile Thr Glu Glu Asp Arg Val Leu Leu Arg Glu His
   290 295 300
   Thr Val Asp Phe Val Ser Phe Ser Tyr Tyr Met Xaa Val Cys Glu Thr
   305 310 315 320
   Val Thr Gln Ser Ala Glu Ala Gly Arg Gly Asn Leu Met Gly Gly Val
   325 330 335
   Pro Asn Pro Thr Leu Glu Ala Ser Glu Trp Gly Trp Gln Ile Asp Pro
   340 345 350
   Ala Gly Leu Arg Thr Ile Leu Asn Asp Tyr Trp Asp Arg Trp Gly Lys
   355 360 365
   Pro Leu Phe Ile Val Glu Asn Gly Leu Gly Ala Lys Asp Val Leu Val
   370 375 380
   Asp Gly Pro Asn Gly Pro Thr Val Glu Asp Asp Tyr Arg Ile Ala Tyr
   385 390 395 400
   Met Asn Asp His Leu Val Gln Val Ala Glu Ala Ile Ala Asp Gly Val
   405 410 415
   Glu Val Leu Gly Tyr Thr Ser Trp Gly Cys Ile Asp Leu Val Ser Ala
   420 425 430
   Ser Thr Ala Gln Met Ser Lys Arg Tyr Gly Phe Ile Tyr Val Asp Arg
           435                 440                 445
   Asp Asp Gly Gly Asn Gly Thr Leu Ala Arg Tyr Arg Lys Lys Ser Phe
       450                 455                 460
   Gly Trp Tyr Arg Asp Val Ile Ala Ser Asn Gly Ala Ser Leu Val Pro
   465                 470                 475                 480
   Pro Val Gln Glu Pro Pro Arg Gly
                   485
<210> 8
   <211> 3072
   <212> DNA
   <213> Streptococcus salivarius
<220>
   <221> CDS
   <222> (392)..(2860)
   <223> 1,6-alpha-glucanhydrolase
<400> 8
   aactgaggcc gttgctccag tagcgacaac agaaataggt ccatcaactg ctactgttgc      60
   gacagatact gcaacaacag cgacagcttc tacaatcttt tcacaagctg tgccagcaga     120
   aagtgctagc tcagaaacgc ttgtagccag tgaagcacta gctcctgagt cagctgctgt     180
   ggaaaccatc acatcatcat ctgataatgc tactgaagca ggacgccatt caactgctca     240
   agtaacacca gttacagaag tgacagagca aaacttgaat ggtgatgcct acttgacaga     300
   tccagaaaca acaaaagcag cttatagcaa gacagatggt gatattaatt attccgttgt     360
   tgtgtctaat ccaacagcag aaactaagac g atg act gtc aac ttg aca ctt       412
                                      Met Thr Val Asn Leu Thr Leu
                                      1               5
   caa cat gct tca gaa att atc ggt caa gat aac gtt gac ctt acg cta      460
   Gln His Ala Ser Glu Ile Ile Gly Gln Asp Asn Val Asp Leu Thr Leu
           10                  15                  20
   gcg gca gga gct tca gcc aag gtt tca aac ttg aca gta gcg tca gag      508
   Ala Ala Gly Ala Ser Ala Lys Val Ser Asn Leu Thr Val Ala Ser Glu
       25                  30                  35
   tgg ttg aca aac aat aca ggt tac ttg gtg aca atc agt gtc aac gat      556
   Trp Leu Thr Asn Asn Thr Gly Tyr Leu Val Thr Ile Ser Val Asn Asp
   40                  45                  50                  55
   aaa tca ggc aat gtc ttg tca agc aag cgc gct ggc ttg tct gtt gaa      604
   Lys Ser Gly Asn Val Leu Ser Ser Lys Arg Ala Gly Leu Ser Val Glu
                   60                  65                  70
   gat gat tgg aca gtt ttc cca cgt tac ggt atc gta gca ggt tca cca      652
   Asp Asp Trp Thr Val Phe Pro Arg Tyr Gly Ile Val Ala Gly Ser Pro
               75                  80                  85
   act gat caa aac agt att ctt gtt aaa aat ctt gaa gcc tac cgt aaa      700
   Thr Asp Gln Asn Ser Ile Leu Val Lys Asn Leu Glu Ala Tyr Arg Lys
           90                  95                  100
   gag ctt gag ctc atg aag tct atg aat atc aac tca tat ttc ttc tat      748
   Glu Leu Glu Leu Met Lys Ser Met Asn Ile Asn Ser Tyr Phe Phe Tyr
       105                 110                 115
   gat gct tat aat gaa gct aca gat cct ttc cca gaa ggt gtc gat agc      796
   Asp Ala Tyr Asn Glu Ala Thr Asp Pro Phe Pro Glu Gly Val Asp Ser
   120                 125                 130                 135
   ttt gtt caa aaa tgg aat acc tgg agt cac act cag gtt gac act aag      844
   Phe Val Gln Lys Trp Asn Thr Trp Ser His Thr Gln Val Asp Thr Lys
                   140                 145                 150
   gct gtt aaa gaa ttg gtt gat caa gtt cat aag tca ggt gct gtt gcc      892
   Ala Val Lys Glu Leu Val Asp Gln Val His Lys Ser Gly Ala Val Ala
               155                 160                 165
   atg ctt tat aac atg att tca gca gat tca aat cca aag aat ccg gcc      940
   Met Leu Tyr Asn Met Ile Ser Ala Asp Ser Asn Pro Lys Asn Pro Ala
           170                 175                 180
   ctt cca ctt gct gct ttg gct tat aac ttc tac gat agc ttt ggt aag      988
   Leu Pro Leu Ala Ala Leu Ala Tyr Asn Phe Tyr Asp Ser Phe Gly Lys
       185                 190                 195
   aag ggt gaa ccg atg act tac act atc ggt gat aac cca act caa gtt     1036
   Lys Gly Glu Pro Met Thr Tyr Thr Ile Gly Asp Asn Pro Thr Gln Val
   200                 205                 210                 215
   tac tat gat ccg gcg aat cca gat tgg caa aaa tac atc gca ggt gtc     1084
   Tyr Tyr Asp Pro Ala Asn Pro Asp Trp Gln Lys Tyr Ile Ala Gly Val
                   220                 225                 230
   atg aaa tca gct atg gat cgt atg gga ttc gat ggt tgg caa ggt gat     1132
   Met Lys Ser Ala Met Asp Arg Met Gly Phe Asp Gly Trp Gln Gly Asp
               235                 240                 245
   aca att ggt gac aac cgt gtg act gat tat gag cac cgt aac agc aca     1180
   Thr Ile Gly Asp Asn Arg Val Thr Asp Tyr Glu His Arg Asn Ser Thr
           250                 255                 260
   gac gag gct gac tca cac atg atg tct gat tca tat gcg tca ttt att     1228
   Asp Glu Ala Asp Ser His Met Met Ser Asp Ser Tyr Ala Ser Phe Ile
       265                 270                 275
   aat gcc atg aag gac ctc atc ggt gaa aag tac tac atc aca atc aat     1276
   Asn Ala Met Lys Asp Leu Ile Gly Glu Lys Tyr Tyr Ile Thr Ile Asn
   280                 285                 290                 295
   gat gtt aat ggt ggt aat gat gat aaa cta gcc aag gca cgt caa gat     1324
   Asp Val Asn Gly Gly Asn Asp Asp Lys Leu Ala Lys Ala Arg Gln Asp
                   300                 305                 310
   gtt gtt tat aat gag ctt tgg aca aac ggt ggt tca gtt att cca gga     1372
   Val Val Tyr Asn Glu Leu Trp Thr Asn Gly Gly Ser Val Ile Pro Gly
               315                 320                 325
   cgt atg cag gtt gcc tat ggt gat ttg aaa gca cgt atc gat atg gta     1420
   Arg Met Gln Val Ala Tyr Gly Asp Leu Lys Ala Arg Ile Asp Met Val
           330                 335                 340
   cgc aat aaa act ggt aaa tca ctt atc gtt ggt gcc tac atg gaa gaa     1468
   Arg Asn Lys Thr Gly Lys Ser Leu Ile Val Gly Ala Tyr Met Glu Glu
       345                 350                 355
   cca ggg att gat tat act gtt cct ggc gga aaa gca act aac ggt gct      1516
   Pro Gly Ile Asp Tyr Thr Val Pro Gly Gly Lys Ala Thr Asn Gly Ala
   360                 365                 370                 375
   ggt aaa gat gcc ctt gct ggt aaa cca ttg caa gct gat gcg act ctt      1564
   Gly Lys Asp Ala Leu Ala Gly Lys Pro Leu Gln Ala Asp Ala Thr Leu
                   380                 385                 390
   ctc gta gat gcg aca gta gct gca gca ggt ggt tat cac atg tcc att      1612
   Leu Val Asp Ala Thr Val Ala Ala Ala Gly Gly Tyr His Met Ser Ile
               395                 400                 405
   gca gcc ctt gca aat gct aat gcg gcc ctt aac gtc ctt caa agt gcc      1660
   Ala Ala Leu Ala Asn Ala Asn Ala Ala Leu Asn Val Leu Gln Ser Ala
           410                 415                 420
   tat tac cca acg caa tac ctc agt gtg gct aaa gac act att cgt aag      1708
   Tyr Tyr Pro Thr Gln Tyr Leu Ser Val Ala Lys Asp Thr Ile Arg Lys
       425                 430                 435
   ctt tac aat tac caa cag ttt atc act gct tat gaa aat ctt ctc cgc      1756
   Leu Tyr Asn Tyr Gln Gln Phe Ile Thr Ala Tyr Glu Asn Leu Leu Arg
   440                 445                 450                 455
   ggt gag ggt gtg aca aac agc act cag gct gta tct aca aag aat gct      1804
   Gly Glu Gly Val Thr Asn Ser Thr Gln Ala Val Ser Thr Lys Asn Ala
                   460                 465                 470
   tct ggt gaa atc ctt tct aaa gat gct ctt ggt gtg aca gga gat caa      1852
   Ser Gly Glu Ile Leu Ser Lys Asp Ala Leu Gly Val Thr Gly Asp Gln
               475                 480                 485
   gtt tgg aca ttt gct aaa tca gga aaa ggt ttc tca act gtt caa atg      1900
   Val Trp Thr Phe Ala Lys Ser Gly Lys Gly Phe Ser Thr Val Gln Met
           490                 495                 500
   att aat atg atg ggc atc aat gcg ggc tgg cat aat gaa gag ggt tat      1948
   Ile Asn Met Met Gly Ile Asn Ala Gly Trp His Asn Glu Glu Gly Tyr
       505                 510                 515
   gcg gac aat aaa aca ccg gac gca caa gaa aat ctc aca gtt cgt ctt      1996
   Ala Asp Asn Lys Thr Pro Asp Ala Gln Glu Asn Leu Thr Val Arg Leu
   520                 525                 530                 535
   agc cta gca ggt aaa aca gcc caa gaa gca gct aaa att gct gat caa      2044
   Ser Leu Ala Gly Lys Thr Ala Gln Glu Ala Ala Lys Ile Ala Asp Gln
                   540                 545                 550
   gtc tat gtg acg tca ccg gat gat tgg gca act tca agc atg aag aag      2092
   Val Tyr Val Thr Ser Pro Asp Asp Trp Ala Thr Ser Ser Met Lys Lys
               555                 560                 565
   gca caa gca agc ctt gaa aca gat gaa aat ggt caa cca gtg ctt gtc      2140
   Ala Gln Ala Ser Leu Glu Thr Asp Glu Asn Gly Gln Pro Val Leu Val
           570                 575                 580
   att tca gtt cct aaa cta act ctt tgg aac atg ctt tat atc aag gaa      2188
   Ile Ser Val Pro Lys Leu Thr Leu Trp Asn Met Leu Tyr Ile Lys Glu
       585                 590                 595
   gac aca aca gca aca ccg gta gaa cca gtt act aac caa gct ggt aag      2236
   Asp Thr Thr Ala Thr Pro Val Glu Pro Val Thr Asn Gln Ala Gly Lys
   600                 605                 610                 615
   aaa gta gat aat acc gta aca tct gaa gca agc tca gaa aca gct aaa      2284
   Lys Val Asp Asn Thr Val Thr Ser Glu Ala Ser Ser Glu Thr Ala Lys
                   620                 625                 630
   tca gaa aat aca aca gta aat aaa ggt tca gag gct cca act gat acg      2332
   Ser Glu Asn Thr Thr Val Asn Lys Gly Ser Glu Ala Pro Thr Asp Thr
               635                 640                 645
   aaa cca tct gtt gaa gct cct aaa cta gat gaa aca act aaa cca gca      2380
   Lys Pro Ser Val Glu Ala Pro Lys Leu Asp Glu Thr Thr Lys Pro Ala
           650                 655                 660
   cca tca gtt gac gag tta gta aac tca gca gct gtt cca gtg gcg ata      2428
   Pro Ser Val Asp Glu Leu Val Asn Ser Ala Ala Val Pro Val Ala Ile
       665                 670                 675
   gct gtg tca gag acc gca cat gat aag aaa gat gac aac tca gta tct      2476
   Ala Val Ser Glu Thr Ala His Asp Lys Lys Asp Asp Asn Ser Val Ser
   680                 685                 690                 695
   aat acg gat caa ggt aca gta gca tca gat tca atc act aca cca gct      2524
   Asn Thr Asp Gln Gly Thr Val Ala Ser Asp Ser Ile Thr Thr Pro Ala
                   700                 705                 710
   tca gag gct gca agc aca gct gcc tca aca gtc tca tca gaa gta tca      2572
   Ser Glu Ala Ala Ser Thr Ala Ala Ser Thr Val Ser Ser Glu Val Ser
               715                 720                 725
   gaa agt gta aca gta tca tcg gaa cca tca gaa act gaa aat agt tca      2620
   Glu Ser Val Thr Val Ser Ser Glu Pro Ser Glu Thr Glu Asn Ser Ser
           730                 735                 740
   gaa gca tca act tca gag tca gca act cca acg acg aca gca att tca      2668
   Glu Ala Ser Thr Ser Glu Ser Ala Thr Pro Thr Thr Thr Ala Ile Ser
       745                 750                 755
   gaa tca cat gca gta gtt gaa cca gtg gct tct ttg aca gaa tca gag      2716
   Glu Ser His Ala Val Val Glu Pro Val Ala Ser Leu Thr Glu Ser Glu
   760                 765                 770                 775
   agt cag gca agc act agc ctt gtt tca gaa act aca agc aca att gtc      2764
   Ser Gln Ala Ser Thr Ser Leu Val Ser Glu Thr Thr Ser Thr Ile Val
                   780                 785                 790
   tca gtt gct ccg tca gaa gta tca gaa agc aca tca gag gaa gtc atc      2812
   Ser Val Ala Pro Ser Glu Val Ser Glu Ser Thr Ser Glu Glu Val Ile
               795                 800                 805
   ctt atg gac tat cag aaa aca tca ata gtt gga ata gac tct ctg tag      2860
   Leu Met Asp Tyr Gln Lys Thr Ser Ile Val Gly Ile Asp Ser Leu
           810                 815                 820
   ctcctcgcgt ctcagaaacc ttaccaagta cttctgaaac gattacagaa gcagcatcac    2920
   tctttagcaa ctatgcaaga tattcagaaa cagcaagctc agaatctcac tctatggtag    2980
   cagcttcttc agaagtttct attgaaaaat tagcagtatc tatcttgaaa gatactgagg    3040
   gaggcttgta tgatgcaaca acaatcagaa at                                  3072
<210> 9
   <211> 822
   <212> PRT
   <213> Streptococcus salivarius
<400> 9
   Met Thr Val Asn Leu Thr Leu Gln His Ala Ser Glu Ile Ile Gly Gln
   1               5                   10                  15
   Asp Asn Val Asp Leu Thr Leu Ala Ala Gly Ala Ser Ala Lys Val Ser
   20 25 30
   Asn Leu Thr Val Ala Ser Glu Trp Leu Thr Asn Asn Thr Gly Tyr Leu
   35 40 45
   Val Thr Ile Ser Val Asn Asp Lys Ser Gly Asn Val Leu Ser Ser Lys
   50 55 60
   Arg Ala Gly Leu Ser Val Glu Asp Asp Trp Thr Val Phe Pro Arg Tyr
   65 70 75 80
   Gly Ile Val Ala Gly Ser Pro Thr Asp Gln Asn Ser Ile Leu Val Lys
   85 90 95
   Asn Leu Glu Ala Tyr Arg Lys Glu Leu Glu Leu Met Lys Ser Met Asn
   100 105 110
   Ile Asn Ser Tyr Phe Phe Tyr Asp Ala Tyr Asn Glu Ala Thr Asp Pro
   115 120 125
   Phe Pro Glu Gly Val Asp Ser Phe Val Gln Lys Trp Asn Thr Trp Ser
   130 135 140
   His Thr Gln Val Asp Thr Lys Ala Val Lys Glu Leu Val Asp Gln Val
   145 150 155 160
   His Lys Ser Gly Ala Val Ala Met Leu Tyr Asn Met Ile Ser Ala Asp
   165 170 175
   Ser Asn Pro Lys Asn Pro Ala Leu Pro Leu Ala Ala Leu Ala Tyr Asn
   180 185 190
   Phe Tyr Asp Ser Phe Gly Lys Lys Gly Glu Pro Met Thr Tyr Thr Ile
   195 200 205
   Gly Asp Asn Pro Thr Gln Val Tyr Tyr Asp Pro Ala Asn Pro Asp Trp
   210 215 220
   Gln Lys Tyr Ile Ala Gly Val Met Lys Ser Ala Met Asp Arg Met Gly
   225 230 235 240
   Phe Asp Gly Trp Gln Gly Asp Thr Ile Gly Asp Asn Arg Val Thr Asp
   245 250 255
   Tyr Glu His Arg Asn Ser Thr Asp Glu Ala Asp Ser His Met Met Ser
   260 265 270
   Asp Ser Tyr Ala Ser Phe Ile Asn Ala Met Lys Asp Leu Ile Gly Glu
   275 280 285
   Lys Tyr Tyr Ile Thr Ile Asn Asp Val Asn Gly Gly Asn Asp Asp Lys
   290 295 300
   Leu Ala Lys Ala Arg Gln Asp Val Val Tyr Asn Glu Leu Trp Thr Asn
   305 310 315 320
   Gly Gly Ser Val Ile Pro Gly Arg Met Gln Val Ala Tyr Gly Asp Leu
   325 330 335
   Lys Ala Arg Ile Asp Met Val Arg Asn Lys Thr Gly Lys Ser Leu Ile
   340 345 350
   Val Gly Ala Tyr Met Glu Glu Pro Gly Ile Asp Tyr Thr Val Pro Gly
   355 360 365
   Gly Lys Ala Thr Asn Gly Ala Gly Lys Asp Ala Leu Ala Gly Lys Pro
   370 375 380
   Leu Gln Ala Asp Ala Thr Leu Leu Val Asp Ala Thr Val Ala Ala Ala
   385 390 395 400
   Gly Gly Tyr His Met Ser Ile Ala Ala Leu Ala Asn Ala Asn Ala Ala
   405 410 415
   Leu Asn Val Leu Gln Ser Ala Tyr Tyr Pro Thr Gln Tyr Leu Ser Val
   420 425 430
   Ala Lys Asp Thr Ile Arg Lys Leu Tyr Asn Tyr Gln Gln Phe Ile Thr
   435 440 445
   Ala Tyr Glu Asn Leu Leu Arg Gly Glu Gly Val Thr Asn Ser Thr Gln
   450 455 460
   Ala Val Ser Thr Lys Asn Ala Ser Gly Glu Ile Leu Ser Lys Asp Ala
   465 470 475 480
   Leu Gly Val Thr Gly Asp Gln Val Trp Thr Phe Ala Lys Ser Gly Lys
   485 490 495
   Gly Phe Ser Thr Val Gln Met Ile Asn Met Met Gly Ile Asn Ala Gly
   500 505 510
   Trp His Asn Glu Glu Gly Tyr Ala Asp Asn Lys Thr Pro Asp Ala Gln
   515 520 525
   Glu Asn Leu Thr Val Arg Leu Ser Leu Ala Gly Lys Thr Ala Gln Glu
   530 535 540
   Ala Ala Lys Ile Ala Asp Gln Val Tyr Val Thr Ser Pro Asp Asp Trp
   545 550 555 560
   Ala Thr Ser Ser Met Lys Lys Ala Gln Ala Ser Leu Glu Thr Asp Glu
   565 570 575
   Asn Gly Gln Pro Val Leu Val Ile Ser Val Pro Lys Leu Thr Leu Trp
   580 585 590
   Asn Met Leu Tyr Ile Lys Glu Asp Thr Thr Ala Thr Pro Val Glu Pro
   595 600 605
   Val Thr Asn Gln Ala Gly Lys Lys Val Asp Asn Thr Val Thr Ser Glu
   610 615 620
   Ala Ser Ser Glu Thr Ala Lys Ser Glu Asn Thr Thr Val Asn Lys Gly
   625 630 635 640
   Ser Glu Ala Pro Thr Asp Thr Lys Pro Ser Val Glu Ala Pro Lys Leu
   645 650 655
   Asp Glu Thr Thr Lys Pro Ala Pro Ser Val Asp Glu Leu Val Asn Ser
   660 665 670
   Ala Ala Val Pro Val Ala Ile Ala Val Ser Glu Thr Ala His Asp Lys
   675 680 685
   Lys Asp Asp Asn Ser Val Ser Asn Thr Asp Gln Gly Thr Val Ala Ser
   690 695 700
   Asp Ser Ile Thr Thr Pro Ala Ser Glu Ala Ala Ser Thr Ala Ala Ser
   705 710 715 720
   Thr Val Ser Ser Glu Val Ser Glu Ser Val Thr Val Ser Ser Glu Pro
   725 730 735
   Ser Glu Thr Glu Asn Ser Ser Glu Ala Ser Thr Ser Glu Ser Ala Thr
   740 745 750
   Pro Thr Thr Thr Ala Ile Ser Glu Ser His Ala Val Val Glu Pro Val
   755 760 765
   Ala Ser Leu Thr Glu Ser Glu Ser Gln Ala Ser Thr Ser Leu Val Ser
   770 775 780
   Glu Thr Thr Ser Thr Ile Val Ser Val Ala Pro Ser Glu Val Ser Glu
   785 790 795 800
   Ser Thr Ser Glu Glu Val Ile Leu Met Asp Tyr Gln Lys Thr Ser Ile
   805 810 815
   Val Gly Ile Asp Ser Leu
   820
<210> 10
   <211> 2220
   <212> DNA
   <213> Trichoderma longibrachiatum
<400> 10
   aaggttagcc aagaacaata gccgataaag atagcctcat taaacggaat gagctagtag 60
   gcaaagtcag cgaatgtgta tatataaagg ttcgaggtcc gtgcctccct catgctctcc 120
   ccatctactc atcaactcag atcctccagg agacttgtac accatctttt gaggcacaga 180
   aacccaatag tcaaccgcgg actggcatca tgtatcggaa gttggccgtc atcacggcct 240
   tcttggccac agctcgtgct cagtcggcct gcactctcca atcggagact cacccgcctc 300
   tgacatggca gaaatgctcg tctggtggca cttgcactca acagacaggc tccgtggtca 360
   tcgacgccaa ctggcgctgg actcacgcta cgaacagcag cacgaactgc tacgatggca 420
   acacttggag ctcgacccta tgtcctgaca acgagacctg cgcgaagaac tgctgtctgg 480
   acggtgccgc ctacgcgtcc acgtacggag ttaccacgag cggtaacagc ctctccattg 540
   gctttgtcac ccagtctgcg cagaagaacg ttggcgctcg cctttacctt atggcgagcg 600
   acacgaccta ccaggaattc accctgcttg gcaacgagtt ctctttcgat gttgatgttt 660
   cgcagctgcc gtaagtgact taccatgaac ccctgacgta tcttcttgtg ggctcccagc 720
   tgactggcca atttaaggtg cggcttgaac ggagctctct acttcgtgtc catggacgcg 780
   gatggtggcg tgagcaagta tcccaccaac aacgctggcg ccaagtacgg cacggggtac 840
   tgtgacagcc agtgtccccg cgatctgaag ttcatcaatg gccaggccaa cgttgagggc 900
   tgggagccgt catccaacaa cgcaaacacg ggcattggag gacacggaag ctgctgctct     960
   gagatggata tctgggaggc caactccatc tccgaggctc ttacccccca cccttgcacg    1020
   actgtcggcc aggagatctg cgagggtgat gggtgcggcg gaacttactc cgataacaga 1080
   tatggcggca cttgcgatcc cgatggctgc gactggaacc cataccgcct gggcaacacc 1140
   agcttctacg gccctggctc aagctttacc ctcgatacca ccaagaaatt gaccgttgtc 1200
   acccagttcg agacgtcggg tgccatcaac cgatactatg tccagaatgg cgtcactttc 1260
   cagcagccca acgccgagct tggtagttac tctggcaacg agctcaacga tgattactgc 1320
   acagctgagg agacagaatt cggcggatct ctttctcaga caagggcggc ctgactcagt 1380
   tcaagaaggc tacctctggc ggcatggttc tggtcatgag tctgtgggat gatgtgagtt 1440
   tgatggacaa acatgcgcgt tgacaaagag tcaagcagct gactgagatg ttacagtact 1500
   acgccaacat gctgtggctg gactccacct acccgacaaa cgagacctcc tccacacccg 1560
   gtgccgtgcg cggaagctgc tccaccagct ccggtgtccc tgctcaggtc gaatctcagt 1620
   ctcccaacgc caaggtcacc ttctccaaca tcaagttcgg acccattggc agcaccggca 1680
   accctagcgg cggcaaccct cccggcggaa accgtggcac caccaccacc cgccgcccag   1740
   ccactaccac tggaagctct cccggaccta cccagtctca ctacggccag tgcggcggta   1800
   ttggctacag cggccccacg gtctgcgcca gcggcacaac ttgccaggtc ctgaaccctt   1860
   actactctca gtgcctgtaa agctccgtgc gaaagcctga cgcaccggta gattcttggt   1920
   gagcccgtat catgacggcg gcgggagcta catggccccg ggtgatttat tttttttgta   1980
   tctacttctg acccttttca aatatacggt caactcatct ttcactggag atgcggcctg   2040
   cttggtattg cgatgttgtc agcttggcaa attgtggctt tcgaaaacac aaaacgattc   2100
   cttagtagcc atgcatttta agataacgga atagaagaaa gaggaaatta aaaaaaaaaa   2160
   aaaaacaaac atcccgttca taacccgtag aatcgccgct cttcgtgtat cccagtacca   2220
<210> 11
   <211> 1263
   <212> DNA
   <213> Phanerochaete chrysosporium
<220>
   <221> CDS
   <222> (34)..(1152)
   <223> ckg4 ligninase precursor
<400> 11
   gctacagctc accgtccggt ctcagcagca gca atg gcg ttc aag cag ctc ctc       54
                                        Met Ala Phe Lys Gln Leu Leu
                                        1               5
   gca gcc ctc tcc gtc gcc ctg acc ctc cag gtc acc caa gct gcc ccg        102
   Ala Ala Leu Ser Val Ala Leu Thr Leu Gln Val Thr Gln Ala Ala Pro
           10                  15                  20
   aac ctc gac aag cgc gtc gct tgc ccc gac ggc gtg cac acc gcc tcc        150
   Asn Leu Asp Lys Arg Val Ala Cys Pro Asp Gly Val His Thr Ala Ser
       25                  30                  35
   aac gcg gcg tgc tgt gca tgg ttc ccg gtc ctc gat gat atc cag cag        198
   Asn Ala Ala Cys Cys Ala Trp Phe Pro Val Leu Asp Asp Ile Gln Gln
   40                  45                  50                  55
   aac ctc ttc cac ggt ggc cag tgc ggt gcc gag gcc cac gag gcc ctt        246
   Asn Leu Phe His Gly Gly Gln Cys Gly Ala Glu Ala His Glu Ala Leu
                   60                  65                  70
   cgt atg gtc ttc cac gac tcc atc gct atc tcg ccc aag ctt cag tcg        294
   Arg Met Val Phe His Asp Ser Ile Ala Ile Ser Pro Lys Leu Gln Ser
               75                  80                  85
   cag ggc aag ttt ggc ggc ggc ggc gcg gac ggc tcg atc att acc ttc        342
   Gln Gly Lys Phe Gly Gly Gly Gly Ala Asp Gly Ser Ile Ile Thr Phe
           90                  95                  100
   tcc tcg atc gag acc acg tac cac ccg aac atc ggc ctc gac gag gtc        390
   Ser Ser Ile Glu Thr Thr Tyr His Pro Asn Ile Gly Leu Asp Glu Val
       105                 110                 115
   gtc gcc atc cag aag ccg ttc atc gcg aag cac ggc gtc acc cgt ggc        438
   Val Ala Ile Gln Lys Pro Phe Ile Ala Lys His Gly Val Thr Arg Gly
   120                 125                 130                 135
   gac ttc atc gca ttc gct ggt gcc gtc ggc gtg agc aac tgc ccg ggc      486
   Asp Phe Ile Ala Phe Ala Gly Ala Val Gly Val Ser Asn Cys Pro Gly
                   140                 145                 150
   gcg ccg cag atg cag ttc ttc ctt ggc cgc ccc gag gca acg cag gcc      534
   Ala Pro Gln Met Gln Phe Phe Leu Gly Arg Pro Glu Ala Thr Gln Ala
               155                 160                 165
   gcc ccc gac ggt ctc gtg ccc gag ccc ttc cac acc atc gat cag gtt      582
   Ala Pro Asp Gly Leu Val Pro Glu Pro Phe His Thr Ile Asp Gln Val
           170                 175                 180
   ctc gct cgc atg ctt gac gct ggt ggc ttc gac gag atc gag act gtc      630
   Leu Ala Arg Met Leu Asp Ala Gly Gly Phe Asp Glu Ile Glu Thr Val
       185                 190                 195
   tgg ctg ctc tct gcc cac tcc atc gcg gct gcg aac gac gtc gac ccg      678
   Trp Leu Leu Ser Ala His Ser Ile Ala Ala Ala Asn Asp Val Asp Pro
   200                 205                 210                 215
   acc atc tcc ggc ctg ccg ttc gac tcc act ccc ggc cag ttc gac tcc      726
   Thr Ile Ser Gly Leu Pro Phe Asp Ser Thr Pro Gly Gln Phe Asp Ser
                   220                 225                 230
   cag ttc ttc gtc gag acg cag ctc cgc ggt acc gca ttc cct ggc aag      774
   Gln Phe Phe Val Glu Thr Gln Leu Arg Gly Thr Ala Phe Pro Gly Lys
               235                 240                 245
   act ggt atc cag ggc acc gtc atg tcc ccg ctc aag ggc gag atg cgt      822
   Thr Gly Ile Gln Gly Thr Val Met Ser Pro Leu Lys Gly Glu Met Arg
           250                 255                 260
   ctg cag acg gac cac ttg ttc gcg cgt gac tcg cgc acg gca tgc gag      870
   Leu Gln Thr Asp His Leu Phe Ala Arg Asp Ser Arg Thr Ala Cys Glu
       265                 270                 275
   tgg cag tcc ttc gtc aac aac cag acg aag ctg cag gag gac ttc cag      918
   Trp Gln Ser Phe Val Asn Asn Gln Thr Lys Leu Gln Glu Asp Phe Gln
   280                 285                 290                 295
   ttc atc ttc acg gcg ctc tcg acg ctc ggc cac gac atg aac gcc atg      966
   Phe Ile Phe Thr Ala Leu Ser Thr Leu Gly His Asp Met Asn Ala Met
                   300                 305                 310
   atc gac tgc tcc gag gtc atc ccc gcg ccc aag ccc gtc aac ttc ggc     1014
   Ile Asp Cys Ser Glu Val Ile Pro Ala Pro Lys Pro Val Asn Phe Gly
               315                 320                 325
   ccg tcg ttc ttc ccc gcc ggt aag acg cac gcc gac atc gag cag gcc     1062
   Pro Ser Phe Phe Pro Ala Gly Lys Thr His Ala Asp Ile Glu Gln Ala
           330                 335                 340
   tgc gca tcc acg ccg ttc ccg acg ctc atc acc gcc ccc ggt ccc tct     1110
   Cys Ala Ser Thr Pro Phe Pro Thr Leu Ile Thr Ala Pro Gly Pro Ser
       345                 350                 355
   gcg tcc gtc gct cgc atc ccc ccg ccg ccg tcc ccc aac taa             1152
   Ala Ser Val Ala Arg Ile Pro Pro Pro Pro Ser Pro Asn
   360                 365                 370
   gctatgtcta tgctggacat gctctcggtt ctacctcgtc ggtatcgtcg cacggttatc   1212
   tcgcgtttgc atcatgtata cctgctcgtg gaatatacaa agtggtctat c            1263
<210> 12
   <211> 372
   <212> PRT
   <213> Phanerochaete chrysosporium
<400> 12
   Met Ala Phe Lys Gln Leu Leu Ala Ala Leu Ser Val Ala Leu Thr Leu
   1 5 10 15
   Gln Val Thr Gln Ala Ala Pro Asn Leu Asp Lys Arg Val Ala Cys Pro
   20 25 30
   Asp Gly Val His Thr Ala Ser Asn Ala Ala Cys Cys Ala Trp Phe Pro
   35 40 45
   Val Leu Asp Asp Ile Gln Gln Asn Leu Phe His Gly Gly Gln Cys Gly
   50 55 60
   Ala Glu Ala His Glu Ala Leu Arg Met Val Phe His Asp Ser Ile Ala
   65 70 75 80
   Ile Ser Pro Lys Leu Gln Ser Gln Gly Lys Phe Gly Gly Gly Gly Ala
   85 90 95
   Asp Gly Ser Ile Ile Thr Phe Ser Ser Ile Glu Thr Thr Tyr His Pro
   100 105 110
   Asn Ile Gly Leu Asp Glu Val Val Ala Ile Gln Lys Pro Phe Ile Ala
   115 120 125
   Lys His Gly Val Thr Arg Gly Asp Phe Ile Ala Phe Ala Gly Ala Val
   130 135 140
   Gly Val Ser Asn Cys Pro Gly Ala Pro Gln Met Gln Phe Phe Leu Gly
   145 150 155 160
   Arg Pro Glu Ala Thr Gln Ala Ala Pro Asp Gly Leu Val Pro Glu Pro
   165 170 175
   Phe His Thr Ile Asp Gln Val Leu Ala Arg Met Leu Asp Ala Gly Gly
   180 185 190
   Phe Asp Glu Ile Glu Thr Val Trp Leu Leu Ser Ala His Ser Ile Ala
   195 200 205
   Ala Ala Asn Asp Val Asp Pro Thr Ile Ser Gly Leu Pro Phe Asp Ser
   210 215 220
   Thr Pro Gly Gln Phe Asp Ser Gln Phe Phe Val Glu Thr Gln Leu Arg
   225 230 235 240
   Gly Thr Ala Phe Pro Gly Lys Thr Gly Ile Gln Gly Thr Val Met Ser
   245 250 255
   Pro Leu Lys Gly Glu Met Arg Leu Gln Thr Asp His Leu Phe Ala Arg
               260                 265                 270
   Asp Ser Arg Thr Ala Cys Glu Trp Gln Ser Phe Val Asn Asn Gln Thr
           275                 280                 285
   Lys Leu Gln Glu Asp Phe Gln Phe Ile Phe Thr Ala Leu Ser Thr Leu
       290                 295                 300
   Gly His Asp Met Asn Ala Met Ile Asp Cys Ser Glu Val Ile Pro Ala
   305                 310                 315                 320
   Pro Lys Pro Val Asn Phe Gly Pro Ser Phe Phe Pro Ala Gly Lys Thr
                   325                 330                 335
   His Ala Asp Ile Glu Gln Ala Cys Ala Ser Thr Pro Phe Pro Thr Leu
               340                 345                 350
   Ile Thr Ala Pro Gly Pro Ser Ala Ser Val Ala Arg Ile Pro Pro Pro
           355                 360                 365
   Pro Ser Pro Asn
       370
<210> 13
   <211> 1285
   <212> DNA
   <213> Phanerochaete chrysosporium
<220>
   <221> CDS
   <222> (34)..(1149)
   <223> CKG5 ligninase precursor
<400> 13
   gtcagactct ccaacggttg cctttggaca gac atg gcc ttc aag aag ctc ctt       54
                                        Met Ala Phe Lys Lys Leu Leu
                                        1               5
   gct gtt ctt acc gcc gct ctc tcc ctc cgc gct gcg cag ggt gcg gcc        102
   Ala Val Leu Thr Ala Ala Leu Ser Leu Arg Ala Ala Gln Gly Ala Ala
           10                  15                  20
   gtc gag aag cgc gcg acc tgc tcg aac ggc aag gtc gtc ccc gcg gcg        150
   Val Glu Lys Arg Ala Thr Cys Ser Asn Gly Lys Val Val Pro Ala Ala
       25                  30                  35
   tct tgc tgc acc tgg ttc aac gtt ctg tcc gat atc cag gag aac ctc        198
   Ser Cys Cys Thr Trp Phe Asn Val Leu Ser Asp Ile Gln Glu Asn Leu
   40                  45                  50                  55
   ttc aat ggc ggc cag tgt ggc gcc gag gct cat gag tcg atc cgt ctc        246
   Phe Asn Gly Gly Gln Cys Gly Ala Glu Ala His Glu Ser Ile Arg Leu
                   60                  65                  70
   gtc ttc cac gac gcc atc gct atc tct ccc gct atg gag ccg cag gcc        294
   Val Phe His Asp Ala Ile Ala Ile Ser Pro Ala Met Glu Pro Gln Ala
               75                  80                  85
   agt tcg gtg cga ggc gcc gat ggt tct atc atg atc ttc gac gag atc      342
   Ser Ser Val Arg Gly Ala Asp Gly Ser Ile Met Ile Phe Asp Glu Ile
           90                  95                  100
   gag acc aac ttc cat ccc aac atc ggt ctc gac gag atc gtc cgc ctg      390
   Glu Thr Asn Phe His Pro Asn Ile Gly Leu Asp Glu Ile Val Arg Leu
       105                 110                 115
   cag aag ccg ttc gtc cag aag cac ggt gtc act ccc ggt gac ttc atc      438
   Gln Lys Pro Phe Val Gln Lys His Gly Val Thr Pro Gly Asp Phe Ile
   120                 125                 130                 135
   gcc ttc gct ggc gcg gtg gcg ctc agt aac tgc ccc ggt gct ccg cag      486
   Ala Phe Ala Gly Ala Val Ala Leu Ser Asn Cys Pro Gly Ala Pro Gln
                   140                 145                 150
   atg aac ttc ttc act ggt cgt gct ccg gca act cag cca gcc cct gac      534
   Met Asn Phe Phe Thr Gly Arg Ala Pro Ala Thr Gln Pro Ala Pro Asp
               155                 160                 165
   ggc ctc gtc cca gag ccc ttc cac tct gtt gac caa atc atc gac cgt      582
   Gly Leu Val Pro Glu Pro Phe His Ser Val Asp Gln Ile Ile Asp Arg
           170                 175                 180
   gtc ttc gat gcc ggt gaa ttc gat gag ctc gag ctc gtc tgg atg ctc      630
   Val Phe Asp Ala Gly Glu Phe Asp Glu Leu Glu Leu Val Trp Met Leu
       185                 190                 195
   tct gca cac tcc gtc gcg gct gcc aac gat atc gac ccg aac atc cag      678
   Ser Ala His Ser Val Ala Ala Ala Asn Asp Ile Asp Pro Asn Ile Gln
   200                 205                 210                 215
   ggc ttg ccc ttc gac tcg acc ccc ggt att ttc gat tcc cag ttc ttc      726
   Gly Leu Pro Phe Asp Ser Thr Pro Gly Ile Phe Asp Ser Gln Phe Phe
                   220                 225                 230
   gtc gag act cag ctt gct ggc acc ggc ttc act ggc ggt tct aac aac      774
   Val Glu Thr Gln Leu Ala Gly Thr Gly Phe Thr Gly Gly Ser Asn Asn
               235                 240                 245
   cag ggc gag gtt tcc tcc ccg ctt cca ggc gag atg cgt ctc cag tct      822
   Gln Gly Glu Val Ser Ser Pro Leu Pro Gly Glu Met Arg Leu Gln Ser
           250                 255                 260
   gac ttc ctg atc gct cgt gac gcg cgc acc gcc tgc gag tgg cag tcg      870
   Asp Phe Leu Ile Ala Arg Asp Ala Arg Thr Ala Cys Glu Trp Gln Ser
       265                 270                 275
   ttc gtc aac aac cag tcc aag ctc gtc tcc gac ttc caa ttc atc ttc      918
   Phe Val Asn Asn Gln Ser Lys Leu Val Ser Asp Phe Gln Phe Ile Phe
   280                 285                 290                 295
   ctc gcc ctc act cag ctc ggc cag gac ccg gat gcg atg acc gac tgc      966
   Leu Ala Leu Thr Gln Leu Gly Gln Asp Pro Asp Ala Met Thr Asp Cys
                   300                 305                 310
   tct gct gtc atc ccc atc tcc aag ccc gcc ccg aac aac acc ccc gga     1014
   Ser Ala Val Ile Pro Ile Ser Lys Pro Ala Pro Asn Asn Thr Pro Gly
               315                 320                 325
   ttc tcc ttc ttc ccg ccc ggc atg acg atg gac gat gtc gag cag gct     1062
   Phe Ser Phe Phe Pro Pro Gly Met Thr Met Asp Asp Val Glu Gln Ala
           330                 335                 340
   tgc gcc gag acg ccc ttc ccg act ctc tcg act ctc cct ggc ccc gcg      1110
   Cys Ala Glu Thr Pro Phe Pro Thr Leu Ser Thr Leu Pro Gly Pro Ala
       345                 350                 355
   acc tcc gtc gct cgc atc cct cct cct cct ggt gct taa gcagccatca      1159
   Thr Ser Val Ala Arg Ile Pro Pro Pro Pro Gly Ala
   360                 365                 370
   gacttcggat cacaccccgg tattggcaac ggaaatttag aacgaagatc gtccagtgtt   1219
   ttgaagtaga aatgtgcttg tactgtgtaa acagctcttt tgacgaaata cactctgatt   1279
   tcgtcg                                                              1285
<210> 14
   <211> 371
   <212> PRT
   <213> *Phanerochaete chrysosporium.*
<400> 14
   Met Ala Phe Lys Lys Leu Leu Ala Val Leu Thr Ala Ala Leu Ser Leu
   1               5                   10                  15
   Arg Ala Ala Gln Gly Ala Ala Val Glu Lys Arg Ala Thr Cys Ser Asn
               20                  25                  30
   Gly Lys Val Val Pro Ala Ala Ser Cys Cys Thr Trp Phe Asn Val Leu
           35                  40                  45
   Ser Asp Ile Gln Glu Asn Leu Phe Asn Gly Gly Gln Cys Gly Ala Glu
       50                  55                  60
   Ala His Glu Ser Ile Arg Leu Val Phe His Asp Ala Ile Ala Ile Ser
   65                  70                  75                  80
   Pro Ala Met Glu Pro Gln Ala Ser Ser Val Arg Gly Ala Asp Gly Ser
                   85                  90                  95
   Ile Met Ile Phe Asp Glu Ile Glu Thr Asn Phe His Pro Asn Ile Gly
               100                 105                 110
   Leu Asp Glu Ile Val Arg Leu Gln Lys Pro Phe Val Gln Lys His Gly
           115                 120                 125
   Val Thr Pro Gly Asp Phe Ile Ala Phe Ala Gly Ala Val Ala Leu Ser
       130                 135                 140
   Asn Cys Pro Gly Ala Pro Gln Met Asn Phe Phe Thr Gly Arg Ala Pro
   145                 150                 155                 160
   Ala Thr Gln Pro Ala Pro Asp Gly Leu Val Pro Glu Pro Phe His Ser
                   165                 170                 175
   Val Asp Gln Ile Ile Asp Arg Val Phe Asp Ala Gly Glu Phe Asp Glu
   180 185 190
   Leu Glu Leu Val Trp Met Leu Ser Ala His Ser Val Ala Ala Ala Asn
   195 200 205
   Asp Ile Asp Pro Asn Ile Gln Gly Leu Pro Phe Asp Ser Thr Pro Gly
   210 215 220
   Ile Phe Asp Ser Gln Phe Phe Val Glu Thr Gln Leu Ala Gly Thr Gly
   225 230 235 240
   Phe Thr Gly Gly Ser Asn Asn Gln Gly Glu Val Ser Ser Pro Leu Pro
   245 250 255
   Gly Glu Met Arg Leu Gln Ser Asp Phe Leu Ile Ala Arg Asp Ala Arg
   260 265 270
   Thr Ala Cys Glu Trp Gln Ser Phe Val Asn Asn Gln Ser Lys Leu Val
   275 280 285
   Ser Asp Phe Gln Phe Ile Phe Leu Ala Leu Thr Gln Leu Gly Gln Asp
   290 295 300
   Pro Asp Ala Met Thr Asp Cys Ser Ala Val Ile Pro Ile Ser Lys Pro
   305 310 315 320
   Ala Pro Asn Asn Thr Pro Gly Phe Ser Phe Phe Pro Pro Gly Met Thr
   325 330 335
   Met Asp Asp Val Glu Gln Ala Cys Ala Glu Thr Pro Phe Pro Thr Leu
   340 345 350
   Ser Thr Leu Pro Gly Pro Ala Thr Ser Val Ala Arg Ile Pro Pro Pro
   355 360 365
   Pro Gly Ala
   370
<210> 15
   <211> 360
   <212> DNA
   <213> solanum tuberosum
<400> 15
   tgaccctaga cttgtccatc ttctggattg gccaagttaa ttaatgtatg aaataaaagg 60
   atgcacacat agtgacatgc taatcactat aatgtgggca tcaaagttgt gtgttatgtg 120
   taataactaa ttatctgaat aagagaaaga gagatcatcc atatttctta tcctaaatga 180
   atgacagtgt ctttataatt ctttgatgaa cagatgcatt ttattaacca attccatata 240
   catataaata ttaatcatat ataattaata tcaattggtt agcaaaaccc aaatctagtc 300
   taggtgtgtt ttgctaatta tgggggatag agcaaaaaag aaactaacgt ctcaagaatc 360
<210> 16
   <211> 2521
   <212> DNA
   <213> Agrobacterium tumefaciens
<220>
   <221> CDS
   <222> (585) .. (1826)
   <223> nopaline synthetase
<400> 16
   tagccgaccc agacgagcca agggatcttt ttggaatgct gctccgtcgt caggctttcc    60
   gacgtttggg tggttgaaca gaagtcatta tcgtacggaa tgccaagcac tcccgagggg   120
   aaccctgtgg ttggcatgca catacaaatg gacgaacgga taaacctttt cacgcccttt   180
   taaatatccg ttattctaat aaacgctctt ttctcttagg tttacccgcc aatatatcct   240
   gtcaaacact gatagtttaa actgaaggcg ggaaacgaca atctgatcat gagcggagaa   300
   ttaagggagt cacgttatga cccccgccga tgacgcggga caagccgttt tacgtttgga   360
   actgacagaa ccgcaacgat tgaaggagcc actcagccgc gggtttctgg agtttaatga   420
   gctaagcaca tacgtcagaa accattattg cgcgttcaaa agtcgcctaa ggtcactatc   480
   agctagcaaa tatttcttgt caaaaatgct ccactgacgt tccataaatt cccctcggta   540
   tccaattaga gtctcatatt cactctcaat ccaaataatc tgca atg gca att acc    596
                                                    Met Ala Ile Thr
                                                    1
   tta tcc gca act tct tta cct att tcc gcc gca gat cac cat ccg ctt      644
   Leu Ser Ala Thr Ser Leu Pro Ile Ser Ala Ala Asp His His Pro Leu
   5                   10                  15                  20
   ccc ttg acc gta ggt gtc ctc ggt tct ggt cac gcg ggg act gca tta      692
   Pro Leu Thr Val Gly Val Leu Gly Ser Gly His Ala Gly Thr Ala Leu
                   25                  30                  35
   gcg gct tgg ttc gcc tcc cgg cat gtt ccc acg gcg ctg tgg gca cca      740
   Ala Ala Trp Phe Ala Ser Arg His Val Pro Thr Ala Leu Trp Ala Pro
               40                  45                  50
   gca gat cat cca gga tcg atc tca gca atc aag gcc aat gaa gga gtt      788
   Ala Asp His Pro Gly Ser Ile Ser Ala Ile Lys Ala Asn Glu Gly Val
           55                  60                  65
   atc acc acc gag gga atg att aac ggt cca ttt agg gtc tca gcc tgt      836
   Ile Thr Thr Glu Gly Met Ile Asn Gly Pro Phe Arg Val Ser Ala Cys
       70                  75                  80
   gat gac ctt gcc gca gtt att cgc tcc agc cgt gta ctg att att gta      884
   Asp Asp Leu Ala Ala Val Ile Arg Ser Ser Arg Val Leu Ile Ile Val
   85                  90                  95                  100
   acc cgt gcg gac gtt cac gac agc ttc gtc aac gaa ctc gcc aac ttc      932
   Thr Arg Ala Asp Val His Asp Ser Phe Val Asn Glu Leu Ala Asn Phe
                   105                 110                 115
   aac ggc gaa ctc gca aca aag gat att gtc gtc gtg tgc ggc cat ggc      980
   Asn Gly Glu Leu Ala Thr Lys Asp Ile Val Val Val Cys Gly His Gly
                   120                 125                 130
   ttc tcc atc aag tac gag aga cag ctg cga ttc aag cga ata ttc gag     1028
   Phe Ser Ile Lys Tyr Glu Arg Gln Leu Arg Phe Lys Arg Ile Phe Glu
           135                 140                 145
   acg gat aat tcg ccc ata acg tct aag cta tcg gat caa aaa aaa tgt     1076
   Thr Asp Asn Ser Pro Ile Thr Ser Lys Leu Ser Asp Gln Lys Lys Cys
       150                 155                 160
   aac gtc aac atc aag gaa atg aaa gcg tct ttc gga ctg tca tgt ttc     1124
   Asn Val Asn Ile Lys Glu Met Lys Ala Ser Phe Gly Leu Ser Cys Phe
   165                 170                 175                 180
   cca att cat cgc gat gat gct ggc gtg att gat cta ccc gaa gat acc     1172
   Pro Ile His Arg Asp Asp Ala Gly Val Ile Asp Leu Pro Glu Asp Thr
                   185                 190                 195
   aag aac atc ttt gcc cag cta ttt tcc gct aga atc atc tgc atc ccg     1220
   Lys Asn Ile Phe Ala Gln Leu Phe Ser Ala Arg Ile Ile Cys Ile Pro
               200                 205                 210
   ccg ttg caa gtg cta ttc ttt tcc aac tgt atc act cat gcg gtt ccg     1268
   Pro Leu Gln Val Leu Phe Phe Ser Asn Cys Ile Thr His Ala Val Pro
           215                 220                 225
   gca gtc atg aac atc gga aga ctc cgc gac cca gcc aat tct ctt act     1316
   Ala Val Met Asn Ile Gly Arg Leu Arg Asp Pro Ala Asn Ser Leu Thr
       230                 235                 240
   aaa aga gct gag aag tgg ctt ctt gaa cta gac gag cga acc cca cga     1364
   Lys Arg Ala Glu Lys Trp Leu Leu Glu Leu Asp Glu Arg Thr Pro Arg
   245                 250                 255                 260
   gcc gag aag ggc ttt ttc ttt tat ggt gaa gga tcc aac act tac gtt     1412
   Ala Glu Lys Gly Phe Phe Phe Tyr Gly Glu Gly Ser Asn Thr Tyr Val
                   265                 270                 275
   tgc aac gtc caa gag caa ata gac cac gaa cgc cgg aag gtt gcc gca     1460
   Cys Asn Val Gln Glu Gln Ile Asp His Glu Arg Arg Lys Val Ala Ala
               280                 285                 290
   gcg tgt gga ttg cgt ctc aat tct ctc ttg cag gaa tgc aat gat gaa     1508
   Ala Cys Gly Leu Arg Leu Asn Ser Leu Leu Gln Glu Cys Asn Asp Glu
           295                 300                 305
   tat gat act gac tat gaa act ttg agg gaa tac tgc cta gca ccg tca     1556
   Tyr Asp Thr Asp Tyr Glu Thr Leu Arg Glu Tyr Cys Leu Ala Pro Ser
       310                 315                 320
   cct cat aac gtg cat cat gca tgc cct gac aac atg gaa cat cgc tat     1604
   Pro His Asn Val His His Ala Cys Pro Asp Asn Met Glu His Arg Tyr
   325                 330                 335                 340
   ttt tct gaa gaa tta tgc tcg ttg gag gat gtc gcg gca att gca gct     1652
   Phe Ser Glu Glu Leu Cys Ser Leu Glu Asp Val Ala Ala Ile Ala Ala
                   345                 350                 355
   att gcc aac atc gaa cta ccc ctc acg cat gca ttc atc aat att att     1700
   Ile Ala Asn Ile Glu Leu Pro Leu Thr His Ala Phe Ile Asn Ile Ile
               360                 365                 370
   cat gcg ggg aaa ggc aag att aat cca act ggc aaa tca tcc agc gtg     1748
   His Ala Gly Lys Gly Lys Ile Asn Pro Thr Gly Lys Ser Ser Ser Val
           375                 380                 385
   att ggt aac ttc agt tcc agc gac ttg att cgt ttt ggt gct acc cac     1796
   Ile Gly Asn Phe Ser Ser Ser Asp Leu Ile Arg Phe Gly Ala Thr His
       390                 395                 400
   gtt ttc aat aag gac gag atg gtg gag taa agaaggagtg cgtcgaagca      1846
   Val Phe Asn Lys Asp Glu Met Val Glu
   405                 410
   gatcgttcaa acatttggca ataaagtttc ttaagattga atcctgttgc cggtcttgcg   1906
   atgattatca tataatttct gttgaattac gttaagcatg taataattaa catgtaatgc   1966
   atgacgttat ttatgagatg ggtttttatg attagagtcc cgcaattata catttaatac   2026
   gcgatagaaa acaaaatata gcgcgcaaac taggataaat tatcgcgcgc ggtgtcatct   2086
   atgttactag atcgatcaaa cttcggtact gtgtaatgac gatgagcaat cgagaggctg   2146
   actaacaaaa ggtatgccca aaaacaacct ctccaaactg tttcgaattg gaagtttctg   2206
   ctcatgccga caggcataac ttagatattc gcgggctatt cccactaatt cgtcctgctg   2266
   gtttgcgcca agataaatca gtgcatctcc ttacaagttc ctctgtcttg tgaaatgaac   2326
   tgctgactgc cccccaagaa agcctcctca tctcccagtt ggcggcggct gatacaccat   2386
   cgaaaaccca cgtccgaaca cttgatacat gtgcctgaga aataggccta cgtccaagag   2446
   caagtccttt ctgtgctcgt cggaaattcc tctcctgtca gacggtcgtg cgcatgtctt   2506
   gcgttgatga agctt                                                    2521
<210> 17
   <211> 413
   <212> PRT
   <213> Agrobacterium tumefaciens
<400> 17
   Met Ala Ile Thr Leu Ser Ala Thr Ser Leu Pro Ile Ser Ala Ala Asp
   1               5                   10                  15
   His His Pro Leu Pro Leu Thr Val Gly Val Leu Gly Ser Gly His Ala
               20                  25                  30
   Gly Thr Ala Leu Ala Ala Trp Phe Ala Ser Arg His Val Pro Thr Ala
           35                  40                  45
   Leu Trp Ala Pro Ala Asp His Pro Gly Ser Ile Ser Ala Ile Lys Ala
       50                  55                  60
   Asn Glu Gly Val Ile Thr Thr Glu Gly Met Ile Asn Gly Pro Phe Arg
   65                  70                  75                  80
   Val Ser Ala Cys Asp Asp Leu Ala Ala Val Ile Arg Ser Ser Arg Val
                   85                  90                  95
   Leu Ile Ile Val Thr Arg Ala Asp Val His Asp Ser Phe Val Asn Glu
               100                 105                 110
   Leu Ala Asn Phe Asn Gly Glu Leu Ala Thr Lys Asp Ile Val Val Val
   115 120 125
   Cys Gly His Gly Phe Ser Ile Lys Tyr Glu Arg Gln Leu Arg Phe Lys
   130 135 140
   Arg Ile Phe Glu Thr Asp Asn Ser Pro Ile Thr Ser Lys Leu Ser Asp
   145 150 155 160
   Gln Lys Lys Cys Asn Val Asn Ile Lys Glu Met Lys Ala Ser Phe Gly
   165 170 175
   Leu Ser Cys Phe Pro Ile His Arg Asp Asp Ala Gly Val Ile Asp Leu
   180 185 190
   Pro Glu Asp Thr Lys Asn Ile Phe Ala Gln Leu Phe Ser Ala Arg Ile
   195 200 205
   Ile Cys Ile Pro Pro Leu Gln Val Leu Phe Phe Ser Asn Cys Ile Thr
   210 215 220
   His Ala Val Pro Ala Val Met Asn Ile Gly Arg Leu Arg Asp Pro Ala
   225 230 235 240
   Asn Ser Leu Thr Lys Arg Ala Glu Lys Trp Leu Leu Glu Leu Asp Glu
   245 250 255
   Arg Thr Pro Arg Ala Glu Lys Gly Phe Phe Phe Tyr Gly Glu Gly Ser
   260 265 270
   Asn Thr Tyr Val Cys Asn Val Gln Glu Gln Ile Asp His Glu Arg Arg
   275 280 285
   Lys Val Ala Ala Ala Cys Gly Leu Arg Leu Asn Ser Leu Leu Gln Glu
   290 295 300
   Cys Asn Asp Glu Tyr Asp Thr Asp Tyr Glu Thr Leu Arg Glu Tyr Cys
   305 310 315 320
   Leu Ala Pro Ser Pro His Asn Val His His Ala Cys Pro Asp Asn Met
   325 330 335
   Glu His Arg Tyr Phe Ser Glu Glu Leu Cys Ser Leu Glu Asp Val Ala
   340 345 350
   Ala Ile Ala Ala Ile Ala Asn Ile Glu Leu Pro Leu Thr His Ala Phe
   355 360 365
   Ile Asn Ile Ile His Ala Gly Lys Gly Lys Ile Asn Pro Thr Gly Lys
   370 375 380
   Ser Ser Ser Val Ile Gly Asn Phe Ser Ser Ser Asp Leu Ile Arg Phe
   385                 390                 395                 400
   Gly Ala Thr His Val Phe Asn Lys Asp Glu Met Val Glu
                   405                 410
<210> 18
   <211> 835
   <212> DNA
   <213> Streptomyces hygroscopicus
<400> 18
   gctcgctgtc attttcgaga cgccatcttt ggaagcggtg gccgaatccg tactgcgcgg      60
   actcgacgac gcgtaaaacg atcgaccacg tacacgagtc cggacacggg gcgaggaggc     120
   ccggttccgg caccgaggaa gaccgaagga agaccacacg tgagcccaga acgacgcccg     180
   gccgacatcc gccgtgccac cgaggcggac atgccggcgg tctgcaccat cgtcaaccac     240
   tacatcgaga caagcacggt caacttccgt accgagccgc aggaaccgca ggagtggacg     300
   gacgacctcg tccgtctgcg ggagcgctat ccctggctcg tcgccgaggt ggacggcgag     360
   gtcgccggca tcgcctacgc gggcccctgg aaggcacgca acgcctacga ctggacggcc     420
   gagtcgaccg tgtacgtctc cccccgccac cagcggacgg gactgggctc cacgctctac     480
   acccacctgc tgaagtccct ggaggcacag ggcttcaaga gcgtggtcgc tgtcatcggg     540
   ctgcccaacg acccgagcgt gcgcatgcac gaggcgctcg gatatgcccc ccgcggcatg     600
   ctgcgggcgg ccggcttcaa gcacgggaac tggcatgacg tgggtttctg gcagctggac     660
   ttcagcctgc cggtaccgcc ccgtccggtc ctgcccgtca ccgagatctg aacggagtgc     720
   gcgtgggcat cgcccgagtt ggagctggta cgggaactca tcgaactcaa ctggcatacc     780
   cgcaatggtg aggtggaacc gcggcggatc gcgtacgacc gtgcccagga ggcct          835
<210> 19
   <211> 623
   <212> DNA
   <213> Oryza sativa
<400> 19
   tatatacata cccccccctc tcctcccatc cccccaaccc taccaccacc accaccacca 60
   cctcctcccc cctcgctgcc ggacgacgag ctcctccccc ctccccctcc gccgccgccg 120
   gtaaccaccc cgcgtccctc tcctctttct ttctccgttt tttttttccg tctcgtctcg 180
   atctttggcc ttggtagttt gggggcgaga ggcggcttcg tcgcccagat cggtgcgcgg 240
   gaggggcggg atctcgcggc tgggtctcgg cgtgcggccg gatcctcgcg gggaatgggg 300
   ctctcggatg tagatctgat ccgccgttgt tgggggagat gatggggcgt ttaaaatttc 360
   gccatgctaa acaagatcag gaagagggga aaagggcact atggtttata tttttatata 420
   tttctgctgc tgctcgtcag gcttagatgt gctagatctt tctttcttct ttttgtgggt 480
   agaatttgaa tccctcagca ttgttcatcg gtagtttttc ttttcatgat ttgtgacaaa 540
   tgcagcctcg tgcggagctt ttttgtaggt agaagatggc tgacgccgag gatgggggat 600
   ccccgggtgg tcagtccctt atg 623
<210> 20
   <211> 1506
   <212> DNA
   <213> Trichoderma longibrachiatum
<400> 20
   accatggctc aatctgcttg tactcttcaa tctgagactc atcctccact tacttggcag 60
   aagtgttcat ctggtggtac ttgtactcaa cagactggat ctgttgttat tgatgctaac 120
   tggagatgga ctcatgctac taactcttct actaactgct atgatggtaa cacttggtca 180
   tctactcttt gtcctgataa cgagacttgt gctaagaact gctgtcttga tggtgctgct 240
   tacgcttcta cttacggagt tactacttct ggaaactctc tttctattgg attcgttact 300
   cagtctgctc agaagaacgt tggtgctagg ttgtacttga tggcttctga tactacttac 360
   caagagttca ctcttcttgg taacgagttc tctttcgatg ttgatgtttc tcaacttcca 420
   tgtggtttga acggagcttt gtacttcgtt tctatggatg ctgatggtgg agtttctaag 480
   tatccaacta acactgctgg agctaagtat ggtactggtt actgtgattc tcagtgtcca 540
   agagatctta agttcattaa cggacaagct aatgttgagg gatgggagcc atcttctaac 600
   aatgctaaca ctggtattgg aggtcatgga tcttgttgct ctgagatgga tatttgggag 660
   gctaactcta tctctgaggc tcttactcca catccatgca ctactgttgg acaagagatt 720
   tgcgagggag atggttgtgg tggaacttac tctgataaca gatacggagg tacttgcgat 780
   ccagatggat gtgattggaa tccatacaga cttggtaaca cttctttcta cggtccagga 840
   tcttcattca ctcttgatac tactaagaag ttgactgttg ttactcagtt cgagacttct 900
   ggtgctatca acagatacta cgttcagaat ggagttactt tccaacaacc taacgctgag 960
   cttggttctt actctggtaa cgagttgaac gatgattact gtactgctga ggaagctgag 1020
   tttggtggat catctttctc tgataagggt ggacttactc agttcaagaa ggctacttct 1080
   ggtggtatgg ttcttgttat gtctctttgg gatgattact acgctaacat gttgtggctt 1140
   gattctactt acccaactaa cgagacttct tctactccag gtgctgttag aggatcttgc 1200
   tctacttctt ctggtgttcc tgctcaagtt gaatctcaat ctcctaatgc taaggttact 1260
   ttctctaaca tcaagttcgg accaattgga tctactggta acccttctgg aggtaatcca 1320
   cctggaggta atccacctgg aactactaca actaggagac cagctactac aactggatca 1380
   tctccaggac ctactcaatc tcattacggt caatgtggag gtattggtta ctctggtcca 1440
   actgtttgtg cttctggaac tacttgtcaa gttcttaacc cttactattc tcaatgcctt 1500
   taatga 1506
<210> 21
   <211> 26
   <212> DNA
   <213> Artificial Sequence
<220>
   <223> PCR primer SP1F
<400> 21
   ccgcctaggc gcatggcccc ctccgt 26
<210> 22
   <211> 25
   <212> DNA
   <213> Artificial Sequence
<220>
   <223> PCR primer SP3R
<400> 22
   cgctgtacac gcacctgatc ctgcc 25
<210> 23
   <211> 3124
   <212> DNA
   <213> Butyrivibrio fibrisolvens
<400> 23
   aacaaacaga attgttggaa acttttttat atgcaatatt actttaacac agaatgtctg 60
   gtgatggtat atgttttttc ttcctgagtt atatatttca aatcttagat gtgctatgct 120
   tttatggcag aaacatgtta tatacaaggt aaagataagt aggataacga ggtatttata 180
   atggagaaat gggcaagaat caaatataca ccaaatcttc cgcttggaga gaatggtgaa 240
   agggttacag cgagtcagaa gcacattgag ctttcatgcg aggcagcatg tgagggaatg 300
   gtacttctca agaatgacag aaacgttctt cctatcagaa agggcacaag agtagccctc 360
   tttggaaagg gagtatttga ctatgtaaaa ggcggcggtg gtagcggaga tgtaacagtt 420
   ccttacatca gaaacctcta cgaaggcctt tctcagtaca catcagacat ttcaatttac 480
   gacaaatctg tcagattcta tcaggaatat gtagcagacc agtacagact tggaattgca 540
   ccaggcatga tcaaagagcc ggctcttccg gaagatattc ttgcagatgc agcagcctat 600
   gcagatactg caatcatcgc aatcagcaga ttctccggag aaggctggga cagaaaggtt 660
   gcaggcgttg acagagaaat caagtgcgaa gccaaggacc tcgtagagca gggcaacaag 720
   atatttgatc atggtgattt ctacctcaca aatgctgaga agaagatggt caagatggta 780
   aaagagaact tctcaagcgt cattgtagtc atgaatgtcg gaggagtcgt agacacaaca 840
   tggtttaaaa aggatgacca gatttcatca gtcctcatgg catggcaggg tggaattgaa 900
   ggcggacttg ccgcagccag gatccttctt ggcaaggtta atccttcagg taagctctca 960
   gatacattcg cagcaaggct tgaagactat ccttcaacag agggcttcca cgaagatgat 1020
   gactacgtgg attacacaga agatatctac gttggctata gatatttcga gaccattccc 1080
   ggggcaaaag agaaagttaa ctaccccttt ggctatggcc tttcctatac aactttcctg 1140
   cttgaagact ataaggcaga gccttttgtg gcttcagcag cagacgaggt cggtaaatct 1200
   gatagcgacc ttgcagatgc aatcgtagcc tcagttacag tcacaaacat tggcaagatt 1260
   ccgggcaaag aggttgttca gctctactac agcgctcctc agggcaagct cggtaagcct 1320
   gctaaagtcc ttggcggcta tgccaagaca aggctactgc agccgggaga gagccagaga 1380
   gtgacaattg ctctttatat ggaggatatg gcatcttacg acgaccttgg caaggttaaa 1440
   aaggctgcct ggctccttga aaaaggtgaa tatcatttct tccttggaac atcagtaaga 1500
   gacacaaggc ttcttgatta cacctatgaa ctttctaaga acataatagt tgaacaggtc 1560
   tcaaacaagc tcgttccaac atctcttccc aagagaatgc ttgctgatgg cacatatgag 1620
   gaacttcctc agacagaacc tgtagatact tatgcaacaa tcttcccaag acctaagaac 1680
   tggaaagaaa caattgagca cgacgtatta aagactcctg tagttcgtcc acaggacaga 1740
   ttccagctct ttttgccacc taaggaaggt gaccctaaga aatttatcga agttgcagaa 1800
   tgcaaggtga cacttgaaga ctttattgca cagctatcta acgagcagct tgcaagcctt 1860
   cttggaggac agccaaatgt cggaatggct aacacctttg gatacggcaa ccttcctgag 1920
   gttggagttc ctaatgccca gacctgtgat ggtcctgcag gtgtccgtat tgcaccggaa 1980
   gttggtgttg tgactacagc attcccatgt tcaacccttc ttgcatgcac atggaatgaa 2040
   gatatctgct acgaagtcgg agttgcaggc ggagaagagg ccaaggagtg caattttggt 2100
   gcatggctta ctcctgctgt taacatccat agaagccctc tttgcggcag aaactttgag 2160
   tactactccg aagatccatt ccttgcaggt aaacaggcag cagctatggt tcgtggtatc 2220
   cagagcaaca acataattgc tacacctaaa cattttgccc tcaacaacaa ggaatccaat 2280
   agaaaaggca gcgattcacg tgcttctgag cgtgcgatca gagaaatata tttaaaggcc 2340
   tttgaaatca ttgttaaaga gcagagccct ggagcatcat gtcttcaata caatatagtt 2400
   aacggtcaga gatcatccga atctcacgac ctcctcacag gaatcctccg cgatgagtgg 2460
   ggctttgaag gtgttgtagt cagcgactgg tggggctttg gtgagcatta caaggaagtc 2520
   cttgcaggca acgatatcaa gatgggctgt ggctatacag aacagctcct tgaagcaatt 2580
   gataagaaag ctcttaagag aaaagatttg gaaaagaggc agagcgagtc ctcaagatgc 2640
   ttctcaaact cgactaagct caaagccgct tagaataaac gtttaagtca taaaaaagta 2700
   atgtattgtt cataaactat caatattttt ctgacaattt gtcgtataat ataaatatga 2760
   tatagttgtt gggaatatct tctgagaatt gtatcaggag aaagttgtag aactacaact 2820
   ctcggaggta tgtctatgaa caaaaaagct attgttggta tttttatgtc cattttgatg 2880
   gcagggctcg ttggatgtgc cggtagcagt gatgcccagg caggagatga cctcaagccg 2940
   gttatttatc tttatccaca ggaagataat accgagattt cagtaagcct tgattataac 3000
   ggaaatctgg ttgacctgat tcctgagttt aatgcagata agacatggaa tgttacagct 3060
   aacaaagatg gcaagattac ctttgaagga cagacttatg actatctgtt ttgggaaggc 3120
   gatc 3124
<210> 24
   <211> 1640
   <212> DNA
   <213> Cochliobolus carbonum
<400> 24
   gatctacacc ctagctaata ttactctagc agatggaata ccgctatcat gacgaggatg     60
   tttggcttgg ctgtgcacaa catccggtct tgtttccact cacatgccat gcgagggcga    120
   gtataaagct atcctgagtc ggctccatgg cacactttcc tcaccaacca tcaccacaac    180
   taccacttcc cttcgcttca ttcactcaac aacaacacca tcaaaatggt ttctttcacc    240
   tccatcatca ccgctgctgt tgcggctacc ggcgctcttg ccgcccccgc cactgatgtg    300
   tctctcgttg cccgtcagaa cacccccaac ggcgagggta cccacaacgg ctgcttctgg    360
   tcttggtggt ctgatggcgg tgcccgcgct acctacacca acggtgccgg tggtagctac    420
   agcgtaagct ggggaagcgg tggcaacctc gtcggtggaa agggatggaa cccaggaact    480
   gcccggtatg gactgtctta ctcaactctg atagactaca tacactaaca ttgatacagt    540
   accatcacct actctggtac ttacaactac aacggcaact cctaccttgc cgtctacggc    600
   tggacccgca acccccttgt cgagtactac gtcgttgaga acttcggcac ctacgacccc    660
   tcttcccagt cccagaacaa gggtaccgtc acctctgatg gatcttccta caagatcgct    720
   cagtcgaccc gtaccaacca gccctccatc gatggcacca ggacctttca gcagtattgg    780
   tctgttcgtc agaacaagcg ctcttccggc tccgtcaata tgaagactca ctttgacgcc    840
   tgggccagca agggcatgaa ccttggccag cactactacc agattgttgc caccgagggt    900
   tacttctcca ctggtaacgc ccagatcacc gtcaactgcc cataaattct tcaccacgaa    960
   gattaaacga atggccctga tggctctctg aacgactgga agatgacagg cgtggttttt   1020
   ggttacgtgc tgtgctagct gagctggtgg attcttttct gtatatacta tctttgtcaa   1080
   cccgatttgt ctatagctta acaatgtcaa aatcttggca ttactcacaa cgcgtctttt   1140
   catttttgcc tctaagtgaa ttgtgatggt cttgacgcat gatgacgtct actacgagca   1200
   ttctctccgg tgtccttcta atgataaaaa taaacaaact tcaatactcg caatatgaaa   1260
   ctccgcagta ccaaatttag atgtttgact tcacaatagg ctggcctatc tacgctgcct   1320
   catctattac atacaacggc atcgtaccag gagtcatcct cagtgcctcc tcaatgattc   1380
   ccaatgcaac atgcaactcc tcttccataa cagtaatcgg gtgtgcaatt ctgaaaacac   1440
   caccaagcga cgccattgtc gccaactgcg cccacaaacc cattttctcc atcttttgct   1500
   acacagcagc tccgaaatca ggagtcccaa ccgtaagaag gtgcagcctt cagtgttact   1560
   ggaggcaccg ctagtgtggt ggtgctttcc caatatagct tagtgcaaat agtaggtggg   1620
   tagcatctcg ctgagagctc                                               1640
<210> 25
   <211> 20
   <212> DNA
   <213> Artificial
<220>
   <223> Synthetic PCR primer
<400> 25
   cgataacctg gtcaagatcc 20
<210> 26
   <211> 20
   <212> DNA
   <213> Artificial
<220>
   <223> synthetic PCR primer
<400> 26
   ctgctcccac atgatgatta 20
<210> 27
   <211> 17
   <212> DNA
   <213> Artificial
<220>
   <223> synthetic PCR primer
<400> 27
   gcgggcggcg gctattg 17
<210> 28
   <211> 20
   <212> DNA
   <213> Artificial
<220>
   <223> synthetic PCR primer
<400> 28
   gccgacagga tcgaaaatcg 20
<210> 29
   <211> 17
   <212> DNA
   <213> Artificial
<220>
   <223> synthetic PCR primer
<400> 29
   atgagcccag aacgacg 17
<210> 30
   <211> 17
   <212> DNA
   <213> Artificial
<220>
   <223> synthetic PCR primer
<400> 30
   tcagatctcg gtgacgg 17
<210> 31
   <211> 17
   <212> DNA
   <213> Artificial
<220>
   <223> synthetic PCR primer
<400> 31
   gcgggcggcg gctattg 17
<210> 32
   <211> 20
   <212> DNA
   <213> Artificial
<220>
   <223> synthetic PCR primer
<400> 32
   gccgacagga tcgaaaatcg 20
<210> 33
   <211> 1461
   <212> DNA
   <213> Cochliobolus carbonum
<400> 33
   cccgggtaga gcgatgttct tttaattatg ttatgcagat ttacgcaagg gctacagatc 60
   tttttgatca ccccgtcgtg gccgttgcta ccgaaactgg atgccgatcg gtgcatgtcc 120
   cctatgtgtc tcgacttggt cattgtggcc gccgcttagg gtgagcttgt cgaagtgaga 180
   ggccttaacc taccaccaat gtgtacacca tcacgaatag agacagcacg cgagatgaga 240
   cctcatgacc accagtgtca tttcatcctg cgattcaggc tcaactatta ctcggcgtag 300
   agatataagt cgatagctgc tcccagtcag tagcatcact gcatccaaca cagatcagca 360
   acactcaaca caagatggtt tccttcaagt ctctgcttct cgccgctgtg gctaccacca 420
   gcgtcctcgc tgctcccttc gatttccttc gtgagcgcga cgatgtcaac gcgactgctc 480
   tccttgagaa gcgtcagtct actcccagcg ccgagggata ccacaatgga tacttctact 540
   cgtggtggac tgatggcggt ggctctgccc agtacactat gggtgagggc agcaggtact 600
   ctgtgacctg gaggaacact ggcaactttg ttggtggaaa ggggtggaac cctggaagcg 660
   gccggtaggt tgcgaagact gtttggtgat gagaatctta ctaatgtgga ttcgtagtgt 720
   catcaactac ggcggagcct tcaaccccca gggcaacgga tacctcgctg tgtacggatg 780
   gacccgcaac ccgcttgtcg agtactacgt gattgaatcc tacggaacct acaaccccag 840
   cagtggagcc caaatcaagg gcagcttcca gaccgacggt ggtacttaca acgttgccgt 900
   ctccacccgt tacaaccagc cctccattga cggaacaagg acctttcagc agtactggta 960
   agtcatttcg gtgtttaatc agacagaagc gtagatgttg actgttatga taggtctgtc 1020
   cgcacccaga agcgtgtcgg tggaagcgtg aacatgcaga accacttcaa cgcttggtct 1080
   cgctatggct tgaaccttgg tcaacactac taccagatcg tcgccactga gggttaccag 1140
   tcctctggaa gctctgacat ctatgtgcag actcagtaga gtagttgttg tctgtgaagc 1200
   gagcaactag tagacgagat tagacaagaa tagtgtctcg gatagttgta agcaggttgg 1260
   agaaagagcg atgtgttcga tttcctgtac atagaaacat gtcaattcac tcgctataaa 1320
   accttccgtc ctcgaatgtg ttctcttttg tattagctga gcattgtatc gtggttcggc 1380
   aaaagcaggt aaagtttagg tgagggcatt tgttataaac agtcatagtc tcgtagccat 1440
   cgaatgctgg tgtgtgaatt c 1461
<210> 34
   <211> 1929
   <212> DNA
   <213> Cochliobolus carbonum
<400> 34
   ctgcatgact ggaccactca gcgcgcggct tccccacaaa agtctatatg aagtatccac 60
   cgccgtgatg gagcttgatt aactgtcggg atggcgactt ggcacgcgcc ttgaaatgca 120
   ggatagcatc acgcctaggg gcagggccga ttgcgctcat tggatacgaa tacatcaaga 180
   cactagtgta gaacgattca gacgattcat ccatgggcaa cgaggggcga gaggtggccc 240
   catgacacag ggaggaagac agcgggtgaa acaaccgcaa tatgtttcct gtggtgtgca 300
   cccacgctct ttaaccgtct ctaagtatat aggtcaagac ttccaagacc gaataagctc 360
   accaccaagc ctttcatctt caacactcaa gcagtcttta cttcactcac cctcttttca 420
   attcactcct agcgagaacc agcttctttt cacacaaaca caaacaaatc ttcaatctat 480
   caaaatggtt gccttcacct ccgtcctcct cggcctctcc gccattggct ctgccttcgc 540
   cgcccccgtt gccgatgtcc ctgacttcga gttctccggc cccaagcact tggctgcccg 600
   ccaggactac aaccagaact acaagactgg tggtaacatc cagtacaacc ccaccagcaa 660
   cggttactcc gtcaccttct ctggcgccca ggacttcgtc cttggcaagg gctggaagca 720
   aggtactacc aggtaagcca cacacagtgt tctagaccca aatatcaatc aatactaact 780
   cctttccttc aggaccgtca agtacactgg ttccacccag gcccaggccg gtactgtcct 840
   cgtcgctctc tacggctgga ccaggggcag caagctcgtc gagtactaca tccaggactt 900
   cacctctggc ggctctggct ccgcccaggg ccagaagatg ggccaagtca cctgcgacgg 960
   ctccgtctac gacatctggc agcacaccca ggtgaaccag ccttccatcg tcggcacgac 1020
   caccttcgtc cagtacatca gcaaccgcgt cagcaagcgc tccaccggcg gtaccatcac 1080
   caccaagtgc cacttcgacg cctgggccaa gctcggcatg aaccttggta accagtggga 1140
   ctaccagacc atctccactg agggttgggg caacgctgct ggaaagtccc agtacaccgt 1200
   ctccgctgct taaattgtgg gcgcggtccg ttctttggct caaggggaat gaaccagggg 1260
   ttcgagagat ttgtgaaaca ttggcttgcg caatcatcaa ttcctctcca gaggagagga 1320
   aatatgatag cttcaagggc tttgtagggg tgttgattta ggggatgctg ccgctgctgt 1380
   tgcttgattc attggtgttt atctttcttt cttcttgatt tgttcagggc cttgacacac 1440
   cctggcttgt acatactttt tttactcgtt tcttgtagaa acgggtaaat cctgaatata 1500
   cacgttgcct tgtttctccc tcgtttggat gacaatagca attgcccggt tatgaaccga 1560
   aaaaaaaact cctcatcttc ttctgtgttt cttatcattg tgacaagtga cttttttttc 1620
   ttttcttact taaatttatt tttctaggtt ttcacaaaag tatatatatt cttagccatg 1680
   ctggctgccc catcttactt agtaaacagg tgaaacttcc aattatcgct gccaaacgca 1740
   ggctcggatg tcggcggcgg cgcgtggatg gagatacata acattgtgtt gcttaaaaca 1800
   aggctaggtt ggctgggctg gggctgaaca gccggtggta ggatgattta tcgggcaaat 1860
   atcaagacac acacacatct tcttaaaacg cccttttggt ggcgaaggtt cttaaccaag 1920
   tgggtaccc 1929
<210> 35
   <211> 2527
   <212> DNA
   <213> Cochliobolus carbonum
<400> 35
   ctttttgtga aggcacctcc gtagacatca gtccttagat ctgacttgcg cagcaccaca      60
   caagcagcag ttattccttg ggctccaatc tggtcacacc accaaacaaa tccaacgggc    120
   taatatcgca aggcagtgca gtaagcggtg ttggcgtgag cattgtagct cgccttgaaa    180
   gttggtccgg cataacaatc ttccacagta aagaacgcac actgtttctc cttattggtg    240
   tgtcaattaa cgtccacatc cattaagcct ggatattcgg ctagccaagt ggagtcttgg    300
   aaagcttgtg gaagacaata agcgcaggta ccttggctgc atatgcagtt tccccagaca    360
   gatgcactcc acaattcctt ctgttccgga gaaatcatcc tcgcaaatcc taacggcttt    420
   actaccccta ccaagtggtc ttatgccaag tcgatcgacg agtacggacc atcgctacgg    480
   aatattctcc acgaatcgtc catccccgca tgccaccacg tagccaaacc aacgaagaac    540
   agaaatggac ccgtagaacc agcactggta ccggacatat cccgtattgc ggtgaatcat    600
   tatcagagct ccgtggatcc agtcattgtt gtgctgcact aattactcgc gcgcagagta    660
   aagcggtcta cgtaccgagc tatggttttg cgtgtgctta cttgcgtact gaacacagcc    720
   cctgttgata tattaatatc ggaaatttat tcagcacaat gatgtcgcgt ttcgagaatt    780
   aggtggaaca agcataggcc ggctgttgtt gtttcgccat gaactcccta ctcgcattta    840
   gccgcttttt ccccgtgcag aaaacgccaa gtacctcaag cagcacaaga accgaccacc    900
   gaagcttgga ataaattaac ccgaaacgcc tggtccccac ttttttactc tatacggtaa    960
   gactacgaat cattcaggcc atgaccgaca tgaacgacgc attgcaggtg cgattgcagg   1020
   cgatgtttga ttgcgttaac gccctatttt cgggggttct ccacaacgtg gtgggtatgc   1080
   ggcttctaga cgacgcctgc cgttgtttga ggcgaattac attgtttaga cgaaatgcgt   1140
   gcgccgaata gtctagggca ctctgggggg acgatggatt ggtataagta gagctgggcc   1200
   catgggcata tcttcgtatc catcacattc attcatttca gcttctccat tctaccaaac 1260
   aatcaaacca atccttttag caaccatgaa gttttctctc atcaccatcc tttccgccag 1320
   cgctttggtc gctgcctctc cctttgctga gccagaggct ttccttgagg agcgccaggc 1380
   tgcgcagagc ctcgatgccg ccatgaaggc caagggcagg aagtacttcg gtaccgccac 1440
   tgacccaggc cgattcaacc agggcaagaa cgctgccatc atcaaggcca actttggtca 1500
   aatcaccccc gagaactcca tgaagtggga tgctaccgag tctacccgtg gcaaattcac 1560
   cttcggtact gccgaccaga ccgctaagtt cgccaaggac aacggcaagc tcatccgtgg 1620
   acacaccacc atctggcact cccagcttcc ttcatgggtt tcttccatca aggacaaggc 1680
   caccttgacc actgtcatgc agaaccacat ctcctccgtc atgggccact tcaagggcca 1740
   gatctacgcc tgggacgtca tcaacgagat gttcgaggag aacggtaact tccgtgccag 1800
   cgtcttctac aacgtcctcg gtgaggactt tgtccgcatc gccttcgagg ctgccaagaa 1860
   ggctgacccc actgctaagc gttacatcaa cgactacaag taagcattga tctatgatta 1920
   ccaagttttt cgatatactg actgcaatat agccttgaca ctgccaacta cgcaaagacc 1980
   caggccatgg ccaagaacgt caagaagtgg atcgccgccg gtatccccat cgacggtatc 2040
   ggttcgcaga ctcaccttac cgccggccag ggtgccgcta ccatcgacgc catgaagctc 2100
   ctctgcagcg ttgcttccga gtgcgccatg accgaggtcg acatccagaa cgcccagcag 2160
   gctgactgga ccaacgtcac caaggcctgc cttaaccaga agaactgcgt tggtatcacc 2220
   gtctggggtg tcagggactc cgactcctgg aggccccagg gcaaccctct cctcttcgac 2280
   aacagctaca accccaagca ggcttacacc actgtcctca acgctctcaa gtaaactgtt 2340
   tgcgatgctg ggcatcctgg gaaccaggaa gactatgtct tttcttgctc ccttacctat 2400
   ccttcttttc gacatattat tgtaaatact ttgattggtg gtggagatat tgtaaatatt 2460
   tgattgaaag gaatgaagaa gttgatcttt cccctataat tgtgtgcttc ttgatctctc 2520
   tactagt 2527